# EUROPEAN PATENT SPECIFICATION

(11) **EP 3 464 296 B1**
(45) Date of publication and mention of the grant of the patent: **14.10.2020**
(21) Application number: 17725286.3
(22) Date of filing: 29.05.2017
(51) Int. Cl.: C07D 513/04, A01N 43/90

(54) **ISOTHIAZOLOPYRIDONES, PROCESSES FOR THEIR PREPARATION AND THEIR USE AS HERBICIDES AND/OR PLANT GROWTH REGULATORS**
ISOTHIAZOLOPYRIDONE, VERFAHREN ZU IHRER HERSTELLUNG UND IHRE VERWENDUNG ALS HERBIZIDE UND/ODER PFLANZENWACHSTUMS-REGULATOREN
ISOTHIAZOLOPYRIDONES, LEURS PROCÉDÉS DE PRÉPARATION ET LEUR UTILISATION COMME HERBICIDES ET/OU RÉGULATEURS DE CROISSANCE VÉGÉTALE

(30) Priority: 02.06.2016 EP 16172609
(43) Date of publication of application: 10.04.2019
(73) Proprietor: Bayer CropScience Aktiengesellschaft, 40789 Monheim am Rhein (DE)
(72) Inventor: REY, Jullien, 68510 Sierentz (FR); TIEBES, Jörg, 60431 Frankfurt (DE); MOSRIN, Marc, 50935 Köln (DE); DIETRICH, Hansjörg, 65835 Liederbach am Taunus (DE); MACHETTIRA, Anu Bheemaiah, 60326 Frankfurt am Main (DE); ROSINGER, Christopher Hugh, 65719 Hofheim (DE); GATZWEILER, Elmar, 61231 Bad Nauheim (DE)
(74) Representative: BIP Patents
(86) International application number: PCT/EP2017/062844
(87) International publication number: WO 2017/207462

(56) References cited:
- EP-A1- 2 123 159
- US-A- 3 707 364
- US-A1- 2008 227 636

## Description

The invention relates to the technical field of the herbicides and/or plant growth regulators. Specifically, the invention primarily relates to novel isothiazolopyridones, and compositions comprising said novel isothiazolopyridones. Further, the present invention relates to processes for the preparation said novel isothiazolopyridones and their use as herbicides and/or plant growth regulators.

In their application, crop protection agents known to date for the selective control of harmful plants in crops of useful plants or active compounds for controlling unwanted vegetation sometimes have disadvantages, be it (a) that they have no or else insufficient herbicidal activity against particular harmful plants, (b) that the spectrum of harmful plants which can be controlled with an active compound is not wide enough, (c) that their selectivity in crops of useful plants is too low and/or (d) that they have a toxicologically unfavourable profile.

Furthermore, some active compounds which can be used as plant growth regulators for a number of useful plants cause unwanted reduced harvest yields in other useful plants or are not compatible with the crop plant, or only within a narrow application rate range. Some of the known active compounds cannot be produced economically on an industrial scale owing to precursors and reagents which are difficult to obtain, or they have only insufficient chemical stabilities.

The prior art discloses several isothiazoles and isothiazolamides and certain pesticidal uses thereof.

US 2011/0201687 A1 discloses various amide derivatives as pest control agents, i.e. insecticides.

WO 2007/014290 discloses various fungicidal carboxamides.

EP 0761654 A1 discloses certain isoxazole- and isothiazole-5-carboxamide derivatives and their use as herbicides.

WO 97/02262 and WO 99/14202 disclose certain (thieno)pyrimidinone-derivatives as fungicides.

WO 98/49899 relates to the use of certain thienopyrimidinones as fungicides.

EP 2123159 describes certain (1,2-benzisothiazol-3yl)(thio)carbamates and -(thio)oxamates as insecticides.

US 3,707,364 discloses certain benzoisothiazole herbicides.

US 2008/0227636 A1 describes certain isothiazolopyridin-3-ylenamines for combating animal pests.

WO 2016/102420 and WO 2016/102435 disclose certain isothiazolamides and their use as herbicides and fungicides, respectively.

In their application, herbicides known to date for controlling harmful plants or unwanted vegetation may have some disadvantages, be it (a) that they have no or else insufficient herbicidal activity against specific harmful plants, (b) that the spectrum of harmful plants which can be controlled with the herbicides is not broad enough, and/or (c) that the selectivity of herbicides in and the compatibility with crop plants is too low, thereby causing unwanted damage and/or unwanted reduced harvest yields of the crops.

Thus, there is still a need for alternative herbicides, in particular highly active herbicides, in particular useful at low application rates and/or having good compatibility with crop plants, for the selective application in plant crops or use on non-crop land. It is also desirable to provide alternative chemical active compounds which may be used in an advantageous manner as herbicides or plant growth regulators.

It is therefore an objective of the present invention to provide compounds having herbicidal activity which are highly effective against economically important harmful plants even at relatively low application rates and that can be used selectively in crop plants.

It has now been found that the compounds of the following formula (G) and/or the salts thereof meet said objective(s).

The present invention primarily relates to compounds of the formula (G) and/or salts thereof 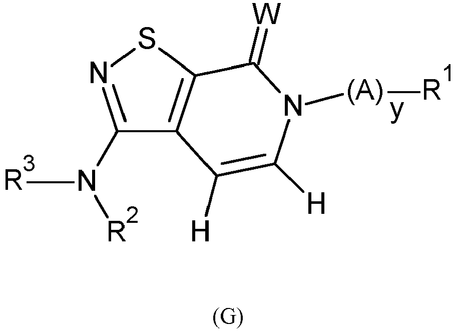 in which
- A: is CR⁶R⁷,
- W: is O or S,
- R¹: is (C₁-C₁₂)-alkyl, (C₁-C₁₂)-haloalkyl, (C₂-C₁₂)-alkenyl, (C₂-C₁₂)-haloalkenyl, (C₂-C₁₂)-alkynyl, (C₂-C₁₂)-haloalkynyl, NR¹³R¹⁴, R¹³R¹⁴N-(C₁-C₆)-alkyl, (C₁-C₆)-alkoxy, (C₁-C₆)-haloalkoxy, (C₁-C₆)-haloalkoxy-(C₁-C₃)-alky|, (C₁-C₆)-alkoxy-(C₁-C₃)-alky|, (C₁-C₆)-alkoxy-(C₂-C₆)-alkoxy-(C₁-C₃)-alkyl, (C₁-C₄)-alkylthio, (C₁-C₄)-alkylsulphoxy, (C₁-C₄)-alkylsulphonyl, (C₁-C₄)-haloalkylthio, (C₁-C₄)-haloalkylsulphoxy, (C₁-C₄)-haloalkylsulphonyl, (C₁-C₄)-alkylthio-(C₁-C₃)-alkyl, (C₁-C₄)-alkylsulphoxy-(C₁-C₃)-alkyl, (C₁-C₄)-alkylsulphonyl-(C₁-C₃)-alkyl, (C₁-C₄)-haloalkylthio-(C₁-C₃)-alkyl, (C₁-C₄)-haloalkylsulphoxy-(C₁-C₃)-alkyl, (C₁-C₄)-haloalkylsulphonyl-(C₁-C₃)-alkyl, (C₃-C₁₂)-cycloalkyl, (C₃-C₈)-cycloalkenyl, (C₃-C₁₂)-cycloalkyl-(C₁-C₆)-alkyl, (C₃-C₈)-cycloalkenyl-(C₁-C₆)-alkyl, (C₃-C₈)-cycloalkoxy, (C₃-C₈)-cycloalkyl-(C₁-C₆)-alkoxy, aryl, aryl-(C₁-C₃)-alkyl, heteroaryl, heteroaryl-(C₁-C₃)-alkyl, heterocyclyl, heterocyclyl-(C₁-C₃)-alkyl, aryloxy, heteroaryloxy, heterocyclyloxy, a bicyclic or a heterobicyclic residue, wherein each of the last-mentioned 17 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, (C₁-C₄)-haloalkylthio, (C₁-C₄)-haloalkylsulphoxy, (C₁-C₄)-haloalkylsulphonyl, (C₁-C₄)-alkoxycarbonyl, (C₁-C₄)-haloalkoxycarbonyl, (C₁-C₄)-alkylcarboxy, (C₃-C₆)-cycloalkyl, (C₃-C₆)-cycloalkyl-(C₁-C₆)-alkyl, (C₁-C₄)-alkoxycarbonyl-(C₁-C₄)-alkyl, hydroxycarbonyl, hydroxycarbonyl-(C₁-C₄)-alkyl, R¹³R¹⁴N-carbonyl, and wherein heterocyclyl has q oxo groups, and wherein each of the aforementioned heterocyclic residues, in addition to the carbon atoms, has in each case p ring members from the group consisting of N(R¹²)ₘ, O and S(O)ₙ,
- R², R³: are each independently hydrogen, (C₁-C₁₂)-alkyl, (C₁-C₁₂)-haloalkyl, (C₂-C₁₂)-alkenyl, (C₂-C₁₂)-haloalkenyl, (C₂-C₁₂)-alkynyl, (C₂-C₁₂)-haloalkynyl, (C₁-C₆)-alkoxy-(C₁-C₃)-alkyl, (C₁-C₆)-alkoxy-(C₂-C₆)-alkoxy-(C₁-C₃)-alkyl, (C₁-C₆)-alkoxy-(C₁-C₃)-alkylcarbonyl, (C₁-C₆)-alkoxy-(C₂-C₆)-alkoxy-(C₁-C₃)-alkylcarbonyl, (C₁-C₆)-alkoxycarbonyl, (C₁-C₆)-haloalkoxycarbonyl, (C₂-C₆)-alkenyloxycarbonyl, (C₂-C₆)-haloalkenyloxycarbonyl, (C₂-C₆)-alkynyloxycarbonyl, (C₂-C₆)-haloalkynyloxycarbonyl, (C₁-C₆)-alkylcarbonyl, (C₁-C₆)-haloalkylcarbonyl, (C₂-C₆)-alkenylcarbonyl, (C₂-C₆)-haloalkenylcarbonyl, (C₂-C₆)-alkynylcarbonyl, (C₂-C₆)-haloalkynylcarbonyl, R¹³R¹⁴N-carbonyl, (C₁-C₄)-alkylthio, (C₁-C₄)-haloalkylthio, (C₁-C₈)-alkylthiocarbonyl, (C₁-C₈)-haloalkylthiocarbonyl, (C₁-C₄)-alkylsulphoxy, (C₁-C₄)-haloalkylsulphoxy, (C₁-C₄)-alkylsulphonyl, (C₁-C₄)-haloalkylsulphonyl, (C₁-C₄)-alkylthio-(C₁-C₃)-alkyl, (C₁-C₄)-alkylsulphoxy-(C₁-C₃)-alkyl, (C₁-C₄)-alkylsulphonyl-(C₁-C₃)-alkyl, (C₁-C₄)-alkylthio-(C₁-C₃)-alkylcarbonyl, (C₁-C₄)-alkylsulphoxy-(C₁-C₃)-alkylcarbonyl, (C₁-C₄)-alkylsulphonyl-(C₁-C₃)-alkylcarbonyl, (C₁-C₁₂)-alkylcarbonyl, (C₁-C₁₂)-haloalkylcarbonyl, (C₂-C₁₂)-alkenylcarbonyl, (C₂-C₁₂)-haloalkenylcarbonyl, (C₂-C₁₂)-alkynylcarbonyl, (C₂-C₁₂)-haloalkynylcarbonyl, (C₁-C₁₂)-alkoxycarbonylcarbonyl, (C₁-C₁₂)-alkoxycarbonyl-(C₁-C₃)-alkylcarbonyl, (C₃-C₈)-cycloalkyl, (C₃-C₈)-cycloalkenyl, (C₃-C₈)-cycloalkyl-(C₁-C₆)-alkyl, (C₃-C₈)-cycloalkenyl-(C₁-C₆)-alkyl, (C₃-C₈)-cycloalkylcarbonyl, (C₃-C₈)-cycloalkenylcarbonyl, (C₃-C₈)-cycloalkyl-(C₁-C₆)-alkylcarbonyl, (C₃-C₈)-cycloalkenyl-(C₁-C₆)-alkylcarbonyl, aryl, aryl-(C₁-C₃)-alkyl, heteroaryl, heteroaryl-(C₁-C₃)-alkyl, heterocyclyl, heterocyclyl-(C₁-C₃)-alkyl, arylcarbonyl, aryl-(C₁-C₆)-alkylcarbonyl, heteroarylcarbonyl, heteroaryl-(C₁-C₆)-alkylcarbonyl, heterocyclylcarbonyl, or heterocyclyl-(C₁-C₆)-alkylcarbonyl, wherein each of the last-mentioned 20 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, (C₁-C₄)-haloalkylthio, (C₁-C₄)-haloalkylsulphoxy, (C₁-C₄)-haloalkylsulphonyl, (C₁-C₄)-alkoxycarbonyl, (C₁-C₄)-haloalkoxycarbonyl, (C₁-C₄)-alkylcarboxy, (C₃-C₆)-cycloalkyl, (C₃-C₆)-cycloalkyl-(C₁-C₆)-alkyl, (C₁-C₄)-alkoxycarbonyl-(C₁-C₄)-alkyl, hydroxycarbonyl, hydroxycarbonyl-(C₁-C₄)-alkyl, R¹³R¹⁴N-carbonyl, and wherein heterocyclyl has q oxo groups,
or
NR²R³ is -N=CR⁸R⁹ or -N=S(O)ₙR¹⁰R¹¹,
- R⁶, R⁷: are each independently hydrogen, cyano, halogen, (C₁-C₆)-alkyl, (C₂-C₆)-alkenyl, (C₂-C₆)-alkynyl, or (C₃-C₈)-cycloalkyl,
or
R⁶ and R⁷, together with the carbon atom to which they are attached, form a 3 - 6-membered carbocyclic or heterocyclic ring, which comprises in each case, in addition to the carbon atoms, p ring members from the group consisting of N(R¹²)ₘ, O and S(O)ₙ and wherein said ring is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, (C₁-C₄)-haloalkylthio, (C₁-C₄)-haloalkylsulphoxy, (C₁-C₄)-haloalkylsulphonyl, (C₁-C₄)-alkoxycarbonyl, (C₁-C₄)-haloalkoxycarbonyl, (C₁-C₄)-alkylcarboxy, (C₃-C₆)-cycloalkyl, (C₃-C₆)-cycloalkyl-(C₁-C₆)-alkyl, (C₁-C₄)-alkoxycarbonyl-(C₁-C₄)-alkyl, hydroxycarbonyl, hydroxycarbonyl-(C₁-C₄)-alkyl, R¹³R¹⁴N-carbonyl and has q oxo groups,
- R⁸, R⁹: are each independently hydrogen, (C₁-C₆)-alkyl, (C₁-C₆)-haloalkyl, (C₂-C₆)-alkenyl, (C₂-C₆)-haloalkenyl, (C₂-C₆)-alkynyl, (C₂-C₆)-haloalkynyl, (C₁-C₆)-alkoxy, (C₁-C₆)-haloalkoxy, (C₁-C₆)-haloalkoxy-(C₁-C₃)-alkyl, (C₂-C₆)-alkenyloxy, (C₂-C₆)-haloalkenyloxy, (C₂-C₆)-alkynyloxy, (C₂-C₆)-haloalkynyloxy, NR¹³R¹⁴, (C₁-C₆)-alkoxy-(C₁-C₃)-alkyl, halogen-(C₁-C₆)-alkoxy-(C₁-C₆)-alkyl, (C₁-C₆)-alkoxy-(C₂-C₆)-alkoxy-(C₁-C₃)-alkyl, (C₁-C₄)-alkylthio-(C₁-C₃)-alkyl, (C₁-C₄)-alkylsulphoxy-(C₁-C₃)-alkyl, (C₁-C₄)-alkylsulphonyl-(C₁-C₃)-alkyl, (C₃-C₈)-cycloalkyl, (C₃-C₈)-cycloalkenyl, (C₃-C₈)-cycloalkyl-(C₁-C₆)-alkyl, (C₃-C₈)-cycloalkenyl-(C₁-C₆)-alkyl, aryl, aryl-(C₁-C₃)-alkyl, heteroaryl, heteroaryl-(C₁-C₃)-alkyl, heterocyclyl, heterocyclyl-(C₁-C₃)-alkyl, wherein each of the last-mentioned 10 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, (C₁-C₄)-haloalkylthio, (C₁-C₄)-haloalkylsulphoxy, (C₁-C₄)-haloalkylsulphonyl, (C₁-C₄)-alkoxycarbonyl, (C₁-C₄)-haloalkoxycarbonyl, (C₁-C₄)-alkylcarboxy, (C₃-C₆)-cycloalkyl, (C₃-C₆)-cycloalkyl-(C₁-C₆)-alkyl, (C₁-C₄)-alkoxycarbonyl-(C₁-C₄)-alkyl, hydroxycarbonyl, hydroxycarbonyl-(C₁-C₄)-alkyl, R¹³R¹⁴N-carbonyl and has q oxo groups,
or
R⁸ and R⁹, together with the carbon atom to which they are attached, form a 3- to 8-membered unsaturated, partially saturated or saturated ring, which comprises in each case, in addition to the carbon atoms, p ring members from the group consisting of N(R¹²)ₘ, O and S(O)ₙ and wherein said ring is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, (C₁-C₄)-haloalkylthio, (C₁-C₄)-haloalkylsulphoxy, (C₁-C₄)-haloalkylsulphonyl, (C₁-C₄)-alkoxycarbonyl, (C₁-C₄)-haloalkoxycarbonyl, (C₁-C₄)-alkylcarboxy, (C₃-C₆)-cycloalkyl, (C₃-C₆)-cycloalkyl-(C₁-C₆)-alkyl, (C₁-C₄)-alkoxycarbonyl-(C₁-C₄)-alkyl, hydroxycarbonyl, hydroxycarbonyl-(C₁-C₄)-alkyl, R¹³R¹⁴N-carbonyl and has q oxo groups,
- R¹⁰, R¹¹: are each independently (C₁-C₆)-alkyl, (C₁-C₆)-haloalkyl, (C₂-C₆)-alkenyl, (C₂-C₆)-haloalkenyl, (C₂-C₆)-alkynyl, (C₂-C₆)-haloalkynyl, (C₁-C₆)-alkoxy-(C₁-C₃)-alkyl, halogen-(C₁-C₆)-alkoxy-(C₁-C₆)-alkyl, (C₁-C₆)-alkoxy-(C₂-C₆)-alkoxy-(C₁-C₃)-alkyl, (C₁-C₄)-alkylthio-(C₁-C₃)-alkyl, (C₁-C₄)-alkylsulphoxy-(C₁-C₃)-alkyl, (C₁-C₄)-alkylsulphonyl-(C₁-C₃)-alkyl, (C₃-C₈)-cycloalkyl, (C₃-C₈)-cycloalkenyl, (C₃-C₈)-cycloalkyl-(C₁-C₆)-alkyl, (C₃-C₈)-cycloalkenyl-(C₁-C₆)-alkyl, aryl, aryl-(C₁-C₃)-alkyl, heteroaryl, heteroaryl-(C₁-C₃)-alkyl, heterocyclyl or heterocyclyl-(C₁-C₃)-alkyl, wherein each of the last-mentioned 10 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, (C₁-C₄)-haloalkylthio, (C₁-C₄)-haloalkylsulphoxy, (C₁-C₄)-haloalkylsulphonyl, (C₁-C₄)-alkoxycarbonyl, (C₁-C₄)-haloalkoxycarbonyl, (C₁-C₄)-alkylcarboxy, (C₃-C₆)-cycloalkyl, (C₃-C₆)-cycloalkyl-(C₁-C₆)-alkyl, (C₁-C₄)-alkoxycarbonyl-(C₁-C₄)-alkyl, hydroxycarbonyl, hydroxycarbonyl-(C₁-C₄)-alkyl, R¹³R¹⁴N-carbonyl and wherein heterocyclyl has q oxo groups,
or R¹⁰ and R¹¹, together with the sulphur atom to which they are attached, form a 3- to 8-membered unsaturated, partially saturated or saturated ring, which comprises in each case, in addition to the carbon atoms and in addition to the sulphur atom, p ring members from the group consisting of N(R¹²)ₘ, O and S(O)ₙ and wherein said ring is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, (C₁-C₄)-haloalkylthio, (C₁-C₄)-haloalkylsulphoxy, (C₁-C₄)-haloalkylsulphonyl, (C₁-C₄)-alkoxycarbonyl, (C₁-C₄)-haloalkoxycarbonyl, (C₁-C₄)-alkylcarboxy, (C₃-C₆)-cycloalkyl, (C₃-C₆)-cycloalkyl-(C₁-C₆)-alkyl, (C₁-C₄)-alkoxycarbonyl-(C₁-C₄)-alkyl, hydroxycarbonyl, hydroxycarbonyl-(C₁-C₄)-alkyl, R¹³R¹⁴N-carbonyl and has q oxo groups,
- R¹²: is hydrogen, (C₁-C₁₂)-alkyl, (C₁-C₁₂)-haloalkyl, (C₂-C₁₂)-alkenyl, (C₂-C₁₂)-haloalkenyl, (C₂-C₁₂)-alkynyl, (C₂-C₁₂)-haloalkynyl, (C₃-C₈)-cycloalkyl, (C₃-C₈)-halocycloalkyl, (C₃-C₈)-cycloalkenyl, (C₃-C₈)-cycloalkyl-(C₁-C₆)-alkyl, (C₃-C₈)-cycloalkenyl-(C₁-C₆)-alkyl, (C₁-C₁₂)-alkylcarbonyl or (C₁-C₁₂)-haloalkylcarbonyl,
- R¹³, R¹⁴: are each independently hydrogen, (C₁-C₁₂)-alkyl, (C₁-C₁₂)-haloalkyl, (C₂-C₁₂)-alkenyl, (C₂-C₁₂)-haloalkenyl, (C₂-C₁₂)-alkynyl, (C₂-C₁₂)-haloalkynyl, (C₁-C₁₂)-alkylcarbonyl, (C₂-C₁₂)-alkenylcarbonyl, (C₂-C₁₂)-alkynylcarbonyl, (C₁-C₁₂)-haloalkylcarbonyl, (C₁-C₄)-alkylsulphonyl, (C₃-C₈)-cycloalkyl, (C₃-C₈)-cycloalkenyl, (C₃-C₈)-cycloalkyl-(C₁-C₆)-alkyl, (C₃-C₈)-cycloalkenyl-(C₁-C₆)-alkyl, (C₃-C₈)-cycloalkylcarbonyl, (C₃-C₈)-cycloalkenylcarbonyl, (C₃-C₈)-cycloalkyl-(C₁-C₆)-alkylcarbonyl, (C₃-C₈)-cycloalkenyl-(C₁-C₆)-alkylcarbonyl, aryl, arylcarbonyl, arylsulphonyl, hetaryl, hetarylcarbonyl, hetarylsulphonyl, heterocyclyl, heterocyclylcarbonyl, heterocyclylsulphonyl, wherein each of the last-mentioned 17 residues is unsubstituted or is substituted by one or more residues from the group consisting of halogen, nitro, hydroxyl, cyano, NH₂, (C₁-C₆)-alkylamine, (C₁-C₆)-dialkylamine, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, (C₁-C₄)-haloalkylthio, (C₁-C₄)-haloalkylsulphoxy, (C₁-C₄)-haloalkylsulphonyl, (C₁-C₄)-alkoxycarbonyl, (C₁-C₄)-haloalkoxycarbonyl, (C₁-C₄)-alkylcarboxy, (C₃-C₆)-cycloalkyl, (C₃-C₆)-cycloalkyl-(C₁-C₆)-alkyl, (C₁-C₄)-alkoxycarbonyl-(C₁-C₄)-alkyl, hydroxycarbonyl, hydroxycarbonyl-(C₁-C₄)-alkyl and wherein heterocyclyl has q oxo groups,
or
R¹³ and R¹⁴, together with the nitrogen atom to which they are attached, form a 3- to 8-membered unsaturated, partially saturated or saturated ring, which comprises in each case, in addition to the carbon atoms and in addition to the nitrogen atom, p ring members from the group consisting of N(R¹²)ₘ, O and S(O)ₙ and wherein said ring is unsubstituted or is substituted by one or more residues from the group consisting of halogen, nitro, hydroxyl, cyano, NH₂, (C₁-C₆)-alkylamine, (C₁-C₆)-dialkylamine, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, (C₁-C₄)-haloalkylthio, (C₁-C₄)-haloalkylsulphoxy, (C₁-C₄)-haloalkylsulphonyl, (C₁-C₄)-alkoxycarbonyl, (C₁-C₄)-haloalkoxycarbonyl, (C₁-C₄)-alkylcarboxy, (C₃-C₆)-cycloalkyl, (C₃-C₆)-cycloalkyl-(C₁-C₆)-alkyl, (C₁-C₄)-alkoxycarbonyl-(C₁-C₄)-alkyl, hydroxycarbonyl, hydroxycarbonyl-(C₁-C₄)-alkyl and has q oxo groups,
- n: is independently selected from 0, 1 or 2,
- m: is independently selected from 0 or 1,
- p: is independently selected from 0, 1, 2 or 3,
- q: is independently selected from 0, 1 or 2,
- y: is 0 or 1.

The compounds of formula (G) and/or the salt thereof as defined in the context of the present invention are useful as herbicides and/or plant growth regulators, preferably in crops of useful plants and/or ornamental plants.

The compounds of the formula (G) according to the invention include all stereoisomers which can occur on the basis of the centres of asymmetry or double bonds in the molecule whose configuration is not designated specifically in the formula or which are not specified explicitly, and mixtures thereof, including the racemic compounds and the mixtures enriched partly with particular stereoisomers. The invention also includes all tautomers, such as keto and enol tautomers, and their mixtures and salts, if appropriate functional groups are present.

In the case of suitable acidic substituents, the compounds of the formula (G) are able to form salts by reaction with bases where the acidic hydrogen is replaced by an agriculturally suitable cation.

By addition of a suitable inorganic or organic acid onto a basic group, such as, for example, amino or alkylamino, the compounds of the formula (G) are able to form salts. Suitable acidic groups present, such as, for example, carboxylic acid groups, are able to form inner salts with groups which for their part can be protonated, such as amino groups.

The compounds of the formula (G) may preferably be present in the form of agriculturally usable salts, where the type of salt is otherwise immaterial. In general, suitable salts are the salts of those cations or the acid additions salts of those acids whose cations and anions, respectively, have no adverse effect on the herbicidal activity of the compounds of formula (G).

Suitable cations are in particular the ions of the alkali metals, preferably lithium, sodium or potassium, of the alkaline earth metals, preferably calcium or magnesium, and of the transition metals, preferably manganese, copper, zinc or iron. The cation used may also be ammonium or substituted ammonium, where one to four hydrogen atoms may be replaced by (C₁-C₄)-alkyl, hydroxy-(C₁-C₄)-alkyl, (C₁-C₄)-alkoxy-(C₁-C₄)-alkyl, hydroxy-(C₁-C₄)-alkoxy-(C₁-C₄)-alkyl, phenyl or benzyl, preferably ammonium, dimethylammonium, diisopropylammonium, tetramethylammonium, tetrabutylammonium, 2-(2-hydroxyeth-1-oxy)eth-1 -ylammonium, di(2-hydroxyeth-1-yl)ammonium, trimethylbenzylammonium. Also suitable are phosphonium ions, sulphonium ions, preferably tri(C₁-C₄)methylsulphonium, or sulphoxonium ions, preferably tri(C₁-C₄)methylsulphoxonium.

Anions of useful acid addition salts are primarily chloride, bromide, fluoride, hydrogensulphate, sulphate, dihydrogenphosphate, hydrogenphosphate, nitrate, bicarbonate, carbonate, hexafluorosilicate, hexafluorophosphate, benzoate and also the anions of (C₁-C₄)-alkanoic acids, preferably formate, acetate, propionate, butyrate or trifluoroacetate.

The indexes n, m, p and q are used in the definitions of different structural elements which may be present in residues R¹, R², R³ and A, and are independently selected from the indexes n, m, p and q, respectively, which are optionally present in the respective other residues R¹, R², R³ and A. For example, q may be 1 in residue R¹, q may be 0 in residue R², and q may be 2 in residue R³.

In formula (G) and in all subsequent formulae, chemical radicals or substituents are referred to by names which are collective terms for the enumeration of individual group members or specifically refer to individual chemical radicals or substituents. In general, terms are used which are familiar to the person skilled in the art and/or in particular have the meanings illustrated below.

A hydrocarbon radical is an aliphatic, cycloaliphatic or aromatic monocyclic or, in the case of an optionally substituted hydrocarbon radical, also a bicyclic or polycyclic organic radical based on the elements carbon and hydrogen, including, for example, the radicals alkyl, alkenyl, alkynyl, cycloalkyl, cycloalkenyl, aryl, phenyl, naphthyl, indanyl, indenyl, etc.; this applies correspondingly to hydrocarbon radicals in composite meanings, such as hydrocarbonoxy radicals or other hydrocarbon radicals attached via heteroatom groups.

Unless defined in more detail, the hydrocarbon radicals preferably have 1 to 20 carbon atoms, more preferably 1 to 16 carbon atoms, in particular 1 to 12 carbon atoms. The hydrocarbon radicals, also in the special radicals alkyl, alkoxy, haloalkyl, haloalkoxy, alkylamino and alkylthio, and also the corresponding unsaturated and/or substituted radicals may in each case be straight-chain or branched in the carbon skeleton.

The expression "(C₁-C₄)-alkyl" is a brief notation for alkyl having from 1 to 4 carbon atoms, i.e. encompasses the methyl, ethyl, 1-propyl, 2-propyl, 1-butyl, 2-butyl, 2-methylpropyl or tert-butyl radicals. General alkyl radicals with a larger specified range of carbon atoms, e.g. "(C₁-C₆)-alkyl", correspondingly also encompass straight-chain or branched alkyl radicals with a greater number of carbon atoms, i.e. according to the example also the alkyl radicals having 5 and 6 carbon atoms.

Unless stated specifically, preference is given to the lower carbon skeletons, for example having from 1 to 6 carbon atoms, or having from 2 to 6 carbon atoms in the case of unsaturated groups, in the case of the hydrocarbyl radicals such as alkyl, alkenyl and alkynyl radicals, including in composite radicals. Alkyl radicals, including in the combined definitions such as alkoxy, haloalkyl, etc., are, for example, methyl, ethyl, n- or i-propyl, n-, i-, t- or 2-butyl, pentyls, hexyls such as n-hexyl, i-hexyl and 1,3-dimethylbutyl, heptyls such as n-heptyl, 1-methylhexyl and 1,4-dimethylpentyl; alkenyl and alkynyl radicals are defined as the possible unsaturated radicals corresponding to the alkyl radicals; alkenyl is, for example, vinyl, allyl, 1-methyl-2-propenyl, 2-methyl-2-propenyl, 2-butenyl, pentenyl, 2-methylpentenyl or hexenyl group, preferably allyl, 1-methylprop-2-en-1-yl, 2-methylprop-2-en-1-yl, but-2-en-1-yl, but-3-en-1-yl, 1-methylbut-3-en-1-yl or 1-methylbut-2-en-1-yl.

Alkenyl also includes in particular straight-chain or branched hydrocarbon radicals having more than one double bond, such as 1,3-butadienyl and 1,4-pentadienyl, but also allenyl or cumulenyl radicals having one or more cumulated double bonds, for example allenyl (1,2-propadienyl), 1,2-butadienyl and 1,2,3-pentatrienyl.

Alkynyl is, for example, propargyl, but-2-yn-1-yl, but-3-yn-1-yl, 1-methylbut-3-yn-1-yl.

Alkynyl also includes, in particular, straight-chain or branched hydrocarbon radicals having more than one triple bond or else having one or more triple bonds and one or more double bonds, for example 1,3-butatrienyl or 3-penten-1-yn-1-yl.

A 3- to 9-membered carbocyclic ring is (C₃-C₉)-cycloalkyl or (C₅-C₉)-cycloalkenyl.

(C₃-C₉)-Cycloalkyl is a carbocyclic saturated ring system having preferably 3-9 carbon atoms, for example cyclopropyl, cyclobutyl, cyclopentyl, cyclohexyl, cycloheptyl, cyclooctyl or cyclononyl. In the case of substituted cycloalkyl, cyclic systems with substituents are included, where the substituents may also be bonded by a double bond on the cycloalkyl radical, for example an alkylidene group such as methylidene.

(C₅-C₉)-Cycloalkenyl is a carbocyclic, nonaromatic, partially unsaturated ring system having 5-9 carbon atoms, for example 1-cyclobutenyl, 2-cyclobutenyl, 1-cyclopentenyl, 2-cyclopentenyl, 3-cyclopentenyl, or 1-cyclohexenyl, 2-cyclohexenyl, 3-cyclohexenyl, 1,3-cyclohexadienyl or 1,4-cyclohexadienyl. In the case of substituted cycloalkenyl, the explanations for substituted cycloalkyl apply correspondingly.

Alkylidene, for example also in the form of (C₁-C₁₀)-alkylidene, is the radical of a straight-chain or branched alkane which is bonded via a double bond, the position of the binding site not being fixed. In the case of a branched alkane, the only positions possible are, of course, those in which two hydrogen atoms can be replaced by the double bond; radicals are, for example, =CH₂, =CH-CH₃, =C(CH₃)-CH₃, =C(CH₃)-C₂H₅ or =C(C₂H₅)-C₂H₅.

Halogen is, for example, fluorine, chlorine, bromine or iodine. Haloalkyl, -alkenyl and -alkynyl are alkyl, alkenyl and alkynyl, respectively, which are partially or fully substituted by identical or different halogen atoms, preferably from the group consisting of fluorine, chlorine, bromine and iodine, in particular from the group consisting of fluorine, chlorine and bromine, very particularly from the group consisting of fluorine and chlorine, for example monohaloalkyl, perhaloalkyl, CF₃, CHF₂, CH₂F, CF₃CF₂, CH₂FCHCl, CCl₃, CHCl₂, CH₂CH₂Cl; haloalkoxy is, for example, OCF₃, OCHF₂, OCH₂F, CF₃CF₂O, OCH₂CF₃ and OCH₂CH₂Cl; this applies correspondingly to haloalkenyl and other halogen-substituted radicals such as, for example, halocycloalkyl.

Aryl is a mono-, bi- or polycyclic aromatic system, for example phenyl, naphthyl, tetrahydronaphthyl, indenyl, indanyl, pentalenyl, fluorenyl and the like, preferably phenyl.

Optionally substituted aryl also includes polycyclic systems, such as tetrahydronaphthyl, indenyl, indanyl, fluorenyl, biphenylyl, where the point of attachment is at the aromatic system.

A heterocyclic radical (heterocyclyl) comprises at least one heterocyclic ring (=carbocyclic ring in which at least one carbon atom is replaced by a heteroatom, preferably by a heteroatom from the group consisting of N, O, S, P, B, Si, Se), which is saturated, unsaturated or heteroaromatic and may be unsubstituted or substituted, where the point of attachment is located at a ring atom.

Unless defined otherwise it preferably contains one or more, in particular 1, 2 or 3, heteroatoms in the heterocyclic ring, preferably from the group consisting of N, O, and S; it is preferably an aliphatic heterocyclyl radical having 3 to 7 ring atoms or a heteroaromatic radical having 5 or 6 ring atoms. The heterocyclic radical may, for example, be a heteroaromatic radical or ring (heteroaryl), such as, for example, a monocyclic, bicyclic or polycyclic aromatic system in which at least 1 ring contains one or more heteroatoms.

If the heterocyclyl radical or the heterocyclic ring is optionally substituted, it can be fused to other carbocyclic or heterocyclic rings. Preference is given to benzo-fused heterocyclic or heteroaromatic rings.

Optionally substituted heterocyclyl also includes polycyclic systems, such as, for example, 8-aza-bicyclo[3.2.1]octanyl or 1-aza-bicyclo[2.2.1]heptyl.

Optionally substituted heterocyclyl also includes spirocyclic systems, such as, for example, 1-oxa-5-aza-spiro[2.3]hexyl.

It is preferably a radical of a heteroaromatic ring having a heteroatom from the group consisting of N, O and S, for example the radical of a five- or six-membered ring, such as pyridyl, pyrrolyl, thienyl or furyl; it is furthermore preferably a radical of a corresponding heteroaromatic ring having 2, 3 or 4 heteroatoms, for example pyrimidinyl, pyridazinyl, pyrazinyl, triazinyl, tetrazinyl, thiazolyl, thiadiazolyl, oxazolyl, isoxazolyl, pyrazolyl, imidazolyl or triazolyl or tetrazolyl.

Here, preference is given to a radical of a heteroaromatic five- or six-membered ring having 1 to 4 heteroatoms, such as, for example, 1,2,3-triazolyl, 1,2,4-triazolyl, tetrazolyl, isothiazolyl, 1,2,3-oxadiazolyl, 1,2,4-oxadiazolyl, 1,3,4-oxadiazolyl, 1,2,5-oxadiazolyl, 1,2,3-thiadiazolyl, 1,2,4-thiadiazolyl, 1,3,4-thiadiazolyl, 1,2,5-thiadiazolyl, tetrazolyl, 1,2,3-triazinyl, 1,2,4-triazinyl, 1,3,5-triazinyl, 1,2,3,4-tetrazinyl, 1,2,3,5-tetrazinyl, 1,2,4,5-tetrazinyl, thiazolyl, isothiazolyl, oxazolyl, isoxazolyl, pyrazolyl, imidazolyl.

More preference is given here to heteroaromatic radicals of five-membered heterocycles having 3 nitrogen atoms, such as 1,2,3-triazol-1-yl, 1,2,3-triazol-4-yl, 1,2,3-triazol-5-yl, 1,2,5-triazol-1-yl, 1,2,5-triazol-3-yl, 1,3,4-triazol-1-yl, 1,3,4-triazol-2-yl, 1,2,4-triazol-3-yl, 1,2,4-triazol-5-yl;
more preference is also given here to heteroaromatic radicals of six-membered heterocycles having 3 nitrogen atoms, such as 1,3,5-triazin-2-yl, 1,2,4-triazin-3-yl, 1,2,4-triazin-5-yl, 1,2,4-triazin-6-yl, 1,2,3-triazin-4-yl, 1,2,3-triazin-5-yl;
more preference is also given here to heteroaromatic radicals of five-membered heterocycles having two nitrogen atoms and one oxygen atom, such as 1,2,4-oxadiazol-3-yl; 1,2,4-oxadiazol-5-yl, 1,3,4-oxadiazol-2-yl, 1,2,3-oxadiazol-4-yl, 1,2,3-oxadiazol-5-yl, 1,2,5-oxadiazol-3-yl,
more preference is also given here to heteroaromatic radicals of five-membered heterocycles having two nitrogen atoms and one sulphur atom, such as 1,2,4-thiadiazol-3-yl, 1,2,4-thiadiazol-5-yl, 1,3,4-thiadiazol-2-yl, 1,2,3-thiadiazol-4-yl, 1,2,3-thiadiazol-5-yl, 1,2,5-thiadiazol-3-yl;
more preference is also given here to heteroaromatic radicals of five-membered heterocycles having four nitrogen atoms, such as 1,2,3,4-tetrazol-1-yl, 1,2,3,4-tetrazol-5-yl, 1,2,3,5-tetrazol-1-yl, 1,2,3,5-tetrazol-4-yl, 2*H*-1,2,3,4-tetrazol-5-yl, 1*H-*1,2,3,4-tetrazol-5-yl,
more preference is also given here to heteroaromatic radicals of six-membered heterocycles such as 1,2,4,5-tetrazin-3-yl;
more preference is also given here to heteroaromatic radicals of five-membered heterocycles having three nitrogen atoms and one oxygen or sulphur atom, such as 1,2,3,4-oxatriazol-5-yl; 1,2,3,5-oxatriazol-4-yl; 1,2,3,4-thiatriazol-5-yl; 1,2,3,5-thiatriazol-4-yl;
more preference is also given here to heteroaromatic radicals of six-membered heterocycles such as, for example, 1,2,4,6-thiatriazin-1-yl; 1,2,4,6-thiatriazin-3-yl; 1,2,4,6-thiatriazin-5-yl.

Furthermore preferably, the heterocyclic radical or ring is a partially or fully hydrogenated heterocyclic radical having one heteroatom from the group consisting of N, O and S, for example oxiranyl, oxetanyl, oxolanyl (= tetrahydrofuryl), oxanyl, pyrrolinyl, pyrrolidyl or piperidyl.

It is also preferably a partially or fully hydrogenated heterocyclic radical having 2 heteroatoms from the group consisting of N, O and S, for example piperazinyl, dioxolanyl, oxazolinyl, isoxazolinyl, oxazolidinyl, isoxazolidinyl and morpholinyl. Suitable substituents for a substituted heterocyclic radical are the substituents specified later on below, and additionally also oxo. The oxo group may also occur on the hetero-ring atoms which are able to exist in different oxidation states, as in the case of N and S, for example.

Preferred examples of heterocyclyl are a heterocyclic radical having from 3 to 6 ring atoms from the group consisting of pyridyl, thienyl, furyl, pyrrolyl, oxiranyl, 2-oxetanyl, 3-oxetanyl, oxolanyl (= tetrahydrofuryl), pyrrolidyl, piperidyl, especially oxiranyl, 2-oxetanyl, 3-oxetanyl or oxolanyl, or is a heterocyclic radical having two or three heteroatoms, for example pyrimidinyl, pyridazinyl, pyrazinyl, triazinyl, thiazolyl, thiadiazolyl, oxazolyl, isoxazolyl, pyrazolyl, triazolyl, piperazinyl, dioxolanyl, oxazolinyl, isoxazolinyl, oxazolidinyl, isoxazolidinyl or morpholinyl.

Preferred heterocyclic radicals are also benzo-fused heteroaromatic rings, for example benzofuryl, benzisofuryl, benzothiophenyl, benzisothiophenyl, isobenzothiophenyl, indolyl, isoindolyl, indazolyl, benzimidazolyl, benzotriazolyl, benzoxazolyl, 1,2-benzisoxazolyl, 2,1-benzisoxazolyl, benzothiazolyl, 1,2-benzisothiazolyl, 2,1-benzisothiazolyl, 1,2,3-benzoxadiazolyl, 2,1,3-benzoxadiazolyl, 1,2,3-benzothiadiazolyl, 2,1,3-benzothiadiazolyl, quinolyl (quinolinyl), isoquinolyl (isoquinolinyl), quinnolinyl, phthalazinyl, quinazolinyl, quinoxalinyl, naphthyridinyl, benzotriazinyl, purinyl, pteridinyl, indolizinyl, benzo-1,3-dioxylyl, 4H-benzo-1,3-dioxinyl and 4H-benzo-1,4-dioxinyl, and, where possible, N-oxides and salts thereof.

When a base structure is substituted by one or more radicals from a list of radicals (= group) or a generically defined group of radicals, this in each case includes simultaneous substitution by a plurality of identical and/or structurally different radicals.

Substituted radicals, such as a substituted alkyl, alkenyl, alkynyl, cycloalkyl, aryl, phenyl, benzyl, heterocyclyl and heteroaryl radical, are, for example, a substituted radical derived from the unsubstituted base structure, where the substituents are, for example, one or more, preferably 1, 2 or 3, radicals from the group consisting of halogen, alkoxy, alkylthio, hydroxyl, amino, nitro, carboxyl, cyano, azido, alkoxycarbonyl, alkylcarbonyl, formyl, carbamoyl, mono- and dialkylaminocarbonyl, substituted amino such as acylamino, mono- and dialkylamino, and alkylsulphinyl, alkylsulphonyl and, in the case of cyclic radicals, also alkyl, haloalkyl, alkylthioalkyl, alkoxyalkyl, optionally substituted mono- and dialkylaminoalkyl and hydroxyalkyl; in the term "substituted radicals", such as substituted alkyl, etc., substituents include, in addition to the saturated hydrocarbon radicals mentioned, corresponding unsaturated aliphatic and aromatic radicals, such as optionally substituted alkenyl, alkynyl, alkenyloxy, alkynyloxy, phenyl and phenoxy. In the case of substituted cyclic radicals having aliphatic moieties in the ring, cyclic systems with those substituents which are bonded on the ring by a double bond are also included, for example substituted by an alkylidene group such as methylidene or ethylidene.

Unless defined in more detail, optionally substituted phenyl is preferably phenyl or phenyl which is unsubstituted or substituted by one or more radicals from the group consisting of halogen, cyano, (C₁-C₄)-alkyl, (C₁-C₄)-haloalkyl, (C₁-C₄)-alkoxy-(C₁-C₄)alkyl, (C₁-C₄)-alkoxy, (C₁-C₄)-haloalkoxy, (C₁-C₄)-alkoxy-(C₁-C₄)-alkoxy, (C₁-C₄)-alkylthio and nitro, in particular phenyl which is optionally substituted by one or more radicals from the group consisting of halogen, (C₁-C₄)-alkyl, (C₁-C₄)-haloalkyl and (C₁-C₄)-alkoxy.

In the case of radicals having carbon atoms, preference is given to those having 1 to 6 carbon atoms, preferably 1 to 4 carbon atoms. Preference is generally given to substituents from the group consisting of halogen, e.g. fluorine and chlorine, (C₁-C₄)-alkyl, preferably methyl or ethyl, (C₁-C₄)-haloalkyl, preferably trifluoromethyl, (C₁-C₄)-alkoxy, preferably methoxy or ethoxy, (C₁-C₄)-haloalkoxy, nitro and cyano. Particular preference is given here to the substituents methyl, methoxy, fluorine and chlorine.

Substituted amino, such as mono- or disubstituted amino, is a radical from the group consisting of the substituted amino radicals which are N-substituted, for example, by one or two identical or different radicals from the group consisting of alkyl, alkoxy, acyl and aryl; preferably mono- and dialkylamino, mono- and diarylamino, acylamino, N-alkyl-N-arylamino, N-alkyl-N-acylamino and N-heterocycles; preference is given to alkyl radicals having from 1 to 4 carbon atoms; aryl is preferably phenyl or substituted phenyl; acyl is as defined below, preferably (C₁-C₄)-alkanoyl. The same applies to substituted hydroxylamino or hydrazino.

Acyl is a radical of an organic acid which arises in a formal sense by removal of a hydroxyl group on the acid function, and the organic radical in the acid may also be bonded to the acid function via a heteroatom. Examples of acyl are the -CO-R radical of a carboxylic acid HO-CO-R and radicals of acids derived therefrom, such as those of thiocarboxylic acid, optionally N-substituted iminocarboxylic acids or the radical of carbonic monoesters, N-substituted carbamic acid, sulphonic acids, sulphinic acids, N-substituted sulphonamide acids, phosphonic acids or phosphinic acids.

Acyl is, for example, formyl, alkylcarbonyl such as [(C₁-C₄)-alkyl]carbonyl, phenylcarbonyl, alkyloxycarbonyl, phenyloxycarbonyl, benzyloxycarbonyl, alkylsulphonyl, alkylsulphinyl, N-alkyl-1-iminoalkyl and other radicals of organic acids. The radicals may each be substituted further in the alkyl or phenyl moiety, for example in the alkyl moiety by one or more radicals from the group consisting of halogen, alkoxy, phenyl and phenoxy; examples of substituents in the phenyl moiety are the substituents already mentioned above in general for substituted phenyl.

Acyl is preferably an acyl radical in the narrower sense, i.e. a radical of an organic acid in which the acid group is bonded directly to the carbon atom of an organic radical, for example formyl, alkylcarbonyl such as acetyl or [(C₁-C₄)-alkyl]carbonyl, phenylcarbonyl, alkylsulphonyl, alkylsulphinyl and other radicals of organic acids.

More preferably, acyl is an alkanoyl radical having 1 to 6 carbon atoms, in particular 1 to 4 carbon atoms. Here, (C₁-C₄)-alkanoyl is the radical of an alkanoic acid having 1 to 4 carbon atoms formed after removal of the OH group of the acid group, i.e. formyl, acetyl, n-propionyl, isopropionyl or n-, i-, sec- or tert-butanoyl.

The "yl position" of a radical denotes the carbon atom having the free bond.

Compounds of the formula (G) according to the invention and compounds of the formula (G) used according to the invention and/or salts thereof are in short also referred to as "compounds (G)".

The invention also provides all stereoisomers which are encompassed by formula (G) and mixtures thereof. Such compounds of the formula (G) may contain one or more asymmetric carbon atoms or may contain double bonds which are not stated separately in the general formulae (G). The possible stereoisomers defined by their specific three-dimensional shape, such as enantiomers, diastereomers, Z-and E-isomers, are all encompassed by the formula (G) and can be obtained from mixtures of the stereoisomers by customary methods or else prepared by stereoselective reactions in combination with the use of stereochemically pure starting materials.

According to the present invention, compounds of the formula (G) and/or a salt thereof are preferred, in which
- A: is CR⁶R⁷,
- W: is O or S,
- R¹: is (C₁-C₆)-alkyl, (C₁-C₆)-haloalkyl, (C₂-C₆)-alkenyl, (C₂-C₆)-alkynyl, NR¹³R¹⁴, R¹³R¹⁴N-(C₁-C₆)-alkyl, (C₁-C₆)-alkoxy, (C₁-C₆)-haloalkoxy, (C₁-C₆)-haloalkoxy-(C₁-C₃)-alkyl, (C₁-C₆)-alkoxy-(C₁-C₃)-alkyl, (C₁-C₆)-alkoxy-(C₂-C₆)-alkoxy-(C₁-C₃)-alkyl, (C₁-C₄)-alkylthio, (C₁-C₄)-alkylsulphoxy, (C₁-C₄)-alkylsulphonyl, (C₁-C₄)-alkylthio-(C₁-C₃)-alkyl, (C₁-C₄)-alkylsulphoxy-(C₁-C₃)-alkyl, (C₁-C₄)-alkylsulphonyl-(C₁-C₃)-alkyl, (C₃-C₈)-cycloalkyl, (C₃-C₆)-cycloalkenyl, (C₃-C₆)-cycloalkyl-(C₁-C₃)-alkyl, (C₃-C₆)-cycloalkenyl-(C₁-C₃)-alkyl, (C₃-C₆)-cycloalkoxy, phenyl, heteroaryl, heterocyclyl, phenoxy, heteroaryloxy, heterocyclyloxy or a carbobicyclic residue, wherein each of the last-mentioned 12 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
- R², R³: are each independently hydrogen, (C₁-C₆)-alkyl, (C₁-C₆)-haloalkyl, (C₂-C₆)-alkenyl, (C₂-C₆)-alkynyl, (C₁-C₆)-alkoxy-(C₁-C₃)-alkyl, (C₁-C₆)-alkoxy-(C₁-C₃)-alkylcarbonyl, (C₁-C₄)-alkylthio, (C₁-C₄)-haloalkylthio, (C₁-C₄)-alkylthiocarbonyl, (C₁-C₄)-haloalkylthiocarbonyl, (C₁-C₄)-alkylthio-(C₁-C₃)-alkyl, (C₁-C₄)-alkylsulphoxy-(C₁-C₃)-alkyl, (C₁-C₄)-alkylsulphonyl-(C₁-C₃)-alkyl, (C₁-C₄)-alkylthio-(C₁-C₃)-alkylcarbonyl, (C₁-C₄)-alkylsulphoxy-(C₁-C₃)-alkylcarbonyl, (C₁-C₄)-alkylsulphonyl-(C₁-C₃)-alkylcarbonyl, (C₁-C₆)-alkylcarbonyl, (C₁-C₆)-haloalkylcarbonyl, (C₂-C₆)-alkenylcarbonyl, (C₂-C₆)-alkynylcarbonyl, (C₁-C₆)-alkoxycarbonylcarbonyl, (C₁-C₆)-alkoxycarbonyl-(C₁-C₃)-alkylcarbonyl, (C₁-C₆)-alkoxycarbonyl, (C₁-C₆)-haloalkoxycarbonyl, (C₂-C₆)-alkenyloxycarbonyl, (C₃-C₆)-cycloalkyl, (C₃-C₆)-cycloalkyl-(C₁-C₆)-alkyl, (C₃-C₆)-cycloalkylcarbonyl, (C₃-C₆)-cycloalkyl-(C₁-C₆)-alkylcarbonyl, phenyl, phenyl-(C₁-C₃)-alkyl, heteroaryl, heteroaryl-(C₁-C₃)-alkyl, heterocyclyl, heterocyclyl-(C₁-C₃)-alkyl, phenylcarbonyl, phenyl-(C₁-C₆)-alkylcarbonyl, hetarylcarbonyl, hetaryl-(C₁-C₆)-alkylcarbonyl, heterocyclylcarbonyl, heterocyclyl-(C₁-C₆)-alkylcarbonyl, wherein each of the last-mentioned 16 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
or
NR²R³ is -N=CR⁸R⁹ or -N=S(O)ₙR¹⁰R¹¹,
- R⁶, R⁷: are each independently hydrogen or (C₁-C₆)-alkyl,
- R⁸, R⁹: are each independently hydrogen, (C₁-C₆)-alkyl, (C₁-C₆)-haloalkyl, (C₂-C₆)-alkenyl, (C₂-C₆)-alkynyl, (C₁-C₆)-alkoxy-(C₁-C₃)-alkyl, (C₁-C₄)-alkylthio-(C₁-C₃)-alkyl, (C₁-C₄)-alkylsulphoxy-(C₁-C₃)-alkyl, (C₁-C₄)-alkylsulphonyl-(C₁-C₃)-alkyl, (C₁-C₆)-alkoxy, (C₁-C₆)-haloalkoxy, (C₂-C₆)-alkenyloxy, NR¹³R¹⁴, (C₃-C₈)-cycloalkyl, (C₃-C₈)-cycloalkyl-(C₁-C₆)-alkyl, phenyl, phenyl-(C₁-C₃)-alkyl, heteroaryl, heteroaryl-(C₁-C₃)-alkyl, heterocyclyl, heterocyclyl-(C₁-C₃)-alkyl, wherein each of the last-mentioned 8 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
or R⁸ and R⁹, together with the carbon atom to which they are attached, form a 3- to 6-membered unsaturated, partially saturated or saturated ring, which comprises in each case, in addition to the carbon atoms, p ring members from the group consisting of N(R¹²)ₘ, O and S(O)ₙ, and wherein said ring is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
- R¹⁰, R¹¹: are each independently (C₁-C₆)-alkyl, (C₂-C₆)-alkenyl, (C₂-C₆)-alkynyl, (C₁-C₆)-alkoxy-(C₁-C₃)-alkyl, (C₁-C₄)-alkylthio-(C₁-C₃)-alkyl, (C₁-C₄)-alkylsulphoxy-(C₁-C₃)-alkyl, (C₁-C₄)-alkylsulphonyl-(C₁-C₃)-alkyl, (C₃-C₈)-cycloalkyl, (C₃-C₈)-cycloalkyl-(C₁-C₆)-alkyl, phenyl, phenyl-(C₁-C₃)-alkyl, heteroaryl, heteroaryl-(C₁-C₃)-alkyl, heterocyclyl, heterocyclyl-(C₁-C₃)-alkyl, wherein each of the last-mentioned 8 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
or
R¹⁰ and R¹¹, together with the sulphur atom to which they are attached, form a 3- to 6-membered unsaturated, partially saturated or saturated ring, which comprises in each case, in addition to the carbon atoms and in addition to the sulphur atom, p ring members from the group consisting of N(R¹²)ₘ, O and S(O)ₙ, and wherein said ring is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy or (C₁-C₄)-alkylsulphonyl,
- R¹²: is hydrogen, (C₁-C₆)-alkyl or (C₁-C₆)-alkylcarbonyl,
- R¹³, R¹⁴: are each independently hydrogen, (C₁-C₆)-alkyl, (C₁-C₆)-alkylcarbonyl, (C₁-C₄)-alkylsulphonyl, phenyl, phenylcarbonyl, wherein each of the last-mentioned two residues is unsubstituted or is substituted by one or more residues from the group consisting of halogen, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl,
or
R¹³ and R¹⁴, together with the nitrogen atom to which they are attached, form a 3- to 8-membered unsaturated, partially saturated or saturated ring, which comprises in each case, in addition to the carbon atoms and in addition to the nitrogen atom, p ring members from the group consisting of N(R¹²)ₘ, O and S(O)ₙ, and wherein said ring is unsubstituted or is substituted by one or more residues from the group consisting of halogen, hydroxyl, (C₁-C₄)-alkyl, (C₁-C₄)-haloalkyl, (C₁-C₄)-alkoxy, (C₁-C₄)-haloalkoxy, and has q oxo groups,
- n: is independently selected from 0, 1 or 2,
- m: is independently selected from 0 or 1,
- p: is independently selected from 0, 1 or 2,
- q: is independently selected from 0, 1 or 2,
- y: is 0 or 1.

Preferred compounds according to the present invention correspond to the formula (G) as defined hereinabove, wherein
- R¹: is (C₁-C₆)-alkyl, (C₁-C₆)-haloalkyl, (C₂-C₆)-alkenyl, (C₂-C₆)-alkynyl, NR¹³R¹⁴, R¹³R¹⁴N-(C₁-C₆)-alkyl, (C₁-C₆)-alkoxy, (C₁-C₆)-haloalkoxy, (C₁-C₆)-haloalkoxy-(C₁-C₃)-alkyl, (C₁-C₆)-alkoxy-(C₁-C₃)-alkyl, (C₁-C₆)-alkoxy-(C₂-C₆)-alkoxy-(C₁-C₃)-alkyl, (C₁-C₄)-alkylthio, (C₁-C₄)-alkylsulphoxy, (C₁-C₄)-alkylsulphonyl, (C₁-C₄)-alkylthio-(C₁-C₃)-alkyl, (C₁-C₄)-alkylsulphoxy-(C₁-C₃)-alkyl, (C₁-C₄)-alkylsulphonyl-(C₁-C₃)-alkyl, (C₃-C₈)-cycloalkyl, (C₃-C₆)-cycloalkenyl, (C₃-C₆)-cycloalkyl-(C₁-C₃)-alkyl, (C₃-C₆)-cycloalkenyl-(C₁-C₃)-alkyl, (C₃-C₆)-cycloalkoxy, phenyl, heteroaryl, heterocyclyl, phenoxy, heteroaryloxy or a heterocyclyloxy residue, wherein each of the last-mentioned 11 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
and wherein A, W, R², R³, n, m, p, q and y each have the meaning defined herein.

According to the present invention, compounds of the formula (G) and/or a salt thereof are preferred, in which
- A: is CR⁶R⁷,
- W: is O or S,
- R¹: is (C₁-C₆)-alkyl, (C₁-C₆)-haloalkyl, (C₂-C₆)-alkenyl, (C₂-C₆)-alkynyl, NR¹³R¹⁴, R¹³R¹⁴N-(C₁-C₆)-alkyl, (C₁-C₆)-alkoxy, (C₁-C₆)-haloalkoxy, (C₁-C₆)-alkoxy-(C₁-C₃)-alkyl, (C₁-C₆)-alkoxy-(C₂-C₆)-alkoxy-(C₁-C₃)-alkyl, (C₁-C₄)-alkylthio, (C₁-C₄)-alkylsulphoxy, (C₁-C₄)-alkylsulphonyl, (C₁-C₄)-alkylthio-(C₁-C₃)-alkyl, (C₁-C₄)-alkylsulphoxy-(C₁-C₃)-alkyl, (C₁-C₄)-alkylsulphonyl-(C₁-C₃)-alkyl, (C₃-C₈)-cycloalkyl, (C₃-C₆)-cycloalkenyl, (C₃-C₆)-cycloalkyl-(C₁-C₃)-alkyl, (C₃-C₆)-cycloalkenyl-(C₁-C₃)-alkyl, (C₃-C₆)-cycloalkoxy, phenyl, heteroaryl, heterocyclyl, phenoxy, heteroaryloxy or heterocyclyloxy, wherein each of the last-mentioned 11 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
- R², R³: are each independently hydrogen, (C₁-C₆)-alkyl, (C₁-C₆)-haloalkyl, (C₂-C₆)-alkenyl, (C₂-C₆)-alkynyl, (C₁-C₆)-alkoxy-(C₁-C₃)-alkyl, (C₁-C₆)-alkoxy-(C₁-C₃)-alkylcarbonyl, (C₁-C₄)-alkylthio, (C₁-C₄)-haloalkylthio, (C₁-C₄)-alkylthiocarbonyl, (C₁-C₄)-haloalkylthiocarbonyl, (C₁-C₄)-alkylthio-(C₁-C₃)-alkyl, (C₁-C₄)-alkylsulphoxy-(C₁-C₃)-alkyl, (C₁-C₄)-alkylsulphonyl-(C₁-C₃)-alkyl, (C₁-C₄)-alkylthio-(C₁-C₃)-alkylcarbonyl, (C₁-C₄)-alkylsulphoxy-(C₁-C₃)-alkylcarbonyl, (C₁-C₄)-alkylsulphonyl-(C₁-C₃)-alkylcarbonyl, (C₁-C₆)-alkylcarbonyl, (C₁-C₆)-haloalkylcarbonyl, (C₂-C₆)-alkenylcarbonyl, (C₂-C₆)-alkynylcarbonyl, (C₁-C₆)-alkoxycarbonylcarbonyl, (C₁-C₆)-alkoxycarbonyl-(C₁-C₃)-alkylcarbonyl, (C₁-C₆)-alkoxycarbonyl, (C₁-C₆)-haloalkoxycarbonyl, (C₂-C₆)-alkenyloxycarbonyl, (C₃-C₆)-cycloalkyl, (C₃-C₆)-cycloalkyl-(C₁-C₆)-alkyl, (C₃-C₆)-cycloalkylcarbonyl, (C₃-C₆)-cycloalkyl-(C₁-C₆)-alkylcarbonyl, phenyl, phenyl-(C₁-C₃)-alkyl, heteroaryl, heteroaryl-(C₁-C₃)-alkyl, heterocyclyl, heterocyclyl-(C₁-C₃)-alkyl, phenylcarbonyl, phenyl-(C₁-C₆)-alkylcarbonyl, hetarylcarbonyl, hetaryl-(C₁-C₆)-alkylcarbonyl, heterocyclylcarbonyl, heterocyclyl-(C₁-C₆)-alkylcarbonyl, wherein each of the last-mentioned 16 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
or
NR²R³ is -N=CR⁸R⁹ or -N=S(O)ₙR¹⁰R¹¹,
- R⁶, R⁷: are each independently hydrogen or (C₁-C₄)-alkyl, preferably R⁶ and R⁷ independently are hydrogen or methyl,
- R⁸, R⁹: are each independently hydrogen, (C₁-C₆)-alkyl, (C₁-C₆)-haloalkyl, (C₂-C₆)-alkenyl, (C₂-C₆)-alkynyl, (C₁-C₆)-alkoxy-(C₁-C₃)-alkyl, (C₁-C₄)-alkylthio-(C₁-C₃)-alkyl, (C₁-C₄)-alkylsulphoxy-(C₁-C₃)-alkyl, (C₁-C₄)-alkylsulphonyl-(C₁-C₃)-alkyl, (C₁-C₆)-alkoxy, (C₁-C₆)-haloalkoxy, (C₂-C₆)-alkenyloxy, NR¹³R¹⁴, (C₃-C₈)-cycloalkyl, (C₃-C₈)-cycloalkyl-(C₁-C₆)-alkyl, phenyl, phenyl-(C₁-C₃)-alkyl, heteroaryl, heteroaryl-(C₁-C₃)-alkyl, heterocyclyl, heterocyclyl-(C₁-C₃)-alkyl, wherein each of the last-mentioned 8 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
or
R⁸ and R⁹, together with the carbon atom to which they are attached, form a 3- to 6-membered unsaturated, partially saturated or saturated ring, which comprises in each case, in addition to the carbon atoms, p ring members from the group consisting of N(R¹²)ₘ, O and S(O)ₙ, and wherein said ring is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl,
- R¹⁰, R¹¹: are each independently (C₁-C₆)-alkyl, (C₂-C₆)-alkenyl, (C₂-C₆)-alkynyl, (C₁-C₆)-alkoxy-(C₁-C₃)-alkyl, (C₁-C₄)-alkylthio-(C₁-C₃)-alkyl, (C₁-C₄)-alkylsulphoxy-(C₁-C₃)-alkyl, (C₁-C₄)-alkylsulphonyl-(C₁-C₃)-alkyl, (C₃-C₈)-cycloalkyl, (C₃-C₈)-cycloalkyl-(C₁-C₆)-alkyl, phenyl, phenyl-(C₁-C₃)-alkyl, heteroaryl, heteroaryl-(C₁-C₃)-alkyl, heterocyclyl, heterocyclyl-(C₁-C₃)-alkyl, wherein each of the last-mentioned 8 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
or
R¹⁰ and R¹¹, together with the sulphur atom to which they are attached, form a 3- to 6-membered unsaturated, partially saturated or saturated ring, which comprises in each case, in addition to the carbon atoms and in addition to the sulphur atom, p ring members from the group consisting of N(R¹²)ₘ, O and S(O)ₙ, and wherein said ring is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy or (C₁-C₄)-alkylsulphonyl,
- R¹²: is hydrogen, (C₁-C₆)-alkyl or (C₁-C₆)-alkylcarbonyl,
- R¹³, R¹⁴: are each independently hydrogen, (C₁-C₆)-alkyl, (C₁-C₆)-alkylcarbonyl, (C₁-C₄)-alkylsulphonyl, phenyl, phenylcarbonyl, wherein each of the last-mentioned two residues is unsubstituted or is substituted by one or more residues from the group consisting of halogen, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl,
or
R¹³ and R¹⁴, together with the nitrogen atom to which they are attached, form a 3- to 8-membered unsaturated, partially saturated or saturated ring, which comprises in each case, in addition to the carbon atoms and in addition to the nitrogen atom, p ring members from the group consisting of N(R¹²)ₘ, O and S(O)ₙ, and wherein said ring is unsubstituted or is substituted by one or more residues from the group consisting of halogen, hydroxyl, (C₁-C₄)-alkyl, (C₁-C₄)-haloalkyl, (C₁-C₄)-alkoxy, (C₁-C₄)-haloalkoxy, and has q oxo groups,
- n: is independently selected from 0, 1 or 2,
- m: is independently selected from 0 or 1,
- p: is independently selected from 0, 1 or 2,
- q: is independently selected from 0 or 1,
- y: is 0 or 1.

According to the present invention, more compounds of the formula (G) and/or a salt thereof are more preferred, in which
- A: is CR⁶R⁷,
- W: is O or S, preferably O,
- R¹: is (C₁-C₆)-alkyl, (C₁-C₆)-haloalkyl, (C₂-C₆)-alkenyl, (C₁-C₆)-alkoxy-(C₁-C₃)-alkyl, (C₁-C₆)-alkoxy-(C₂-C₆)-alkoxy-(C₁-C₃)-alkyl, (C₁-C₄)-alkylsulphonyl, (C₁-C₄)-alkylthio-(C₁-C₃)-alkyl, (C₁-C₄)-alkylsulphoxy-(C₁-C₃)-alkyl, (C₁-C₄)-alkylsulphonyl-(C₁-C₃)-alkyl, (C₃-C₈)-cycloalkyl, (C₃-C₆)-cycloalkenyl, (C₃-C₆)-cycloalkyl-(C₁-C₃)-alkyl, (C₃-C₆)-cycloalkenyl-(C₁-C₃)-alkyl, (C₃-C₆)-cycloalkoxy, phenyl, heteroaryl, heterocyclyl, phenoxy, heteroaryloxy or heterocyclyloxy, wherein each of the last-mentioned 11 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
- R², R³: are each independently hydrogen, (C₂-C₆)-alkynyl, (C₁-C₄)-alkylthio, (C₁-C₄)-haloalkylthio, (C₁-C₄)-alkylthiocarbonyl, (C₁-C₄)-haloalkylthiocarbonyl, (C₁-C₆)-alkoxy-(C₁-C₃)-alkylcarbonyl, (C₁-C₄)-alkylthio-(C₁-C₃)-alkylcarbonyl, (C₁-C₄)-alkylsulphoxy-(C₁-C₃)-alkylcarbonyl, (C₁-C₄)-alkylsulphonyl-(C₁-C₃)-alkylcarbonyl, (C₁-C₆)-alkylcarbonyl, (C₁-C₆)-haloalkylcarbonyl, (C₂-C₆)-alkenylcarbonyl, (C₂-C₆)-alkynylcarbonyl, (C₁-C₆)-alkoxycarbonylcarbonyl, (C₁-C₆)-alkoxycarbonyl-(C₁-C₃)-alkylcarbonyl, (C₁-C₆)-alkoxycarbonyl, (C₁-C₆)-haloalkoxycarbonyl, (C₂-C₆)-alkenyloxycarbonyl, (C₃-C₆)-cycloalkylcarbonyl, (C₃-C₆)-cycloalkyl-(C₁-C₆)-alkylcarbonyl, phenylcarbonyl, phenyl-(C₁-C₆)-alkylcarbonyl, hetarylcarbonyl, hetaryl-(C₁-C₆)-alkylcarbonyl, heterocyclylcarbonyl, heterocyclyl-(C₁-C₆)-alkylcarbonyl, wherein each of the last-mentioned 8 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
or
NR²R³ is -N=CR⁸R⁹ or -N=S(O)ₙR¹⁰R¹¹,
- R⁶: is hydrogen,
- R⁷: is hydrogen or methyl,
- R⁸, R⁹: are each independently hydrogen, (C₁-C₆)-alkyl, (C₁-C₆)-haloalkyl, (C₂-C₆)-alkenyl, (C₂-C₆)-alkynyl, (C₁-C₆)-alkoxy-(C₁-C₃)-alkyl, (C₁-C₄)-alkylthio-(C₁-C₃)-alkyl, (C₁-C₄)-alkylsulphoxy-(C₁-C₃)-alkyl, (C₁-C₄)-alkylsulphonyl-(C₁-C₃)-alkyl, (C₁-C₆)-alkoxy, (C₁-C₆)-haloalkoxy, (C₂-C₆)-alkenyloxy, NR¹³R¹⁴, (C₃-C₈)-cycloalkyl, (C₃-C₈)-cycloalkyl-(C₁-C₆)-alkyl, phenyl, phenyl-(C₁-C₃)-alkyl, heteroaryl, heteroaryl-(C₁-C₃)-alkyl, heterocyclyl, heterocyclyl-(C₁-C₃)-alkyl, wherein each of the last-mentioned 8 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
or
R⁸ and R⁹, together with the carbon atom to which they are attached, form a 3- to 6-membered unsaturated, partially saturated or saturated ring, which comprises in each case, in addition to the carbon atoms, p ring members from the group consisting of N(R¹²)ₘ, O and S(O)ₙ, and wherein said ring is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl,
- R¹⁰, R¹¹: are each independently (C₁-C₆)-alkyl, (C₂-C₆)-alkenyl, (C₂-C₆)-alkynyl, (C₁-C₆)-alkoxy-(C₁-C₃)-alkyl, (C₁-C₄)-alkylthio-(C₁-C₃)-alkyl, (C₁-C₄)-alkylsulphoxy-(C₁-C₃)-alkyl, (C₁-C₄)-alkylsulphonyl-(C₁-C₃)-alkyl, (C₃-C₈)-cycloalkyl, (C₃-C₈)-cycloalkyl-(C₁-C₆)-alkyl, phenyl, phenyl-(C₁-C₃)-alkyl, heteroaryl, heteroaryl-(C₁-C₃)-alkyl, heterocyclyl, heterocyclyl-(C₁-C₃)-alkyl, wherein each of the last-mentioned 8 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
or
R¹⁰ and R¹¹, together with the sulphur atom to which they are attached, form a 3- to 6-membered unsaturated, partially saturated or saturated ring, which comprises in each case, in addition to the carbon atoms and in addition to the sulphur atom, p ring members from the group consisting of N(R¹²)ₘ, O and S(O)ₙ, and wherein said ring is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy or (C₁-C₄)-alkylsulphonyl,
- R¹²: is hydrogen, (C₁-C₆)-alkyl or (C₁-C₆)-alkylcarbonyl,
- R¹³, R¹⁴: are each independently hydrogen, (C₁-C₆)-alkyl, (C₁-C₆)-alkylcarbonyl, (C₁-C₄)-alkylsulphonyl, phenyl, phenylcarbonyl, wherein each of the last-mentioned two residues is unsubstituted or is substituted by one or more residues from the group consisting of halogen, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl,
or
R¹³ and R¹⁴, together with the nitrogen atom to which they are attached, form a 3- to 8-membered unsaturated, partially saturated or saturated ring, which comprises in each case, in addition to the carbon atoms and in addition to the nitrogen atom, p ring members from the group consisting of N(R¹²)ₘ, O and S(O)ₙ, and wherein said ring is unsubstituted or is substituted by one or more residues from the group consisting of halogen, hydroxyl, (C₁-C₄)-alkyl, (C₁-C₄)-haloalkyl, (C₁-C₄)-alkoxy, (C₁-C₄)-haloalkoxy, and has q oxo groups,
- n: is independently selected from 0, 1 or 2,
- m: is independently selected from 0 or 1,
- p: is independently selected from 0, 1 or 2,
- q: is independently selected from 0 or 1,
- y: is 0 or 1.

Particularly preferred compounds according to the present invention correspond to the formula (G), wherein y = 1, and wherein W, R¹, R², R³ and A each have, independently from one another, the meaning as defined in the context of the formula (G), preferably each have, independently from one another, the meaning as defined in one of the preferred, more preferred, or particularly preferred embodiments.

Particularly preferred compounds according to the present invention correspond to the formula (G), wherein y = 1, A is CHR⁷ (i.e. R⁶ = H), wherein R⁷ is hydrogen or methyl,
and
wherein W, R¹, R² and R³ each have, independently from one another, the meaning as defined in the context of the formula (G), preferably each have, independently from one another, the meaning as defined in one of the preferred, more preferred, or particularly preferred embodiments.

In a preferred embodiment, in the compounds of formula (G), and the formulae (II) and (III), as defined hereinafter, the residues R² and R³ are not both an alkyl residue.

In a more preferred embodiment, the compounds according to the present invention correspond to the formula (G), wherein
- R², R³: are each independently hydrogen, (C₂-C₆)-alkynyl, (C₁-C₄)-alkylthio, (C₁-C₄)-haloalkylthio, (C₁-C₆)-alkoxy-(C₁-C₃)-alkylcarbonyl, (C₁-C₄)-alkylthio-(C₁-C₃)-alkylcarbonyl, (C₁-C₄)-alkylsulphoxy-(C₁-C₃)-alkylcarbonyl, (C₁-C₄)-alkylsulphonyl-(C₁-C₃)-alkylcarbonyl, (C₁-C₆)-alkylcarbonyl, (C₁-C₆)-haloalkylcarbonyl, (C₂-C₆)-alkenylcarbonyl, (C₂-C₆)-alkynylcarbonyl, (C₁-C₆)-alkoxycarbonylcarbonyl, (C₁-C₆)-alkoxycarbonyl-(C₁-C₃)-alkylcarbonyl, (C₁-C₆)-alkoxycarbonyl, (C₁-C₆)-haloalkoxycarbonyl, (C₂-C₆)-alkenyloxycarbonyl, (C₃-C₆)-cycloalkylcarbonyl, (C₃-C₆)-cycloalkyl-(C₁-C₆)-alkylcarbonyl, phenylcarbonyl, phenyl-(C₁-C₆)-alkylcarbonyl, hetarylcarbonyl, hetaryl-(C₁-C₆)-alkylcarbonyl, heterocyclylcarbonyl, heterocyclyl-(C₁-C₆)-alkylcarbonyl, wherein each of the last-mentioned 8 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
or
NR²R³ is -N=S(O)ₙR¹⁰R¹¹, wherein R¹⁰ and R¹¹ each have, independently from one another, the meaning as defined in the context of the formula (G), preferably each have, independently from one another, the meaning as defined in one of the preferred, more preferred, or particularly preferred embodiments,
and wherein the other structural elements in the formula (G) each have, independently from one another, the meaning as defined in the context of the formula (G), preferably each have, independently from one another, the meaning as defined in one of the preferred, more preferred, or particularly preferred embodiments.

Preferred compounds according to the present invention correspond to the formula (G), or to the formulae (II) and (III), as defined hereinafter, wherein
- n: is independently selected from 0 or 1, more preferably n is 0,
- m: is independently selected from 0 or 1, preferably m is 0,
- p: is independently selected from 0, 1 or 2, preferably p is independently selected from 0 or 1, and
- q: is independently selected from 0 or 1, preferably q is 0.

According to the present invention, compounds of the formula (G) and/or a salt thereof are particularly preferred, in which
- A: is CR⁶R⁷,
- W: is O or S, preferably O,
- R¹: is (C₃-C₈)-cycloalkyl, (C₃-C₆)-cycloalkenyl, (C₃-C₆)-cycloalkyl-(C₁-C₃)-alkyl, (C₃-C₆)-cycloalkenyl-(C₁-C₃)-alkyl, phenyl, heteroaryl, heterocyclyl, wherein each of the last-mentioned 7 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
- R², R³: are each independently hydrogen, (C₂-C₆)-alkynyl, (C₁-C₄)-alkylthio, (C₁-C₄)-haloalkylthio (wherein (C₁-C₄)-haloalkylthio more preferably is SCF₃), (C₁-C₄)-alkylthiocarbonyl, (C₁-C₄)-haloalkylthiocarbonyl, (C₁-C₆)-alkoxy-(C₁-C₃)-alkylcarbonyl, (C₁-C₄)-alkylthio-(C₁-C₃)-alkylcarbonyl, (C₁-C₄)-alkylsulphoxy-(C₁-C₃)-alkylcarbonyl, (C₁-C₄)-alkylsulphonyl-(C₁-C₃)-alkylcarbonyl, (C₁-C₆)-alkylcarbonyl, (C₁-C₆)-haloalkylcarbonyl, (C₂-C₆)-alkenylcarbonyl, (C₂-C₆)-alkynylcarbonyl, (C₁-C₆)-alkoxycarbonylcarbonyl, (C₁-C₆)-alkoxycarbonyl-(C₁-C₃)-alkylcarbonyl, (C₁-C₆)-alkoxycarbonyl, (C₁-C₆)-haloalkoxycarbonyl, (C₂-C₆)-alkenyloxycarbonyl, (C₃-C₆)-cycloalkylcarbonyl, (C₃-C₆)-cycloalkyl-(C₁-C₆)-alkylcarbonyl, phenylcarbonyl, phenyl-(C₁-C₆)-alkylcarbonyl, hetarylcarbonyl, hetaryl-(C₁-C₆)-alkylcarbonyl, heterocyclylcarbonyl, heterocyclyl-(C₁-C₆)-alkylcarbonyl, wherein each of the last-mentioned 8 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
or
NR²R³ is -N=CR⁸R⁹ or -N=S(O)ₙR¹⁰R¹¹,
- R⁶: is hydrogen,
- R⁷: is hydrogen or methyl,
- R⁸, R⁹: are each independently hydrogen, (C₁-C₆)-alkyl, (C₁-C₆)-haloalkyl, (C₂-C₆)-alkenyl, (C₂-C₆)-alkynyl, (C₁-C₆)-alkoxy-(C₁-C₃)-alkyl, (C₁-C₄)-alkylthio-(C₁-C₃)-alkyl, (C₁-C₄)-alkylsulphoxy-(C₁-C₃)-alkyl, (C₁-C₄)-alkylsulphonyl-(C₁-C₃)-alkyl, (C₁-C₆)-alkoxy, (C₁-C₆)-haloalkoxy, (C₂-C₆)-alkenyloxy, NR¹³R¹⁴, (C₃-C₈)-cycloalkyl, (C₃-C₈)-cycloalkyl-(C₁-C₆)-alkyl, phenyl, phenyl-(C₁-C₃)-alkyl, heteroaryl, heteroaryl-(C₁-C₃)-alkyl, heterocyclyl, heterocyclyl-(C₁-C₃)-alkyl, wherein each of the last-mentioned 8 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
or
R⁸ and R⁹, together with the carbon atom to which they are attached, form a 3- to 6-membered unsaturated, partially saturated or saturated ring, which comprises in each case, in addition to the carbon atoms, p ring members from the group consisting of N(R¹²)ₘ, O and S(O)ₙ, and wherein said ring is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl,
- R¹⁰, R¹¹: are each independently (C₁-C₆)-alkyl, (C₂-C₆)-alkenyl, (C₂-C₆)-alkynyl, (C₃-C₈)-cycloalkyl, (C₃-C₈)-cycloalkyl-(C₁-C₆)-alkyl, phenyl, phenyl-(C₁-C₃)-alkyl, heteroaryl, heteroaryl-(C₁-C₃)-alkyl, heterocyclyl, heterocyclyl-(C₁-C₃)-alkyl, wherein each of the last-mentioned 8 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
or
R¹⁰ and R¹¹, together with the sulphur atom to which they are attached, form a 3- to 6-membered unsaturated, partially saturated or saturated ring, which comprises in each case, in addition to the carbon atoms and in addition to the sulphur atom, p ring members from the group consisting of N(R¹²)ₘ, O and S(O)ₙ, and wherein said ring is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy or (C₁-C₄)-alkylsulphonyl,
- R¹²: is hydrogen, (C₁-C₆)-alkyl or (C₁-C₆)-alkylcarbonyl,
- R¹³, R¹⁴: are each independently hydrogen, (C₁-C₆)-alkyl, (C₁-C₆)-alkylcarbonyl, (C₁-C₄)-alkylsulphonyl, phenyl, phenylcarbonyl, wherein each of the last-mentioned two residues is unsubstituted or is substituted by one or more residues from the group consisting of halogen, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl,
or
R¹³ and R¹⁴, together with the nitrogen atom to which they are attached, form a 3- to 8-membered unsaturated, partially saturated or saturated ring, which comprises in each case, in addition to the carbon atoms and in addition to the nitrogen atom, p ring members from the group consisting of N(R¹²)ₘ, O and S(O)ₙ, and wherein said ring is unsubstituted or is substituted by one or more residues from the group consisting of halogen, hydroxyl, (C₁-C₄)-alkyl, (C₁-C₄)-haloalkyl, (C₁-C₄)-alkoxy, (C₁-C₄)-haloalkoxy, and has q oxo groups,
- n: is independently selected from 0 or 1, preferably n is 0,
- m: is independently selected from 0 or 1,
- p: is independently selected from 0, 1 or 2,
- q: is independently selected from 0 or 1,
- y: is 0 or 1.

According to the present invention, compounds of the formula (G) and/or a salt thereof are particularly preferred, in which
- A: is CR⁶R⁷,
- W: is O,
- R¹: is (C₃-C₆)-cycloalkyl, (C₃-C₆)-cycloalkenyl, (C₃-C₆)-cycloalkyl-(C₁-C₃)-alkyl, (C₃-C₆)-cycloalkenyl-(C₁-C₃)-alkyl, phenyl, heteroaryl, heterocyclyl, wherein each of the last-mentioned 7 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
- R², R³: are each independently hydrogen, (C₁-C₄)-alkylthiocarbonyl, (C₁-C₄)-haloalkylthiocarbonyl, (C₁-C₆)-alkoxy-(C₁-C₃)-alkylcarbonyl, (C₁-C₄)-alkylthio-(C₁-C₃)-alkylcarbonyl, (C₁-C₄)-alkylsulphoxy-(C₁-C₃)-alkylcarbonyl, (C₁-C₄)-alkylsulphonyl-(C₁-C₃)-alkylcarbonyl, (C₁-C₆)-alkylcarbonyl, (C₁-C₆)-haloalkylcarbonyl, (C₂-C₆)-alkenylcarbonyl, (C₂-C₆)-alkynylcarbonyl, (C₁-C₆)-alkoxycarbonylcarbonyl, (C₁-C₆)-alkoxycarbonyl-(C₁-C₃)-alkylcarbonyl, (C₁-C₆)-alkoxycarbonyl, (C₁-C₆)-haloalkoxycarbonyl, (C₂-C₆)-alkenyloxycarbonyl, (C₃-C₆)-cycloalkylcarbonyl, (C₃-C₆)-cycloalkyl-(C₁-C₆)-alkylcarbonyl, phenylcarbonyl, phenyl-(C₁-C₆)-alkylcarbonyl, hetarylcarbonyl, hetaryl-(C₁-C₆)-alkylcarbonyl, heterocyclylcarbonyl, heterocyclyl-(C₁-C₆)-alkylcarbonyl, wherein each of the last-mentioned 8 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
or
NR²R³ is -N=CR⁸R⁹ or -N=S(O)ₙR¹⁰R¹¹,
- R⁶: is hydrogen,
- R⁷: is hydrogen or methyl,
- R⁸, R⁹: are each independently hydrogen, (C₁-C₆)-alkyl, (C₁-C₆)-haloalkyl, (C₂-C₆)-alkenyl, (C₂-C₆)-alkynyl, (C₁-C₆)-alkoxy-(C₁-C₃)-alkyl, (C₁-C₄)-alkylthio-(C₁-C₃)-alkyl, (C₁-C₄)-alkylsulphoxy-(C₁-C₃)-alkyl, (C₁-C₄)-alkylsulphonyl-(C₁-C₃)-alkyl, (C₁-C₆)-alkoxy, (C₁-C₆)-haloalkoxy, (C₂-C₆)-alkenyloxy, NR¹³R¹⁴, (C₃-C₈)-cycloalkyl, (C₃-C₈)-cycloalkyl-(C₁-C₆)-alkyl, phenyl, phenyl-(C₁-C₃)-alkyl, heteroaryl, heteroaryl-(C₁-C₃)-alkyl, heterocyclyl, heterocyclyl-(C₁-C₃)-alkyl, wherein each of the last-mentioned 8 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
or R⁸ and R⁹, together with the carbon atom to which they are attached, form a 3- to 6-membered unsaturated, partially saturated or saturated ring, which comprises in each case, in addition to the carbon atoms, p ring members from the group consisting of N(R¹²)ₘ, O and S(O)ₙ, and wherein said ring is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl,
- R¹⁰, R¹¹: are each independently (C₁-C₆)-alkyl, (C₂-C₆)-alkenyl, (C₂-C₆)-alkynyl, (C₃-C₈)-cycloalkyl, (C₃-C₈)-cycloalkyl-(C₁-C₆)-alkyl, phenyl, phenyl-(C₁-C₃)-alkyl, heteroaryl, heteroaryl-(C₁-C₃)-alkyl, heterocyclyl, heterocyclyl-(C₁-C₃)-alkyl, wherein each of the last-mentioned 8 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
or
R¹⁰ and R¹¹, together with the sulphur atom to which they are attached, form a 3- to 6-membered unsaturated, partially saturated or saturated ring, which comprises in each case, in addition to the carbon atoms and in addition to the sulphur atom, p ring members from the group consisting of N(R¹²)ₘ, O and S(O)ₙ, and wherein said ring is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy or (C₁-C₄)-alkylsulphonyl,
- R¹²: is hydrogen, (C₁-C₆)-alkyl or (C₁-C₆)-alkylcarbonyl,
- R¹³, R¹⁴: are each independently hydrogen, (C₁-C₆)-alkyl, (C₁-C₆)-alkylcarbonyl, (C₁-C₄)-alkylsulphonyl, phenyl, phenylcarbonyl, wherein each of the last-mentioned two residues is unsubstituted or is substituted by one or more residues from the group consisting of halogen, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl,
or
R¹³ and R¹⁴, together with the nitrogen atom to which they are attached, form a 3- to 8-membered unsaturated, partially saturated or saturated ring, which comprises in each case, in addition to the carbon atoms and in addition to the nitrogen atom, p ring members from the group consisting of N(R¹²)ₘ, O and S(O)ₙ, and wherein said ring is unsubstituted or is substituted by one or more residues from the group consisting of halogen, hydroxyl, (C₁-C₄)-alkyl, (C₁-C₄)-haloalkyl, (C₁-C₄)-alkoxy, (C₁-C₄)-haloalkoxy, and has q oxo groups,
- n: is 0,
- m: is independently selected from 0 or 1,
- p: is independently selected from 0, 1 or 2,
- q: is independently selected from 0 or 1,
- y: is 0 or 1.

According to the present invention, compounds of the formula (G) and/or a salt thereof are particularly preferred, in which
- A: is CR⁶R⁷,
- W: is O,
- R¹: is (C₃-C₆)-cycloalkyl, (C₃-C₆)-cycloalkenyl, (C₃-C₆)-cycloalkyl-(C₁-C₃)-alkyl, (C₃-C₆)-cycloalkenyl-(C₁-C₃)-alkyl, phenyl, heteroaryl, wherein each of the last-mentioned 6 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl,
- R², R³: are each independently hydrogen, (C₁-C₄)-alkylthiocarbonyl, (C₁-C₄)-haloalkylthiocarbonyl, (C₁-C₆)-alkoxy-(C₁-C₃)-alkylcarbonyl, (C₁-C₄)-alkylthio-(C₁-C₃)-alkylcarbonyl, (C₁-C₄)-alkylsulphoxy-(C₁-C₃)-alkylcarbonyl, (C₁-C₄)-alkylsulphonyl-(C₁-C₃)-alkylcarbonyl, (C₁-C₆)-alkylcarbonyl, (C₁-C₆)-haloalkylcarbonyl, (C₂-C₆)-alkenylcarbonyl, (C₂-C₆)-alkynylcarbonyl, (C₁-C₆)-alkoxycarbonylcarbonyl, (C₁-C₆)-alkoxycarbonyl-(C₁-C₃)-alkylcarbonyl, (C₁-C₆)-alkoxycarbonyl, (C₁-C₆)-haloalkoxycarbonyl, (C₂-C₆)-alkenyloxycarbonyl, (C₃-C₆)-cycloalkylcarbonyl, (C₃-C₆)-cycloalkyl-(C₁-C₆)-alkylcarbonyl, phenylcarbonyl, phenyl-(C₁-C₆)-alkylcarbonyl, hetarylcarbonyl, hetaryl-(C₁-C₆)-alkylcarbonyl, heterocyclylcarbonyl, heterocyclyl-(C₁-C₆)-alkylcarbonyl, wherein each of the last-mentioned 8 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
or
NR²R³ is -N=S(O)ₙR¹⁰R¹¹,
- R⁶: is hydrogen,
- R⁷: is hydrogen or methyl,
- R¹⁰, R¹¹: are each independently (C₁-C₆)-alkyl, (C₂-C₆)-alkenyl, (C₂-C₆)-alkynyl, (C₃-C₈)-cycloalkyl, (C₃-C₈)-cycloalkyl-(C₁-C₆)-alkyl, phenyl, phenyl-(C₁-C₃)-alkyl, heteroaryl, heteroaryl-(C₁-C₃)-alkyl, heterocyclyl, heterocyclyl-(C₁-C₃)-alkyl, wherein each of the last-mentioned 8 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
or
R¹⁰ and R¹¹, together with the sulphur atom to which they are attached, form a 3- to 6-membered unsaturated, partially saturated or saturated ring, which comprises in each case, in addition to the carbon atoms and in addition to the sulphur atom, p ring members from the group consisting of N(R¹²)ₘ, O and S(O)ₙ, and wherein said ring is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy or (C₁-C₄)-alkylsulphonyl,
- R¹²: is hydrogen, (C₁-C₆)-alkyl or (C₁-C₆)-alkylcarbonyl,
- R¹³, R¹⁴: are each independently hydrogen, (C₁-C₆)-alkyl, (C₁-C₆)-alkylcarbonyl, (C₁-C₄)-alkylsulphonyl, phenyl, phenylcarbonyl, wherein each of the last-mentioned two residues is unsubstituted or is substituted by one or more residues from the group consisting of halogen, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl,
or
R¹³ and R¹⁴, together with the nitrogen atom to which they are attached, form a 3- to 8-membered unsaturated, partially saturated or saturated ring, which comprises in each case, in addition to the carbon atoms and in addition to the nitrogen atom, p ring members from the group consisting of N(R¹²)ₘ, O and S(O)ₙ, and wherein said ring is unsubstituted or is substituted by one or more residues from the group consisting of halogen, hydroxyl, (C₁-C₄)-alkyl, (C₁-C₄)-haloalkyl, (C₁-C₄)-alkoxy, (C₁-C₄)-haloalkoxy, and has q oxo groups,
- n: is 0,
- m: is independently selected from 0 or 1,
- p: is independently selected from 0 or 1,
- q: is independently selected from 0 or 1,
- y: is 0 or 1.

More preferred compounds according to the present invention correspond to the formula (G), or to the formulae (II) and (III), as defined hereinafter, wherein
- n: is independently selected from 0 or 1, preferably n is 0,
- m: is independently selected from 0 or 1, preferably m is 0,
- p: is independently selected from 0 or 1, and
- q: is independently selected from 0 or 1, preferably q is 0.

Paticularly preferred compounds according to the present invention correspond to the formula (G), or to the formulae (II) and (III), as defined hereinafter, wherein
- n: is 0,
- m: is 0,
- p: is independently selected from 0 or 1, and
- q: is 0.

Many preferred compounds according to the present invention correspond to the formula (G), wherein W = O, y = 1, A = CH₂, and at least one of R² and R³ = H.

The following compounds of the formulae (I), (II) and (III) are preferred compounds of the formula (G) according to the present invention.

Preferred compounds according to the present invention correspond to the formula (G), wherein W = O, R² = H, R³ = H, y = 1, and A = CH₂.

These preferred compounds of the formula (G) are compounds of the formula (I): 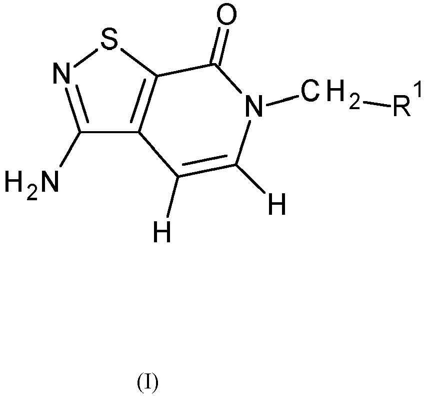 wherein R¹ has the meaning as defined in the context of the formula (G), preferably has the meaning as defined in one of the preferred, more preferred, or particularly preferred embodiments.

Preferred compounds according to the present invention correspond to the formula (G), wherein W = O, y = 1, and A = CH₂.

These preferred compounds of formula (G) are compounds of the formula (II): 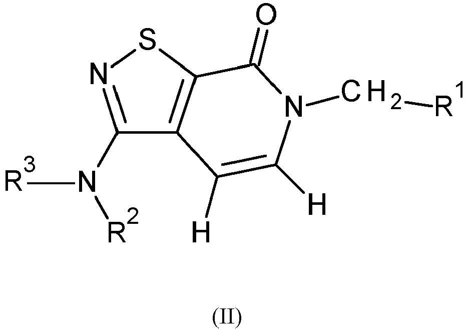 wherein R¹, R² and R³ each have, independently from one another, the meaning as defined in the context of the formula (G), preferably each have, independently from one another, the meaning as defined in one of the preferred, more preferred, or particularly preferred embodiments.

Preferred compounds according to the present invention correspond to the formula (G), wherein W = O.

These preferred compounds of formula (G) are compounds of the formula (III): 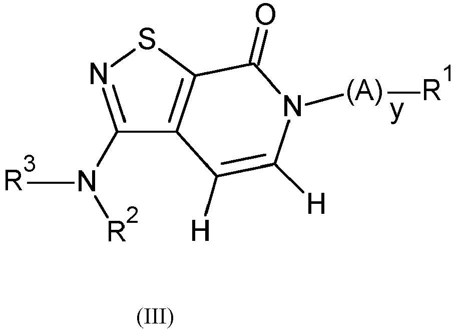 wherein R¹, R², R³, A and y each have, independently from one another, the meaning as defined in the context of the formula (G), preferably each have, independently from one another, the meaning as defined in one of the preferred, more preferred, or particularly preferred embodiments.

Preferred compounds according to the present invention correspond to the formula (G), wherein W = S.

In the following Tables 1 to 3 specific and preferred definitions of R¹, R², R³ and A, respectively, are mentioned.

The abbreviations and numerations of the substituent positions used in the context of the present invention and in Tables 1 to 3 are explained in detail in the section Examples hereinafter.

R¹ in the context of the formula (G) and the formulae (I), (II) and (III), respectively, particularly preferably is selected from the group consisting of the moieties mentioned in Table 1, Table 2 and Table 3 for R¹.

R² and R³ the context of the formula (G) and the formulae (II) and (III), respectively, particularly preferably are selected from the group consisting of the moieties mentioned in Table 2 and Table 3 for NR²R³.

A in the context of the formula (G) particularly preferably is selected from the group consisting of CH₂, CHMe, (*S*)-CHMe, (*R*)-CHMe, i.e. the moieties mentioned in Table 3 for A, and y is 0 or 1.

Specific preferred compounds of the formula (I) are shown in Table 1.

**Table 1: Preferred compounds of the formula (I):**

| **No**. | **R¹** |
|---|---|
| I-1 | cPr |
| I-2 | cBu |
| I-3 | cPentyl |
| I-4 | cHexyl |
| I-5 | cHeptyl |
| I-6 | cOctyl |
| I-7 | 4-CF₃-cHexyl |
| I-8 | Tetrahydro-2H-pyran-2-yl |
| I-9 | Tetrahydro-2H-pyran-3-yl |
| I-10 | Tetrahydro-2H-pyran-4-yl |
| I-11 | Tetrahydrofuran-2-yl |
| I-12 | 2-F-Phenyl |
| I-13 | 3-F-Phenyl |
| I-14 | 4-F-Phenyl |
| I-15 | 2-Cl-Phenyl |
| I-16 | 3-Cl-Phenyl |
| I-17 | 4-Cl-Phenyl |
| I-18 | 2-CHF₂-Phenyl |
| I-19 | 3-CHF₂-Phenyl |
| I-20 | 4-CHF₂-Phenyl |
| I-21 | 2,3-diF-Phenyl |
| I-22 | 2,4-diF-Phenyl |
| I-23 | 2,5-diF-Phenyl |
| I-24 | 2,6-diF-Phenyl |
| I-25 | 3,4-diF-Phenyl |
| I-26 | 3,5-diF-Phenyl |
| I-27 | 2-F-4-Cl-Phenyl |
| I-28 | 2-Me-3,4-diF-Phenyl |
| I-29 | 4-Me-2,3-diF-Phenyl |
| I-30 | 2-F-4-Me-Phenyl |
| I-31 | 5-F-2-Me-Phenyl |
| I-32 | 2,4,6-triF-Phenyl |
| I-33 | 2,3,5-triF-Phenyl |
| I-34 | 2,4,5-triF-Phenyl |
| I-35 | 2,3,6-triF-Phenyl |
| I-36 | 3,4,5-triF-Phenyl |
| I-37 | 2,3,4-triF-Phenyl |
| I-38 | 2-Cl-Pyridin-3-yl |
| I-39 | 6-Cl-Pyridin-3-yl |
| I-40 | 5-F-Pyridin-3-yl |
| I-41 | 2-F-Pyridin-4-yl |
| I-42 | 1 -Ethyl-3-methyl- 1 H-pyrazol-4-yl |
| I-43 | (cis) 2-Cl-cHexyl |
| I-44 | (trans) 2-Cl-cHexyl |
| I-45 | (R,R) 2-Cl-cHexyl |
| I-46 | (S,S) 2-Cl-cHexyl |
| I-47 | (S,R) 2-Cl-cHexyl |
| I-48 | (R,S) 2-Cl-cHexyl |
| I-49 | (cis) 2-Methyl-cHexyl |
| I-50 | (trans) 2-Methyl-cHexyl |
| I-51 | (R,R) 2-Methyl-cHexyl |
| I-52 | (S,S) 2-Methyl-cHexyl |
| I-53 | (S,R) 2-Methyl-cHexyl |
| I-54 | (R,S) 2-Methyl-cHexyl |
| I-55 | 1 -Methyl-cHexyl |
| I-56 | 1-F-cHexyl |
| I-57 | 1-Cl-cHexyl |
| I-58 | 1 -Br-cHexyl |
| I-59 | 1-OH-cHexyl |
| I-60 | (cis) 2-Br-cHexyl |
| I-61 | (trans) 2-Br-cHexyl |
| I-62 | (R,R) 2-Br-cHexyl |
| I-63 | (S,S) 2-Br-cHexyl |
| I-64 | (S,R) 2-Br-cHexyl |
| I-65 | (R,S) 2-Br-cHexyl |
| I-66 | (cis) 2-F-cHexyl |
| I-67 | (trans) 2-F-cHexyl |
| I-68 | (R,R) 2-F-cHexyl |
| I-69 | (S,S) 2-F-cHexyl |
| I-70 | (S,R) 2-F-cHexyl |
| I-71 | (R,S) 2-F-cHexyl |
| I-72 | (cis) 3-Cl-cHexyl |
| I-73 | (trans) 3-Cl-cHexyl |
| I-74 | (R,R) 3-Cl-cHexyl |
| I-75 | (S,S) 3-Cl-cHexyl |
| I-76 | (S,R) 3-Cl-cHexyl |
| I-77 | (R,S) 3-Cl-cHexyl |
| I-78 | (cis) 4-Cl-cHexyl |
| I-79 | (trans) 4-Cl-cHexyl |
| I-80 | (cis) 4-F-cHexyl |
| I-81 | (trans) 4-F-cHexyl |
| I-82 | (cis) 4-Methyl-cHexyl |
| I-83 | (trans) 4-Methyl-cHexyl |
| I-84 | 2,2-F,F-cHexyl |
| I-85 | 3,3-F,F-cHexyl |
| I-86 | 4,4-F,F-cHexyl |
| I-87 | 2-F-cHex-1-ene |
| I-88 | 2-Cl-cHex-1-ene |
| I-89 | 2-Br-cHex-1-ene |
| I-90 | 2-Methyl-cHex-1-ene |
| I-91 | cHex-1-ene |

Specific preferred compounds of the formula (II) are shown in Table 2.

**Table 2: Preferred compounds of the formula (II):**

| **No.** | **R¹** | **NR²R³** |
|---|---|---|
| II-1 | cPentyl | NHCOCH₃ |
| II-2 | cHexyl | NHCOCH₃ |
| II-3 | cHeptyl | NHCOCH₃ |
| II-4 | 4-F-Phenyl | NHCOCH₃ |
| II-5 | 4-Cl-Phenyl | NHCOCH₃ |
| II-6 | 2,4-diF-Phenyl | NHCOCH₃ |
| II-7 | 2,4,6-triF-Phenyl | NHCOCH₃ |
| II-8 | 2,4,5-triF-Phenyl | NHCOCH₃ |
| II-9 | (cis) 2-Cl-cHexyl | NHCOCH₃ |
| II-10 | (trans) 2-Cl-cHexyl | NHCOCH₃ |
| II-11 | (R,R) 2-Cl-cHexyl | NHCOCH₃ |
| II-12 | (S,S) 2-Cl-cHexyl | NHCOCH₃ |
| II-13 | (S,R) 2-Cl-cHexyl | NHCOCH₃ |
| II-14 | (R,S) 2-Cl-cHexyl | NHCOCH₃ |
| II-22 | (cis) 2-Methyl-cHexyl | NHCOCH₃ |
| II-23 | (trans) 2-Methyl-cHexyl | NHCOCH₃ |
| II-24 | 1-F-cHexyl | NHCOCH₃ |
| II-25 | cPentyl | NHCOCH₂CH₃ |
| II-26 | cHexyl | NHCOCH₂CH₃ |
| II-27 | cHeptyl | NHCOCH₂CH₃ |
| II-28 | 4-F-Phenyl | NHCOCH₂CH₃ |
| II-29 | 4-Cl-Phenyl | NHCOCH₂CH₃ |
| II-30 | 2,4-diF-Phenyl | NHCOCH₂CH₃ |
| II-31 | 2,4,6-triF-Phenyl | NHCOCH₂CH₃ |
| II-32 | 2,4,5-triF-Phenyl | NHCOCH₂CH₃ |
| II-33 | (cis) 2-Cl-cHexyl | NHCOCH₂CH₃ |
| II-34 | (trans) 2-Cl-cHexyl | NHCOCH₂CH₃ |
| II-35 | (R,R) 2-Cl-cHexyl | NHCOCH₂CH₃ |
| II-36 | (S,S) 2-Cl-cHexyl | NHCOCH₂CH₃ |
| II-37 | (S,R) 2-Cl-cHexyl | NHCOCH₂CH₃ |
| II-38 | (R,S) 2-Cl-cHexyl | NHCOCH₂CH₃ |
| II-39 | (cis) 2-Methyl-cHexyl | NHCOCH₂CH₃ |
| II-40 | (trans) 2-Methyl-cHexyl | NHCOCH₂CH₃ |
| II-41 | 1-F-cHexyl | NHCOCH₂CH₃ |
| II-42 | cPentyl | NHCOnPr |
| II-43 | cHexyl | NHCOnPr |
| II-44 | cHeptyl | NHCOnPr |
| II-45 | 4-F-Phenyl | NHCOnPr |
| II-46 | 4-Cl-Phenyl | NHCOnPr |
| II-47 | 2,4-diF-Phenyl | NHCOnPr |
| II-48 | 2,4,6-triF-Phenyl | NHCOnPr |
| II-49 | 2,4,5-triF-Phenyl | NHCOnPr |
| II-50 | (cis) 2-Cl-cHexyl | NHCOnPr |
| II-51 | (trans) 2-Cl-cHexyl | NHCOnPr |
| II-52 | (R,R) 2-Cl-cHexyl | NHCOnPr |
| II-53 | (S,S) 2-Cl-cHexyl | NHCOnPr |
| II-54 | (S,R) 2-Cl-cHexyl | NHCOnPr |
| II-55 | (R,S) 2-Cl-cHexyl | NHCOnPr |
| II-56 | (cis) 2-Methyl-cHexyl | NHCOnPr |
| II-57 | (trans) 2-Methyl-cHexyl | NHCOnPr |
| II-58 | 1-F-cHexyl | NHCOnPr |
| II-59 | cPentyl | NHCOCH₂Cl |
| II-60 | cHexyl | NHCOCH₂Cl |
| II-61 | cHeptyl | NHCOCH₂Cl |
| II-62 | 4-F-Phenyl | NHCOCH₂Cl |
| II-63 | 4-Cl-Phenyl | NHCOCH₂Cl |
| II-64 | 2,4-diF-Phenyl | NHCOCH₂Cl |
| II-65 | 2,4,6-triF-Phenyl | NHCOCH₂Cl |
| II-66 | 2,4,5-triF-Phenyl | NHCOCH₂Cl |
| II-67 | (cis) 2-Cl-cHexyl | NHCOCH₂Cl |
| II-68 | (trans) 2-Cl-cHexyl | NHCOCH₂Cl |
| II-69 | (R,R) 2-Cl-cHexyl | NHCOCH₂Cl |
| II-70 | (S,S) 2-Cl-cHexyl | NHCOCH₂Cl |
| II-71 | (S,R) 2-Cl-cHexyl | NHCOCH₂Cl |
| II-72 | (R,S) 2-Cl-cHexyl | NHCOCH₂Cl |
| II-73 | (cis) 2-Methyl-cHexyl | NHCOCH₂Cl |
| II-74 | (trans) 2-Methyl-cHexyl | NHCOCH₂Cl |
| II-75 | 1-F-cHexyl | NHCOCH₂Cl |
| II-76 | cPentyl | NHCOCH₂F |
| II-77 | cHexyl | NHCOCH₂F |
| II-78 | cHeptyl | NHCOCH₂F |
| II-79 | 4-F-Phenyl | NHCOCH₂F |
| II-80 | 4-Cl-Phenyl | NHCOCH₂F |
| II-81 | 2,4-diF-Phenyl | NHCOCH₂F |
| II-82 | 2,4,6-triF-Phenyl | NHCOCH₂F |
| II-83 | 2,4,5-triF-Phenyl | NHCOCH₂F |
| II-84 | (cis) 2-Cl-cHexyl | NHCOCH₂F |
| II-85 | (trans) 2-Cl-cHexyl | NHCOCH₂F |
| II-86 | (R,R) 2-Cl-cHexyl | NHCOCH₂F |
| II-87 | (S,S) 2-Cl-cHexyl | NHCOCH₂F |
| II-88 | (S,R) 2-Cl-cHexyl | NHCOCH₂F |
| II-89 | (R,S) 2-Cl-cHexyl | NHCOCH₂F |
| II-90 | (cis) 2-Methyl-cHexyl | NHCOCH₂F |
| II-91 | (trans) 2-Methyl-cHexyl | NHCOCH₂F |
| II-92 | 1-F-cHexyl | NHCOCH₂F |
| II-93 | cPentyl | NHCOCH₂Br |
| II-94 | cHexyl | NHCOCH₂Br |
| II-95 | cHeptyl | NHCOCH₂Br |
| II-96 | 4-F-Phenyl | NHCOCH₂Br |
| II-97 | 4-Cl-Phenyl | NHCOCH₂Br |
| II-98 | 2,4-diF-Phenyl | NHCOCH₂Br |
| II-99 | 2,4,6-triF-Phenyl | NHCOCH₂Br |
| II-100 | 2,4,5-triF-Phenyl | NHCOCH₂Br |
| II-101 | (cis) 2-Cl-cHexyl | NHCOCH₂Br |
| II-102 | (trans) 2-Cl-cHexyl | NHCOCH₂Br |
| II-103 | (R,R) 2-Cl-cHexyl | NHCOCH₂Br |
| II-104 | (S,S) 2-Cl-cHexyl | NHCOCH₂Br |
| II-105 | (S,R) 2-Cl-cHexyl | NHCOCH₂Br |
| II-106 | (R,S) 2-Cl-cHexyl | NHCOCH₂Br |
| II-107 | (cis) 2-Methyl-cHexyl | NHCOCH₂Br |
| II-108 | (trans) 2-Methyl-cHexyl | NHCOCH₂Br |
| II-109 | 1-F-cHexyl | NHCOCH₂Br |
| II-110 | cPentyl | NHCOCH₂CF₃ |
| II-111 | cHexyl | NHCOCH₂CF₃ |
| II-112 | cHeptyl | NHCOCH₂CF₃ |
| II-113 | 4-F-Phenyl | NHCOCH₂CF₃ |
| II-114 | 4-Cl-Phenyl | NHCOCH₂CF₃ |
| II-115 | 2,4-diF-Phenyl | NHCOCH₂CF₃ |
| II-116 | 2,4,6-triF-Phenyl | NHCOCH₂CF₃ |
| II-117 | 2,4,5-triF-Phenyl | NHCOCH₂CF₃ |
| II-118 | (cis) 2-Cl-cHexyl | NHCOCH₂CF₃ |
| II-119 | (trans) 2-Cl-cHexyl | NHCOCH₂CF₃ |
| II-120 | (R,R) 2-Cl-cHexyl | NHCOCH₂CF₃ |
| II-121 | (S,S) 2-Cl-cHexyl | NHCOCH₂CF₃ |
| II-122 | (S,R) 2-Cl-cHexyl | NHCOCH₂CF₃ |
| II-123 | (R,S) 2-Cl-cHexyl | NHCOCH₂CF₃ |
| II-124 | (cis) 2-Methyl-cHexyl | NHCOCH₂CF₃ |
| II-125 | (trans) 2-Methyl-cHexyl | NHCOCH₂CF₃ |
| II-126 | 1-F-cHexyl | NHCOCH₂CF₃ |
| II-127 | cPentyl | NHCOCHF₂ |
| II-128 | cHexyl | NHCOCHF₂ |
| II-129 | cHeptyl | NHCOCHF₂ |
| II-130 | 4-F-Phenyl | NHCOCHF₂ |
| II-131 | 4-Cl-Phenyl | NHCOCHF₂ |
| II-132 | 2,4-diF-Phenyl | NHCOCHF₂ |
| II-133 | 2,4,6-triF-Phenyl | NHCOCHF₂ |
| II-134 | 2,4,5-triF-Phenyl | NHCOCHF₂ |
| II-135 | (cis) 2-Cl-cHexyl | NHCOCHF₂ |
| II-136 | (trans) 2-Cl-cHexyl | NHCOCHF₂ |
| II-137 | (R,R) 2-Cl-cHexyl | NHCOCHF₂ |
| II-138 | (S,S) 2-Cl-cHexyl | NHCOCHF₂ |
| II-139 | (S,R) 2-Cl-cHexyl | NHCOCHF₂ |
| II-140 | (R,S) 2-Cl-cHexyl | NHCOCHF₂ |
| II-141 | (cis) 2-Methyl-cHexyl | NHCOCHF₂ |
| II-142 | (trans) 2-Methyl-cHexyl | NHCOCHF₂ |
| II-143 | 1-F-cHexyl | NHCOCHF₂ |
| II-144 | cPentyl | NHCOCF₃ |
| II-145 | cHexyl | NHCOCF₃ |
| II-146 | cHeptyl | NHCOCF₃ |
| II-147 | 4-F-Phenyl | NHCOCF₃ |
| II-148 | 4-Cl-Phenyl | NHCOCF₃ |
| II-149 | 2,4-diF-Phenyl | NHCOCF₃ |
| II-150 | 2,4,6-triF-Phenyl | NHCOCF₃ |
| II-151 | 2,4,5-triF-Phenyl | NHCOCF₃ |
| II-152 | (cis) 2-Cl-cHexyl | NHCOCF₃ |
| II-153 | (trans) 2-Cl-cHexyl | NHCOCF₃ |
| II-154 | (R,R) 2-Cl-cHexyl | NHCOCF₃ |
| II-155 | (S,S) 2-Cl-cHexyl | NHCOCF₃ |
| II-156 | (S,R) 2-Cl-cHexyl | NHCOCF₃ |
| II-157 | (R,S) 2-Cl-cHexyl | NHCOCF₃ |
| II-158 | (cis) 2-Methyl-cHexyl | NHCOCF₃ |
| II-159 | (trans) 2-Methyl-cHexyl | NHCOCF₃ |
| II-160 | 1-F-cHexyl | NHCOCF₃ |
| II-161 | cPentyl | NHCOCF₂CF₃ |
| II-162 | cHexyl | NHCOCF₂CF₃ |
| II-163 | cHeptyl | NHCOCF₂CF₃ |
| II-164 | 4-F-Phenyl | NHCOCF₂CF₃ |
| II-165 | 4-Cl-Phenyl | NHCOCF₂CF₃ |
| II-166 | 2,4-diF-Phenyl | NHCOCF₂CF₃ |
| II-167 | 2,4,6-triF-Phenyl | NHCOCF₂CF₃ |
| II-168 | 2,4,5-triF-Phenyl | NHCOCF₂CF₃ |
| II-169 | (cis) 2-Cl-cHexyl | NHCOCF₂CF₃ |
| II-170 | (trans) 2-Cl-cHexyl | NHCOCF₂CF₃ |
| II-171 | (R,R) 2-Cl-cHexyl | NHCOCF₂CF₃ |
| II-172 | (S,S) 2-Cl-cHexyl | NHCOCF₂CF₃ |
| II-173 | (S,R) 2-Cl-cHexyl | NHCOCF₂CF₃ |
| II-174 | (R,S) 2-Cl-cHexyl | NHCOCF₂CF₃ |
| II-175 | (cis) 2-Methyl-cHexyl | NHCOCF₂CF₃ |
| II-176 | (trans) 2-Methyl-cHexyl | NHCOCF₂CF₃ |
| II-177 | 1-F-cHexyl | NHCOCF₂CF₃ |
| II-178 | cPentyl | NHCOCF₂CF₂CF₃ |
| II-179 | cHexyl | NHCOCF₂CF₂CF₃ |
| II-180 | cHeptyl | NHCOCF₂CF₂CF₃ |
| II-181 | 4-F-Phenyl | NHCOCF₂CF₂CF₃ |
| II-182 | 4-Cl-Phenyl | NHCOCF₂CF₂CF₃ |
| II-183 | 2,4-diF-Phenyl | NHCOCF₂CF₂CF₃ |
| II-184 | 2,4,6-triF-Phenyl | NHCOCF₂CF₂CF₃ |
| II-185 | 2,4,5-triF-Phenyl | NHCOCF₂CF₂CF₃ |
| II-186 | (cis) 2-Cl-cHexyl | NHCOCF₂CF₂CF₃ |
| II-187 | (trans) 2-Cl-cHexyl | NHCOCF₂CF₂CF₃ |
| II-188 | (R,R) 2-Cl-cHexyl | NHCOCF₂CF₂CF₃ |
| II-189 | (S,S) 2-Cl-cHexyl | NHCOCF₂CF₂CF₃ |
| II-190 | (S,R) 2-Cl-cHexyl | NHCOCF₂CF₂CF₃ |
| II-191 | (R,S) 2-Cl-cHexyl | NHCOCF₂CF₂CF₃ |
| II-192 | (cis) 2-Methyl-cHexyl | NHCOCF₂CF₂CF₃ |
| II-193 | (trans) 2-Methyl-cHexyl | NHCOCF₂CF₂CF₃ |
| II-194 | 1-F-cHexyl | NHCOCF₂CF₂CF₃ |
| II-195 | cPentyl | NHCOCH₂OMe |
| II-196 | cHexyl | NHCOCH₂OMe |
| II-197 | cHeptyl | NHCOCH₂OMe |
| II-198 | 4-F-Phenyl | NHCOCH₂OMe |
| II-199 | 4-Cl-Phenyl | NHCOCH₂OMe |
| II-200 | 2,4-diF-Phenyl | NHCOCH₂OMe |
| II-201 | 2,4,6-triF-Phenyl | NHCOCH₂OMe |
| II-202 | 2,4,5-triF-Phenyl | NHCOCH₂OMe |
| II-203 | (cis) 2-Cl-cHexyl | NHCOCH₂OMe |
| II-204 | (trans) 2-Cl-cHexyl | NHCOCH₂OMe |
| II-205 | (R,R) 2-Cl-cHexyl | NHCOCH₂OMe |
| II-206 | (S,S) 2-Cl-cHexyl | NHCOCH₂OMe |
| II-207 | (S,R) 2-Cl-cHexyl | NHCOCH₂OMe |
| II-208 | (R,S) 2-Cl-cHexyl | NHCOCH₂OMe |
| II-209 | (cis) 2-Methyl-cHexyl | NHCOCH₂OMe |
| II-210 | (trans) 2-Methyl-cHexyl | NHCOCH₂OMe |
| II-211 | 1-F-cHexyl | NHCOCH₂OMe |
| II-212 | cPentyl | NHCOCH₂SMe |
| II-213 | cHexyl | NHCOCH₂SMe |
| II-214 | cHeptyl | NHCOCH₂SMe |
| II-215 | 4-F-Phenyl | NHCOCH₂SMe |
| II-216 | 4-Cl-Phenyl | NHCOCH₂SMe |
| II-217 | 2,4-diF-Phenyl | NHCOCH₂SMe |
| II-218 | 2,4,6-triF-Phenyl | NHCOCH₂SMe |
| II-219 | 2,4,5-triF-Phenyl | NHCOCH₂SMe |
| II-220 | (cis) 2-Cl-cHexyl | NHCOCH₂SMe |
| II-221 | (trans) 2-Cl-cHexyl | NHCOCH₂SMe |
| II-222 | (R,R) 2-Cl-cHexyl | NHCOCH₂SMe |
| II-223 | (S,S) 2-Cl-cHexyl | NHCOCH₂SMe |
| II-224 | (S,R) 2-Cl-cHexyl | NHCOCH₂SMe |
| II-225 | (R,S) 2-Cl-cHexyl | NHCOCH₂SMe |
| II-226 | (cis) 2-Methyl-cHexyl | NHCOCH₂SMe |
| II-227 | (trans) 2-Methyl-cHexyl | NHCOCH₂SMe |
| II-228 | 1-F-cHexyl | NHCOCH₂SMe |
| II-229 | cPentyl | NHCOCO₂Me |
| II-230 | cHexyl | NHCOCO₂Me |
| II-231 | cHeptyl | NHCOCO₂Me |
| II-232 | 4-F-Phenyl | NHCOCO₂Me |
| II-233 | 4-Cl-Phenyl | NHCOCO₂Me |
| II-234 | 2,4-diF-Phenyl | NHCOCO₂Me |
| II-235 | 2,4,6-triF-Phenyl | NHCOCO₂Me |
| II-236 | 2,4,5-triF-Phenyl | NHCOCO₂Me |
| II-237 | (cis) 2-Cl-cHexyl | NHCOCO₂Me |
| II-238 | (trans) 2-Cl-cHexyl | NHCOCO₂Me |
| II-239 | (R,R) 2-Cl-cHexyl | NHCOCO₂Me |
| II-240 | (S,S) 2-Cl-cHexyl | NHCOCO₂Me |
| II-241 | (S,R) 2-Cl-cHexyl | NHCOCO₂Me |
| II-242 | (R,S) 2-Cl-cHexyl | NHCOCO₂Me |
| II-243 | (cis) 2-Methyl-cHexyl | NHCOCO₂Me |
| II-244 | (trans) 2-Methyl-cHexyl | NHCOCO₂Me |
| II-245 | 1-F-cHexyl | NHCOCO₂Me |
| II-246 | cPentyl | NHCOCH₂CO₂Me |
| II-247 | cHexyl | NHCOCH₂CO₂Me |
| II-248 | cHeptyl | NHCOCH₂CO₂Me |
| II-249 | 4-F-Phenyl | NHCOCH₂CO₂Me |
| II-250 | 4-Cl-Phenyl | NHCOCH₂CO₂Me |
| II-251 | 2,4-diF-Phenyl | NHCOCH₂CO₂Me |
| II-252 | 2,4,6-triF-Phenyl | NHCOCH₂CO₂Me |
| II-253 | 2,4,5-triF-Phenyl | NHCOCH₂CO₂Me |
| II-254 | (cis) 2-Cl-cHexyl | NHCOCH₂CO₂Me |
| II-255 | (trans) 2-Cl-cHexyl | NHCOCH₂CO₂Me |
| II-256 | (R,R) 2-Cl-cHexyl | NHCOCH₂CO₂Me |
| II-257 | (S,S) 2-Cl-cHexyl | NHCOCH₂CO₂Me |
| II-258 | (S,R) 2-Cl-cHexyl | NHCOCH₂CO₂Me |
| II-259 | (R,S) 2-Cl-cHexyl | NHCOCH₂CO₂Me |
| II-260 | (cis) 2-Methyl-cHexyl | NHCOCH₂CO₂Me |
| II-261 | (trans) 2-Methyl-cHexyl | NHCOCH₂CO₂Me |
| II-262 | 1-F-cHexyl | NHCOCH₂CO₂Me |
| II-263 | cPentyl | NHCOC₂H₄CO₂Me |
| II-264 | cHexyl | NHCOC₂H₄CO₂Me |
| II-265 | cHeptyl | NHCOC₂H₄CO₂Me |
| II-266 | 4-F-Phenyl | NHCOC₂H₄CO₂Me |
| II-267 | 4-Cl-Phenyl | NHCOC₂H₄CO₂Me |
| II-268 | 2,4-diF-Phenyl | NHCOC₂H₄CO₂Me |
| II-269 | 2,4,6-triF-Phenyl | NHCOC₂H₄CO₂Me |
| II-270 | 2,4,5-triF-Phenyl | NHCOC₂H₄CO₂Me |
| II-271 | (cis) 2-Cl-cHexyl | NHCOC₂H₄CO₂Me |
| II-272 | (trans) 2-Cl-cHexyl | NHCOC₂H₄CO₂Me |
| II-273 | (R,R) 2-Cl-cHexyl | NHCOC₂H₄CO₂Me |
| II-274 | (S,S) 2-Cl-cHexyl | NHCOC₂H₄CO₂Me |
| II-275 | (S,R) 2-Cl-cHexyl | NHCOC₂H₄CO₂Me |
| II-276 | (R,S) 2-Cl-cHexyl | NHCOC₂H₄CO₂Me |
| II-277 | (cis) 2-Methyl-cHexyl | NHCOC₂H₄CO₂Me |
| II-278 | (trans) 2-Methyl-cHexyl | NHCOC₂H₄CO₂Me |
| II-279 | 1-F-cHexyl | NHCOC₂H₄CO₂Me |
| II-280 | cPentyl | NHCOC₃H₆CO₂Me |
| II-281 | cHexyl | NHCOC₃H₆CO₂Me |
| II-282 | cHeptyl | NHCOC₃H₆CO₂Me |
| II-283 | 4-F-Phenyl | NHCOC₃H₆CO₂Me |
| II-284 | 4-Cl-Phenyl | NHCOC₃H₆CO₂Me |
| II-285 | 2,4-diF-Phenyl | NHCOC₃H₆CO₂Me |
| II-286 | 2,4,6-triF-Phenyl | NHCOC₃H₆CO₂Me |
| II-287 | 2,4,5-triF-Phenyl | NHCOC₃H₆CO₂Me |
| II-288 | (cis) 2-Cl-cHexyl | NHCOC₃H₆CO₂Me |
| II-289 | (trans) 2-Cl-cHexyl | NHCOC₃H₆CO₂Me |
| II-290 | (R,R) 2-Cl-cHexyl | NHCOC₃H₆CO₂Me |
| II-291 | (S,S) 2-Cl-cHexyl | NHCOC₃H₆CO₂Me |
| II-292 | (S,R) 2-Cl-cHexyl | NHCOC₃H₆CO₂Me |
| II-293 | (R,S) 2-Cl-cHexyl | NHCOC₃H₆CO₂Me |
| II-294 | (cis) 2-Methyl-cHexyl | NHCOC₃H₆CO₂Me |
| II-295 | (trans) 2-Methyl-cHexyl | NHCOC₃H₆CO₂Me |
| II-296 | 1-F-cHexyl | NHCOC₃H₆CO₂Me |
| II-297 | cPentyl | NHCOCF₂CClF₂ |
| II-298 | cHexyl | NHCOCF₂CClF₂ |
| II-299 | cHeptyl | NHCOCF₂CClF₂ |
| II-300 | 4-F-Phenyl | NHCOCF₂CClF₂ |
| II-301 | 4-Cl-Phenyl | NHCOCF₂CClF₂ |
| II-302 | 2,4-diF-Phenyl | NHCOCF₂CClF₂ |
| II-303 | 2,4,6-triF-Phenyl | NHCOCF₂CClF₂ |
| II-304 | 2,4,5-triF-Phenyl | NHCOCF₂CClF₂ |
| II-305 | (cis) 2-Cl-cHexyl | NHCOCF₂CClF₂ |
| II-306 | (trans) 2-Cl-cHexyl | NHCOCF₂CClF₂ |
| II-307 | (R,R) 2-Cl-cHexyl | NHCOCF₂CClF₂ |
| II-308 | (S,S) 2-Cl-cHexyl | NHCOCF₂CClF₂ |
| II-309 | (S,R) 2-Cl-cHexyl | NHCOCF₂CClF₂ |
| II-310 | (R,S) 2-Cl-cHexyl | NHCOCF₂CClF₂ |
| II-311 | (cis) 2-Methyl-cHexyl | NHCOCF₂CClF₂ |
| II-312 | (trans) 2-Methyl-cHexyl | NHCOCF₂CClF₂ |
| II-313 | 1-F-cHexyl | NHCOCF₂CClF₂ |
| II-314 | cPentyl | NHCOCClFCF₃ |
| II-315 | cHexyl | NHCOCClFCF₃ |
| II-316 | cHeptyl | NHCOCClFCF₃ |
| II-317 | 4-F-Phenyl | NHCOCClFCF₃ |
| II-318 | 4-Cl-Phenyl | NHCOCClFCF₃ |
| II-319 | 2,4-diF-Phenyl | NHCOCClFCF₃ |
| II-320 | 2,4,6-triF-Phenyl | NHCOCClFCF₃ |
| II-321 | 2,4,5-triF-Phenyl | NHCOCClFCF₃ |
| II-322 | (cis) 2-Cl-cHexyl | NHCOCClFCF₃ |
| II-323 | (trans) 2-Cl-cHexyl | NHCOCClFCF₃ |
| II-324 | (R,R) 2-Cl-cHexyl | NHCOCClFCF₃ |
| II-325 | (S,S) 2-Cl-cHexyl | NHCOCClFCF₃ |
| II-326 | (S,R) 2-Cl-cHexyl | NHCOCClFCF₃ |
| II-327 | (R,S) 2-Cl-cHexyl | NHCOCClFCF₃ |
| II-328 | (cis) 2-Methyl-cHexyl | NHCOCClFCF₃ |
| II-329 | (trans) 2-Methyl-cHexyl | NHCOCClFCF₃ |
| II-330 | 1-F-cHexyl | NHCOCClFCF₃ |
| II-331 | cPentyl | N(COCH₂CH₃)₂ |
| II-332 | cHexyl | N(COCH₂CH₃)₂ |
| II-333 | cHeptyl | N(COCH₂CH₃)₂ |
| II-334 | 4-F-Phenyl | N(COCH₂CH₃)₂ |
| II-335 | 4-Cl-Phenyl | N(COCH₂CH₃)₂ |
| II-336 | 2,4-diF-Phenyl | N(COCH₂CH₃)₂ |
| II-337 | 2,4,6-triF-Phenyl | N(COCH₂CH₃)₂ |
| II-338 | 2,4,5-triF-Phenyl | N(COCH₂CH₃)₂ |
| II-339 | (cis) 2-Cl-cHexyl | N(COCH₂CH₃)₂ |
| II-340 | (trans) 2-Cl-cHexyl | N(COCH₂CH₃)₂ |
| II-341 | (R,R) 2-Cl-cHexyl | N(COCH₂CH₃)₂ |
| II-342 | (S,S) 2-Cl-cHexyl | N(COCH₂CH₃)₂ |
| II-343 | (S,R) 2-Cl-cHexyl | N(COCH₂CH₃)₂ |
| II-344 | (R,S) 2-Cl-cHexyl | N(COCH₂CH₃)₂ |
| II-345 | (cis) 2-Methyl-cHexyl | N(COCH₂CH₃)₂ |
| II-346 | (trans) 2-Methyl-cHexyl | N(COCH₂CH₃)₂ |
| II-347 | 1-F-cHexyl | N(COCH₂CH₃)₂ |
| II-348 | cPentyl | N(COCH₃)₂ |
| II-349 | cHexyl | N(COCH₃)₂ |
| II-350 | cHeptyl | N(COCH₃)₂ |
| II-351 | 4-F-Phenyl | N(COCH₃)₂ |
| II-352 | 4-Cl-Phenyl | N(COCH₃)₂ |
| II-353 | 2,4-diF-Phenyl | N(COCH₃)₂ |
| II-354 | 2,4,6-triF-Phenyl | N(COCH₃)₂ |
| II-355 | 2,4,5-triF-Phenyl | N(COCH₃)₂ |
| II-356 | (cis) 2-Cl-cHexyl | N(COCH₃)₂ |
| II-357 | (trans) 2-Cl-cHexyl | N(COCH₃)₂ |
| II-358 | (R,R) 2-Cl-cHexyl | N(COCH₃)₂ |
| II-359 | (S,S) 2-Cl-cHexyl | N(COCH₃)₂ |
| II-360 | (S,R) 2-Cl-cHexyl | N(COCH₃)₂ |
| II-361 | (R,S) 2-Cl-cHexyl | N(COCH₃)₂ |
| II-362 | (cis) 2-Methyl-cHexyl | N(COCH₃)₂ |
| II-363 | (trans) 2-Methyl-cHexyl | N(COCH₃)₂ |
| II-364 | 1-F-cHexyl | N(COCH₃)₂ |
| II-365 | cHexyl | NHCOCO₂Et |
| II-366 | cHexyl | NHCOCH₂CO₂Et |
| II-367 | cHexyl | NHCOC₂H₄CO₂Et |
| II-368 | cHexyl | NHCOC₃H₆CO₂Et |
| II-369 | 2-Cl-cHex-1-ene | NHCOCH₂CH₃ |
| II-370 | 2-Cl-cHex-1-ene | NHCOC₂H₄CO₂Me |
| II-371 | cHexyl | N=SMe₂ |
| II-372 | cHexyl | N=S(Me)(4-Me-Ph) |
| II-373 | cHexyl | N=S(CH₂Ph)₂ |
| II-374 | cHexyl | N=S(nPr)₂ |
| II-375 | cHexyl | N=S(Et)₂ |
| II-376 | cHexyl | N=S(Ph)₂ |
| II-377 | cHexyl | N=S(Et)(nPr) |
| II-378 | (trans) 4-F-cHexyl | NHCOCF₂CF₃ |
| II-379 | (cis) 4-F-cHexyl | NHCOCF₂CF₃ |
| II-380 | (trans) 4-F-cHexyl | NHCOCH₂CH₃ |
| II-381 | (cis) 4-F-cHexyl | NHCOCH₂CH₃ |
| II-382 | cHexyl | 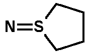 |
| II-383 | cHexyl | 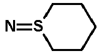 |
| II-384 | cHexyl | 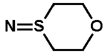 |
| II-385 | cHexyl | N(COC₃H₆CO₂Et)₂ |
| II-386 | cHexyl | N(COC₃H₆CO₂Me)₂ |
| II-387 | cHexyl | N(COC₂H₄CO₂Me)₂ |
| II-388 | cHeptyl | NHCOC₂H₄CO₂Et |
| II-389 | cHexyl | NHCOCH₂OEt |
| II-390 | cHexyl | N(COCH₂OEt)₂ |
| II-391 | 1-F-cHexyl | NHCOC₂H₄CO₂Et |
| II-392 | cHexyl | N(COCH₂OMe)₂ |

Specific preferred compounds of formula (III) are shown in Table 3.

**Table 3: Preferred compounds of formula (III):**

| **No.** | **y** | **A** | **R¹** | **NR²R³** |
|---|---|---|---|---|
| III-1 | 0 | - | cPentyl | NH₂ |
| III-2 | 0 | - | cHexyl | NH₂ |
| III-3 | 0 | - | cHeptyl | NH₂ |
| III-4 | 0 | - | (cis) 2-Cl-cHexyl | NH₂ |
| III-5 | 0 | - | Phenyl | NH₂ |
| III-6 | 0 | - | cPentyl | NHCOCH₂CH₃ |
| III-7 | 0 | - | cHexyl | NHCOCH₂CH₃ |
| III-8 | 0 | - | cHeptyl | NHCOCH₂CH₃ |
| III-9 | 0 | - | (cis) 2-Cl-cHexyl | NHCOCH₂CH₃ |
| III-10 | 0 | - | Phenyl | NHCOCH₂CH₃ |
| III-11 | 0 | - | cPentyl | NHCOCF₂CF₃ |
| III-12 | 0 | - | cHexyl | NHCOCF₂CF₃ |
| III-13 | 0 | - | cHeptyl | NHCOCF₂CF₃ |
| III-14 | 0 | - | (cis) 2-Cl-cHexyl | NHCOCF₂CF₃ |
| III-15 | 0 | - | Phenyl | NHCOCF₂CF₃ |
| III-16 | 1 | CHMe | cHexyl | NH₂ |
| III-17 | 1 | (*R*)-CHMe | cHexyl | NH₂ |
| III-18 | 1 | (*S*)-CHMe | cHexyl | NH₂ |
| III-19 | 1 | CHMe | cHexyl | NHCOCH₂CH₃ |
| III-20 | 1 | (*R*)-CHMe | cHexyl | NHCOCH₂CH₃ |
| III-21 | 1 | (*S*)-CHMe | cHexyl | NHCOCH₂CH₃ |
| III-22 | 1 | CHMe | cHexyl | NHCOCF₂CF₃ |
| III-23 | 1 | (*R*)-CHMe | cHexyl | NHCOCF₂CF₃ |
| III-24 | 1 | (*S*)-CHMe | cHexyl | NHCOCF₂CF₃ |
| III-25 | 1 | CHMe | cHexyl | NHCOCH₃ |
| III-26 | 1 | (*R*)-CHMe | cHexyl | NHCOCH₃ |
| III-27 | 1 | (*S*)-CHMe | cHexyl | NHCOCH₃ |
| III-28 | 1 | CHMe | cHexyl | NHCOCF₃ |
| III-29 | 1 | (*R*)-CHMe | cHexyl | NHCOCF₃ |
| III-30 | 1 | (*S*)-CHMe | cHexyl | NHCOCF₃ |

Preferably, the one or more compounds of the formulae (G), (I), (II) and (III), each as defined above, and the salts thereof, are used in the context of the present invention as herbicides and/or plant growth regulators, preferably in crops of useful plants and/or ornamental plants, wherein the structural elements in the formulae (G), (I), (II) and (III), each have, independently from one another, the meaning as defined in the context of the meaning as defined in one of the preferred, more preferred, or particularly preferred embodiments.

The present invention also provides processes for preparing the compounds of the general formula (G) and/or their salts. This includes processes which can be carried out analogously to known methods.

Compounds according to the invention may be obtained using different synthetic routes shown in the following Schemes 1 to 3.

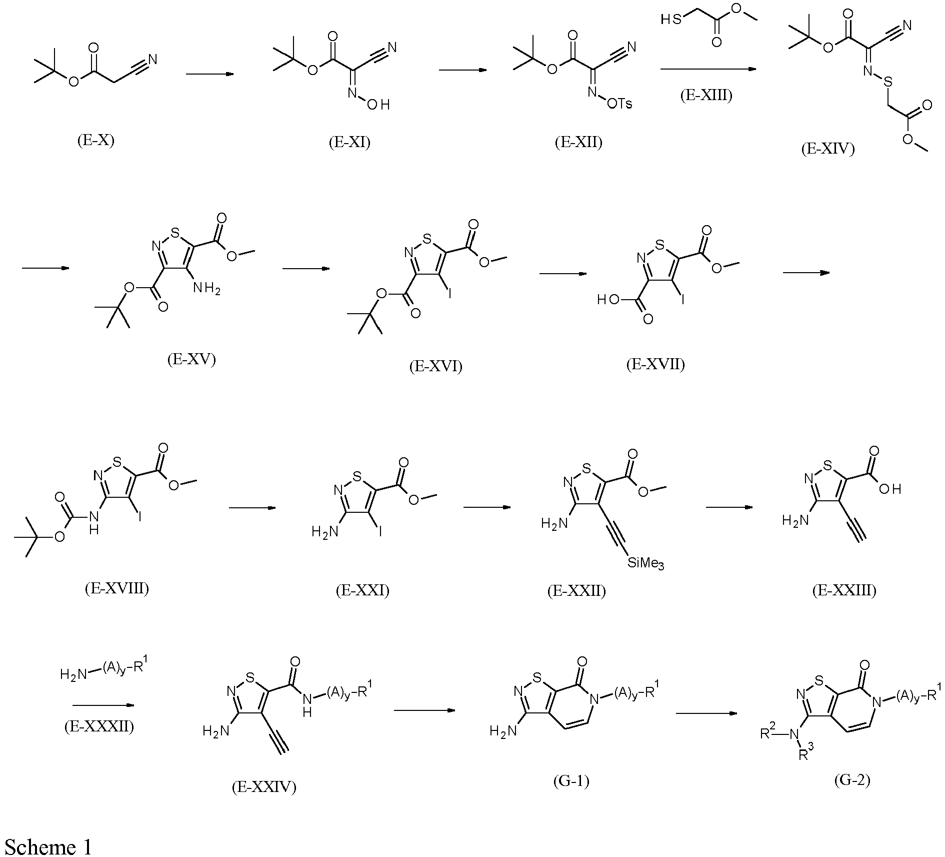

Compound (E-XIV) required for the cyclization can be readily prepared in three steps from the cyanoacetic ester (E-X). For this purpose, (E-X) is initially reacted with NaNO₂ in aqueous acetic acid, which forms the oxime (E-XI), which may be converted in a second step to the para-tolylsulphonate. For this purpose, (E-XI) is stirred with a suitable sulphonylating reagent, for example para-tolylsulphonyl chloride, and an organic base, for example pyridine. The resulting tosylate (E-XII) is reacted in the third step with the thioglycolate (E-XIII), forming a N-S bond, to give the cyclization precursor (E-XIV). This reaction generally takes place in a commonly used organic solvent such as ethanol, with the aid of an organic base such as pyridine.

The amino compound (E-XV) may be synthesized from the compound (E-XIV) by cyclization, by firstly treating the latter with a weak base, for example triethylamine or other organic bases, and directly after with ethanolic HCl.

The ester (E-XVI) may be obtained from the amino compound (E-XV) by the Sandmeyer reaction or related reactions. For instance, (E-XV) may be reacted, for example, with an alkyl nitrite, such as isoamyl nitrite, and iodine in an inert solvent, such as acetonitrile, at temperatures between 20 °C and 150 °C.

The acid (E-XVII) may be obtained, for example, from the tertiary butyl ester (E-XVI) by the action of acid, such as, for example, trifluoroacetic acid (TFA) or dilute mineral acid in the presence of triethylsilane.

The compound (E-XVIII) can be obtained, for example, from the acid (E-XVII) by Hoffman degradation, Curtius or Schmidt rearrangement or by a related reaction, wherein the tertiary butyl carbamate, which is readily isolatable, is directly obtained using a suitable reaction procedure (t-BuOH as solvent or solvent constituent), preferably in the presence of t-BuOH, T3P (propylphosphonic anhydride), trimethylsilyl azide and NEt₃ in a solvent like THF (tetrahydrofuran) at elevated temperatures (typically 70 °C).

This tertiary butyl carbamate (E-XVIII) may be cleaved to the free amine (E-XXI) by treatment with acid, such as, for example, trifluoroacetic acid or dilute mineral acid.

The ester (E-XXII) can be obtained from compound (E-XXI) by reaction of ethynyltrimethylsilane in the presence of palladium (II) diphenylphosphine dichloride Pd(PPh₂)₂Cl₂, CuI and NEt₃ in a suitable solvent (e.g. DMF) at elevated temperatures (e.g. 100 °C).

The acid (E-XXIII) in turn is available from the corresponding ester (E-XXII) by basic ester cleavage, for example, with the aid of inorganic bases such as NaOH or LiOH or other bases in aqueous solvents or solvent mixtures like MeOH and THF (tetrahydrofuran).

Intermediate (E-XXIV) may be obtained from the corresponding acid (E-XXIII) by the common amidation reactions with suitable amines (E-XXXII), preferably in the presence of T3P (propylphosphonic anhydride) and NEt₃ in a solvent like THF.

The ring closure of compound (E-XXIV) is effected in the presence of TBAF (tetra-n-butylammonium fluoride) (see e.g. Tetrahedron 2001, 51, 9697-9710) in a suitable solvent, e.g. THF, yielding the isothiazolopyridones having a free amine group (i.e. R² = R³ = H). This compound of formula (G-1) may be converted to compound of the formula (G-2) in which R² and/or R³ are not H using suitable known reactions for converting free amine groups to correspondingly substituted amine groups. For example, suitable conversions are achieved with the corresponding acyl halide(s), acid anhydride(s) or the like, preferably acyl chlorides R²COCl and/or R³COCl, or anyhdrides (R²CO)₂O, (R³CO)₂O and/or R²CO(O)OCR³ using an amine like NEt₃, preferably in the presence of DMAP (4-dimethylaminopyridine) in a suitable solvent like DCM (dichloromethane).

The compounds (E-X), (E-XI), (E-XII), (E-XIII), (E-XIV), (E-XV), (E-XVI), (E-XVII), (E-XVIII), (E-XXI) and (E-XXXII) are known and have been described in the prior art. Also, the synthetic route for obtaining (E-XXI) depicted in Scheme 1 above has been described in the two European Patent Applications relating to isothiazolamides and processes for their preparation, and which were filed electronically on 22.12.2014 at the European Patent Office, of which Bayer CropScience AG is the applicant, and which have received the Application numbers EP14199545.6 and EP14199548.0, respectively.

The compound (E-XXI) described in Scheme 1 may also be used as starting material for another synthetic route for obtaining compounds of the formula (G) according to the present invention. This alternative route to obtain the compounds of formula (G) according to the present invention is shown in the following Scheme 2.

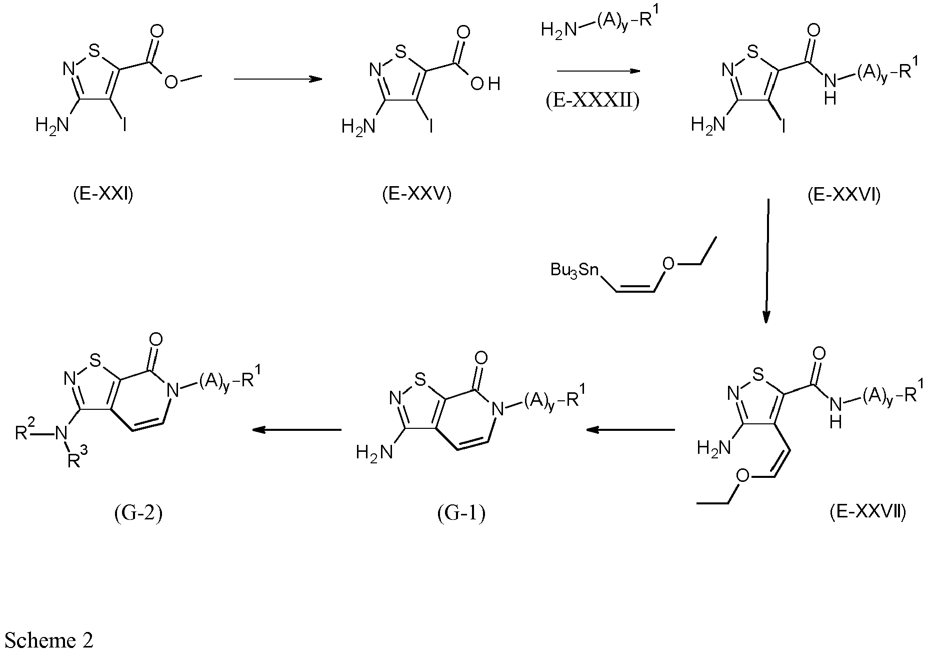

As shown in Scheme 2, starting from compound (E-XXI), in a first step the ester group is hydrolyzed (e.g. with NaOH in MeOH and THF) to yield compound (E-XXV) which is subsequently reacted with amine (XXXII) under conditions as described above for Scheme 1 to give compound (E-XXVI).

Compound (E-XXVI) is reacted in a cross-coupling reaction to afford the compound (E-XXVII). This cross-coupling reaction with tributyl-[(Z)-2-ethoxyvinyl]stannane is generally carried out with the aid of a transition metal catalyst or transition metal precatalyst (Pd₂dba₃, PdCl₂(PPh₃)₂, etc.) in a suitable solvent (e.g. DMF etc.), generally at temperatures in the range of 40 °C and 120 °C. The subsequent ring closure is effected in the presence of an acid (e.g. 2M HCl, in dioxane) yielding the isothiazolopyridones having a free amine group (i.e. R² = R³ = H). This compound of formula (G-1) may be converted to compound of the formula (G-2) in which R² and/or R³ are not H using the reactions described in Scheme 1 above.

The following Scheme 3 shows the synthetic route suitable for obtaining compounds of the formula (G) according to the present invention, in which W = sulphur.

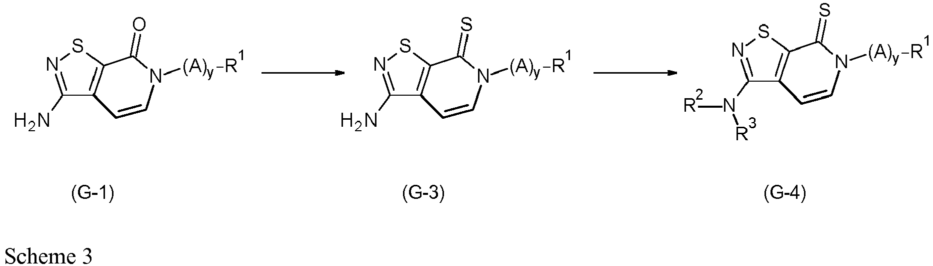

As shown in Scheme 3, the amine compounds of formula (G-1) can be converted in a further step into the corresponding amine compounds (i.e. R² = R³ = H) of formula (G-3), by reaction with a sulphur-transferring reagent (thionation agent) such as, for example, P₄S₁₀ or Lawesson's reagent [2,4-Bis(4-methoxyphenyl)-1,3,2,4-dithiadiphosphetane-2,4-disulphide]. If desired, compounds of formula (G-3) can be converted to compounds of formula (G-4) in which R² and/or R³ are not H with the corresponding reagents like acyl halide(s), acid anhydride(s) or the like, preferably acyl chlorides R²COCl and/or R³COCl, or anyhdrides (R²CO)₂O, (R³CO)₂O and/or R²CO(O)OCR³ using an amine like NEt₃, pereferably in the presence of DMAP (4-dimethylaminopyridine) in a suitable solvent like DCM (dichloromethane).

The present invention further relates to a process for preparing a compound of the formulae (G) as defined herein, and/or a salt thereof, wherein W is oxygen, characterized in that
(a) a compound of formula (G) wherein R² and R³ both are H is obtained in a chemical synthesis comprising the step of cyclization of a compound of the formula (E-XXIV) 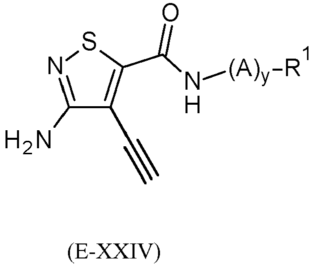 in which R¹, A and y each have the meaning as defined in formula (G), wherein the cyclization preferably is performed in the presence of a quaternary ammonium salt, preferably a tetra-n-butylammonium salt, in particular tetra-n-butylammonium fluoride,
   or
(b) a compound of formula (G) wherein R² and R³ both are H is obtained in a chemical synthesis comprising the step of cyclization of a compound of the formula (E- XXVII) 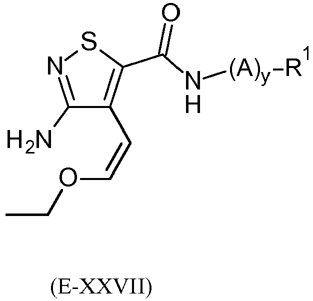 in which R¹, A and y each have the meaning as defined in formula (G), wherein the cyclization preferably is performed in the presence of an acid catalyst, preferably a mineral acid, preferably HCl,
   or
(c) a compound of formula (G), wherein R² and/or R³ are not H is obtained in a chemical synthesis comprising the step of reacting a compound of the formula (G-1) 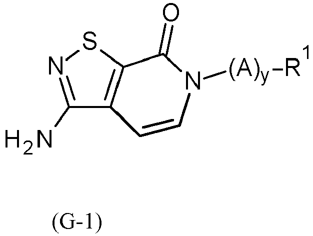 in which R¹, A and y each have the meaning as defined in formula (G),
   with an acyl halogenide R²COHal and/or R³COHal, wherein Hal in each case is Cl, Br or I, or an anyhdride (R²CO)₂O, (R³CO)₂O and/or R²CO(O)OCR³, preferably in the presence of an amine like NEt₃, wherein R² and R³ have the meaning as defined in formula (G), and wherein R² and/or R³ are not H.

Depending on the type of reaction and the reaction conditions used, the skilled person will select suitable organic solvents, such as:
- aliphatic hydrocarbons such as pentane, hexane, cyclohexane or petroleum ether;
- aromatic hydrocarbons such as toluene, o-, m- or p-xylene,
- halogenated hydrocarbons such as methylene chloride, chloroform or chlorobenzene,
- ethers, such as diethyl ether, diisopropyl ether, tert-butyl methyl ether, dioxane, anisole and tetrahydrofuran (THF),
- nitriles such as acetonitrile or propionitrile,
- ketones such as acetone, methyl ethyl ketone, diethyl ketone and tert-butyl methyl ketone,
- alcohols such as methanol, ethanol, n-propanol, isopropanol, n-butanol and tert-butanol, and also
- dimethyl sulphoxide (DMSO), dimethylformamide (DMF), dimethylacetamide, sulpholane,
- mixtures of the organic solvents mentioned.

If the compounds described in the context of the present invention, in particular the intermediates and compounds (G) of the present invention, are obtained as solids, the purification can also be carried out by recrystallization or digestion.

The following acids are generally suitable for preparing the acid addition salts of the compounds of the formula (G): hydrohalic acids, such as hydrochloric acid or hydrobromic acid, furthermore phosphoric acid, nitric acid, sulphuric acid, mono- or bifunctional carboxylic acids and hydroxycarboxylic acids, such as acetic acid, maleic acid, succinic acid, fumaric acid, tartaric acid, citric acid, salicylic acid, sorbic acid, or lactic acid, and also sulphonic acids, such as p-toluenesulphonic acid and 1,5-naphthalenedisulphonic acid. The acid addition compounds of the formula (G) can be obtained in a simple manner by the customary methods for forming salts, for example by dissolving a compound of the formula (G) in a suitable organic solvent, such as, for example, methanol, acetone, methylene chloride or benzene, and adding the acid at temperatures of from 0 to 100 °C, and they can be isolated in a known manner, for example by filtration, and, if appropriate, purified by washing with an inert organic solvent.

The base addition salts of the compounds of the formula (G) are preferably prepared in inert polar solvents, such as, for example, water, methanol or acetone, at temperatures of from 0 to 100 °C. Examples of bases which are suitable for the preparation of the salts according to the invention are alkali metal carbonates, such as potassium carbonate, alkali metal hydroxides and alkaline earth metal hydroxides, for example NaOH or KOH, alkali metal hydrides and alkaline earth metal hydrides, for example NaH, alkali metal alkoxides and alkaline earth metal alkoxides, for example sodium methoxide or potassium tert-butoxide, or ammonia, ethanolamine or quaternary ammonium hydroxide.

What is meant by the "inert solvents" referred to in the above process variants are in each case solvents which are inert under the respective reaction conditions.

Collections of compounds of the formula (G) which can be synthesized by the aforementioned process can also be prepared in a parallel manner, it being possible for this to take place in a manual, partly automated or completely automated manner. In this connection, it is possible to automate the reaction procedure, the work-up or the purification of the products and/or intermediates. Overall, this is understood as meaning a procedure as described, for example, by S. H. DeWitt in "Annual Reports in Combinatorial Chemistry and Molecular Diversity: Automated Synthesis", Volume 1, Verlag Escom, 1997, pages 69 to 77.

For the parallelized reaction procedure and workup it is possible to use a range of commercially available instruments, of the kind offered by, for example, the companies Stem Corporation, Woodrolfe Road, Tollesbury, Essex, CM9 8SE, England, or H + P Labortechnik GmbH, Bruckmannring 28, 85764 Oberschleißheim, Germany. For the parallel purification of compounds (G) or of intermediates produced during the preparation, there are available, inter alia, chromatography apparatuses, for example from ISCO, Inc., 4700 Superior Street, Lincoln, NE 68504, USA. The apparatuses listed allow a modular procedure in which the individual process steps are automated, but between the process steps manual operations have to be carried out. This can be circumvented by using partly or completely integrated automation systems in which the respective automation modules are operated, for example, by robots. Automation systems of this type can be acquired, for example, from Zymark Corporation, Zymark Center, Hopkinton, MA 01748, USA.

Besides the methods described here, the preparation of compounds of the formula (G) can take place completely or partially by solid-phase supported methods. For this purpose, individual intermediates or all intermediates in the synthesis or a synthesis adapted for the corresponding procedure are bonded to a synthesis resin. Solid-phase-supported synthesis methods are described extensively in the specialist literature, for example Barry A. Bunin in "The Combinatorial Index", Academic Press, 1998.

The use of solid-phase-supported synthesis methods permits a number of protocols, which are known from the literature and which for their part may be performed manually or in an automated manner, to be carried out. For example, the "teabag method" (Houghten, US 4,631,211; Houghten et al., Proc. Natl. Acad. Sci, 1985, 82, 5131-5135) in which products from IRORI, 11149 North Torrey Pines Road, La Jolla, CA 92037, USA, are employed, may be semiautomated. The automation of solid-phase-supported parallel syntheses is performed successfully, for example, by apparatuses from Argonaut Technologies, Inc., 887 Industrial Road, San Carlos, CA 94070, USA or MultiSynTech GmbH, Wullener Feld 4, 58454 Witten, Germany.

The preparation according to the processes described herein produces compounds of the formula (G) in the form of substance collections or libraries. Accordingly, the present invention also provides libraries of compounds of the formula (G) which comprise at least two compounds of the formula (G), and precursors thereof.

The compounds of the formula (G) used in the context of the present invention or according to the invention (and/or their salts) have excellent herbicidal efficacy against a broad spectrum of economically important monocotyledonous and dicotyledonous annual harmful plants. The active compounds of the formula (G) also provide good control over perennial harmful plants which are difficult to control and produce shoots from rhizomes, root stocks or other perennial organs.

The present invention therefore also relates to a method for controlling unwanted plants or for regulating the growth of plants, preferably in crops of plants, where one or more compound(s) according to the invention is/are applied to the plants (for example harmful plants such as monocotyledonous or dicotyledonous weeds or undesired crop plants), to the seed (for example grains, seeds or vegetative propagules such as tubers or shoot parts with buds), to the soil in or on which the plants grow (for example the soil of cropland or non-cropland) or to the area on which the plants grow (for example the area under cultivation).

Thus, in a further aspect, the present invention relates to a method for controlling harmful plants or for regulating the growth of plants, characterized in that an effective amount of
- one or more compounds of the formula (G) and/or salts thereof as defined hereinabove, preferably in one of the preferred, more preferred or particularly preferred embodiments,
   or
- a herbicidal and/or plant growth-regulating composition as defined hereinafter comprising one or more compounds of the formula (G) and/or salts thereof as defined hereinabove, preferably in one of the preferred, more preferred or particularly preferred embodiments,
is applied to the plants, seeds of plants, the soil in which or on which the plants grow or the area under cultivation.

Likewise, the present invention relates to the use of one or more compounds of the formula (G) and/or salts thereof as defined in the context of the present invention for controlling harmful plants or for regulating the growth of plants, wherein one or more compounds of the formula (G) and/or salts thereof are applied onto the harmful plants, plant seeds, the soil in which or on which the plants grow or the area under cultivation.

The compounds according to the invention can be deployed, for example, prior to sowing (if appropriate also by incorporation into the soil), prior to emergence or after emergence. Specific examples may be mentioned of some representatives of the monocotyledonous and dicotyledonous weed flora which can be controlled by the compounds according to the invention, without the enumeration being restricted to certain species.

Monocotyledonous harmful plants of the genera: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dicotyledonous weeds of the genera: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

The compounds of the formula (G) to be used according to the invention or the compounds of the formula (G) according to the invention and/or their salts were found to be highly effective in the control of harmful plants such as Alopecurus myosuroides, Echinochloa crus-galli, Setaria viridis, Abutilon theophrasti, Amaranthus retroflexus, Pharbitis purpurea, Polygonum convolvulus (= Fallopia convolvulus), Stellaria media, and Viola tricolor.

When the compounds (G) according to the invention are applied to the soil surface before germination, either the weed seedlings are prevented completely from emerging or the weeds grow until they have reached the cotyledon stage, but then stop growing and eventually, after three to four weeks have elapsed, die completely.

If the compounds (G) are applied post-emergence to the green parts of the plants, growth stops after the treatment, and the harmful plants remain at the growth stage of the time of application, or die completely after a certain time, such that competition by the weeds, which is harmful to the crop plants, is thus eliminated very early and in a lasting manner.

Although the compounds according to the invention display an outstanding herbicidal activity against monocotyledonous and dicotyledonous weeds, crop plants of economically important crops, for example dicotyledonous crops of the genera Arachis, Beta, Brassica, Cucumis, Cucurbita, Helianthus, Daucus, Glycine, Gossypium, Ipomoea, Lactuca, Linum, Lycopersicon, Miscanthus, Nicotiana, Phaseolus, Pisum, Solanum, Vicia, or monocotyledonous crops of the genera Allium, Ananas, Asparagus, Avena, Hordeum, Oryza, Panicum, Saccharum, Secale, Sorghum, Triticale, Triticum, Zea, in particular Zea and Triticum, are damaged only to an insignificant extent, or not at all, depending on the structure of the respective compound according to the invention and its application rate. For these reasons, the present compounds are very suitable for selective control of unwanted plant growth in plant crops such as agriculturally useful plants or ornamentals.

Furthermore, it has been found that the compounds of the formula (G) to be used according to the invention or the compounds of the formula (G) according to the invention and/or their salts show excellent or very good pre-emergence and post-emergence action, and are particularly selectively in certain crops, in particular in oilseed rape, soya beans, cotton and cereals (and here in particular in maize, barley, wheat, rye, oats, triticale, millet varieties, rice).

In addition, the compounds according to the invention (depending on their particular structure and their application rate) have outstanding growth-regulating properties in crop plants. They intervene to regulate the plant's metabolism and can thus be used for controlled influence on plant constituents and to facilitate harvesting, for example by triggering desiccation and stunted growth. Moreover, they are also suitable for generally controlling and inhibiting unwanted vegetative growth without destroying the plants in the process. Inhibiting the vegetative growth plays an important role in many monocotyledonous and dicotyledonous crops since for example lodging can be reduced, or prevented completely, hereby.

By virtue of their herbicidal and/or plant growth-regulating properties, the active compounds (G) can also be used for control of harmful plants in crops of genetically modified plants or plants modified by conventional mutagenesis. In general, transgenic plants are notable for special advantageous properties, for example for resistances to certain pesticides, in particular certain herbicides, resistances to plant diseases or organisms that cause plant diseases, such as certain insects or microorganisms such as fungi, bacteria or viruses. Other specific characteristics relate, for example, to the harvested material with regard to quantity, quality, storability, composition and specific constituents. Thus, transgenic plants are known whose starch content is increased, or whose starch quality is altered, or those where the harvested material has a different fatty acid composition.

It is preferred with a view to transgenic crops to use the compounds according to the invention and/or their salts in economically important transgenic crops of useful plants and ornamentals, for example of cereals such as wheat, barley, rye, oats, millet, rice and corn or else crops of sugar beet, cotton, soybean, oilseed rape, potato, tomato, peas and other vegetables.

It is preferred to employ the compounds according to the invention as herbicides in crops of useful plants which are resistant, or have been made resistant by recombinant means, to the phytotoxic effects of the herbicides.

By virtue of their herbicidal and/or plant-growth-regulatory properties, the active compounds of the formula (G) can also be employed for controlling harmful plants in crops of known genetically modified plants or genetically modified plants still to be developed. In general, the transgenic plants are distinguished by especially advantageous properties, for example by resistances to certain pesticides, mainly certain herbicides, resistances to plant diseases or causative organisms of plant diseases, such as certain insects or microorganisms such as fungi, bacteria or viruses. Other specific characteristics relate, for example, to the harvested material with regard to quantity, quality, storability, composition and specific constituents. Thus, transgenic plants are known whose starch content is increased, or whose starch quality is altered, or those where the harvested material has a different fatty acid composition. Other particular properties may be tolerance or resistance to abiotic stressors, for example heat, low temperatures, drought, salinity and ultraviolet radication.

It is preferred to use the compounds of the formula (G) according to the invention and/or salts thereof in economically important transgenic crops of useful plants and ornamental plants, for example of cereals such as wheat, barley, rye, oats, sorghum and millet, rice, cassava and corn or else crops of sugar beet, cotton, soybean, oilseed rape, potato, tomato, peas and other vegetables.

It is preferred to employ the compounds of the formula (G) according to the invention as herbicides in crops of useful plants which are resistant, or have been made resistant by recombinant means, to the phytotoxic effects of the herbicides.

On employment of the active compounds (G) according to the invention in transgenic crops, not only do the effects toward harmful plants observed in other crops occur, but often also effects which are specific to application in the particular transgenic crop, for example an altered or specifically widened spectrum of weeds which can be controlled, altered application rates which can be used for the application, preferably good combinability with the herbicides to which the transgenic crop is resistant, and influencing of growth and yield of the transgenic crop plants.

The invention therefore also relates to the use of the compounds of the formula (G) according to the invention and/or their salts as herbicides for controlling harmful plants in crops of useful plants or ornamentals, optionally in transgenic crop plants.

Preference is given to the use by the pre- or post-emergence method in cereals such as wheat, barley, rye, oats, millet and rice, in particular in wheat by the post-emergence method.

Preference is also given to the use by the pre- or post-emergence method in corn, in particular by the pre-emergence method in corn.

Preference is also given to the use by the pre- or post-emergence method in soybeans, in particular by the post-emergence method in soybeans.

The use according to the invention for the control of harmful plants or for the growth regulation of plants also includes the case in which the active compound of the formula (G) or its salt is not formed from a precursor substance ("prodrug") until after application on the plant, in the plant or in the soil.

The invention also provides the method (application method) for controlling harmful plants or for regulating the growth of plants which comprises applying an effective amount of one or more compounds of the formula (G) and/or salts thereof onto the plants (harmful plants, if appropriate together with the useful plants), plant seeds, the soil in which or on which the plants grow or the area under cultivation.

The compounds (G) according to the invention can be used in the form of wettable powders, emulsifiable concentrates, sprayable solutions, dusting products or granules in the customary formulations. The invention therefore also provides herbicidal and/or plant growth-regulating compositions which comprise (an herbicidal and/or plant growth-regulating effective amount of) compounds of the formula (G) and/or salts thereof.

Thus, in a further aspect, the present invention relates to a herbicidal and/or plant growth-regulating composition, characterized in that said composition comprises (an herbicidal and/or plant growth-regulating effective amount of) one or more compounds of the formula (G) and/or salts thereof as defined hereinabove, preferably in one of the preferred, more preferred or particularly preferred embodiments,
and one or more further substances selected from groups (i) and/or (ii):
(i) one or more further agrochemically active substances, preferably selected from the group consisting of insecticides, acaricides, nematicides, further herbicides, fungicides, safeners, fertilizers and/or further growth regulators,
(ii) one or more formulation auxiliaries customary in crop protection.

The compounds of the formula (G) and/or salts thereof can be formulated in various ways according to which biological and/or physicochemical parameters are required. Possible formulations include, for example: wettable powders (WP), water-soluble powders (SP), water-soluble concentrates, emulsifiable concentrates (EC), emulsions (EW), such as oil-in-water and water-in-oil emulsions, sprayable solutions, suspension concentrates (SC), oil- or water-based dispersions, oil-miscible solutions, capsule suspensions (CS), dusting products (DP), seed-dressing products, granules for broadcasting and soil application, granules (GR) in the form of microgranules, sprayable granules, coated granules and adsorption granules, water-dispersible granules (WG), water-soluble granules (SG), ULV formulations, microcapsules and waxes.

These individual formulation types are known in principle and are described, for example, in: Winnacker-Küchler, "Chemische Technologie" [Chemical technology], volume 7, C. Hanser Verlag Munich, 4th ed. 1986; Wade van Valkenburg, "Pesticide Formulations", Marcel Dekker, N.Y., 1973; K. Martens, "Spray Drying" Handbook, 3rd ed. 1979, G. Goodwin Ltd. London.

The necessary formulation auxiliaries, such as inert materials, surfactants, solvents and further additives are likewise known and are described, for example, in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte" [Interface-active Ethylene Oxide Adducts], Wiss. Verlagsgesell., Stuttgart 1976; Winnacker-Küchler, "Chemische Technologie", volume 7, C. Hanser Verlag Munich, 4th ed. 1986.

Wettable powders are preparations which can be dispersed uniformly in water and, as well as the active compound, apart from a diluent or inert substance, also comprise surfactants of the ionic and/or nonionic type (wetting agents, dispersants), for example polyoxyethylated alkylphenols, polyoxyethylated fatty alcohols, polyoxyethylated fatty amines, fatty alcohol polyglycol ether sulphates, alkanesulphonates, alkylbenzenesulphonates, sodium lignosulphonate, sodium 2,2'-dinaphthylmethane-6,6'-disulphonate, sodium dibutylnaphthalenesulphonate or else sodium oleoylmethyltaurinate. To prepare the wettable powders, the herbicidally active compounds are ground finely, for example in customary apparatus such as hammer mills, blower mills and air-jet mills, and simultaneously or subsequently mixed with the formulation assistants.

Emulsifiable concentrates are produced by dissolving the active compound in an organic solvent, for example butanol, cyclohexanone, dimethylformamide, xylene or else relatively high-boiling aromatics or hydrocarbons or mixtures of the organic solvents, with addition of one or more surfactants of the ionic and/or nonionic type (emulsifiers). The emulsifiers used may, for example, be: alkylarylsulphonic calcium salts, such as calcium dodecylbenzenesulphonate, or nonionic emulsifiers such as fatty acid polyglycol esters, alkylaryl polyglycol ethers, fatty alcohol polyglycol ethers, propylene oxide-ethylene oxide condensation products, alkyl polyethers, sorbitan esters, such as, for example, sorbitan fatty acid esters, or polyoxyethylene sorbitan esters, such as, for example, polyoxyethylene sorbitan fatty acid esters.

Dusting products are obtained by grinding the active compound with finely distributed solid substances, for example talc, natural clays, such as kaolin, bentonite and pyrophyllite, or diatomaceous earth.

Suspension concentrates may be water- or oil-based. They can be produced, for example, by wet grinding by means of commercial bead mills with optional addition of surfactants as already listed above, for example, for the other formulation types.

Emulsions, e.g. oil-in-water emulsions (EW), can be prepared, for example, by means of stirrers, colloid mills and/or static mixers using aqueous organic solvents and if appropriate surfactants, as have for example already been listed above in connection with the other types of formulation.

Granules can be produced either by spraying the active compound onto adsorptive granulated inert material or by applying active compound concentrates by means of adhesives, for example polyvinyl alcohol, sodium polyacrylate or mineral oils, to the surface of carrier substances, such as sand, kaolinites or of granulated inert material. Suitable active compounds can also be granulated in the manner customary for the production of fertilizer granules - if desired as a mixture with fertilizers.

Water-dispersible granules are produced generally by the customary processes such as spray-drying, fluidized bed granulation, pan granulation, mixing with high-speed mixers and extrusion without solid inert material.

For the production of pan granules, fluidized bed granules, extruder granules and spray granules, see, for example, processes in "Spray-Drying Handbook" 3rd ed. 1979, G. Goodwin Ltd., London; J.E. Browning, "Agglomeration", Chemical and Engineering 1967, pages 147 ff.; "Perry's Chemical Engineer's Handbook", 5th Ed., McGraw-Hill, New York 1973, pp. 8-57.

For further details regarding the formulation of crop protection agents, see, for example, G.C. Klingman, "Weed Control as a Science", John Wiley and Sons, Inc., New York, 1961, pages 81-96 and J.D. Freyer, S.A. Evans, "Weed Control Handbook", 5th ed., Blackwell Scientific Publications, Oxford, 1968, pages 101-103.

The agrochemical formulations comprise generally from 0.1 to 99% by weight, in particular from 0.1 to 95% by weight, of active compound of the formula (G) and/or salts thereof.

In wettable powders, the active compound concentration is, for example, about 10 to 90% by weight; the remainder to 100% by weight consists of the customary formulation constituents. In the case of emulsifiable concentrates, the active compound concentration can be from about 1 to 90, preferably from 5 to 80, % by weight. Dust-type formulations contain from 1 to 30% by weight of active compound, preferably usually from 5 to 20% by weight of active compound; sprayable solutions contain from about 0.05 to 80% by weight, preferably from 2 to 50% by weight of active compound. In the case of water-dispersible granules, the active compound content depends partly on whether the active compound is present in liquid or solid form and on which granulation assistants, fillers, etc., are used. In the water-dispersible granules, the content of active compound is, for example, between 1 and 95% by weight, preferably between 10 and 80% by weight.

In addition, the active compound formulations mentioned optionally comprise the respective customary tackifiers, wetting agents, dispersants, emulsifiers, penetrants, preservatives, antifreeze agents and solvents, fillers, carriers and dyes, defoamers, evaporation inhibitors and agents which influence the pH and the viscosity. Examples of formulation auxiliaries are described, inter alia, in "Chemistry and Technology of Agrochemical Formulations", ed. D. A. Knowles, Kluwer Academic Publishers (1998).

The compounds of the formula (G) and/or salts thereof can be employed as such or in the form of their preparations (formulations) combined with other pesticidally active compounds, such as, for example, insecticides, acaricides, nematicides, herbicides, fungicides, safeners, fertilizers and/or growth regulators, for example as finished formulation or as tank mixes. The combination formulations can be prepared on the basis of the abovementioned formulations, while taking account of the physical properties and stabilities of the active compounds to be combined.

The weight ratios of herbicide (mixture) to safener depend generally on the herbicide application rate and the efficacy of the safener in question and may vary within wide limits, for example in the range from 200:1 to 1:200, preferably 100:1 to 1:100, in particular 20:1 to 1:20. Analogously to the compounds (G) or mixtures thereof, the safeners can be formulated with further herbicides/pesticides and be provided and employed as a finished formulation or tankmix with the herbicides.

For application, the herbicide or herbicide/safener formulations present in commercial form are, if appropriate, diluted in a customary manner, for example in the case of wettable powders, emulsifiable concentrates, dispersions and water-dispersible granules with water. Preparations in the form of dusts, granules for soil application or granules for broadcasting and sprayable solutions are usually not diluted further with other inert substances prior to application.

The application rate of the compounds of the formula (G) and/or salts thereof can vary within wide limits. For the application as herbicide for controlling harmful plants, for example, generally the range of from 0.001 to 10.0 kg/ha of active substance is suitable, preferably the compounds of the formula (G) and/or salts thereof are applied in the range of from 0.005 to 5 kg/ha, in particular in the range of from 0.01 to 1 kg/ha. This applies both to the pre-emergence and the post-emergence application.

When used as plant growth regulator, for example as culm stabilizer for crop plants like those mentioned above, preferably cereal plants, such as wheat, barley, rye, triticale, millet, rice or corn, the application rate of the compounds of the formula (G) and/or salts thereof is, for example, in the range of from 0.001 to 2 kg/ha or more of active substance, preferably in the range of from 0.005 to 1 kg/ha, in particular in the range of from 10 to 500 g/ha of active substance. This applies both to application by the pre-emergence method and the post-emergence method, the post-emergence treatment generally being preferred.

The application as culm stabilizer may take place at various stages of the growth of the plants. Preferred is, for example, the application after the tillering phase, at the beginning of the longitudinal growth.

As an alternative, application as plant growth regulator is also possible by treating the seed, which includes various techniques for dressing and coating seed. Here, the application rate depends on the particular techniques and can be determined in preliminary tests.

### Examples

In an exemplary manner, some synthesis examples of compounds of the general formula (G) are described below. In the examples, the amounts (including percentages) refer to the weight, unless especially stated otherwise.

The symbols ">" and "<" mean "greater than" and "smaller than", respectively. The symbol "≥" means "greater than or equal to", the symbol "≤" means "smaller than or equal to".

If, in the context of the description and the examples, the terms "R" and "S" are given for the absolute configuration on a centre of chirality of the stereoisomers of the formula (G), this RS nomenclature follows, unless defined differently, the Cahn-Ingold-Prelog rule.

In the context of the present invention and in the Tables 1 to 3 mentioning specific and preferred compounds according to the present invention, the following abbreviations are used:
- H: = hydrogen
- Me: = methyl or CH₃
- Et: = ethyl
- Pr: = propyl
- Bu: = butyl
- nAlkyl: = n-alkyl, e.g. nPr = n-propyl

- cAlkyl: = cycloalkyl, e.g. cPr = cyclopropyl, cHexyl = cyclohexyl
- iAlkyl: = isooalkyl, e.g. iPr = isopropyl
- tAlkyl: = tertiary alkyl, e.g. tBu = tert-butyl
- Ac: = acetyl
- F, Cl, Br, I: = fluorine, chlorine, bromine and iodine, respectively, in accordance with the conventional chemical atom symbol
- MeO or OMe: = methoxy
- CN: = cyano
- NO₂: = nitro
- Ph: = phenyl
- diHal: = diHal, e.g. diF = difluoro
- triHal: = triHal, e.g. triF = trifluoro
- -CCH: = ethinyl (-C≡CH)

The position of a substituent, e.g. at the phenyl ring in position 2, is stated as a prefix to the symbol or the abbreviation of the radical, for example
- 2-Cl: = 2-chloro
- 2-Me: = 2-methyl

Numerations of the substituent positions for di- or trisubstituted substitution patterns are analogously stated as a prefix, for example
- 2,3-Cl₂: = 2,3-dichloro (e.g. as substitution at the phenyl ring)
- 2,4-diF: = 2,4-difluoro (e.g. as substitution at the phenyl ring)
- 2,4-F₂: = 2,4-difluoro (e.g. as substitution at the phenyl ring)
- 2,4,6-triF: = 2,4,6-trifluoro (e.g. as substitution at the phenyl ring)
- 2-F-4-Cl: = 2-fluoro, 4-chloro (e.g. as substitution at the phenyl ring)
- 5-F-2-Me: = 5-fluoro, 2-methyl (e.g. as substitution at the phenyl ring)

Other abbreviations are to be understood analogously to the examples stated above.

In addition, the customary chemical symbols and formulae apply, such as, for example, CH₂ for methylene or CF₃ for trifluoromethyl or OH for hydroxyl.

Correspondingly, composite meanings are defined as composed of the abbreviations mentioned, for example
- 4-CF₃-cHexyl: = 4- trifluoromethyl-cyclohexyl

Further, the following abbreviations are used:
- DCM =: dichloromethane
- DMF =: dimethylformamide
- DMSO =: dimethylsulfoxide
- T3P =: propylphosphonic anhydride
- THF =: tetrahydrofuran

### NMR-Peak lists

1H-NMR data of selected examples are written in form of 1H-NMR-peak lists. To each signal peak are listed the δ-value in ppm and the signal intensity in round brackets. Between the δ-value - signal intensity pairs are semicolons as delimiters.

The peak list of an example has therefore the form:
δ1 (intensity 1); δ2 (intensity2);........; δi (intensityi);......; δn (intensityn)
Intensity of sharp signals correlates with the height of the signals in a printed example of a NMR spectrum in cm and shows the real relations of signal intensities. From broad signals several peaks or the middle of the signal and their relative intensity in comparison to the most intensive signal in the spectrum can be shown.

For calibrating chemical shift for 1H spectra, tetramethylsilane and/or the chemical shift of the solvent was used, especially in the case of spectra measured in DMSO (Dimethyl sulphoxide). Therefore in NMR peak lists, tetramethylsilane peak can occur, but not necessarily
The 1H-NMR peak lists are similar to classical 1H-NMR prints and contains therefore usually all peaks, which are listed at classical NMR-interpretation.

Additionally they can show like classical 1H-NMR prints signals of solvents, stereoisomers of the target compounds, which are also object of the invention, and/or peaks of impurities.

To show compound signals in the delta-range of solvents and/or water the usual peaks of solvents, for example peaks of DMSO in DMSO-D₆ and the peak of water are shown in our 1H-NMR peak lists and have usually on average a high intensity .

The peaks of stereoisomers of the target compounds and/or peaks of impurities have usually on average a lower intensity than the peaks of target compounds (for example with a purity >90%).

Such stereoisomers and/or impurities can be typical for the specific preparation process. Therefore their peaks can help to recognize the reproduction of our preparation process via "side-products-fingerprints".

An expert, who calculates the peaks of the target compounds with known methods (MestreC, ACD-simulation, but also with empirically evaluated expectation values) can isolate the peaks of the target compounds as needed optionally using additional intensity filters. This isolation would be similar to relevant peak picking at classical 1H-NMR interpretation.

Further details of NMR-data description with peak lists can be found in the publication "Citation of NMR Peaklist Data within Patent Applications" of the Research Disclosure Database Number 564025.

The compounds according to the present invention, such as described in the Tables 1 to 3, are obtained according to or analogously to the following chemical synthesis examples.

### (A) Chemical synthesis examples:

### 1. Synthesis of N-[6-(cyclopentylmethyl)-7-oxo-isothiazolo[5,4-c]pyridin-3-yl]propanamide (compound No. II-25) via the compound No. I-3):

The following scheme illustrates the steps (i) to (v) described in detail in the examples 1.1 to 1.5:

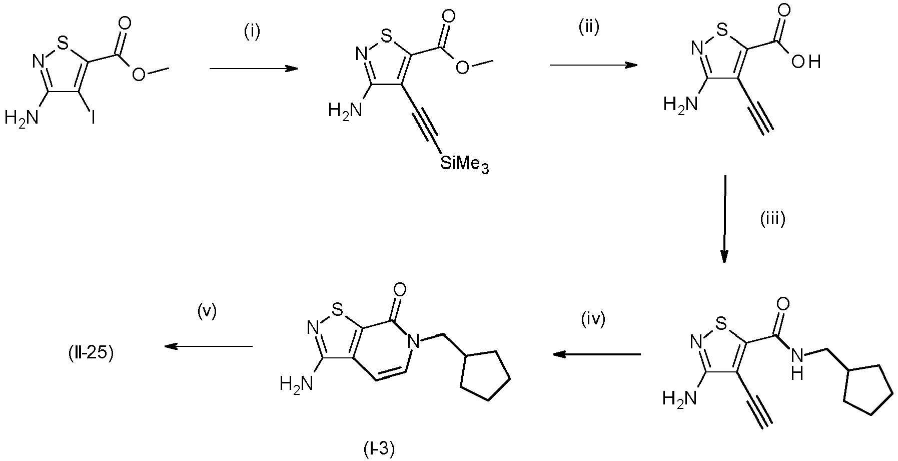

### 1.1 Step (i) = Synthesis of methyl 3-amino-4-(2-trimethylsilylethynyl)isothiazole-5-carboxylate:

To a stirred degassed solution of 650 mg of methyl 3-amino-4-iodo-isothiazole-5-carboxylate (2.3 mmol) in dry DMF (11 mL) were successively added 161 mg of palladium (II) diphenylphosphine dichloride Pd(PPh₂)₂Cl₂ (0.23 mmol), 44 mg of copper iodide CuI (0.23 mmol), 0.64 mL of triethylamine NEt₃ (4.6 mmol) and 0.65 mL of ethynyltrimethylsilane (4.6 mmol). The reaction mixture was then stirred at 100 °C for 1 h. The solution was cooled to room temperature (about 20 °C) and partitioned between a saturated aqueous solution of ammonium chloride and 10% dichloromethane in heptane. The aqueous phase was extracted twice with 10% dichloromethane in heptane and the combined organic extracts were dried over sodium sulphate, filtered, concentrated and purified by column chromatography. Yield: 486 mg (83% of theory).
¹H-NMR (400 MHz, CDCl₃ δ, ppm) 4.92 (br. s, 2H), 3.92 (s, 3H), 0.30 (s, 9H).

### 1.2 Step (ii) = Synthesis of 3-amino-4-ethynyl-isothiazole-5-carboxylic acid:

To a stirred solution of 8.0 g of methyl 3-amino-4-(2-trimethylsilylethynyl)isothiazole-5-carboxylate (31 mmol) in THF (80 mL) and MeOH (80 mL) was added 42 mL of a 2 M aqueous solution of sodium hydroxide (94 mmol). The reaction mixture was then stirred at room temperature (about 20 °C) for 2 h. The mixture was concentrated and the residue partitioned between a 2 M aqueous solution of HCl and EtOAc. The aqueous phase was extracted twice with EtOAc and the combined organic extracts were dried over sodium sulphate, filtered and concentrated. The corresponding acid was used in the next step without further purification. Yield: 5.2 g (99% of theory).
¹H-NMR (400 MHz, DMSO δ, ppm) 13.9 (br. s, 1H), 6.42 (br. s, 2H), 4.68 (s, 1H).

### 1.3 Step (iii) = Synthesis of 3-amino-N-(cyclopentylmethyl)-4-ethynyl-isothiazole-5-carboxamide:

To a stirred solution of 2.2 g of 3-amino-4-ethynyl-isothiazole-5-carboxylic acid (13 mmol) in THF (59 mL) was added successively 15 mL of T3P (26 mmol, 50% in THF), 5.4 mL of triethylamine NEt₃ (39 mmol) and 1.4 g of cyclopentylmethanamine (14 mmol). The reaction mixture was then stirred at 55 °C for 2 h. The mixture was concentrated and the residue partitioned between a 2 M aqueous solution of HCl and EtOAc. The aqueous phase was extracted twice with EtOAc and the combined organic extracts were dried over sodium sulphate, filtered, concentrated and purified by column chromatography. Yield: 1.6 g (49% of theory).
¹H-NMR (400 MHz, DMSO δ, ppm) 8.09 (t, 1H), 6.40 (br. s, 2H), 4.83 (s, 1H), 3.20 (t, 2H), 2.13-2.06 (m, 1H), 1.74-1.47 (m, 6H), 1.27-1.19 (m, 2H).

### 1.4 Step (iv) = Synthesis of 3-amino-6-(cyclopentylmethyl)isothiazolo[5,4-c]pyridin-7-one (compound No. 1-3):

To a stirred solution of 1.6 g of 3-amino-N-(cyclopentylmethyl)-4-ethynyl-isothiazole-5-carboxamide (6.4 mmol) in THF (65 mL) was added 13 mL of a 1 M solution of tetrabutylammonium fluoride in THF (13 mmol). The reaction mixture was then stirred at room temperature (about 20 °C) for 30 min. Water was added and the resulting precipitate was collected by filtration. Yield: 1.6 g (100% of theory).
¹H-NMR (400 MHz, DMSO δ, ppm) 7.61 (d, 1H), 6.87 (d, 1H), 6.83 (br. s, 2H), 3.92 (d, 2H), 2.38-2.31 (m, 1H), 1.65-1.49 (m, 6H), 1.29-1.24 (m, 2H).

### 1.5 Step (v) = Synthesis ofN-[6-(cyclopentylmethyl)-7-oxo-isothiazolo[5,4-c]pyridin-3-yl]propanamide (example II-25):

To a stirred solution of 120 mg of 3-amino-6-(cyclopentylmethyl)isothiazolo[5,4-c]pyridin-7-one (0.48 mmol) in DCM (6 mL) was added successively 5.9 mg of dimethylaminopyridine (0.048 mmol), 0.13 mL of triethylamine NEt₃ (0.96 mmol) and 0.089 mL of propionyl chloride (0.96 mmol). The reaction mixture was then stirred at room temperature (about 20 °C) for 3 h. The mixture was partitioned between water and EtOAc. The aqueous phase was extracted twice with EtOAc and the combined organic extracts were dried over sodium sulphate, filtered and concentrated. The residue was purified by HPLC. Yield: 81 mg (54% of theory).
¹H-NMR (400 MHz, DMSO δ, ppm) 10.80 (s, 1H), 7.64 (d, 1H), 6.78 (d, 1H), 3.95 (d, 2H), 2.47-2.41 (m, 2H), 2.37-2.32 (m, 1H), 1.64-1.49 (m, 6H), 1.30-1.24 (m, 2H), 1.11 (t, 3H).

### 2. Synthesis of 3-amino-6-[(4-chlorophenyl)methyl]isothiazolo[5,4-c]pyridin-7-one (compound No. I-17):

The following scheme illustrates the steps (i) to (v) described in detail in the examples 2.1 to 2.4:

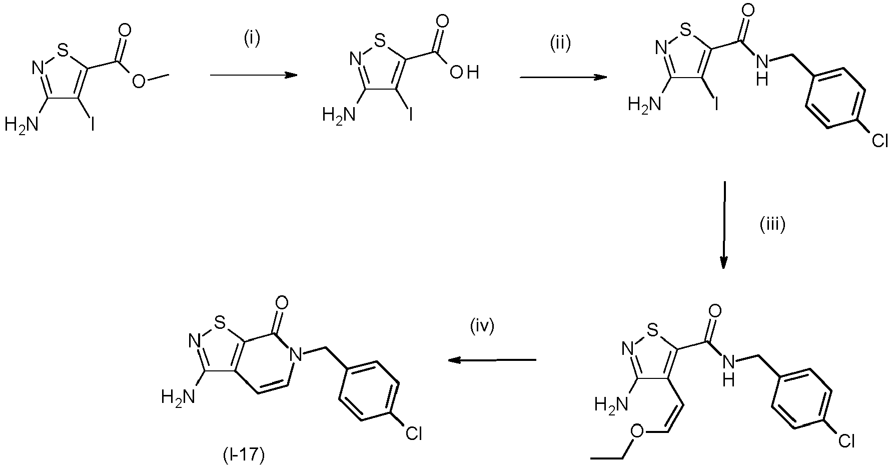

### 2.1 Step (i) = Synthesis of 3-amino-4-iodo-isothiazole-5-carboxylic acid:

To a stirred solution of 20.5 g of methyl 3-amino-4-iodo-isothiazole-5-carboxylate (72 mmol) in THF (150 mL) and MeOH (150 mL) was added 108 mL of a 2 M aqueous solution of sodium hydroxide (216 mmol). The reaction mixture was then stirred at room temperature (about 20 °C) for 2 h. The mixture was concentrated and the residue partitioned between a 2 M aqueous solution of HCl and EtOAc. The aqueous phase was extracted twice with EtOAc and the combined organic extracts were dried over sodium sulphate, filtered and concentrated. The corresponding acid was engaged in the next step without further purification. Yield: 18.9 g (97% of theory).
¹H-NMR (400 MHz, DMSO δ, ppm) 13.9 (br. s, 1H), 6.32 (br. s, 2H).

### 2.2 Step (ii) = Synthesis of 3-amino-N-[(4-chlorophenyl)methyl]-4-iodo-isothiazole-5-carboxamide:

To a stirred solution of 47 mg of 3-amino-4-iodo-isothiazole-5-carboxylic acid (0.17 mmol) in THF (1.2 mL) was added successively 0.26 mL of T3P (0.44 mmol, 50% in THF), 0.073 mL of triethylamine NEt₃ (0.52 mmol) and 0.053 mL of 4-chlorobenzylamine (0.44 mmol). The reaction mixture was then stirred at 55 °C for 2 h. The mixture was concentrated and the residue partitioned between a 2 M aqueous solution of HCl and EtOAc. The aqueous phase was extracted twice with EtOAc and the combined organic extracts were dried over sodium sulphate, filtered, concentrated and purified by column chromatography. Yield: 55 mg (80% of theory).
¹H-NMR (400 MHz, CDCl₃ δ, ppm) 7.36-7.27 (m, 4H), 6.73 (br. s, 1H), 4.84 (br. s, 2H), 4.63 (d, 2H).

### 2.3 Step (iii) = Synthesis of 3-amino-N-[(4-chlorophenyl)methyl]-4-[(Z)-2-ethoxyvinyl]isothiazole-5-carboxamide:

To a stirred degassed solution of 2.5 g of 3-amino-N-[(4-chlorophenyl)methyl]-4-iodo-isothiazole-5-carboxamide (6.4 mmol) in dry DMF (34 mL) were successively added 2.1 mL of tributyl-[(Z)-2-ethoxyvinyl]stannane (6.4 mmol) and 223 mg of palladium (II) diphenylphosphine dichloride Pd(PPh₂)₂Cl₂ (0.32 mmol). The reaction mixture was then stirred at 80 °C for 3 h. The solution was cooled to room temperature (about 20 °C), concentrated and the residue partitioned between a saturated aqueous solution of sodium hydrogenocarbonate and EtOAc. The aqueous phase was extracted twice with EtOAc and the combined organic extracts were dried over sodium sulphate, filtered, concentrated and purified by column chromatography. Yield: 1.1 g (51% of theory).
¹H-NMR (400 MHz, CDCl₃ δ, ppm) 7.34-7.25 (m, 4H), 6.58 (br. s, 1H), 6.32 (d, 1H), 5.35 (d, 1H), 4.90 (br. s, 2H), 4.56 (d, 2H), 3.93 (q, 2H), 1.26 (t, 3H).

### 2.4 Step (iv) = Synthesis of 3-amino-6-[(4-chlorophenyl)methyl]isothiazolo[5,4-c]pyridin-7-one (example 1-17):

To a stirred solution of 0.50 g of 3-amino-N-[(4-chlorophenyl)methyl]-4-[(Z)-2-ethoxyvinyl]isothiazole-5-carboxamide (1.5 mmol) in 1,4-dioxane (16 mL) was added 15 mL of a 2 M aqueous solution of HCl (30 mmol). The reaction mixture was then stirred at 100 °C for 1 h. The mixture was cooled to room temperature (about 20 °C) and the dioxane was removed. A 2 M aqueous solution of sodium hydroxide was then slowly added and the resulting precipitate was collected by filtration. Yield: 0.19 g (44% of theory).
¹H-NMR (400 MHz, DMSO δ, ppm) 7.73 (d, 1H), 7.41 (d, 2H), 7.34 (d, 2H), 6.94 (d, 1H), 6.88 (br. s, 2H), 5.19 (s, 2H).

### NMR peak lists

NMR peak lists for compounds according to formula (G) in the context of the present invention. The numbering refers to Tables 1 to 3 above.

| |
|---|
| Example I-4: ¹H-NMR(400,6 MHz, d₆-DMSO): δ= 7,555(10,1); 7,537(10,6); 6,881(11,5); 6,864(12,0); 6,849(13,6); 3,835(12,3); 3,817(12,4); 3,327(93,5); 2,891(0 ,6); 2,731(0,6); 2,676(0,9); 2,671(1,2); 2,667(0,9); 2,542(4,9); 2,525(6,2); 2,512(75,7); 2,507(153,8); 2,503(208,9); 2,498(147,7); 2,494(68,4); 2,476(1,5); 2,47 1(1,2); 2,334(0,9); 2,330(1,3); 2,325(0,8); 1,813(1,2); 1,803(1,4); 1,794(1,6); 1,785(2,0); 1,776(1,6); 1,767(1,4); 1,758(1,3); 1,677(3,4); 1,662(4,1); 1,610(2,7); 1,591(16,0); 1,559(4,1); 1,527(4,2); 1,148(7,6); 1,128(4,6); 1,032(1,6); 1,001(3,3); 0,973(2,7); 0,008(0,6); 0,000(18,5); -0,009(0,6) |
| Example I-22: ¹H-NMR(600,0 MHz, d₆-DMSO): δ= 7,670(1,7); 7,658(1,8); 7,300(0,6); 7,296(0,6); 7,288(1,0); 7,282(0,9); 7,278(1,4); 7,267(0,6); 7,263(1,0); 7,077 (0,5); 7,073(0,5); 7,063(0,9); 7,059(0,8); 7,048(0,4); 7,045(0,4); 6,946(2,5); 6,934(2,4); 6,883(3,5); 5,213(4,7); 3,568(1,2); 3,324(10,3); 2,614(0,3); 2,523(1,0); 2,520(1,2); 2,516(1,5); 2,508(19,8); 2,505(37,8); 2,502(50,0); 2,499(36,5); 2,496(17,5); 2,386(0,3); 1,572(1,7); 0,000(0,3) |
| Example I-17: ¹H-NMR(400,6 MHz, d₆-DMSO): δ = 7,740(6,7); 7,723(6,9); 7,422(7,2); 7,417(2,8); 7,406(4,0); 7,401(13,2); 7,395(2,0); 7,352(11,4); 7,331(6,0); 6, 947(7,1); 6,929(7,0); 6,882(9,0); 5,191(14,8); 4,038(0,5); 4,021(0,6); 3,335(34,0); 2,676(0,6); 2,671(0,8); 2,511(54,3); 2,507(104,6); 2,502(136,9); 2,498(96,2); 2,493(44,4); 2,334(0,7); 2,329(0,8); 1,989(2,4); 1,569(16,0); 1,193(0,6); 1,175(1,3); 1,157(0,6); 0,000(1,4) |
| Example I-45: ¹H-NMR(400.0 MHz, d₆-DMSO): δ= 7.537(8.0);7.519(8.5);7.440(0.6);6.896(9.3);6.878(9.3);6.853(10.1);4.487(0. 6);4.436(3.4);4.429(3.4);4.031(2.1);4.013(2.3);3.999(3.0);3.980(3.1);3.863(3.1);3.847(3.2);3.830(2.3);3.814(2.4);3.314(76.2);3. 182(2.3);3.162(2.0);3.141(2.4);2.675(1.1);2.670(1.5);2.666(1.1);2.536(0.7);2.523(4.9);2.519(7.0);2.510(87.5);2.506(186.2);2.5 01(256.1);2.496(180.5);2.492(82.6);2.468(1.4);2.456(1.8);2.451(2.1);2.447(1.5);2.400(0.6);2.332(1.3);2.328(1.8);2.323(1.4);2. 279(1.5);2.268(1.5);1.944(1.3);1.913(1.8);1.816(1.1);1.794(1.3);1.786(1.7);1.751(1.0);1.687(1.4);1.655(2.1);1.633(1.4);1.625(1 .6);1.592(2.8);1.569(2.6);1.494(2.1);1.462(1.5);1.439(1.3);1.406(1.9);1.378(3.3);1.370(3.2);1.357(1.9);1.338(2.5);1.320(3.5);1. 301(3.4);1.283(2.0);1.264(1.2);1.232(2.0);1.210(1.1);1.199(1.5);1.180(1.0);1.169(0.8);0.954(7.6);0.936(16.0);0.918(6.2);0.882( 0.5);0.864(0.9);0.008(0.8);0.000(23.7);-0.009(0.8) |
| Example III-2: ¹H-NMR(400,0 MHz, d₆-DMSO): δ= 7,684(13,2); 7,665(13,2); 6,938(14,4); 6,919(13,8); 6,826(16,0); 4,752(3,8); 4,723(2,7); 3,311(423,5); 2,670(3, 5); 2,523(11,1); 2,510(203,1); 2,505(437,8); 2,501(609,3); 2,496(428,6); 2,492(194,3); 2,327(3,2); 2,073(2,2); 1,861(4,8); 1,829(5,6); 1,741(8,9); 1,701(7,7); 1,671(6,9); 1,431(4,4); 1,400(4,6); 1,238(4,1); 1,206(2,7); 0,000(25,2) |
| Example II-2: ¹H-NMR(400.0 MHz, d₆-DMSO): δ= 10.857(4.1);8.316(8.2);7.589(5.9);7.571(6.3);7.554(0.7);7.536(0.7);6.869(0. 7);6.852(0.7);6.809(2.4);6.791(2.4);3.865(7.3);3.846(7.6);3.835(1.8);3.816(1.8);3.726(3.3);2.676(0.6);2.671(0.9);2.667(0.6);2.5 56(0.8);2.551(1.8);2.546(1.9);2.542(1.4);2.525(2.2);2.520(3.1);2.511(59.4);2.507(130.2);2.502(180.8);2.497(129.1);2.493(61.6 );2.463(4.3);2.459(4.3);2.454(3.9);2.449(3.1);2.445(2.6);2.439(2.4);2.333(1.0);2.329(1.3);2.324(1.0);2.159(16.0);2.075(0.6);1.9 89(0.6);1.909(9.7);1.822(0.7);1.813(0.8);1.803(1.0);1.794(1.2);1.785(1.1);1.775(0.9);1.767(0.9);1.755(0.7);1.663(2.6);1.611(1. 7);1.572(2.6);1.542(2.6);1.235(1.0);1.193(0.8);1.175(2.0);1.153(4.5);1.133(3.0);1.042(1.0);1.014(2.0);0.985(1.7);0.049(0.7);0.0 08(2.0);0.000(69.4);-0.009(2.6);-0.049(0.6) |
| Example I-34: ¹H-NMR(400.0 MHz, d₆-DMSO): δ= 7.677(6.1);7.675(6.0);7.659(6.6);7.657(6.0);7.630(2.1);7.613(2.2);7.604(2.7 );7.589(2.8);7.579(2.1);7.562(2.0);7.412(1.7);7.395(2.0);7.389(2.0);7.385(1.9);7.372(1.9);7.368(2.0);7.362(1.7);7.345(1.5);6.95 1(12.5);6.933(12.3);6.888(12.0);5.199(16.0);3.321(57.6);2.675(1.3);2.671(1.9);2.666(1.4);2.610(0.6);2.551(0.7);2.524(4.8);2.5 20(7.0);2.511(104.8);2.506(228.7);2.502(317.6);2.497(221.8);2.492(98.3);2.456(1.4);2.452(1.7);2.447(1.3);2.333(1.5);2.328(2. 0);2.324(1.4);2.074(1.4);1.989(1.3);1.535(8.5);1.175(0.7);0.008(2.3);0.000(90.0);-0.009(2.8) |
| Example II-145: ¹H-NMR(400.0 MHz, d₆-DMSO): δ= 12.450(4.8);8.314(12.9);7.654(12.8);7.636(13.6);6.740(15.9);6.722(16.0); 3.888(12.5);3.870(12.7);3.379(1.1);3.329(322.7);3.279(2.9);2.676(0.9);2.671(1.3);2.666(1.0);2.551(0.7);2.525(3.7);2.520(5.2); 2.511(75.0);2.507(164.9);2.502(232.8);2.497(162.3);2.493(71.5);2.461(0.9);2.457(1.6);2.452(2.2);2.447(1.7);2.443(1.0);2.338( 0.6);2.333(1.0);2.329(1.5);2.324(1.1);2.074(0.7);1.989(0.6);1.829(1.1);1.821(1.3);1.811(1.5);1.802(1.9);1.794(1.6);1.784(1.3);1 .775(1.3);1.666(3.8);1.614(2.4);1.577(4.0);1.547(3.9);1.279(0.8);1.247(3.5);1.175(2.3);1.157(7.2);1.136(4.5);1.051(1.5);1.020( 3.1);0.992(2.5);0.875(1.8);0.858(7.3);0.841(2.4);0.000(4.2) |
| Example I-37: ¹H-NMR(400.0 MHz, d₆-DMSO): δ= 7.701(6.4);7.683(6.9);7.322(1.1);7.316(1.2);7.298(2.5);7.275(2.6);7.268(1.6 );7.255(1.2);7.250(1.2);7.119(1.1);7.113(1.2);7.098(2.5);7.077(2.1);7.062(0.9);6.961(11.8);6.943(11.6);6.894(12.2);5.257(16.0) ;4.020(0.5);3.568(4.7);3.320(70.8);2.675(1.3);2.671(1.8);2.666(1.2);2.524(4.7);2.519(7.2);2.511(102.5);2.506(221.1);2.502(305 .9);2.497(215.7);2.492(98.5);2.452(2.1);2.393(0.9);2.333(1.5);2.328(2.0);2.324(1.5);1.989(1.9);1.536(10.9);1.193(0.5);1.175(1. 1);0.008(4.0);0.000(137.0);-0.009(4.7);-0.050(0.6) |
| Example I-50: ¹H-NMR(400.0 MHz, d₆-DMSO): δ= 7.540(5.8);7.522(6.1);6.882(6.8);6.864(6.8);6.842(8.4);4.275(1.9);4.264(2.0 );4.243(2.2);4.232(2.1);3.699(2.1);3.673(2.3);3.667(2.1);3.641(1.9);3.387(0.9);3.337(215.8);3.287(2.5);2.672(0.5);2.526(1.7);2. 512(30.2);2.508(62.5);2.503(84.6);2.499(60.7);2.494(28.5);2.458(0.8);2.453(1.0);2.330(0.5);1.989(1.5);1.674(1.2);1.636(1.7);1 .617(2.3);1.592(6.6);1.519(1.1);1.503(1.0);1.493(1.2);1.468(0.5);1.327(1.2);1.295(1.3);1.272(0.5);1.247(1.2);1.230(1.3);1.220( 1.4);1.206(1.7);1.193(1.2);1.175(2.1);1.158(0.8);1.144(0.6);1.054(1.1);1.029(1.7);1.009(16.0);0.993(14.9);0.968(2.5);0.939(1.4 );0.000(1.6) |
| Example I-49: ¹H-NMR(400.0 MHz, d₆-DMSO): δ= 7.570(6.1);7.552(6.3);6.887(7.0);6.869(6.9);6.841(8.7);3.936(0.8);3.920(1.0 );3.903(3.5);3.889(5.1);3.868(3.0);3.836(0.8);3.332(155.0);3.283(2.1);2.676(0.8);2.671(0.9);2.552(0.6);2.525(3.4);2.511(59.2); 2.507(121.6);2.502(163.8);2.498(117.1);2.493(54.6);2.457(1.5);2.452(1.8);2.448(1.4);2.329(1.0);2.324(0.8);1.989(1.5);1.790(1. 3);1.783(1.3);1.773(1.2);1.620(1.2);1.576(10.5);1.478(0.8);1.429(5.0);1.417(3.3);1.401(1.9);1.348(1.3);1.334(1.4);1.320(1.3);1. 284(1.1);1.261(2.4);1.250(2.9);1.237(3.1);1.203(1.2);1.193(1.0);1.175(1.2);1.147(0.9);1.009(0.6);0.993(0.6);0.959(16.0);0.942( 15.4);0.000(8.3) |
| Example II-128: ¹H-NMR(400.0 MHz, d₆-DMSO): δ= 11.752(1.9);8.314(16.0);7.647(8.3);7.628(8.6);6.871(0.6);6.757(3.4);6.739 (3.2);6.649(0.8);6.516(1.6);6.383(0.8);3.880(8.4);3.862(8.5);3.371(1.0);3.357(0.5);3.321(243.6);3.271(2.0);2.676(0.7);2.671(1. 1);2.666(0.8);2.524(2.4);2.520(3.6);2.511(57.9);2.506(128.5);2.502(181.2);2.497(126.3);2.493(55.8);2.456(1.2);2.452(1.5);2.44 7(1.0);2.333(0.8);2.329(1.1);2.324(0.8);2.183(1.1);1.829(0.7);1.820(0.8);1.810(1.0);1.801(1.3);1.792(1.0);1.782(0.9);1.773(0.8) ;1.681(2.1);1.666(2.6);1.614(1.6);1.578(2.7);1.547(2.6);1.355(9.3);1.179(1.4);1.157(4.8);1.136(3.0);1.047(1.0);1.019(2.1);0.99 1(1.7);0.000(2.3) |
| Example II-43: ¹H-NMR(400.0 MHz, d₆-DMSO): δ= 10.817(2.9);7.585(4.2);7.567(4.4);6.753(2.4);6.735(2.3);3.866(4.2);3.847(4 .3);3.317(116.1);3.267(0.9);2.675(0.5);2.670(0.8);2.666(0.5);2.524(1.8);2.519(2.7);2.511(41.7);2.506(91.9);2.501(129.2);2.497 (90.0);2.492(39.6);2.461(0.6);2.456(1.0);2.451(1.4);2.447(1.3);2.442(2.2);2.424(3.6);2.405(2.1);2.333(0.6);2.328(0.8);2.324(0. 6);2.183(0.9);1.803(0.5);1.794(0.7);1.785(0.5);1.776(0.5);1.766(0.5);1.760(0.6);1.688(1.3);1.669(3.4);1.651(5.0);1.632(4.4);1.6 14(3.0);1.596(1.1);1.571(1.4);1.541(1.4);1.355(7.2);1.153(2.4);1.132(1.5);1.044(0.5);1.014(1.1);0.985(0.9);0.964(7.6);0.946(16 .0);0.937(0.8);0.927(6.7);0.008(1.9);0.004(0.6);0.000(77.5);-0.009(2.4);-0.050(0.7) |
| Example II-26: ¹H-NMR(400.0 MHz, d₆-DMSO): δ= 10.801(2.8);8.315(0.7);7.583(4.1);7.565(4.2);6.777(2.5);6.759(2.4);3.866(4 .3);3.848(4.4);3.317(118.1);3.268(1.2);2.671(0.6);2.524(1.8);2.519(2.9);2.511(34.1);2.506(72.9);2.501(101.0);2.497(71.0);2.49 2(32.4);2.474(3.5);2.456(3.8);2.447(1.1);2.437(1.2);2.328(0.6);1.803(0.5);1.795(0.7);1.786(0.6);1.663(1.4);1.612(0.9);1.573(1. 4);1.539(1.5);1.154(2.6);1.127(7.8);1.108(16.0);1.089(6.7);1.042(0.6);1.014(1.2);0.986(1.0);0.008(1.4);0.006(0.5);0.005(0.6);0. 000(43.8);-0.006(0.8);-0.007(0.7);-0.009(1.4) |
| Example II-298: ¹H-NMR(400.6 MHz, d₆-DMSO): δ= 7.953(0.5);7.664(0.9);7.646(0.9);6.572(1.0);6.554(1.1);3.887(0.9);3.869(0 .9);3.533(16.0);2.891(4.0);2.732(3.4);2.731(3.5);2.511(12.3);2.507(25.8);2.502(35.7);2.498(25.0);2.493(11.2);1.755(0.8);1.152 (0.6);0.000(2.9) |
| Example II-315: ¹H-NMR(400.6 MHz, d₆-DMSO): δ= 12.389(2.9);9.987(1.3);8.314(8.3);7.812(2.6);7.792(3.5);7.673(8.9);7.655( 9.4);7.601(2.4);7.580(1.9);6.514(10.9);6.496(11.2);3.888(9.6);3.870(9.9);3.432(3.7);3.381(9.7);3.357(41.4);3.332(2825.1);3.30 3(15.2);3.280(4.4);3.232(2.7);2.890(1.3);2.675(3.4);2.671(4.9);2.666(3.7);2.532(20.3);2.511(314.6);2.507(664.7);2.502(918.9); 2.497(649.3);2.493(295.7);2.480(7.0);2.475(5.3);2.470(4.3);2.334(4.1);2.329(5.9);2.325(4.2);2.179(1.5);1.909(1.4);1.798(1.9); 1.754(8.5);1.668(3.9);1.576(4.1);1.548(3.8);1.316(2.5);1.307(8.0);1.300(3.3);1.292(8.8);1.235(16.0);1.153(7.4);1.017(3.1);0.99 6(2.7);0.854(2.6);0.000(55.5) |
| Example II-152: ¹H-NMR(400.0 MHz, d₆-DMSO): δ= 12.454(4.1);7.631(13.8);7.613(14.4);6.756(16.0);6.737(15.4);4.459(5.7);4. 079(4.1);4.061(3.9);4.047(5.2);4.028(5.4);3.909(5.2);3.892(5.5);3.876(3.7);3.859(3.8);3.359(2.0);3.311(840.3);3.261(6.6);3.21 1(1.7);2.674(5.5);2.670(7.9);2.665(5.6);2.535(2.6);2.523(20.5);2.518(29.8);2.510(427.2);2.505(934.9);2.500(1305.1);2.496(907 .4);2.491(397.7);2.455(8.3);2.450(11.5);2.446(8.4);2.441(4.5);2.400(1.7);2.332(6.6);2.327(8.9);2.323(6.4);2.073(3.1);1.950(2.2 );1.908(3.1);1.818(1.7);1.790(2.8);1.754(1.7);1.692(2.3);1.660(3.4);1.628(2.4);1.596(3.0);1.499(3.1);1.452(2.5);1.419(3.3);1.38 5(4.9);1.308(1.3);1.297(1.8);1.259(2.5);1.236(5.1);1.206(2.3);0.146(2.3);0.008(19.5);0.000(738.8);-0.009(20.7);-0.033(1.3);-0.050(5.0);-0.150(2.0) |
| Example II-196: ¹H-NMR(400.0 MHz, d₆-DMSO): δ= 10.768(5.7);8.313(1.7);7.603(7.1);7.585(7.8);7.551(2.5);7.534(2.7);6.869( 2.5);6.851(2.6);6.831(2.9);6.773(4.8);6.755(4.8);4.168(16.0);4.118(0.9);3.873(8.2);3.854(8.7);3.835(3.2);3.817(3.2);3.444(0.9); 3.394(60.7);3.366(4.7);3.344(2.3);3.316(260.5);3.281(4.6);3.266(11.8);3.233(2.4);2.670(0.9);2.556(1.9);2.551(2.4);2.547(1.6); 2.524(1.4);2.511(56.2);2.506(121.3);2.502(169.7);2.497(128.2);2.492(68.0);2.461(8.7);2.456(10.2);2.452(11.1);2.447(9.1);2.33 3(2.4);2.328(2.5);2.073(0.9);1.806(1.5);1.797(1.9);1.789(1.8);1.779(1.6);1.769(1.5);1.664(4.5);1.611(3.2);1.572(4.2);1.540(4.5) ;1.299(0.8);1.259(1.0);1.236(2.4);1.151(7.1);1.133(5.3);1.015(3.3);0.988(3.1);0.854(0.9);0.000(11.7) |
| Example II-162: ¹H-NMR(400.0 MHz, d₆-DMSO): δ= 12.543(3.7);8.313(1.0);7.665(13.2);7.647(14.0);6.594(15.5);6.576(15.6);3. 888(15.2);3.869(16.0);3.367(11.0);3.317(794.8);3.267(31.9);3.235(6.8);2.670(2.6);2.555(5.1);2.551(6.2);2.524(4.6);2.510(158. 2);2.506(340.8);2.501(481.2);2.497(362.6);2.492(192.3);2.460(24.6);2.456(28.2);2.451(30.3);2.447(24.9);2.328(5.8);2.073(2.0) ;2.013(1.0);1.800(2.8);1.791(2.6);1.665(6.2);1.612(4.4);1.576(6.9);1.544(6.9);1.235(2.3);1.154(10.5);1.135(7.5);1.019(5.1);0.9 91(4.5);0.000(11.1) |
| Example II-332: ¹H-NMR(400.0 MHz, d₆-DMSO): δ= 7.681(2.5);7.663(2.6);6.764(2.5);6.746(2.4);5.754(1.4);3.889(2.6);3.871(2 .7);3.309(66.4);3.259(0.5);2.679(0.6);2.674(1.2);2.670(1.8);2.665(1.2);2.572(1.7);2.554(6.1);2.536(6.7);2.523(5.5);2.518(9.0);2 .510(98.9);2.505(214.0);2.500(297.1);2.496(206.6);2.491(91.1);2.460(1.3);2.455(2.0);2.451(2.4);2.446(1.6);2.332(1.4);2.327(1. 8);2.323(1.3);2.318(0.7);1.671(0.9);1.591(1.0);1.560(0.9);1.159(1.6);1.141(1.1);1.019(7.2);1.001(16.0);0.983(6.7);0.008(4.0);0. 000(159.8);-0.009(5.2);-0.050(0.9);-0.150(0.6) |
| Example I-46: ¹H-NMR(400.0 MHz, d₆-DMSO): δ= 8.313(0.8);7.537(13.1);7.520(13.8);7.459(0.8);7.440(0.8);6.893(15.8);6.876 (15.8);6.850(16.0);4.484(0.9);4.429(5.3);4.031(3.5);4.012(3.7);3.998(4.7);3.980(5.1);3.863(4.9);3.846(5.1);3.830(3.7);3.814(3. 8);3.317(450.6);3.282(1.9);3.267(4.6);3.218(1.0);3.183(1.3);3.163(1.2);3.139(1.2);2.675(2.2);2.670(3.0);2.666(2.3);2.524(8.9); 2.519(12.9);2.510(177.1);2.506(383.9);2.501(534.1);2.497(375.5);2.492(170.5);2.456(3.4);2.451(4.8);2.446(3.8);2.420(1.4);2.3 33(2.8);2.328(3.4);2.278(2.4);2.268(2.4);2.073(0.9);1.944(2.1);1.912(2.9);1.814(1.8);1.786(2.7);1.757(1.5);1.750(1.7);1.690(2. 2);1.655(3.4);1.624(2.4);1.591(3.2);1.560(2.2);1.494(3.3);1.461(2.3);1.438(2.0);1.406(3.0);1.378(4.9);1.370(4.8);1.338(2.0);1.3 20(2.0);1.301(2.0);1.283(1.7);1.242(4.7);1.231(3.5);1.221(2.2);1.210(1.7);1.199(2.4);0.954(3.9);0.936(8.1);0.918(3.1);0.875(1. 8);0.858(5.9);0.841(2.3);0.008(5.7);0.000(199.7);-0.009(6.5);-0.050(1.4);-0.150(0.7) |
| Example I-3: ¹H-NMR(400.0 MHz, d₆-DMSO): δ= 7.618(13.1);7.600(13.8);6.880(15.9);6.862(16.0);6.832(12.3);6.731(0.9);5.53 3(0.8);5.525(0.8);5.207(0.7);5.200(0.8);3.930(15.3);3.911(15.6);3.597(1.1);3.578(1.2);3.312(81.9);3.262(0.5);2.675(0.8);2.671( 1.1);2.666(0.7);2.524(2.8);2.520(4.1);2.511(59.4);2.506(129.4);2.502(179.9);2.497(123.9);2.492(54.3);2.472(0.7);2.468(0.7);2. 457(1.1);2.452(1.2);2.447(0.8);2.381(1.1);2.363(2.4);2.344(3.1);2.333(1.5);2.324(2.7);2.307(1.0);1.989(0.8);1.651(2.3);1.630(5 .0);1.622(5.1);1.614(6.8);1.603(4.9);1.596(4.0);1.584(4.3);1.564(2.6);1.533(1.6);1.525(1.9);1.513(3.2);1.506(3.1);1.501(3.7);1. 489(3.8);1.452(0.7);1.286(3.1);1.271(3.6);1.255(3.0);1.241(2.4);1.175(0.7);1.170(1.3);1.157(0.5);0.936(0.6);0.008(1.4);0.000(4 9.4);-0.009(1.4) |
| Example I-88: ¹H-NMR(400.0 MHz, d₆-DMSO): δ= 7.456(13.8);7.438(14.9);6.919(15.3);6.901(15.0);6.868(13.9);6.777(0.8);5.5 37(0.7);5.530(0.7);4.994(0.7);4.987(0.6);4.792(16.0);4.485(1.0);3.602(0.7);3.314(31.9);2.672(0.5);2.525(1.5);2.520(2.3);2.512( 31.7);2.507(68.5);2.502(94.9);2.498(66.1);2.493(29.7);2.470(0.6);2.453(0.5);2.383(3.4);2.367(6.9);2.363(5.4);2.357(4.1);2.352 (3.8);2.334(0.9);2.329(0.9);2.325(0.7);1.867(3.3);1.852(7.2);1.838(4.5);1.760(0.9);1.664(1.5);1.656(1.7);1.649(4.0);1.640(4.2); 1.635(6.2);1.626(4.6);1.620(6.3);1.610(2.2);1.604(2.5);1.545(2.5);1.540(2.2);1.531(6.5);1.525(4.8);1.516(6.4);1.510(4.5);1.502 (4.1);1.487(1.3);0.008(1.4);0.000(47.8);-0.009(1.5) |
| Example II-342: ¹H-NMR(400.0 MHz, CDCl₃): δ= 7.370(2.4);7.352(2.6);7.261(45.7);6.349(3.0);6.331(2.9);5.299(1.4);4.262(0.7) :4.255(0.7);4.166(0.6);4.152(0.7);4.133(0.8);4.119(0.8);3.827(0.8);3.806(0.8);3.794(0.7);3.772(0.7);2.632(0.8);2.626(0.7);2.613(2.9);2.608(2.5);2.595(3.0);2.590(2.6);2.577(0.9);2.572(0.9);1.784(0.6);1.748(0.6);1.564(0.7);1.551(1.4);1.544(13.9);1.155(7. 0);1.137(16.0);1.119(7.0);0.008(0.5);0.000(19.3);-0.009(0.6) |
| Example II-36: ¹H-NMR(400.0 MHz, CDCl₃): δ= 7.294(0.7);7.276(0.8);7.260(67.0);1.540(16.0);1.307(1.7);1.288(3.5);1.269(1.8) ;0.882(0.6);0.008(0.8);0.000(28.8);-0.009(0.8) |
| Example II-206: ¹H-NMR(400.0 MHz, d₆-DMSO): δ= 10.779(4.8);7.675(0.7);7.657(0.7);7.588(7.4);7.569(7.7);6.870(0.8);6.852( 0.8);6.794(4.6);6.776(4.5);4.452(2.6);4.446(2.7);4.269(3.4);4.167(16.0);4.068(1.8);4.049(2.1);4.035(2.5);4.016(2.6);3.897(2.5); 3.881(2.6);3.864(1.8);3.847(1.8);3.394(73.3);3.344(0.7);3.310(143.3);3.261(14.7);3.256(2.0);2.674(1.2);2.670(1.8);2.665(1.3); 2.523(4.6);2.518(6.6);2.510(95.5);2.505(208.4);2.501(289.8);2.496(200.0);2.491(87.6);2.460(1.4);2.455(2.1);2.451(2.5);2.446( 1.8);2.332(1.7);2.327(2.2);2.323(1.8);2.318(1.2);2.298(1.2);2.287(1.2);2.073(0.6);1.952(1.0);1.916(1.5);1.822(0.9);1.792(1.5);1 .756(0.9);1.692(1.1);1.656(1.7);1.636(1.1);1.627(1.2);1.595(1.4);1.563(0.8);1.497(1.7);1.462(1.1);1.448(1.1);1.417(1.7);1.390( 2.6);1.381(2.5);1.246(2.8);1.237(2.8);1.204(1.1);1.175(0.5);0.875(1.0);0.858(4.0);0.841(1.3);0.008(2.5);0.000(84.7);-0.009(2.5);-0.050(0.6) |
| Example II-274: ¹H-NMR(400.0 MHz, CDCl₃): δ= 7.304(0.9);7.286(1.0);7.262(51.8);6.809(1.1);6.791(1.1);5.299(1.6);4.260(0.9) ;4.253(0.9);4.147(0.7);4.133(0.8);4.114(0.9);4.100(0.9);3.832(0.9);3.811(0.9);3.799(0.8);3.778(0.8);3.736(16.0);3.731(1.6);3.7 16(0.7);3.710(12.2);3.703(0.7);3.701(0.8);3.699(0.7);3.692(1.1);3.686(1.2);3.681(0.6);3.673(0.7);3.666(0.6);3.662(0.8);3.024(0 .6);2.803(1.3);2.792(1.5);2.787(1.4);2.726(0.6);2.719(0.7);2.712(0.7);2.705(2.0);2.702(1.3);2.691(1.8);2.689(2.1);2.668(1.7);2. 666(1.5);2.655(1.1);2.652(1.5);2.645(0.6);2.061(0.5);1.778(0.7);1.770(0.7);1.762(0.6);1.753(0.8);1.747(0.8);1.739(0.6);1.723(1 .1);1.697(0.6);1.557(1.1);1.550(1.0);1.541(1.9);1.533(1.6);1.519(1.1);0.008(0.6);0.000(22.4);-0.009(0.7) |
| Example II-172: ¹H-NMR(400.0 MHz, CDCl₃): δ= 7.519(1.4);7.351(3.3);7.333(3.6);7.275(0.5);7.274(0.6);7.2734(0.6);7.2727(0. 7);7.272(0.8);7.271(0.8);7.2703(1.0);7.2695(1.1);7.269(1.1);7.268(1.4);7.267(1.7);7.2663(2.0);7.2655(2.3);7.265(3.0);7.264(3. 8);7.263(5.3);7.260(244.4);7.257(4.5);7.256(3.1);7.255(2.3);7.254(1.8);7.253(1.4);7.2524(1.1);7.2516(1.0);7.251(0.9);7.250(0. 7);7.249(0.6);7.248(0.6);7.210(1.8);6.996(1.4);6.749(4.0);6.730(3.8);4.240(1.0);4.163(0.9);4.149(1.0);4.130(1.2);4.116(1.1);3.8 49(1.2);3.828(1.2);3.816(1.0);3.795(1.0);2.044(0.9);1.777(0.7);1.758(0.6);1.752(0.6);1.729(1.0);1.702(0.6);1.539(16.0);1.259(0 .6);0.008(2.9);0.006(0.7);0.0054(0.7);0.0046(0.9);0.000(113.5);-0.003(4.9);-0.004(2.0);-0.005(1.5);-0.006(1.2);-0.007(1.1);-0.009(3.4);-0.011(0.5);-0.050(0.8) |
| Example II-161: ¹H-NMR(400.0 MHz, d₆-DMSO): δ= 12.543(3.5);7.736(12.7);7.718(13.3);6.611(15.5);6.593(16.0);3.984(14.4); 3.965(14.7);3.663(1.3);3.623(1.2);3.619(2.1);3.616(1.6);3.612(1.5);3.608(1.7);3.602(3.8);3.596(1.7);3.592(1.3);3.588(1.3);3.58 5(1.9);3.510(0.5);2.672(0.7);2.525(1.7);2.520(2.6);2.512(40.9);2.507(90.2);2.502(126.9);2.498(89.7);2.493(40.7);2.457(0.9);2. 452(1.2);2.447(0.9);2.393(1.2);2.374(2.4);2.356(3.1);2.337(2.5);2.329(1.5);2.325(1.1);2.319(1.3);1.910(2.7);1.777(1.3);1.769(1 .2);1.760(3.9);1.756(0.8);1.752(1.2);1.744(1.3);1.651(3.7);1.640(6.5);1.635(5.9);1.630(7.1);1.623(6.2);1.614(4.2);1.606(4.4);1. 594(3.6);1.586(2.3);1.579(2.2);1.560(0.8);1.550(1.2);1.508(3.9);1.496(4.0);1.488(2.5);1.356(1.1);1.319(1.2);1.304(2.7);1.299(2 .9);1.285(3.7);1.267(3.6);1.259(2.9);1.235(4.9);0.854(0.7);0.008(1.0);0.000(37.3);-0.009(1.2) |
| Example I-5: ¹H-NMR(400.0 MHz, d₆-DMSO): δ= 7.577(12.5);7.559(13.2);6.879(16.0);6.861(16.0);6.834(13.9);3.831(14.8);3.8 12(14.9);3.314(47.1);2.672(0.7);2.525(2.1);2.520(3.0);2.512(38.4);2.507(83.2);2.503(115.3);2.498(80.0);2.493(35.3);2.457(0.8 );2.452(1.0);2.448(0.7);2.334(0.5);2.329(0.7);2.325(0.5);2.075(0.7);2.032(1.2);2.027(1.2);2.018(1.6);2.009(2.1);1.999(1.7);1.98 5(1.3);1.760(0.5);1.661(1.3);1.643(3.0);1.635(3.1);1.627(3.2);1.618(3.1);1.610(3.4);1.602(3.3);1.589(6.2);1.578(4.1);1.559(5.3) ;1.553(6.3);1.543(6.1);1.535(5.1);1.528(5.4);1.519(5.3);1.502(5.0);1.495(4.1);1.485(3.5);1.478(3.8);1.472(4.8);1.463(4.1);1.45 2(2.4);1.448(2.4);1.439(3.0);1.429(1.4);1.417(1.0);1.381(1.8);1.356(3.6);1.340(2.6);1.330(3.3);1.325(3.4);1.307(1.7);1.301(1.8) ;1.225(3.2);1.206(4.3);1.200(5.4);1.182(2.4);1.173(4.7);1.167(3.4);1.148(2.0);1.141(1.7);0.008(0.9);0.000(31.8);-0.009(0.9) |
| Example II-369: ¹H-NMR(400.0 MHz, CDCl₃): δ= 8.036(1.7);7.261(85.2);7.211(0.5);7.198(3.6);7.180(4.0);6.847(1.0);6.829(0.9) ;5.299(12.2);4.908(8.7);2.572(1.1);2.443(1.7);2.427(3.2);2.411(1.7);2.044(1.8);2.029(3.4);2.014(1.9);1.741(0.8);1.725(2.1);1.7 17(1.9);1.711(3.0);1.702(2.0);1.696(3.0);1.687(0.9);1.680(1.1);1.618(1.3);1.612(1.1);1.603(3.2);1.597(2.1);1.588(3.2);1.582(2. 0);1.574(2.2);1.567(1.2);1.556(8.3);1.302(7.8);1.284(16.0);1.265(7.6);0.069(0.7);0.008(1.5);0.000(37.0);-0.009(1.3) |
| Example II-370: ¹H-NMR(400.0 MHz, CDCl₃): δ= 7.263(27.3);7.213(0.6);7.210(0.7);7.192(0.7);6.839(1.4);6.821(1.3);5.300(5.8) ;4.910(2.8);3.731(11.3);3.714(1.5);3.709(16.0);3.698(0.7);3.693(1.4);3.684(1.0);3.681(0.7);3.675(0.6);3.665(0.9);2.796(0.9);2. 785(1.1);2.780(1.0);2.724(0.7);2.718(0.9);2.710(1.0);2.704(2.5);2.700(1.7);2.690(2.3);2.687(2.6);2.665(2.2);2.662(1.9);2.652(1 .3);2.648(1.9);2.642(0.8);2.634(0.6);2.628(0.5);2.443(0.6);2.427(1.1);2.412(0.6);2.039(0.6);2.023(1.1);2.018(0.7);2.008(0.6);1. 725(0.7);1.717(0.7);1.711(1.0);1.702(0.7);1.696(1.0);1.602(1.0);1.596(0.7);1.588(1.0);1.582(0.7);1.574(0.7);0.000(12.4) |
| Example II-195: ¹H-NMR(400.0 MHz, d₆-DMSO): δ= 10.768(4.6);7.718(0.6);7.700(0.6);7.670(7.1);7.652(7.6);6.917(0.8);6.899( 0.7);6.785(4.6);6.767(4.5);5.334(1.6);4.348(0.8);4.169(16.0);4.002(0.5);3.967(9.9);3.948(10.0);3.929(2.1);3.911(0.7);3.601(0.7 );3.508(1.3);3.441(0.6);3.394(64.5);3.388(6.4);3.345(0.5);3.331(0.9);3.297(0.8);3.290(2.7);3.249(0.8);2.675(0.6);2.671(0.9);2.6 66(0.6);2.524(1.3);2.519(2.6);2.511(63.1);2.506(141.6);2.501(200.2);2.497(143.5);2.492(67.6);2.456(2.4);2.451(2.5);2.393(1.3 );2.374(2.0);2.355(2.5);2.337(2.3);2.328(2.1);2.323(1.7);2.319(1.5);2.179(0.6);1.988(0.7);1.909(9.0);1.760(0.8);1.636(4.6);1.62 6(5.4);1.611(3.9);1.599(3.6);1.573(2.0);1.507(3.1);1.494(3.3);1.356(0.9);1.298(3.6);1.282(3.4);1.266(3.6);1.259(4.8);1.235(12. 6);0.906(0.6);0.890(0.7);0.869(0.8);0.854(2.1);0.836(1.1);0.807(0.6);0.008(1.5);0.000(53.4);-0.009(1.9) |
| Example II-168: ¹H-NMR(400.0 MHz, d₆-DMSO): δ= 12.572(0.8);7.767(1.9);7.749(1.9);7.635(0.6);7.618(0.7);7.609(0.9);7.593( 0.9);7.583(0.7);7.566(0.6);7.494(0.6);7.471(0.7);7.454(0.7);7.444(0.6);7.426(0.5);6.670(4.1);6.652(4.0);5.248(5.2);4.056(1.1);4 .038(3.4);4.021(3.4);4.003(1.1);3.313(73.7);3.264(0.6);2.675(0.7);2.670(1.0);2.665(0.7);2.524(3.0);2.519(4.3);2.510(53.0);2.50 6(113.7);2.501(158.0);2.496(109.3);2.492(47.7);2.456(1.0);2.451(1.2);2.447(0.8);2.332(0.7);2.328(1.0);2.323(0.7);1.988(16.0); 1.235(1.1);1.193(4.5);1.175(9.3);1.157(4.6);0.008(1.2);0.000(40.9);-0.009(1.2) |
| Example II-202: ¹H-NMR(400.0 MHz, d₆-DMSO): δ= 10.803(4.8);7.713(4.3);7.711(4.3);7.695(4.6);7.693(4.3);7.631(1.4);7.614( 1.5);7.605(2.0);7.589(2.0);7.580(1.5);7.563(1.3);7.474(1.2);7.456(1.4);7.451(1.5);7.446(1.4);7.434(1.4);7.429(1.4);7.424(1.3);7 .407(1.2);6.845(4.9);6.827(4.8);5.235(11.5);4.168(16.0);3.509(0.5);3.394(65.9);3.344(0.5);3.314(39.3);2.671(0.6);2.525(2.2);2. 520(3.0);2.511(33.2);2.507(70.7);2.502(97.2);2.498(67.3);2.493(29.5);2.457(0.6);2.453(0.7);2.329(0.6);1.300(0.5);1.259(1.0);1 .235(4.0);0.854(0.7);0.008(1.1);0.000(34.5);-0.009(1.0) |
| Example II-385: ¹H-NMR(400.0 MHz, d₆-DMSO): δ= 7.697(1.4);7.679(1.5);6.768(1.6);6.750(1.6);4.039(1.9);4.021(6.0);4.003(6 .0);3.986(1.9);3.891(1.3);3.873(1.3);3.311(18.3);2.604(1.3);2.586(2.8);2.568(1.5);2.524(0.7);2.519(1.0);2.510(11.2);2.506(24.0 );2.501(33.1);2.496(23.0);2.492(10.1);2.316(2.0);2.297(4.6);2.279(2.3);1.798(0.6);1.780(1.8);1.762(2.5);1.743(1.7);1.671(0.5); 1.593(0.6);1.561(0.6);1.181(0.5);1.174(0.5);1.157(8.1);1.139(16.0);1.121(7.1);0.008(0.6);0.000(21.2);-0.009(0.7) |
| Example II-365: ¹H-NMR(400.0 MHz, d₆-DMSO): δ= 11.702(1.2);9.764(0.7);7.634(4.9);7.615(4.9);6.778(1.5);4.489(0.7);4.471( 0.8);4.329(5.9);4.312(5.8);4.056(0.6);4.038(1.9);4.021(1.9);4.003(0.6);3.878(15.9);3.860(16.0);3.362(1.1);3.311(372.4);3.261( 2.0);2.675(2.2);2.670(3.1);2.665(2.3);2.524(9.3);2.519(13.1);2.510(174.9);2.506(377.6);2.501(527.0);2.496(370.9);2.492(170.2 );2.456(2.4);2.451(3.1);2.446(2.4);2.397(0.6);2.332(2.4);2.328(3.2);2.323(2.3);1.988(8.5);1.817(1.8);1.808(2.0);1.799(2.6);1.79 1(2.1);1.772(1.7);1.665(5.6);1.613(3.7);1.576(5.7);1.546(5.6);1.389(0.9);1.371(1.9);1.312(6.9);1.248(4.8);1.193(3.9);1.175(7.8) ;1.157(12.2);1.135(6.5);1.018(4.4);0.989(3.8);0.875(2.2);0.858(8.4);0.841(2.9);0.008(3.6);0.000(138.3);-0.009(4.8);-0.050(0.7) |
| Example II-213: ¹H-NMR(400.0 MHz, d₆-DMSO): δ= 10.984(1.9);7.614(2.3);7.595(2.4);6.770(0.9);6.752(0.9);5.755(1.7);3.870( 2.6);3.852(2.7);3.404(2.4);3.314(12.6);2.520(0.7);2.512(7.9);2.507(16.8);2.503(23.1);2.498(16.7);2.493(8.0);2.202(16.0);1.665 (1.0);1.612(0.6);1.576(1.0);1.545(1.0);1.178(0.5);1.155(1.7);1.135(1.1);1.017(0.8);0.988(0.6);0.000(6.7) |
| Example II-111: ¹H-NMR(400.0 MHz, d₆-DMSO): δ= 11.254(11.0);7.635(14.5);7.617(15.2);6.734(2.7);6.716(2.6);5.755(0.5);3.8 72(15.6);3.854(16.0);3.712(2.9);3.685(2.9);3.314(80.9);2.676(0.7);2.671(1.0);2.667(0.7);2.539(0.5);2.534(0.8);2.525(2.8);2.52 0(3.9);2.511(58.6);2.507(129.0);2.502(182.7);2.498(129.4);2.493(59.6);2.462(1.0);2.457(1.2);2.452(1.4);2.448(1.0);2.334(0.9); 2.329(1.2);2.324(0.8);2.074(1.0);1.835(0.8);1.826(1.4);1.817(1.7);1.807(2.0);1.798(2.5);1.790(2.1);1.780(1.8);1.771(1.7);1.761 (1.0);1.680(4.2);1.665(5.1);1.658(4.8);1.612(3.2);1.601(3.1);1.575(5.3);1.543(5.2);1.155(9.5);1.135(5.8);1.045(2.0);1.016(4.1); 0.988(3.3);0.008(2.2);0.000(82.1);-0.009(2.7) |
| Example II-60: ¹H-NMR(400.0 MHz, d₆-DMSO): δ= 11.223(7.3);7.628(9.8);7.610(10.3);6.794(4.1);6.775(4.0);5.754(16.0);4.449 (9.9);3.872(10.9);3.854(11.2);3.314(59.9);2.675(0.6);2.671(0.8);2.666(0.6);2.524(1.4);2.520(2.3);2.511(47.7);2.506(105.4);2.5 02(148.7);2.497(105.6);2.493(48.4);2.465(0.5);2.461(0.6);2.456(1.0);2.452(1.2);2.447(0.9);2.333(0.7);2.329(1.0);2.324(0.7);1. 827(1.0);1.818(1.2);1.808(1.3);1.799(1.8);1.790(1.4);1.781(1.3);1.771(1.1);1.665(3.6);1.612(2.2);1.602(2.2);1.575(3.8);1.544(3 .7);1.234(0.9);1.177(1.9);1.155(6.7);1.135(4.2);1.047(1.4);1.017(2.9);0.988(2.4);0.008(1.5);0.000(54.3);-0.009(1.8) |
| Example II-94: ¹H-NMR(400.0 MHz, d₆-DMSO): δ= 11.283(8.3);11.224(0.6);7.630(11.8);7.612(12.4);6.768(3.9);6.750(3.8);5.75 5(16.0);4.664(0.6);4.448(0.8);4.198(8.9);3.871(13.1);3.853(13.4);3.319(52.0);2.676(0.7);2.671(1.0);2.666(0.7);2.525(2.4);2.52 0(3.5);2.511(56.2);2.507(124.4);2.502(177.1);2.497(126.7);2.493(59.7);2.456(1.5);2.452(1.5);2.447(1.1);2.384(0.9);2.333(0.8); 2.329(1.2);2.324(0.9);1.827(1.2);1.818(1.5);1.808(1.7);1.799(2.1);1.790(1.8);1.780(1.5);1.771(1.4);1.680(3.6);1.664(4.4);1.612 (2.8);1.576(4.6);1.544(4.5);1.235(1.6);1.178(2.6);1.155(8.1);1.135(5.3);1.046(1.8);1.016(3.6);0.988(3.0);0.008(2.0);0.000(77.6) ;-0.009(2.9) |
| Example II-366: ¹H-NMR(400.0 MHz, d₆-DMSO): δ= 11.110(5.3);7.625(6.1);7.607(6.3);6.790(0.9);4.269(0.9);4.251(0.9);4.247( 1.2);4.229(1.2);4.167(2.3);4.150(6.8);4.132(7.2);4.125(2.0);4.115(3.0);4.107(4.0);4.098(0.6);4.089(3.8);4.071(1.2);3.869(7.2);3 .851(7.5);3.836(1.5);3.782(1.6);3.775(0.8);3.695(0.7);3.613(2.9);2.525(1.3);2.520(1.8);2.511(24.1);2.507(52.2);2.502(73.0);2.4 98(50.6);2.493(22.1);1.827(0.6);1.818(0.8);1.808(0.9);1.799(1.1);1.790(0.9);1.780(0.8);1.771(0.7);1.680(1.8);1.665(2.3);1.612( 1.5);1.574(2.4);1.543(2.4);1.279(1.2);1.262(2.9);1.258(1.6);1.244(1.6);1.241(3.4);1.231(7.8);1.223(2.4);1.213(16.0);1.202(5.6); 1.195(8.3);1.192(6.0);1.184(10.5);1.174(3.1);1.166(5.7);1.155(4.5);1.134(2.7);1.046(0.9);1.016(1.9);0.987(1.5);0.008(1.3);0.00 0(51.0);-0.009(1.5) |
| Example II-247: ¹H-NMR(400.0 MHz, d₆-DMSO): δ= 11.117(2.6);7.622(3.0);7.603(3.1);5.755(2.2);3.869(3.6);3.851(3.6);3.679( 16.0);3.636(1.6);3.314(17.6);2.525(0.5);2.520(0.8);2.511(10.7);2.507(23.1);2.502(32.4);2.498(22.6);2.493(10.1);1.799(0.6);1.6 80(0.9);1.664(1.2);1.612(0.7);1.602(0.7);1.574(1.2);1.544(1.2);1.178(0.7);1.154(2.2);1.134(1.4);1.016(0.9);0.987(0.8);0.008(0. 6);0.000(21.5);-0.009(0.7) |
| Example II-368: ¹H-NMR(400.0 MHz, d₆-DMSO): δ= 10.845(3.7);7.588(3.5);7.570(3.7);6.778(2.3);6.760(2.2);4.096(2.2);4.078( 6.9);4.060(7.0);4.042(2.4);3.867(4.5);3.849(4.6);3.315(17.5);2.525(2.7);2.512(14.8);2.507(29.1);2.503(36.0);2.498(25.2);2.494 (12.3);2.401(3.1);2.382(6.5);2.364(3.5);1.899(0.8);1.881(2.9);1.862(4.0);1.844(2.8);1.825(1.0);1.814(0.7);1.804(0.7);1.795(0.9) ;1.786(0.7);1.776(0.6);1.768(0.6);1.679(1.5);1.664(1.8);1.611(1.2);1.572(1.9);1.541(1.8);1.207(7.8);1.189(16.0);1.171(8.6);1.1 53(3.4);1.134(2.1);1.046(0.7);1.014(1.4);0.985(1.2);0.008(0.7);0.000(14.9);-0.009(0.6) |
| Example II-281: ¹H-NMR(400.0 MHz, d₆-DMSO): δ= 10.846(1.6);7.588(1.9);7.570(2.0);6.777(1.0);6.759(1.0);5.755(0.8);3.867( 2.0);3.849(2.1);3.607(16.0);3.316(8.6);2.526(0.9);2.521(0.8);2.513(5.5);2.508(12.1);2.503(15.8);2.499(10.8);2.494(5.0);2.422( 1.5);2.404(3.2);2.385(1.7);1.885(1.3);1.867(1.9);1.849(1.3);1.679(0.6);1.664(0.7);1.572(0.8);1.541(0.8);1.154(1.3);1.133(0.8);1 .015(0.6);0.000(1.0) |
| Example II-386: ¹H-NMR(400.0 MHz, d₆-DMSO): δ= 7.698(0.9);7.680(1.0);6.767(1.0);6.749(1.0);3.892(0.9);3.874(0.9);3.554(1 6.0);3.313(7.4);2.607(0.9);2.589(1.9);2.571(1.0);2.511(3.9);2.506(8.6);2.502(11.9);2.497(8.4);2.492(3.7);2.338(1.3);2.320(3.0); 2.301(1.5);1.782(1.2);1.764(1.7);1.746(1.1);1.161(0.6);0.000(0.8) |
| Example II-367: ¹H-NMR(400.0 MHz, d₆-DMSO): δ= 10.933(2.5);7.591(3.0);7.573(3.1);6.759(1.1);6.741(1.0);4.098(1.9);4.080(6.1);4.062(6.2);4.044(2.0);3.864(3.3);3.846(3.3);3.314(19.6);2.756(0.7);2.741(1.5);2.724(1.1);2.639(2.2);2.627(1.8);2.622(2.8); 2.606(1.1);2.525(0.6);2.520(0.8);2.511(11.2);2.507(24.3);2.502(33.9);2.498(23.7);2.493(10.5);1.796(0.5);1.678(0.9);1.662(1.1) ;1.611(0.7);1.569(1.1);1.538(1.1);1.205(7.5);1.188(16.0);1.170(7.7);1.153(2.1);1.132(1.3);1.012(0.9);0.984(0.7);0.000(2.3) |
| Example II-264: ¹H-NMR(400.0 MHz, d₆-DMSO): δ= 10.941(1.7);7.593(1.9);7.575(2.0);6.761(0.7);6.743(0.6);3.865(2.1);3.846( 2.2);3.612(16.0);3.317(6.7);2.754(1.0);2.737(0.8);2.658(1.5);2.645(1.2);2.641(1.8);2.624(0.7);2.513(4.5);2.508(9.5);2.503(13.1 );2.499(9.2);2.494(4.1);1.678(0.6);1.663(0.7);1.571(0.8);1.539(0.8);1.153(1.4);1.132(0.9);1.013(0.6);0.000(7.9) |
| Example II-387: ¹H-NMR(400.0 MHz, CDCl₃): δ= 7.260(35.1);7.187(1.2);7.169(1.3);6.573(1.5);6.555(1.4);5.299(0.6);3.866(1.3) ;3.848(1.3);3.682(16.0);2.890(0.7);2.875(1.2);2.859(0.9);2.680(1.5);2.664(1.7);2.649(1.1);1.735(0.7);0.000(15.1) |
| Example II-77: ¹H-NMR(400.0 MHz, d₆-DMSO): δ= 11.108(7.3);7.626(14.4);7.608(15.0);6.841(3.9);6.824(3.6);5.243(6.6);5.127 (6.7);4.056(0.8);4.038(2.5);4.020(2.5);4.003(0.8);3.873(15.9);3.855(16.0);3.313(190.2);3.262(1.7);2.675(1.7);2.670(2.4);2.665( 1.7);2.524(8.7);2.519(11.9);2.510(136.5);2.506(292.3);2.501(409.0);2.496(283.1);2.492(123.7);2.455(2.4);2.451(3.5);2.446(2.6 );2.442(1.5);2.332(1.8);2.328(2.5);2.323(1.7);2.073(0.7);1.988(11.4);1.827(1.4);1.818(1.7);1.808(2.0);1.799(2.6);1.790(2.0);1.7 81(1.7);1.772(1.6);1.665(5.0);1.613(3.3);1.575(5.2);1.542(5.0);1.245(3.7);1.193(4.2);1.175(9.4);1.157(11.9);1.134(5.8);1.044(2 .0);1.017(4.1);0.989(3.3);0.875(1.8);0.858(7.2);0.840(2.5);0.008(2.6);0.000(86.1);-0.009(2.4);-0.050(0.6) |
| Example I-56: ¹H-NMR(400.0 MHz, d₆-DMSO): δ= 7.451(5.9);7.446(6.3);7.433(6.7);7.428(6.4);6.888(15.1);6.870(16.0);6.858( 14.5);6.747(1.3);5.554(0.6);5.550(0.6);5.230(0.6);4.274(13.5);4.219(13.7);3.859(1.1);3.805(1.1);3.313(78.9);3.263(0.5);2.676( 0.6);2.671(0.9);2.666(0.6);2.524(1.8);2.520(3.0);2.511(48.8);2.507(108.3);2.502(153.5);2.497(110.2);2.493(52.8);2.452(1.9);2. 333(0.7);2.329(1.0);2.324(0.8);1.623(4.5);1.602(4.9);1.569(7.8);1.562(7.5);1.535(7.7);1.486(4.1);1.459(4.9);1.444(4.0);1.415(2 .8);1.273(1.7);1.247(1.7);0.008(2.9);0.000(105.6);-0.009(3.9);-0.050(0.5) |
| Example II-3: ¹H-NMR(400.0 MHz, d₆-DMSO): δ= 10.853(3.8);7.613(5.7);7.595(6.0);6.818(2.3);6.800(2.3);5.755(9.2);3.860(6. 6);3.841(6.8);3.317(16.2);2.522(0.6);2.513(10.9);2.508(24.3);2.504(34.2);2.499(23.8);2.494(10.5);2.162(16.0);2.042(0.6);2.038 (0.6);2.028(0.7);2.019(1.0);2.009(0.8);1.999(0.7);1.995(0.6);1.665(0.5);1.647(1.2);1.640(1.3);1.631(1.4);1.623(1.3);1.614(1.5); 1.600(3.6);1.582(1.2);1.573(2.2);1.566(2.2);1.556(2.8);1.547(2.3);1.539(1.9);1.530(1.8);1.521(2.3);1.505(2.3);1.497(1.7);1.488 (1.5);1.481(1.7);1.475(2.1);1.466(1.9);1.455(1.0);1.451(1.1);1.442(1.4);1.433(0.6);1.387(0.8);1.362(1.6);1.355(1.4);1.344(1.1); 1.330(1.6);1.312(0.8);1.308(0.8);1.237(1.7);1.218(1.9);1.212(2.6);1.195(1.1);1.186(2.3);1.180(1.5);1.161(0.9);1.154(0.8);0.008 (0.6);0.000(21.1);-0.009(0.6) |
| Example II-32: ¹H-NMR(400.0 MHz, d₆-DMSO): δ= 10.828(2.9);7.693(2.5);7.675(2.7);7.632(0.8);7.615(0.9);7.607(1.2);7.590(1 .2);7.581(0.8);7.564(0.7);7.465(0.7);7.447(0.9);7.420(0.8);7.397(0.7);6.848(2.9);6.830(2.8);5.229(6.7) ;3.311 (82.8);3.262(0.8);2 .675(1.4);2.670(2.0);2.665(1.5);2.523(5.9);2.519(9.0);2.510(106.2);2.505(227.9);2.501(318.0);2.496(225.8);2.492(106.5);2.475 (8.5);2.456(6.7);2.451(4.1);2.446(2.8);2.437(1.7);2.332(1.4);2.328(1.9);2.323(1.4);1.298(0.7);1.259(1.3);1.235(3.6);1.126(7.1); 1.107(16.0);1.088(7.0);0.854(0.5);0.146(0.6);0.008(5.7);0.000(217.4);-0.009(8.4);-0.050(1.7);-0.150(0.8) |
| Example II-25: ¹H-NMR(400.0 MHz, d₆-DMSO): δ= 10.797(2.5);8.314(4.5);7.649(4.1);7.631(4.4);6.790(2.6);6.772(2.6);3.961(4 .9);3.942(5.0);3.508(2.7);2.675(0.6);2.670(0.9);2.665(0.6);2.524(1.9);2.519(2.6);2.510(48.0);2.506(106.4);2.501(150.2);2.496( 104.9);2.492(46.2);2.476(3.7);2.457(4.1);2.451(1.9);2.446(1.3);2.438(1.3);2.371(0.8);2.353(1.1);2.333(1.3);2.328(1.2);2.323(0. 8);2.318(0.5);1.636(1.9);1.625(2.2);1.598(1.4);1.571(0.7);1.505(1.2);1.494(1.2);1.298(1.0);1.281(1.2);1.266(1.2);1.235(1.5);1.1 28(7.0);1.109(16.0);1.090(6.8);0.008(3.0);0.006(0.8);0.000(109.7);-0.009(3.1);-0.050(0.7) |
| Example II-331: ¹H-NMR(400.0 MHz, d₆-DMSO): δ= 8.314(0.7);7.749(2.6);7.731(2.7);6.777(2.6);6.760(2.5);3.985(2.9);3.966(3 .0);3.350(16.1);2.674(1.0);2.670(1.5);2.665(1.1);2.571(1.6);2.553(6.1);2.535(6.2);2.523(3.5);2.518(5.7);2.510(79.8);2.505(176. 6);2.501(249.0);2.496(173.8);2.491(77.2);2.455(2.3);2.450(2.7);2.446(2.1);2.401(0.6);2.383(0.6);2.364(0.7);2.346(0.6);2.332(1 .3);2.327(1.8);2.323(1.2);1.643(1.6);1.510(0.9);1.499(1.0);1.296(0.9);1.278(0.9);1.259(0.9);1.236(2.2);1.108(0.9);1.019(7.0);1. 001(16.0);0.983(6.7);0.008(4.6);0.006(1.4);0.000(163.9);-0.009(4.6);-0.050(1.3);-0.149(0.5) |
| Example II-177: ¹H-NMR(400.0 MHz, d₆-DMSO): δ= 12.569(2.2);7.553(7.1);7.534(7.7);6.623(11.1);6.605(10.9);4.319(16.0);4.2 65(15.8);4.040(0.5);4.022(0.5);3.316(17.1);2.671(1.0);2.552(4.1);2.507(108.2);2.502(141.6);2.499(109.0);2.429(1.2);2.329(1.0 );1.989(2.1);1.647(8.2);1.630(7.8);1.621(7.7);1.592(7.0);1.567(11.1);1.543(13.2);1.512(6.5);1.482(6.5);1.451(5.9);1.424(4.9);1. 279(5.3);1.247(12.7);1.194(1.2);1.176(1.6);1.158(0.9);0.875(4.6);0.858(12.2);0.841(5.7);0.000(23.4) |
| ExampleII-112:¹H-NMR(400.0 MHz,d₆-DMSO): δ=11.258(10.1);7.662(12.6);7.644(13.2);6.741(2.4);6.724(2.4);5.755(16.0);3. 867(14.3);3.848(14.6);3.712(2.6);3.688(2.7);3.316(43.6);2.672(0.7);2.525(1.8);2.521(2.5);2.512(36.7);2.507(81.4);2.503(114.2 );2.498(80.4);2.494(37.0);2.453(0.8);2.334(0.5);2.330(0.7);2.325(0.5);2.044(1.3);2.030(1.7);2.020(2.2);2.011(1.8);1.666(1.3);1. 648(2.9);1.641(3.1);1.632(3.3);1.624(3.2);1.601(8.3);1.574(5.3);1.567(5.3);1.558(6.6);1.548(5.5);1.540(4.6);1.531(4.5);1.522(5 .6);1.506(5.5);1.499(4.2);1.490(3.7);1.483(4.1);1.476(5.0);1.468(4.4);1.457(2.5);1.452(2.7);1.444(3.3);1.434(1.5);1.424(1.1);1. 388(1.9);1.363(3.7);1.332(3.8);1.314(2.0);1.310(2.0);1.240(3.8);1.220(4.4);1.215(5.9);1.197(2.6);1.188(5.1);1.163(2.3);1.156(1 .9);0.008(1.3);0.000(47.6);-0.005(1.0);-0.007(0.7);-0.009(1.6) |
| Example II-146: ¹H-NMR(400.0 MHz, d₆-DMSO): δ= 12.443(4.2);7.680(12.4);7.661(13.0);6.748(15.5);6.730(16.0);5.755(11.5); 3.884(12.9);3.865(13.4);3.317(29.1);2.676(0.6);2.671(0.8);2.666(0.6);2.525(1.6);2.520(2.3);2.511(44.6);2.507(100.7);2.502(14 3.4);2.497(103.3);2.493(49.3);2.333(0.8);2.329(1.1);2.324(0.8);2.049(1.1);2.034(1.5);2.025(2.0);2.015(1.7);1.668(1.1);1.650(2. 5);1.643(2.8);1.634(2.9);1.626(2.8);1.604(7.2);1.577(4.9);1.570(4.5);1.560(5.5);1.550(4.7);1.543(4.1);1.532(4.1);1.523(5.1);1.5 07(5.1);1.500(3.9);1.491(3.5);1.484(3.8);1.478(4.7);1.469(4.1);1.458(2.4);1.454(2.6);1.445(3.1);1.435(1.6);1.422(1.2);1.390(1. 8);1.365(3.3);1.334(3.5);1.311(1.9);1.244(3.4);1.225(3.9);1.219(5.3);1.201(2.5);1.192(4.7);1.167(2.6);1.161(2.2);0.008(1.3);0.0 00(49.3);-0.009(1.7) |
| Example II-27: ¹H-NMR(400.0 MHz, d₆-DMSO): δ= 10.800(2.7);7.608(3.8);7.590(4.0);6.785(2.7);6.767(2.7);5.756(0.9);3.862(4 .6);3.843(4.7);3.315(9.2);2.521(0.5);2.512(10.9);2.507(24.5);2.503(34.9);2.498(25.0);2.493(11.8);2.477(3.4);2.458(3.5);2.439( 1.2);2.027(0.5);2.017(0.7);2.007(0.6);1.648(0.9);1.641(0.9);1.632(1.0);1.623(1.0);1.615(1.1);1.599(2.5);1.581(0.9);1.572(1.5);1 .566(1.6);1.556(2.0);1.547(1.7);1.538(1.4);1.531(1.4);1.521(1.7);1.505(1.7);1.498(1.3);1.489(1.1);1.482(1.2);1.476(1.5);1.467( 1.3);1.456(0.7);1.452(0.8);1.443(1.0);1.387(0.6);1.362(1.1);1.355(1.0);1.344(0.8);1.330(1.2);1.312(0.6);1.308(0.6);1.238(1.2);1 .218(1.4);1.213(1.8);1.195(0.8);1.186(1.6);1.180(1.1);1.161(0.7);1.154(0.6);1.128(7.1);1.110(16.0);1.091(6.9);0.008(0.5);0.000 (22.5);-0.009(0.7) |
| Example II-333: ¹H-NMR(400.0 MHz, CDCl₃): δ= 7.261(29.1);7.176(2.6);7.158(2.7);6.335(2.9);6.317(2.8);3.872(3.6);3.853(3.7) ;2.629(1.9);2.610(6.3);2.592(6.5);2.574(2.1);1.714(1.5);1.694(1.5);1.686(1.6);1.673(0.9);1.619(0.5);1.611(0.6);1.594(0.7);1.57 9(0.8);1.566(0.6);1.551(2.3);1.540(0.7);1.532(0.7);1.525(0.9);1.517(0.7);1.500(0.6);1.493(0.6);1.438(0.9);1.415(0.8);1.293(0.7) ;1.267(1.4);1.259(0.8);1.241(1.4);1.216(0.5);1.153(7.5);1.135(16.0);1.117(7.3);0.000(11.1) |
| Example II-388: ¹H-NMR(400.0 MHz, d₆-DMSO): δ= 10.936(2.4);7.617(2.8);7.599(2.9);6.765(1.0);6.747(1.0);5.755(1.9);4.098( 1.8);4.080(6.0);4.062(6.0);4.044(1.9);3.859(3.2);3.840(3.3);3.313(9.5);2.756(0.7);2.741(1.4);2.725(1.1);2.639(2.1);2.627(1.7);2 .622(2.8);2.606(1.1);2.511(9.6);2.507(21.6);2.502(30.7);2.498(21.6);2.493(9.7);2.018(0.5);1.646(0.7);1.640(0.7);1.631(0.7);1.6 22(0.7);1.614(0.8);1.597(1.9);1.570(1.1);1.563(1.2);1.554(1.5);1.546(1.3);1.536(1.0);1.531(1.1);1.521(1.3);1.504(1.2);1.497(1. 0);1.488(0.8);1.481(0.9);1.475(1.2);1.466(1.0);1.455(0.6);1.451(0.6);1.442(0.8);1.362(0.9);1.344(0.7);1.330(0.9);1.236(0.9);1.2 17(1.0);1.206(7.8);1.188(16.0);1.170(7.1);1.159(0.6);1.152(0.5);0.008(0.5);0.000(20.9);-0.009(0.7) |
| Example II-197: ¹H-NMR(400.0 MHz, d₆-DMSO): δ= 10.769(5.3);7.629(7.8);7.611(8.2);6.781(4.8);6.763(4.7);5.755(9.0);4.170( 16.0);3.868(8.2);3.849(8.4);3.395(78.3);3.345(0.5);3.314(22.5);2.525(0.6);2.520(0.9);2.512(18.1);2.507(40.4);2.502(57.3);2.49 8(40.0);2.493(17.7);2.453(0.6);2.044(0.7);2.030(0.9);2.020(1.2);2.010(1.0);1.666(0.7);1.648(1.6);1.641(1.7);1.632(1.7);1.624(1 .7);1.615(1.9);1.600(4.5);1.573(2.8);1.567(2.8);1.557(3.6);1.548(2.9);1.539(2.4);1.531(2.4);1.522(3.0);1.505(3.0);1.498(2.3);1. 489(2.0);1.482(2.2);1.476(2.8);1.467(2.4);1.456(1.3);1.452(1.4);1.443(1.8);1.433(0.8);1.422(0.6);1.387(1.0);1.362(2.0);1.345(1 .4);1.331(2.1);1.313(1.1);1.308(1.1);1.239(2.1);1.220(2.4);1.214(3.3);1.197(1.4);1.187(2.9);1.182(2.0);1.163(1.3);1.156(1.0);0. 008(1.2);0.000(44.1);-0.009(1.2) |
| Example II-350: ¹H-NMR(400.0 MHz, CDCl₃): δ= 7.261(15.9);7.187(1.2);7.169(1.2);6.358(1.4);6.340(1.3);3.876(1.5);3.857(1.6) ;2.323(16.0);1.713(0.6);1.686(0.6);1.553(0.9);1.268(0.6);1.242(0.6);0.000(6.2) |
| Example II-163: ¹H-NMR(400.0 MHz, d₆-DMSO): δ= 12.539(0.8);7.689(7.7);7.671(8.1);6.602(8.8);6.584(9.1);3.883(8.7);3.864( 8.9);3.318(3.7);2.526(0.9);2.521(1.3);2.513(19.2);2.508(42.7);2.503(60.5);2.499(42.9);2.494(20.0);2.458(0.5);2.454(0.5);2.075 (16.0);2.046(0.8);2.032(1.0);2.022(1.3);2.012(1.1);1.999(0.9);1.667(0.8);1.649(1.8);1.642(2.0);1.633(2.0);1.625(2.0);1.603(5.0) ;1.576(3.3);1.570(3.0);1.559(3.6);1.548(3.1);1.542(2.7);1.531(2.7);1.522(3.5);1.506(3.4);1.499(2.6);1.490(2.3);1.483(2.6);1.47 7(3.1);1.468(2.7);1.457(1.6);1.453(1.7);1.444(2.1);1.434(1.0);1.423(0.7);1.389(1.2);1.365(2.3);1.357(2.1);1.333(2.4);1.314(1.3) ;1.259(0.6);1.243(2.5);1.223(2.7);1.218(3.7);1.200(1.7);1.191(3.1);1.166(1.3);1.159(1.1);0.008(1.5);0.004(0.5);0.000(55.0);-0.007(0.9);-0.009(1.9) |
| Example II-389: ¹H-NMR(400.0 MHz, CDCl₃): δ= 9.056(0.9);7.263(31.8);7.101(4.2);7.082(5.0);6.891(3.8);6.873(3.2);5.299(5.2) ;4.150(10.1);3.867(5.7);3.849(5.8);3.727(2.3);3.710(7.3);3.692(7.5);3.675(2.4);1.874(0.6);1.866(0.5);1.746(0.9);1.740(0.9);1.7 18(2.8);1.683(1.9);1.584(0.6);1.344(7.8);1.327(16.0);1.309(7.6);1.256(0.8);1.242(0.8);1.234(0.8);1.205(2.0);1.180(1.4);1.048(1 .1);1.025(0.9);0.070(2.8);0.000(11.8) |
| Example II-390: ¹H-NMR(400.0 MHz, CDCl₃): δ= 7.261(50.1);7.155(2.2);7.137(2.4);6.347(2.7);6.329(2.7);4.355(13.2);3.880(2. 4);3.862(2.4);3.511(2.1);3.494(7.4);3.476(7.5);3.459(2.3);1.722(0.7);1.714(0.8);1.677(0.9);1.261(0.6);1.254(0.7);1.243(0.8);1.2 07(1.0);1.185(0.7);1.133(7.5);1.116(16.0);1.098(7.5);1.045(0.6);0.000(20.0);-0.009(0.6) |
| Example II-41: ¹H-NMR(400.0 MHz, d₆-DMSO): δ= 10.821(3.3);7.476(2.1);7.471(2.2);7.457(2.3);7.453(2.2);6.792(3.0);6.774(2 .9);4.300(4.5);4.245(4.6);3.311(94.2);3.261(0.8);2.670(0.7);2.665(0.5);2.524(2.4);2.519(3.5);2.510(40.4);2.506(86.0);2.501(11 9.5);2.496(86.0);2.492(41.5);2.476(6.3);2.457(5.1);2.438(2.0);2.332(0.6);2.328(0.8);2.323(0.6);2.073(0.9);1.639(1.7);1.624(1.7 );1.586(1.7);1.561(2.2);1.541(2.7);1.504(1.3);1.476(1.6);1.450(1.3);1.421(1.0);1.283(0.6);1.256(0.6);1.130(7.0);1.111(16.0);1.0 92(7.0);0.008(1.6);0.000(52.0);-0.009(2.1) |
| Example II-391: ¹H-NMR(400.0 MHz, d₆-DMSO): δ= 10.959(2.5);7.479(1.4);7.475(1.5);7.461(1.6);7.457(1.4);6.774(1.1);6.756( 1.0);5.754(8.9);4.298(2.8);4.243(2.9);4.098(1.8);4.080(6.0);4.062(6.1);4.044(2.0);3.313(39.5);2.758(0.6);2.743(1.4);2.726(1.1); 2.640(2.0);2.628(1.6);2.623(2.6);2.607(1.0);2.524(0.6);2.519(0.9);2.511(13.7);2.506(30.3);2.502(42.8);2.497(29.7);2.492(13.2) ;1.638(1.0);1.620(1.0);1.611(0.9);1.584(1.1);1.575(1.1);1.558(1.3);1.540(1.6);1.501(0.8);1.474(1.0);1.449(0.8);1.422(0.6);1.20 4(7.3);1.186(16.0);1.168(7.3);0.000(3.3) |
| Example II-392: ¹H-NMR(400.0 MHz, CDCl₃): δ= 7.261(21.8):7.164(1.0):7.146(1.1):6.335(1.2):6.317(1.2):4.305(5.7):3.879(1.1) ;3.861(1.2);3.549(0.9);3.380(16.0);1.721(0.5);1.553(0.9);0.000(8.3) |
| Example III-7: ¹H-NMR(400.0 MHz, CDCl₃): δ= 8.092(1.5);7.520(0.6);7.316(0.7);7.297(0.8);7.261(109.9);7.239(2.9);7.221(3.2); 7.211(0.7);6.997(0.6);6.895(0.9);6.876(1.5);6.855(0.9);5.299(11.4);4.939(1.0);4.931(0.9);4.910(0.5);2.574(1.0);2.298(1.0);1.96 4(2.1);1.943(3.3);1.934(3.0);1.912(2.2);1.798(1.0);1.768(1.1);1.598(0.7);1.566(2.5);1.538(4.4);1.510(1.9);1.304(7.7);1.286(16. 0);1.267(7.9);1.249(1.2);1.226(0.8);1.216(0.7);1.195(0.7);0.981(0.7);0.963(1.4);0.944(0.6);0.008(1.2);0.000(41.5);-0.009(1.2) |
| Example III-12: ¹H-NMR(400.0 MHz, CDCl₃): δ= 9.080(1.9);7.291(9.7);7.272(10.7);7.262(81.3);6.786(8.7);6.767(8.1);5.299(16. 0);4.942(1.1);4.933(1.6);4.925(1.3);4.905(0.8);1.969(2.9);1.946(5.5);1.922(3.2);1.804(1.5);1.772(1.7);1.608(3.7);1.566(3.5);1.5 38(6.6);1.511(2.6);1.474(0.7);1.284(0.9);1.258(2.0);1.252(2.1);1.228(1.1);1.219(1.1);0.899(0.6);0.882(2.1);0.864(0.8);0.008(0.8);0.000(30.2);-0.009(0.9) |
| Example I-80: ¹H-NMR(400.0 MHz, d₆-DMSO): δ= 7.584(9.6);7.567(10.2);7.554(4.0);7.536(4.2);6.883(5.0);6.876(11.3);6.865( 5.2);6.858(11.5);6.837(16.0);6.733(3.0);5.540(2.2);5.532(2.2);5.225(1.7);5.218(1.6);5.204(0.6);5.196(0.5);4.866(1.8);4.744(1.8 );4.569(0.6);4.446(0.6);3.877(10.5);3.859(13.5);3.842(4.6);3.549(2.3);3.531(2.7);3.512(0.6);3.313(188.9);3.263(1.1);2.675(0.9) ;2.671(1.3);2.666(0.9);2.524(3.2);2.520(4.4);2.511(73.4);2.506(161.5);2.502(227.3);2.497(160.8);2.492(73.0);2.457(1.5);2.452( 1.7);2.447(1.3);2.333(1.1);2.329(1.5);2.324(1.1);2.006(1.4);1.989(2.8);1.888(4.4);1.799(1.1);1.760(0.9);1.642(1.2);1.610(1.3);1 .572(1.3);1.539(2.0);1.500(1.3);1.462(1.5);1.430(4.2);1.420(2.8);1.396(5.7);1.387(4.7);1.367(2.8);1.356(3.6);1.330(4.4);1.300( 3.8);1.271(1.5);1.234(1.0);1.193(0.7);1.175(1.3);1.170(1.2);1.157(1.2);1.125(1.5);1.092(1.6);1.066(0.7);0.955(1.0);0.936(2.1);0 .918(0.8);0.008(0.6);0.000(22.1);-0.009(0.7) |
| Example III-4: ¹H-NMR(400.0 MHz, d₆-DMSO): δ= 7.549(10.4);7.530(10.8);7.388(0.6);6.925(13.4);6.907(13.1);6.898(1.6);6.87 6(16.0);5.000(2.3);4.993(3.8);4.986(2.6);4.968(2.6);4.962(3.9);4.954(2.4);4.748(5.6);3.364(0.9);3.314(147.5);3.264(0.8);2.676( 0.6);2.671(0.8);2.666(0.6);2.524(2.9);2.520(4.3);2.511(50.0);2.506(105.0);2.502(143.5);2.497(101.6);2.493(46.4);2.456(0.8);2. 452(1.0);2.447(0.9);2.329(1.0);2.324(0.7);2.304(0.9);2.294(1.0);2.273(2.5);2.265(1.9);2.242(2.7);2.210(1.2);2.113(0.8);2.105(0 .8);2.076(2.5);2.069(1.9);2.041(5.1);2.004(1.1);1.930(2.3);1.903(2.3);1.760(0.6);1.702(2.9);1.675(3.3);1.633(2.4);1.604(5.5);1. 597(5.0);1.557(2.2);1.549(2.0);1.517(0.7);0.000(2.1) |
| Example II-371: ¹H-NMR(400.0 MHz, d₆-DMSO): δ= 7.440(1.1);7.423(1.2);6.522(1.5);6.504(1.5);3.830(1.3);3.812(1.3);3.309(9 .5);2.822(16.0);2.510(6.1);2.506(13.3);2.501(18.5);2.496(13.0);2.492(5.9);2.086(6.8);1.909(0.8);1.143(0.7);0.000(1.5) |
| Example II-382: ¹H-NMR(400.0 MHz, d₆-DMSO): δ= 7.550(0.7);7.532(0.7);7.427(11.1);7.410(11.8);6.870(0.8);6.852(0.8);6.829 (1.1);6.507(12.8);6.489(12.9);3.826(15.7);3.808(16.0);3.374(2.9);3.356(6.0);3.341(6.1);3.323(7.7);3.311(64.1);3.259(1.1);3.08 7(3.5);3.072(5.9);3.056(6.1);3.040(5.0);3.025(3.3);2.877(1.4);2.872(1.2);2.856(2.8);2.837(3.2);2.822(4.3);2.806(2.1);2.801(2.3) ;2.725(2.3);2.720(2.7);2.711(3.7);2.704(3.0);2.699(3.0);2.694(2.2);2.686(2.0);2.680(2.8);2.671(2.7);2.667(2.4);2.506(91.1);2.5 02(121.9);2.497(90.9);2.493(46.8);2.455(2.9);2.451(3.1);2.328(1.5);2.295(3.8);2.283(5.5);2.271(6.0);2.255(5.4);2.230(3.3);2.2 18(4.1);2.209(3.1);2.205(3.6);2.199(3.0);2.187(3.0);2.176(1.4);2.158(0.9);2.086(3.1);2.074(2.3);2.058(4.7);2.043(6.0);2.034(5. 4);2.020(3.7);2.000(1.7);1.990(1.7);1.982(1.8);1.963(2.7);1.946(3.7);1.943(3.2);1.938(3.1);1.928(2.5);1.907(2.4);1.792(1.8);1.7 83(2.2);1.774(2.5);1.765(3.1);1.756(2.6);1.746(2.3);1.737(2.2);1.668(5.6);1.653(6.6);1.602(4.4);1.546(6.3);1.515(6.3);1.234(1. 7);1.140(11.3);1.121(7.2);1.017(2.6);0.990(5.1);0.960(4.1);0.937(2.0);0.000(20.7);-0.009(1.2) |
| Example II-377: ¹H-NMR(400.0 MHz, d₆-DMSO): δ= 7.445(3.6);7.427(3.8);6.566(4.4);6.549(4.4);3.832(4.3);3.814(4.3);3.310(1 9.2);3.193(0.7);3.177(1.7);3.158(4.5);3.146(1.4);3.140(4.6);3.126(1.3);3.121(1.5);3.075(1.0);3.057(1.4);3.055(1.3);3.044(0.7);3 .036(1.1);3.025(0.9);3.005(0.6);2.776(1.5);2.762(0.7);2.757(1.9);2.743(2.3);2.738(1.1);2.724(2.8);2.705(1.4);2.696(0.6);2.675( 0.9);2.664(1.6);2.656(0.6);2.644(2.1);2.639(0.9);2.631(1.7);2.624(1.9);2.621(2.3);2.616(2.5);2.611(1.9);2.606(0.8);2.602(2.4);2 .596(2.0);2.588(1.5);2.583(1.7);2.578(0.9);2.569(1.4);2.564(0.9);2.551(1.0);2.546(0.8);2.524(1.6);2.519(2.1);2.511(21.7);2.506 (46.2);2.502(64.3);2.497(46.4);2.492(22.2);1.909(3.8);1.777(0.6);1.768(0.8);1.759(0.6);1.749(0.8);1.740(0.7);1.730(1.1);1.721( 1.2);1.709(1.4);1.703(1.9);1.696(1.6);1.690(2.0);1.685(2.8);1.677(2.5);1.672(2.6);1.666(3.1);1.659(3.3);1.653(2.5);1.651(2.5);1 .648(2.5);1.640(2.4);1.632(1.3);1.621(1.4);1.613(1.0);1.605(1.2);1.587(0.7);1.560(1.5);1.530(1.5);1.243(5.6);1.236(1.2);1.225( 12.4);1.206(5.7);1.192(7.4);1.186(3.2);1.173(15.1);1.167(6.4);1.154(7.9);1.148(4.7);1.125(1.7);1.021(6.9);1.018(8.2);1.003(14. 2);0.999(16.0);0.984(6.5);0.981(7.4);0.000(14.6);-0.009(0.6) |
| Example II-375: ¹H-NMR(400.0 MHz, d₆-DMSO): δ= 7.451(2.0);7.434(2.1);6.579(2.3);6.562(2.3);4.040(0.7);4.022(0.7);3.834(2 .8);3.816(2.8);3.311(2.9);3.165(1.3);3.147(4.0);3.128(4.1);3.110(1.4);2.525(0.7);2.520(1.0);2.511(10.0);2.507(21.0);2.502(29.1 );2.497(20.9);2.493(10.0);1.989(3.0);1.910(3.3);1.672(0.8);1.658(1.0);1.651(0.9);1.607(0.6);1.596(0.6);1.561(1.0);1.529(1.0);1. 253(6.9);1.234(16.0);1.216(6.7);1.194(1.1);1.186(0.5);1.176(2.1);1.168(1.1);1.158(1.4);1.144(1.8);1.125(1.1);0.999(0.8);0.969( 0.6);0.000(6.7) |
| Example III-14: ¹H-NMR(400.0 MHz, d₆-DMSO): δ= 12.581(4.9);7.672(11.4);7.653(11.7);6.678(0.6);6.632(14.4);6.613(14.6);5. 028(4.0);5.021(3.0);4.997(4.2);4.989(2.8);4.974(0.9);4.769(6.3);4.056(1.0);4.038(3.6);4.021(3.6);4.003(1.2);3.684(0.6);3.471(6 .7);3.318(2.1);2.674(2.0);2.670(2.9);2.665(2.2);2.556(2.8);2.551(3.2);2.547(3.3);2.523(12.9);2.518(16.4);2.510(171.5);2.505(3 65.8);2.501(508.8);2.496(372.7);2.491(184.2);2.450(3.1);2.332(2.4);2.328(3.1);2.323(2.4);2.295(1.1);2.263(2.6);2.232(3.2);2.2 00(1.6);2.097(2.9);2.050(4.8);2.040(4.6);2.019(1.8);1.988(16.0);1.938(2.6);1.908(4.0);1.730(3.0);1.705(3.2);1.671(1.8);1.636(3 .0);1.603(6.8);1.571(3.0);1.298(1.2);1.284(1.1);1.234(1.4);1.193(4.4);1.175(8.7);1.157(4.2);0.146(1.4);0.051(0.9);0.008(11.3);0 .000(332.2);-0.009(14.5);-0.034(2.4);-0.050(1.3);-0.149(1.2) |
| Example II-379: ¹H-NMR(400.0 MHz, d₆-DMSO): δ= 12.545(4.1);7.698(8.8);7.680(9.1);7.665(2.6);7.647(2.5);6.607(3.8);6.603( 10.7);6.589(4.0);6.585(10.7);5.754(16.0);4.870(1.8);4.749(1.8);3.930(10.1);3.912(12.8);3.894(3.1);3.330(15.5);2.674(1.5);2.67 0(2.0);2.665(1.4);2.601(0.7);2.551(5.1);2.546(5.1);2.523(9.3);2.518(12.7);2.510(120.9);2.505(253.0);2.501(349.8);2.496(252.8 );2.492(120.8);2.462(2.7);2.457(2.2);2.452(1.0);2.332(1.4);2.328(2.0);2.323(1.5);2.041(1.0);2.011(1.1);1.917(3.2);1.891(4.1);1. 628(1.0);1.577(1.0);1.544(1.8);1.517(1.1);1.475(1.2);1.439(4.8);1.414(5.9);1.382(2.6);1.344(3.8);1.321(2.7);1.284(1.0);1.236(2 .4);1.175(0.7);1.140(1.1);1.109(0.9);0.146(0.8);0.050(2.4);0.008(8.4);0.000(243.1);-0.009(9.7);-0.049(1.0);-0.150(0.8) |
| Example II-383: ¹H-NMR(400.0 MHz, d₆-DMSO): δ= 7.452(8.3);7.434(8.8);6.578(8.9);6.560(8.9);4.039(1.6);4.021(1.6);4.003(0 .5);3.835(10.3);3.817(10.5);3.311(59.6);3.257(2.0);3.231(4.0);3.224(3.0);3.207(2.9);3.200(2.7);3.125(2.8);3.113(2.7);3.105(2.8 );3.080(1.8);2.675(1.0);2.670(1.3);2.665(0.9);2.524(3.0);2.519(4.5);2.510(67.8);2.506(146.1);2.501(203.0);2.496(143.5);2.492( 65.3);2.455(0.6);2.450(1.0);2.446(0.9);2.332(1.0);2.328(1.3);2.323(0.9);2.179(1.8);2.158(2.2);2.146(2.3);2.085(10.7);1.988(7.2 );1.909(16.0);1.800(1.0);1.791(1.2);1.782(1.4);1.773(1.8);1.764(1.5);1.754(1.3);1.745(1.3);1.736(1.3);1.710(2.3);1.700(2.9);1.6 82(4.3);1.674(4.5);1.664(4.9);1.615(3.2);1.603(5.4);1.596(4.8);1.589(4.9);1.575(3.6);1.564(4.2);1.525(3.6);1.259(0.6);1.236(2.7);1.193(2.5);1.175(5.7);1.157(4.1);1.145(6.3);1.126(3.8);1.026(1.3);0.998(2.8);0.969(2.2);0.000(2.5) |

### B) Formulation examples

a) A dust is obtained by mixing 10 parts by weight of a compound of the formula (G) and 90 parts by weight of talc as inert substance and comminuting the mixture in a hammer mill.
b) A wettable powder which is readily dispersible in water is obtained by mixing 25 parts by weight of a compound of the formula (G), 64 parts by weight of kaolin-containing quartz as inert substance, 10 parts by weight of potassium lignosulphonate and 1 part by weight of sodium oleoylmethyltaurate as wetting agent and dispersant, and grinding the mixture in a pinned-disk mill.
c) A readily water-dispersible dispersion concentrate is obtained by mixing 20 parts by weight of a compound of the formula (G) with 6 parts by weight of alkylphenol polyglycol ether (®Triton X 207), 3 parts by weight of isotridecanol polyglycol ether (8 EO) and 71 parts by weight of paraffinic mineral oil (boiling range for example about 255 to above 277 °C) and grinding the mixture in a ball mill to a fineness of below 5 microns.
d) An emulsifiable concentrate is obtained from 15 parts by weight of a compound of the formula (G), 75 parts by weight of cyclohexanone as solvent and 10 parts by weight of oxyethylated nonylphenol as emulsifier.
e) Water-dispersible granules are obtained by mixing
   75 parts by weight of a compound of the formula (G),
   10 parts by weight of calcium lignosulphonate,
   5 parts by weight of sodium laurylsulphate,
   3 parts by weight of polyvinyl alcohol and
   7 parts by weight of kaolin,
   grinding the mixture in a pinned-disk mill, and granulating the powder in a fluidized bed by spray application of water as a granulating liquid.
f) Water-dispersible granules are also obtained by homogenizing and precomminuting
   25 parts by weight of a compound of the formula (G),
   5 parts by weight of sodium 2,2'-dinaphthylmethane-6,6'-disulphonate,
   2 parts by weight of sodium oleoylmethyltaurinate,
   1 part by weight of polyvinyl alcohol,
   17 parts by weight of calcium carbonate and
   50 parts by weight of water,
   on a colloid mill, subsequently grinding the mixture in a bead mill and atomizing and drying the resulting suspension in a spray tower by means of a single-substance nozzle.

### (C) Biological examples

Herbicidal activities of the compounds of the formula (G) according to the invention were investigated against the following harmful plants:
- ALOMY =: Alopecurus myosuroides
- ECHCG =: Echinochloa crus-galli
- SETVI =: Setaria viridis
- ABUTH =: Abutilon theophrasti
- AMARE =: Amaranthus retroflexus
- PHBPU =: Pharbitis purpurea
- POLCO =: Polygonum convolvulus (= Fallopia convolvulus)
- STEME =: Stellaria media
- VIOTR =: Viola tricolor

### 1. Herbicidal pre-emergence action

Seeds of monocotyledonous and dicotyledonous weed plants and crop plants were placed in wood-fibre pots in sandy loam and covered with soil. The compounds (G) according to the invention, formulated in the form of wettable powders (WP), were then applied as aqueous suspension or emulsion at a water application rate of 600 l/ha (converted) with the addition of 0.2% of wetting agent to the surface of the covering soil.

After the treatment, the pots were placed in a greenhouse and kept under good growth conditions for the test plants. After about 3 weeks, the effect of the preparations was scored visually in comparison with untreated controls as percentages. For example, 100% activity = the plants have died, 50% herbicidal activity or damage = the plants have been reduced by 50% or the plant mass has been reduced by 50%, 0% activity = like control plants.

Compounds (G) according to the invention such as, for example, the compounds No. I-17, I-22, I-34, I-37, I-4, I-49, I-50, I-56, II-111, II-112, II-128, II-145, II-152, II-161, II-162, II-163, II-168, II-172, II-177, II-195, II-196, II-197, II-2, II-202, II-206, II-213, II-247, II-25, II-26, II-264, II-27, II-274, II-281, II-298, II-3, II-315, II-32, II-331, II-332, II-333, II-342, II-350, II-365, II-366, II-367, II-368, II-369, II-370, II-385, II-386, II-387, II-388, II-389, II-390, II-43, II-60, II-77, II-94, and III-2 from the above Tables 1 to 3, have good herbicidal efficacy (80% to 100% activity) against a plurality of harmful plants at an application rate of 320 g of active substance per hectare when applied by the pre-emergence method, in particular against one, two, three, four, five, six or even all of the harmful plants selected from the group consisting of ALOMY, ECHCG, SETVI, AMARE, POLCO, STEME and VIOTR.

What was determined was the respective herbicidal activity, in each case at the same point in time after application of the formulation in question, i.e. the damage to the respective harmful plant in % compared to untreated control plants.

Further, the compounds No. I-17, I-22, I-34, I-4, I-49, I-50, I-56, II-111, II-112, II-128, II-145, II-152, II-161, II-162, II-163, II-168, II-172, II-177, II-195, II-196, II-197, II-2, II-202, II-206, II-213, II-247, II-25, II-26, II-264, II-27, II-274, II-281, II-298, II-3, II-315, II-32, II-331, II-332, II-333, II-342, II-350, II-365, II-366, II-367, II-368, II-369, II-385, II-386, II-387, II-388, II-389, II-390, II-43, II-60, II-77, II-94, and III-2 from the above Tables 1 to 3, showed excellent activity (90-100%) against several, in particular three or more, of the mentioned harmful plants when applied by the pre-emergence method at an application rate of 320 g of active substance per hectare.

Generally, the compounds according to the invention displayed particularly good herbicidal activity in pre-emergence application method against several harmful plants selected from the group AMARE = Amaranthus retroflexus, POLCO = Polygonum convolvulus, STEME = Stellaria media and VIOTR = Viola tricolor.

### 2. Herbicidal post-emergence action

Seeds of monocotyledonous and dicotyledonous weeds and crop plants were placed in sandy loam in wood-fibre pots, covered with soil and cultivated in a greenhouse under good growth conditions. 2 to 3 weeks after sowing, the test plants were treated at the one-leaf stage, where the compounds (G) according to the invention, formulated in the form of wettable powders (WP), were applied by spraying as aqueous suspension or emulsion at a water application rate of 600 l/ha (converted) with the addition of 0.2% of wetting agent to the green parts of the plants. After the test plants had been kept in the greenhouse under optimum growth conditions for about 3 weeks, the activity of the preparations was rated visually in comparison to untreated controls in per cent (%): For example, 100% activity = the plants have died, 50% herbicidal activity or damage = the plants have been reduced by 50% or the plant mass has been reduced by 50%, 0% activity = like control plants.

Compounds (G) according to the invention such as, for example, the compounds No. I-22, I-34, I-4, I-49, I-50, I-56, II-111, II-112, II-128, II-145, II-152, II-161, II-162, II-163, II-168, II-172, II-177, II-195, II-196, II-197, II-2, II-202, II-206, II-213, II-247, II-25, II-26, II-264, II-27, II-274, II-281, II-298, II-3, II-315, II-32, II-331, II-332, II-333, II-342, II-350, II-365, II-366, II-367, II-368, II-369, II-370, II-385, II-386, II-387, II-388, II-389, II-390, II-43, II-60, II-77, II-94, and III-2 from the above Tables 1 to 3, have good herbicidal efficacy (80% to 100% activity) against a plurality of harmful plants at an application rate of 320 g of active substance per hectare when applied by the post-emergence method, in particular against against one, two, three, four, five, six, seven, or even all of the harmful plants selected from the group consisting of ECHCG, SETVI, ABUTH, AMARE, PHBPU, POLCO, STEME, and VIOTR.

What was determined was the respective herbicidal activity, in each case at the same point in time after application of the formulation in question, i.e. the damage to the respective harmful plant in % compared to untreated control plants.

Further, the compounds No. I-4, I-49, I-56, II-111, II-112, II-128, II-145, II-152, II-161, II-162, II-163, II-168, II-172, II-177, II-195, II-196, II-197, II-2, II-202, II-206, II-213, II-247, II-25, II-26, II-264, II-27, II-274, II-281, II-298, II-3, II-315, II-32, II-331, II-332, II-333, II-342, II-350, II-365, II-366, II-367, II-368, II-369, II-385, II-386, II-387, II-388, II-389, II-390, II-43, II-60, II-77, and II-94, from the above Tables 1 to 3, showed excellent activity (90-100%) against several, in particular three or more, of the mentioned harmful plants when applied by the post-emergence method at an application rate of 320 g of active substance per hectare.

Generally, the compounds according to the invention displayed particularly good herbicidal activity in post-emergence application method against several harmful plants selected from the group AMARE = Amaranthus retroflexus, PHBPU = Pharbitis purpurea, and VIOTR = Viola tricolor.

### 3. Herbicidal action and crop plant compatibility

In further trials in the greenhouse, seeds of crop plants are placed in pots in sandy loam soil, covered with soil and cultivated under good growth conditions and treated by the pre-emergence method and scored analogously to the harmful plants mentioned in above section (C) examples 1 and 2, respectively.

### 3.1 The results showed that the compounds according to the invention did not cause significant

damage when applied by the pre-emergence method at the same application rates as mentioned above to monocotyledonous crops, such as
ORYSA = Oryza sativa (common rice)
TRZAS = Triticum aestivum (spring) (summer wheat)
ZEAMX = Zea mays (maize)
BRSNW = Brassica napus subsp. napus (winter) (winter oilseed rape)

Here, the observed damage to the respective useful plants was within the acceptable range and was generally assessed as low (generally in a range from 0 to 10%).

### 3.2 The results showed that the compounds according to the invention did not cause significant damage when applied by the post-emergence method at the same application rates as mentioned above to monocotyledonous crops, such as those mentioned in above section (C) 3.1.

Here, the observed damage to the respective useful plants was within the acceptable range and was generally assessed as low (generally in a range from 0 to 20%).

## Claims

1. Compound of the formula (G) and/or a salt thereof, 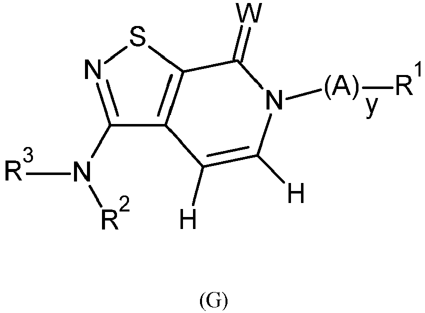 in which
A is CR⁶R⁷,
W is O or S,
R¹ is (C₁-C₁₂)-alkyl, (C₁-C₁₂)-haloalkyl, (C₂-C₁₂)-alkenyl, (C₂-C₁₂)-haloalkenyl, (C₂-C₁₂)-alkynyl, (C₂-C₁₂)-haloalkynyl, NR¹³R¹⁴, R¹³R¹⁴N-(C₁-C₆)-alkyl, (C₁-C₆)-alkoxy, (C₁-C₆)-haloalkoxy, (C₁-C₆)-haloalkoxy-(C₁-C₃)-alkyl, (C₁-C₆)-alkoxy-(C₁-C₃)-alkyl, (C₁-C₆)-alkoxy-(C₂-C₆)-alkoxy-(C₁-C₃)-alkyl, (C₁-C₄)-alkylthio, (C₁-C₄)-alkylsulphoxy, (C₁-C₄)-alkylsulphonyl, (C₁-C₄)-haloalkylthio, (C₁-C₄)-haloalkylsulphoxy, (C₁-C₄)-haloalkylsulphonyl, (C₁-C₄)-alkylthio-(C₁-C₃)-alkyl, (C₁-C₄)-alkylsulphoxy-(C₁-C₃)-alkyl, (C₁-C₄)-alkylsulphonyl-(C₁-C₃)-alkyl, (C₁-C₄)-haloalkylthio-(C₁-C₃)-alkyl, (C₁-C₄)-haloalkylsulphoxy-(C₁-C₃)-alkyl, (C₁-C₄)-haloalkylsulphonyl-(C₁-C₃)-alkyl, (C₃-C₁₂)-cycloalkyl, (C₃-C₈)-cycloalkenyl, (C₃-C₁₂)-cycloalkyl-(C₁-C₆)-alkyl, (C₃-C₈)-cycloalkenyl-(C₁-C₆)-alkyl, (C₃-C₈)-cycloalkoxy, (C₃-C₈)-cycloalkyl-(C₁-C₆)-alkoxy, aryl, aryl-(C₁-C₃)-alkyl, heteroaryl, heteroaryl-(C₁-C₃)-alkyl, heterocyclyl, heterocyclyl-(C₁-C₃)-alkyl, aryloxy, heteroaryloxy, heterocyclyloxy, a bicyclic or a heterobicyclic residue, wherein each of the last-mentioned 17 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, (C₁-C₄)-haloalkylthio, (C₁-C₄)-haloalkylsulphoxy, (C₁-C₄)-haloalkylsulphonyl, (C₁-C₄)-alkoxycarbonyl, (C₁-C₄)-haloalkoxycarbonyl, (C₁-C₄)-alkylcarboxy, (C₃-C₆)-cycloalkyl, (C₃-C₆)-cycloalkyl-(C₁-C₆)-alkyl, (C₁-C₄)-alkoxycarbonyl-(C₁-C₄)-alkyl, hydroxycarbonyl, hydroxycarbonyl-(C₁-C₄)-alkyl, R¹³R¹⁴N-carbonyl, and wherein heterocyclyl has q oxo groups, and wherein each of the aforementioned heterocyclic residues, in addition to the carbon atoms, has in each case p ring members from the group consisting of N(R¹²)ₘ, O and S(O)ₙ,
R², R³ are each independently hydrogen, (C₁-C₁₂)-alkyl, (C₁-C₁₂)-haloalkyl, (C₂-C₁₂)-alkenyl, (C₂-C₁₂)-haloalkenyl, (C₂-C₁₂)-alkynyl, (C₂-C₁₂)-haloalkynyl, (C₁-C₆)-alkoxy-(C₁-C₃)-alkyl, (C₁-C₆)-alkoxy-(C₂-C₆)-alkoxy-(C₁-C₃)-alkyl, (C₁-C₆)-alkoxy-(C₁-C₃)-alkylcarbonyl, (C₁-C₆)-alkoxy-(C₂-C₆)-alkoxy-(C₁-C₃)-alkylcarbonyl, (C₁-C₆)-alkoxycarbonyl, (C₁-C₆)-haloalkoxycarbonyl, (C₂-C₆)-alkenyloxycarbonyl, (C₂-C₆)-haloalkenyloxycarbonyl, (C₂-C₆)-alkynyloxycarbonyl, (C₂-C₆)-haloalkynyloxycarbonyl, (C₁-C₆)-alkylcarbonyl, (C₁-C₆)-haloalkylcarbonyl, (C₂-C₆)-alkenylcarbonyl, (C₂-C₆)-haloalkenylcarbonyl, (C₂-C₆)-alkynylcarbonyl, (C₂-C₆)-haloalkynylcarbonyl, R¹³R¹⁴N-carbonyl, (C₁-C₄)-alkylthio, (C₁-C₄)-haloalkylthio, (C₁**-**C₈)-alkylthiocarbonyl, (C₁-C₈)-haloalkylthiocarbonyl, (C₁-C₄)-alkylsulphoxy, (C₁-C₄)-haloalkylsulphoxy, (C₁-C₄)-alkylsulphonyl, (C₁-C₄)-haloalkylsulphonyl, (C₁-C₄)-alkylthio-(C₁-C₃)-alkyl, (C₁-C₄)-alkylsulphoxy-(C₁-C₃)-alkyl, (C₁-C₄)-alkylsulphonyl-(C₁-C₃)-alkyl, (C₁-C₄)-alkylthio-(C₁-C₃)-alkylcarbonyl, (C₁-C₄)-alkylsulphoxy-(C₁-C₃)-alkylcarbonyl, (C₁-C₄)-alkylsulphonyl-(C₁-C₃)-alkylcarbonyl, (C₁-C₁₂)-alkylcarbonyl, (C₁-C₁₂)-haloalkylcarbonyl, (C₂-C₁₂)-alkenylcarbonyl, (C₂-C₁₂)-haloalkenylcarbonyl, (C₂-C₁₂)-alkynylcarbonyl, (C₂-C₁₂)-haloalkynylcarbonyl, (C₁-C₁₂)-alkoxycarbonylcarbonyl, (C₁-C₁₂)-alkoxycarbonyl-(C₁-C₃)-alkylcarbonyl, (C₃-C₈)-cycloalkyl, (C₃-C₈)-cycloalkenyl, (C₃-C₈)-cycloalkyl-(C₁-C₆)-alkyl, (C₃-C₈)-cycloalkenyl-(C₁-C₆)-alkyl, (C₃-C₈)-cycloalkylcarbonyl, (C₃-C₈)-cycloalkenylcarbonyl, (C₃-C₈)-cycloalkyl-(C₁-C₆)-alkylcarbonyl, (C₃-C₈)-cycloalkenyl-(C₁-C₆)-alkylcarbonyl, aryl, aryl-(C₁-C₃)-alkyl, heteroaryl, heteroaryl-(C₁-C₃)-alkyl, heterocyclyl, heterocyclyl-(C₁-C₃)-alkyl, arylcarbonyl, aryl-(C₁-C₆)-alkylcarbonyl, heteroarylcarbonyl, heteroaryl-(C₁-C₆)-alkylcarbonyl, heterocyclylcarbonyl, or heterocyclyl-(C₁-C₆)-alkylcarbonyl, wherein each of the last-mentioned 20 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, (C₁-C₄)-haloalkylthio, (C₁-C₄)-haloalkylsulphoxy, (C₁-C₄)-haloalkylsulphonyl, (C₁-C₄)-alkoxycarbonyl, (C₁-C₄)-haloalkoxycarbonyl, (C₁-C₄)-alkylcarboxy, (C₃-C₆)-cycloalkyl, (C₃-C₆)-cycloalkyl-(C₁-C₆)-alkyl, (C₁-C₄)-alkoxycarbonyl-(C₁-C₄)-alkyl, hydroxycarbonyl, hydroxycarbonyl-(C₁-C₄)-alkyl, R¹³R¹⁴N-carbonyl, and wherein heterocyclyl has q oxo groups,
or
NR²R³ is -N=CR⁸R⁹ or -N=S(O)ₙR¹⁰R¹¹,
R⁶, R⁷ are each independently hydrogen, cyano, halogen, (C₁-C₆)-alkyl, (C₂-C₆)-alkenyl, (C₂-C₆)-alkynyl, or (C₃-C₈)-cycloalkyl,
or
R⁶ and R⁷, together with the carbon atom to which they are attached, form a 3 - 6-membered carbocyclic or heterocyclic ring, which comprises in each case, in addition to the carbon atoms, p ring members from the group consisting of N(R¹²)ₘ, O and S(O)ₙ and wherein said ring is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, (C₁-C₄)-haloalkylthio, (C₁-C₄)-haloalkylsulphoxy, (C₁-C₄)-haloalkylsulphonyl, (C₁-C₄)-alkoxycarbonyl, (C₁-C₄)-haloalkoxycarbonyl, (C₁-C₄)-alkylcarboxy, (C₃-C₆)-cycloalkyl, (C₃-C₆)-cycloalkyl-(C₁-C₆)-alkyl, (C₁-C₄)-alkoxycarbonyl-(C₁-C₄)-alkyl, hydroxycarbonyl, hydroxycarbonyl-(C₁-C₄)-alkyl, R¹³R¹⁴N-carbonyl and has q oxo groups,
R⁸, R⁹ are each independently hydrogen, (C₁-C₆)-alkyl, (C₁-C₆)-haloalkyl, (C₂-C₆)-alkenyl, (C₂-C₆)-haloalkenyl, (C₂-C₆)-alkynyl, (C₂-C₆)-haloalkynyl, (C₁-C₆)-alkoxy, (C₁-C₆)-haloalkoxy, (C₁-C₆)-haloalkoxy-(C₁-C₃)-alkyl, (C₂-C₆)-alkenyloxy, (C₂-C₆)-haloalkenyloxy, (C₂-C₆)-alkynyloxy, (C₂-C₆)-haloalkynyloxy, NR¹³R¹⁴, (C₁-C₆)-alkoxy-(C₁-C₃)-alkyl, halogen-(C₁-C₆)-alkoxy-(C₁-C₆)-alkyl, (C₁-C₆)-alkoxy-(C₂-C₆)-alkoxy-(C₁-C₃)-alkyl, (C₁-C₄)-alkylthio-(C₁-C₃)-alkyl, (C₁-C₄)-alkylsulphoxy-(C₁-C₃)-alkyl, (C₁-C₄)-alkylsulphonyl-(C₁-C₃)-alkyl, (C₃-C₈)-cycloalkyl, (C₃-C₈)-cycloalkenyl, (C₃-C₈)-cycloalkyl-(C₁-C₆)-alkyl, (C₃-C₈)-cycloalkenyl-(C₁-C₆)-alkyl, aryl, aryl-(C₁-C₃)-alkyl, heteroaryl, heteroaryl-(C₁-C₃)-alkyl, heterocyclyl, heterocyclyl-(C₁-C₃)-alkyl, wherein each of the last-mentioned 10 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, (C₁-C₄)-haloalkylthio, (C₁-C₄)-haloalkylsulphoxy, (C₁-C₄)-haloalkylsulphonyl, (C₁-C₄)-alkoxycarbonyl, (C₁-C₄)-haloalkoxycarbonyl, (C₁-C₄)-alkylcarboxy, (C₃-C₆)-cycloalkyl, (C₃-C₆)-cycloalkyl-(C₁-C₆)-alkyl, (C₁-C₄)-alkoxycarbonyl-(C₁-C₄)-alkyl, hydroxycarbonyl, hydroxycarbonyl-(C₁-C₄)-alkyl, R¹³R¹⁴N-carbonyl and has q oxo groups,
or
R⁸ and R⁹, together with the carbon atom to which they are attached, form a 3- to 8-membered unsaturated, partially saturated or saturated ring, which comprises in each case, in addition to the carbon atoms, p ring members from the group consisting of N(R¹²)ₘ, O and S(O)ₙ and wherein said ring is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, (C₁-C₄)-haloalkylthio, (C₁-C₄)-haloalkylsulphoxy, (C₁-C₄)-haloalkylsulphonyl, (C₁-C₄)-alkoxycarbonyl, (C₁-C₄)-haloalkoxycarbonyl, (C₁-C₄)-alkylcarboxy, (C₃-C₆)-cycloalkyl, (C₃-C₆)-cycloalkyl-(C₁-C₆)-alkyl, (C₁-C₄)-alkoxycarbonyl-(C₁-C₄)-alkyl, hydroxycarbonyl, hydroxycarbonyl-(C₁-C₄)-alkyl, R¹³R¹⁴N-carbonyl and has q oxo groups,
R¹⁰, R¹¹ are each independently (C₁-C₆)-alkyl, (C₁-C₆)-haloalkyl, (C₂-C₆)-alkenyl, (C₂-C₆)-haloalkenyl, (C₂-C₆)-alkynyl, (C₂-C₆)-haloalkynyl, (C₁-C₆)-alkoxy-(C₁-C₃)-alkyl, halogen-(C₁-C₆)-alkoxy-(C₁-C₆)-alkyl, (C₁-C₆)-alkoxy-(C₂-C₆)-alkoxy-(C₁-C₃)-alkyl, (C₁-C₄)-alkylthio-(C₁-C₃)-alkyl, (C₁-C₄)-alkylsulphoxy-(C₁-C₃)-alkyl, (C₁-C₄)-alkylsulphonyl-(C₁-C₃)-alkyl, (C₃-C₈)-cycloalkyl, (C₃-C₈)-cycloalkenyl, (C₃-C₈)-cycloalkyl-(C₁-C₆)-alkyl, (C₃-C₈)-cycloalkenyl-(C₁-C₆)-alkyl, aryl, aryl-(C₁-C₃)-alkyl, heteroaryl, heteroaryl-(C₁-C₃)-alkyl, heterocyclyl or heterocyclyl-(C₁-C₃)-alkyl, wherein each of the last-mentioned 10 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, (C₁-C₄)-haloalkylthio, (C₁-C₄)-haloalkylsulphoxy, (C₁-C₄)-haloalkylsulphonyl, (C₁-C₄)-alkoxycarbonyl, (C₁-C₄)-haloalkoxycarbonyl, (C₁-C₄)-alkylcarboxy, (C₃-C₆)-cycloalkyl, (C₃-C₆)-cycloalkyl-(C₁-C₆)-alkyl, (C₁-C₄)-alkoxycarbonyl-(C₁-C₄)-alkyl, hydroxycarbonyl, hydroxycarbonyl-(C₁-C₄)-alkyl, R¹³R¹⁴N-carbonyl and wherein heterocyclyl has q oxo groups,
or
R¹⁰ and R¹¹, together with the sulphur atom to which they are attached, form a 3- to 8-membered unsaturated, partially saturated or saturated ring, which comprises in each case, in addition to the carbon atoms and in addition to the sulphur atom, p ring members from the group consisting of N(R¹²)ₘ, O and S(O)ₙ and wherein said ring is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, (C₁-C₄)-haloalkylthio, (C₁-C₄)-haloalkylsulphoxy, (C₁-C₄)-haloalkylsulphonyl, (C₁-C₄)-alkoxycarbonyl, (C₁-C₄)-haloalkoxycarbonyl, (C₁-C₄)-alkylcarboxy, (C₃-C₆)-cycloalkyl, (C₃-C₆)-cycloalkyl-(C₁-C₆)-alkyl, (C₁-C₄)-alkoxycarbonyl-(C₁-C₄)-alkyl, hydroxycarbonyl, hydroxycarbonyl-(C₁-C₄)-alkyl, R¹³R¹⁴N-carbonyl and has q oxo groups,
R¹² is hydrogen, (C₁-C₁₂)-alkyl, (C₁-C₁₂)-haloalkyl, (C₂-C₁₂)-alkenyl, (C₂-C₁₂)-haloalkenyl, (C₂-C₁₂)-alkynyl, (C₂-C₁₂)-haloalkynyl, (C₃-C₈)-cycloalkyl, (C₃-C₈)-halocycloalkyl, (C₃-C₈)-cycloalkenyl, (C₃-C₈)-cycloalkyl-(C₁-C₆)-alkyl, (C₃-C₈)-cycloalkenyl-(C₁-C₆)-alkyl, (C₁-C₁₂)-alkylcarbonyl or (C₁-C₁₂)-haloalkylcarbonyl,
R¹³, R¹⁴ are each independently hydrogen, (C₁-C₁₂)-alkyl, (C₁-C₁₂)-haloalkyl, (C₂-C₁₂)-alkenyl, (C₂-C₁₂)-haloalkenyl, (C₂-C₁₂)-alkynyl, (C₂-C₁₂)-haloalkynyl, (C₁-C₁₂)-alkylcarbonyl, (C₂-C₁₂)-alkenylcarbonyl, (C₂-C₁₂)-alkynylcarbonyl, (C₁-C₁₂)-haloalkylcarbonyl, (C₁-C₄)-alkylsulphonyl, (C₃-C₈)-cycloalkyl, (C₃-C₈)-cycloalkenyl, (C₃-C₈)-cycloalkyl-(C₁-C₆)-alkyl, (C₃-C₈)-cycloalkenyl-(C₁-C₆)-alkyl, (C₃-C₈)-cycloalkylcarbonyl, (C₃-C₈)-cycloalkenylcarbonyl, (C₃-C₈)-cycloalkyl-(C₁-C₆)-alkylcarbonyl, (C₃-C₈)-cycloalkenyl-(C₁-C₆)-alkylcarbonyl, aryl, arylcarbonyl, arylsulphonyl, hetaryl, hetarylcarbonyl, hetarylsulphonyl, heterocyclyl, heterocyclylcarbonyl, heterocyclylsulphonyl, wherein each of the last-mentioned 17 residues is unsubstituted or is substituted by one or more residues from the group consisting of halogen, nitro, hydroxyl, cyano, NH₂, (C₁-C₆)-alkylamine, (C₁-C₆)-dialkylamine, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, (C₁-C₄)-haloalkylthio, (C₁-C₄)-haloalkylsulphoxy, (C₁-C₄)-haloalkylsulphonyl, (C₁-C₄)-alkoxycarbonyl, (C₁-C₄)-haloalkoxycarbonyl, (C₁-C₄)-alkylcarboxy, (C₃-C₆)-cycloalkyl, (C₃-C₆)-cycloalkyl-(C₁-C₆)-alkyl, (C₁-C₄)-alkoxycarbonyl-(C₁-C₄)-alkyl, hydroxycarbonyl, hydroxycarbonyl-(C₁-C₄)-alkyl and wherein heterocyclyl has q oxo groups,
or
R¹³ and R¹⁴, together with the nitrogen atom to which they are attached, form a 3- to 8-membered unsaturated, partially saturated or saturated ring, which comprises in each case, in addition to the carbon atoms and in addition to the nitrogen atom, p ring members from the group consisting of N(R¹²)ₘ, O and S(O)ₙ and wherein said ring is unsubstituted or is substituted by one or more residues from the group consisting of halogen, nitro, hydroxyl, cyano, NH₂, (C₁-C₆)-alkylamine, (C₁-C₆)-dialkylamine, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, (C₁-C₄)-haloalkylthio, (C₁-C₄)-haloalkylsulphoxy, (C₁-C₄)-haloalkylsulphonyl, (C₁-C₄)-alkoxycarbonyl, (C₁-C₄)-haloalkoxycarbonyl, (C₁-C₄)-alkylcarboxy, (C₃-C₆)-cycloalkyl, (C₃-C₆)-cycloalkyl-(C₁-C₆)-alkyl, (C₁-C₄)-alkoxycarbonyl-(C₁-C₄)-alkyl, hydroxycarbonyl, hydroxycarbonyl-(C₁-C₄)-alkyl and has q oxo groups,
n is independently selected from 0, 1 or 2,
m is independently selected from 0 or 1,
p is independently selected from 0, 1, 2 or 3,
q is independently selected from 0, 1 or 2,
y is 0 or 1.

2. Compound of the formula (G) according to Claim 1 and/or a salt thereof, in which
A is CR⁶R⁷,
W is O or S,
R¹ is (C₁-C₆)-alkyl, (C₁-C₆)-haloalkyl, (C₂-C₆)-alkenyl, (C₂-C₆)-alkynyl, NR¹³R¹⁴, R¹³R¹⁴N-(C₁-C₆)-alkyl, (C₁-C₆)-alkoxy, (C₁-C₆)-haloalkoxy, (C₁-C₆)-haloalkoxy-(C₁-C₃)-alkyl, (C₁-C₆)-alkoxy-(C₁-C₃)-alkyl, (C₁-C₆)-alkoxy-(C₂-C₆)-alkoxy-(C₁-C₃)-alkyl, (C₁-C₄)-alkylthio, (C₁-C₄)-alkylsulphoxy, (C₁-C₄)-alkylsulphonyl, (C₁-C₄)-alkylthio-(C₁-C₃)-alkyl, (C₁-C₄)-alkylsulphoxy-(C₁-C₃)-alkyl, (C₁-C₄)-alkylsulphonyl-(C₁-C₃)-alkyl, (C₃-C₈)-cycloalkyl, (C₃-C₆)-cycloalkenyl, (C₃-C₆)-cycloalkyl-(C₁-C₃)-alkyl, (C₃-C₆)-cycloalkenyl-(C₁-C₃)-alkyl, (C₃-C₆)-cycloalkoxy, phenyl, heteroaryl, heterocyclyl, phenoxy, heteroaryloxy, heterocyclyloxy or a carbobicyclic residue, wherein each of the last-mentioned 12 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
R², R³ are each independently hydrogen, (C₁-C₆)-alkyl, (C₁-C₆)-haloalkyl, (C₂-C₆)-alkenyl, (C₂-C₆)-alkynyl, (C₁-C₆)-alkoxy-(C₁-C₃)-alkyl, (C₁-C₆)-alkoxy-(C₁-C₃)-alkylcarbonyl, (C₁-C₄)-alkylthio, (C₁-C₄)-haloalkylthio, (C₁-C₄)-alkylthiocarbonyl, (C₁-C₄)-haloalkylthiocarbonyl, (C₁-C₄)-alkylthio-(C₁-C₃)-alkyl, (C₁-C₄)-alkylsulphoxy-(C₁-C₃)-alkyl, (C₁-C₄)-alkylsulphonyl-(C₁-C₃)-alkyl, (C₁-C₄)-alkylthio-(C₁-C₃)-alkylcarbonyl, (C₁-C₄)-alkylsulphoxy-(C₁-C₃)-alkylcarbonyl, (C₁-C₄)-alkylsulphonyl-(C₁-C₃)-alkylcarbonyl, (C₁-C₆)-alkylcarbonyl, (C₁-C₆)-haloalkylcarbonyl, (C₂-C₆)-alkenylcarbonyl, (C₂-C₆)-alkynylcarbonyl, (C₁-C₆)-alkoxycarbonylcarbonyl, (C₁-C₆)-alkoxycarbonyl-(C₁-C₃)-alkylcarbonyl, (C₁-C₆)-alkoxycarbonyl, (C₁-C₆)-haloalkoxycarbonyl, (C₂-C₆)-alkenyloxycarbonyl, (C₃-C₆)-cycloalkyl, (C₃-C₆)-cycloalkyl-(C₁-C₆)-alkyl, (C₃-C₆)-cycloalkylcarbonyl, (C₃-C₆)-cycloalkyl-(C₁-C₆)-alkylcarbonyl, phenyl, phenyl-(C₁-C₃)-alkyl, heteroaryl, heteroaryl-(C₁-C₃)-alkyl, heterocyclyl, heterocyclyl-(C₁-C₃)-alkyl, phenylcarbonyl, phenyl-(C₁-C₆)-alkylcarbonyl, hetarylcarbonyl, hetaryl-(C₁-C₆)-alkylcarbonyl, heterocyclylcarbonyl, heterocyclyl-(C₁-C₆)-alkylcarbonyl, wherein each of the last-mentioned 16 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
or
NR²R³ is -N=CR⁸R⁹ or -N=S(O)ₙR¹⁰R¹¹,
R⁶, R⁷ are each independently hydrogen or (C₁-C₆)-alkyl,
R⁸, R⁹ are each independently hydrogen, (C₁-C₆)-alkyl, (C₁-C₆)-haloalkyl, (C₂-C₆)-alkenyl, (C₂-C₆)-alkynyl, (C₁-C₆)-alkoxy-(C₁-C₃)-alkyl, (C₁-C₄)-alkylthio-(C₁-C₃)-alkyl, (C₁-C₄)-alkylsulphoxy-(C₁-C₃)-alkyl, (C₁-C₄)-alkylsulphonyl-(C₁-C₃)-alkyl, (C₁-C₆)-alkoxy, (C₁-C₆)-haloalkoxy, (C₂-C₆)-alkenyloxy, NR¹³R¹⁴, (C₃-C₈)-cycloalkyl, (C₃-C₈)-cycloalkyl-(C₁-C₆)-alkyl, phenyl, phenyl-(C₁-C₃)-alkyl, heteroaryl, heteroaryl-(C₁-C₃)-alkyl, heterocyclyl, heterocyclyl-(C₁-C₃)-alkyl, wherein each of the last-mentioned 8 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
or
R⁸ and R⁹, together with the carbon atom to which they are attached, form a 3- to 6-membered unsaturated, partially saturated or saturated ring, which comprises in each case, in addition to the carbon atoms, p ring members from the group consisting of N(R¹²)ₘ, O and S(O)ₙ, and wherein said ring is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
R¹⁰, R¹¹ are each independently (C₁-C₆)-alkyl, (C₂-C₆)-alkenyl, (C₂-C₆)-alkynyl, (C₁-C₆)-alkoxy-(C₁-C₃)-alkyl, (C₁-C₄)-alkylthio-(C₁-C₃)-alkyl, (C₁-C₄)-alkylsulphoxy-(C₁-C₃)-alkyl, (C₁-C₄)-alkylsulphonyl-(C₁-C₃)-alkyl, (C₃-C₈)-cycloalkyl, (C₃-C₈)-cycloalkyl-(C₁-C₆)-alkyl, phenyl, phenyl-(C₁-C₃)-alkyl, heteroaryl, heteroaryl-(C₁-C₃)-alkyl, heterocyclyl, heterocyclyl-(C₁-C₃)-alkyl, wherein each of the last-mentioned 8 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
or
R¹⁰ and R¹¹, together with the sulphur atom to which they are attached, form a 3- to 6-membered unsaturated, partially saturated or saturated ring, which comprises in each case, in addition to the carbon atoms and in addition to the sulphur atom, p ring members from the group consisting of N(R¹²)ₘ, O and S(O)ₙ, and wherein said ring is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy or (C₁-C₄)-alkylsulphonyl,
R¹² is hydrogen, (C₁-C₆)-alkyl or (C₁-C₆)-alkylcarbonyl,
R¹³, R¹⁴ are each independently hydrogen, (C₁-C₆)-alkyl, (C₁-C₆)-alkylcarbonyl, (C₁-C₄)-alkylsulphonyl, phenyl, phenylcarbonyl, wherein each of the last-mentioned two residues is unsubstituted or is substituted by one or more residues from the group consisting of halogen, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl,
or
R¹³ and R¹⁴, together with the nitrogen atom to which they are attached, form a 3- to 8-membered unsaturated, partially saturated or saturated ring, which comprises in each case, in addition to the carbon atoms and in addition to the nitrogen atom, p ring members from the group consisting of N(R¹²)ₘ, O and S(O)ₙ, and wherein said ring is unsubstituted or is substituted by one or more residues from the group consisting of halogen, hydroxyl, (C₁-C₄)-alkyl, (C₁-C₄)-haloalkyl, (C₁-C₄)-alkoxy, (C₁-C₄)-haloalkoxy, and has q oxo groups,
n is independently selected from 0, 1 or 2,
m is independently selected from 0 or 1,
p is independently selected from 0, 1 or 2,
q is independently selected from 0, 1 or 2,
y is 0 or 1.

3. Compound of the formula (G) according to Claim 1 or 2, and/or a salt thereof, in which
A is CR⁶R⁷,
W is O or S,
R¹ is (C₁-C₆)-alkyl, (C₁-C₆)-haloalkyl, (C₂-C₆)-alkenyl, (C₂-C₆)-alkynyl, NR¹³R¹⁴, R¹³R¹⁴N-(C₁-C₆)-alkyl, (C₁-C₆)-alkoxy, (C₁-C₆)-haloalkoxy, (C₁-C₆)-alkoxy-(C₁-C₃)-alkyl, (C₁-C₆)-alkoxy-(C₂-C₆)-alkoxy-(C₁-C₃)-alkyl, (C₁-C₄)-alkylthiO, (C₁-C₄)-alkylsulphoxy, (C₁-C₄)-alkylsulphonyl, (C₁-C₄)-alkylthio-(C₁-C₃)-alkyl, (C₁-C₄)-alkylsulphoxy-(C₁-C₃)-alkyl, (C₁-C₄)-alkylsulphonyl-(C₁-C₃)-alkyl, (C₃-C₈)-cycloalkyl, (C₃-C₆)-cycloalkenyl, (C₃-C₆)-cycloalkyl-(C₁-C₃)-alkyl, (C₃-C₆)-cycloalkenyl-(C₁-C₃)-alkyl, (C₃-C₆)-cycloalkoxy, phenyl, heteroaryl, heterocyclyl, phenoxy, heteroaryloxy or heterocyclyloxy, wherein each of the last-mentioned 11 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
R², R³ are each independently hydrogen, (C₁-C₆)-alkyl, (C₁-C₆)-haloalkyl, (C₂-C₆)-alkenyl, (C₂-C₆)-alkynyl, (C₁-C₆)-alkoxy-(C₁-C₃)-alkyl, (C₁-C₆)-alkoxy-(C₁-C₃)-alkylcarbonyl, (C₁-C₄)-alkylthio, (C₁-C₄)-haloalkylthio, (C₁-C₄)-alkylthiocarbonyl, (C₁-C₄)-haloalkylthiocarbonyl, (C₁-C₄)-alkylthio-(C₁-C₃)-alkyl, (C₁-C₄)-alkylsulphoxy-(C₁-C₃)-alkyl, (C₁-C₄)-alkylsulphonyl-(C₁-C₃)-alkyl, (C₁-C₄)-alkylthio-(C₁-C₃)-alkylcarbonyl, (C₁-C₄)-alkylsulphoxy-(C₁-C₃)-alkylcarbonyl, (C₁-C₄)-alkylsulphonyl-(C₁-C₃)-alkylcarbonyl, (C₁-C₆)-alkylcarbonyl, (C₁-C₆)-haloalkylcarbonyl, (C₂-C₆)-alkenylcarbonyl, (C₂-C₆)-alkynylcarbonyl, (C₁-C₆)-alkoxycarbonylcarbonyl, (C₁-C₆)-alkoxycarbonyl-(C₁-C₃)-alkylcarbonyl, (C₁-C₆)-alkoxycarbonyl, (C₁-C₆)-haloalkoxycarbonyl, (C₂-C₆)-alkenyloxycarbonyl, (C₃-C₆)-cycloalkyl, (C₃-C₆)-cycloalkyl-(C₁-C₆)-alkyl, (C₃-C₆)-cycloalkylcarbonyl, (C₃-C₆)-cycloalkyl-(C₁-C₆)-alkylcarbonyl, phenyl, phenyl-(C₁-C₃)-alkyl, heteroaryl, heteroaryl-(C₁-C₃)-alkyl, heterocyclyl, heterocyclyl-(C₁-C₃)-alkyl, phenylcarbonyl, phenyl-(C₁-C₆)-alkylcarbonyl, hetarylcarbonyl, hetaryl-(C₁-C₆)-alkylcarbonyl, heterocyclylcarbonyl, heterocyclyl-(C₁-C₆)-alkylcarbonyl, wherein each of the last-mentioned 16 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
or
NR²R³ is -N=CR⁸R⁹ or -N=S(O)ₙR¹⁰R¹¹,
R⁶, R⁷ are each independently hydrogen or (C₁-C₄)-alkyl, preferably R⁶ and R⁷ independently are hydrogen or methyl,
R⁸, R⁹ are each independently hydrogen, (C₁-C₆)-alkyl, (C₁-C₆)-haloalkyl, (C₂-C₆)-alkenyl, (C₂-C₆)-alkynyl, (C₁-C₆)-alkoxy-(C₁-C₃)-alkyl, (C₁-C₄)-alkylthio-(C₁-C₃)-alkyl, (C₁-C₄)-alkylsulphoxy-(C₁-C₃)-alkyl, (C₁-C₄)-alkylsulphonyl-(C₁-C₃)-alkyl, (C₁-C₆)-alkoxy, (C₁-C₆)-haloalkoxy, (C₂-C₆)-alkenyloxy, NR¹³R¹⁴, (C₃-C₈)-cycloalkyl, (C₃-C₈)-eycloalkyl-(C₁-C₆)-alkyl, phenyl, phenyl-(C₁-C₃)-alkyl, heteroaryl, heteroaryl-(C₁-C₃)-alkyl, heterocyclyl, heterocyclyl-(C₁-C₃)-alkyl, wherein each of the last-mentioned 8 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
or
R⁸ and R⁹, together with the carbon atom to which they are attached, form a 3- to 6-membered unsaturated, partially saturated or saturated ring, which comprises in each case, in addition to the carbon atoms, p ring members from the group consisting of N(R¹²)ₘ, O and S(O)ₙ, and wherein said ring is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl,
R¹⁰, R¹¹ are each independently (C₁-C₆)-alkyl, (C₂-C₆)-alkenyl, (C₂-C₆)-alkynyl, (C₁-C₆)-alkoxy-(C₁-C₃)-alkyl, (C₁-C₄)-alkylthio-(C₁-C₃)-alkyl, (C₁-C₄)-alkylsulphoxy-(C₁-C₃)-alkyl, (C₁-C₄)-alkylsulphonyl-(C₁-C₃)-alkyl, (C₃-C₈)-cycloalkyl, (C₃-C₈)-cycloalkyl-(C₁-C₆)-alkyl, phenyl, phenyl-(C₁-C₃)-alkyl, heteroaryl, heteroaryl-(C₁-C₃)-alkyl, heterocyclyl, heterocyclyl-(C₁-C₃)-alkyl, wherein each of the last-mentioned 8 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
or
R¹⁰ and R¹¹, together with the sulphur atom to which they are attached, form a 3- to 6-membered unsaturated, partially saturated or saturated ring, which comprises in each case, in addition to the carbon atoms and in addition to the sulphur atom, p ring members from the group consisting of N(R¹²)ₘ, O and S(O)ₙ, and wherein said ring is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy or (C₁-C₄)-alkylsulphonyl,
R¹² is hydrogen, (C₁-C₆)-alkyl or (C₁-C₆)-alkylcarbonyl,
R¹³, R¹⁴ are each independently hydrogen, (C₁-C₆)-alkyl, (C₁-C₆)-alkylcarbonyl, (C₁-C₄)-alkylsulphonyl, phenyl, phenylcarbonyl, wherein each of the last-mentioned two residues is unsubstituted or is substituted by one or more residues from the group consisting of halogen, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl,
or
R¹³ and R¹⁴, together with the nitrogen atom to which they are attached, form a 3- to 8-membered unsaturated, partially saturated or saturated ring, which comprises in each case, in addition to the carbon atoms and in addition to the nitrogen atom, p ring members from the group consisting of N(R¹²)ₘ, O and S(O)ₙ, and wherein said ring is unsubstituted or is substituted by one or more residues from the group consisting of halogen, hydroxyl, (C₁-C₄)-alkyl, (C₁-C₄)-haloalkyl, (C₁-C₄)-alkoxy, (C₁-C₄)-haloalkoxy, and has q oxo groups,
n is independently selected from 0, 1 or 2,
m is independently selected from 0 or 1,
p is independently selected from 0, 1 or 2,
q is independently selected from 0 or 1,
y is 0 or 1.

4. Compound of the formula (G) according to any of Claims 1 to 3, and/or a salt thereof, in which
A is CR⁶R⁷,
W is O or S, preferably O,
R¹ is (C₁-C₆)-alkyl, (C₁-C₆)-haloalkyl, (C₂-C₆)-alkenyl, (C₁-C₆)-alkoxy-(C₁-C₃)-alkyl, (C₁-C₆)-alkoxy-(C₂-C₆)-alkoxy-(C₁-C₃)-alkyl, (C₁-C₄)-alkylsulphonyl, (C₁-C₄)-alkylthio-(C₁-C₃)-alkyl, (C₁-C₄)-alkylsulphoxy-(C₁-C₃)-alkyl, (C₁-C₄)-alkylsulphonyl-(C₁-C₃)-alkyl, (C₃-C₈)-cycloalkyl, (C₃-C₆)-cycloalkenyl, (C₃-C₆)-cycloalkyl-(C₁-C₃)-alkyl, (C₃-C₆)-cycloalkenyl-(C₁-C₃)-alkyl, (C₃-C₆)-cycloalkoxy, phenyl, heteroaryl, heterocyclyl, phenoxy, heteroaryloxy or heterocyclyloxy, wherein each of the last-mentioned 11 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
R², R³ are each independently hydrogen, (C₂-C₆)-alkynyl, (C₁-C₄)-alkylthio, (C₁-C₄)-haloalkylthio, (C₁-C₄)-alkylthiocarbonyl, (C₁-C₄)-haloalkylthiocarbonyl, (C₁-C₆)-alkoxy-(C₁-C₃)-alkylcarbonyl, (C₁-C₄)-alkylthio-(C₁-C₃)-alkylcarbonyl, (C₁-C₄)-alkylsulphoxy-(C₁-C₃)-alkylcarbonyl, (C₁-C₄)-alkylsulphonyl-(C₁-C₃)-alkylcarbonyl, (C₁-C₆)-alkylcarbonyl, (C₁-C₆)-haloalkylcarbonyl, (C₂-C₆)-alkenylcarbonyl, (C₂-C₆)-alkynylcarbonyl, (C₁-C₆)-alkoxycarbonylcarbonyl, (C₁-C₆)-alkoxycarbonyl-(C₁-C₃)-alkylcarbonyl, (C₁-C₆)-alkoxycarbonyl, (C₁-C₆)-haloalkoxycarbonyl, (C₂-C₆)-alkenyloxycarbonyl, (C₃-C₆)-cycloalkylcarbonyl, (C₃-C₆)-cycloalkyl-(C₁-C₆)-alkylcarbonyl, phenylcarbonyl, phenyl-(C₁-C₆)-alkylcarbonyl, hetarylcarbonyl, hetaryl-(C₁-C₆)-alkylcarbonyl, heterocyclylcarbonyl, heterocyclyl-(C₁-C₆)-alkylcarbonyl, wherein each of the last-mentioned 8 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
or
NR²R³ is -N=CR⁸R⁹ or -N=S(O)ₙR¹⁰R¹¹,
R⁶ is hydrogen,
R⁷ is hydrogen or methyl,
R⁸, R⁹ are each independently hydrogen, (C₁-C₆)-alkyl, (C₁-C₆)-haloalkyl, (C₂-C₆)-alkenyl, (C₂-C₆)-alkynyl, (C₁-C₆)-alkoxy-(C₁-C₃)-alkyl, (C₁-C₄)-alkylthio-(C₁-C₃)-alkyl, (C₁-C₄)-alkylsulphoxy-(C₁-C₃)-alkyl, (C₁-C₄)-alkylsulphonyl-(C₁-C₃)-alkyl, (C₁-C₆)-alkoxy, (C₁-C₆)-haloalkoxy, (C₂-C₆)-alkenyloxy, NR¹³R¹⁴, (C₃-C₈)-cycloalkyl, (C₃-C₈)-cycloalkyl-(C₁-C₆)-alkyl, phenyl, phenyl-(C₁-C₃)-alkyl, heteroaryl, heteroaryl-(C₁-C₃)-alkyl, heterocyclyl, heterocyclyl-(C₁-C₃)-alkyl, wherein each of the last-mentioned 8 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
or
R⁸ and R⁹, together with the carbon atom to which they are attached, form a 3- to 6-membered unsaturated, partially saturated or saturated ring, which comprises in each case, in addition to the carbon atoms, p ring members from the group consisting of N(R¹²)ₘ, O and S(O)ₙ, and wherein said ring is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl,
R¹⁰, R¹¹ are each independently (C₁-C₆)-alkyl, (C₂-C₆)-alkenyl, (C₂-C₆)-alkynyl, (C₁-C₆)-alkoxy-(C₁-C₃)-alkyl, (C₁-C₄)-alkylthio-(C₁-C₃)-alkyl, (C₁-C₄)-alkylsulphoxy-(C₁-C₃)-alkyl, (C₁-C₄)-alkylsulphonyl-(C₁-C₃)-alkyl, (C₃-C₈)-cycloalkyl, (C₃-C₈)-cycloalkyl-(C₁-C₆)-alkyl, phenyl, phenyl-(C₁-C₃)-alkyl, heteroaryl, heteroaryl-(C₁-C₃)-alkyl, heterocyclyl, heterocyclyl-(C₁-C₃)-alkyl, wherein each of the last-mentioned 8 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
or
R¹⁰ and R¹¹, together with the sulphur atom to which they are attached, form a 3- to 6-membered unsaturated, partially saturated or saturated ring, which comprises in each case, in addition to the carbon atoms and in addition to the sulphur atom, p ring members from the group consisting of N(R¹²)ₘ, O and S(O)ₙ, and wherein said ring is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy or (C₁-C₄)-alkylsulphonyl,
R¹² is hydrogen, (C₁-C₆)-alkyl or (C₁-C₆)-alkylcarbonyl,
R¹³, R¹⁴ are each independently hydrogen, (C₁-C₆)-alkyl, (C₁-C₆)-alkylcarbonyl, (C₁-C₄)-alkylsulphonyl, phenyl, phenylcarbonyl, wherein each of the last-mentioned two residues is unsubstituted or is substituted by one or more residues from the group consisting of halogen, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl,
or
R¹³ and R¹⁴, together with the nitrogen atom to which they are attached, form a 3- to 8-membered unsaturated, partially saturated or saturated ring, which comprises in each case, in addition to the carbon atoms and in addition to the nitrogen atom, p ring members from the group consisting of N(R¹²)ₘ, O and S(O)ₙ, and wherein said ring is unsubstituted or is substituted by one or more residues from the group consisting of halogen, hydroxyl, (C₁-C₄)-alkyl, (C₁-C₄)-haloalkyl, (C₁-C₄)-alkoxy, (C₁-C₄)-haloalkoxy, and has q oxo groups,
n is independently selected from 0, 1 or 2,
m is independently selected from 0 or 1,
p is independently selected from 0, 1 or 2,
q is independently selected from 0 or 1,
y is 0 or 1.

5. Compound of the formula (G) according to any of Claims 1 to 4, and/or a salt thereof, in which
A is CR⁶R⁷,
W is O or S, preferably O,
R¹ is (C₃-C₈)-cycloalkyl, (C₃-C₆)-cycloalkenyl, (C₃-C₆)-cycloalkyl-(C₁-C₃)-alkyl, (C₃-C₆)-cycloalkenyl-(C₁-C₃)-alkyl, phenyl, heteroaryl, heterocyclyl, wherein each of the last-mentioned 7 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
R², R³ are each independently hydrogen, (C₂-C₆)-alkynyl, (C₁-C₄)-alkylthio, (C₁-C₄)-haloalkylthio (wherein (C₁-C₄)-haloalkylthio more preferably is SCF₃), (C₁-C₄)-alkylthiocarbonyl, (C₁-C₄)-haloalkylthiocarbonyl, (C₁-C₆)-alkoxy-(C₁-C₃)-alkylcarbonyl, (C₁-C₄)-alkylthio-(C₁-C₃)-alkylcarbonyl, (C₁-C₄)-alkylsulphoxy-(C₁-C₃)-alkylcarbonyl, (C₁-C₄)-alkylsulphonyl-(C₁-C₃)-alkylcarbonyl, (C₁-C₆)-alkylcarbonyl, (C₁-C₆)-haloalkylcarbonyl, (C₂-C₆)-alkenylcarbonyl, (C₂-C₆)-alkynylcarbonyl, (C₁-C₆)-alkoxycarbonylcarbonyl, (C₁-C₆)-alkoxycarbonyl-(C₁-C₃)-alkylcarbonyl, (C₁-C₆)-alkoxycarbonyl, (C₁-C₆)-haloalkoxycarbonyl, (C₂-C₆)-alkenyloxycarbonyl, (C₃-C₆)-cycloalkylcarbonyl, (C₃-C₆)-cycloalkyl-(C₁-C₆)-alkylcarbonyl, phenylcarbonyl, phenyl-(C₁-C₆)-alkylcarbonyl, hetarylcarbonyl, hetaryl-(C₁-C₆)-alkylcarbonyl, heterocyclylcarbonyl, heterocyclyl-(C₁-C₆)-alkylcarbonyl, wherein each of the last-mentioned 8 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
or
NR²R³ is -N=CR⁸R⁹ or -N=S(O)ₙR¹⁰R¹¹,
R⁶ is hydrogen,
R⁷ is hydrogen or methyl,
R⁸, R⁹ are each independently hydrogen, (C₁-C₆)-alkyl, (C₁-C₆)-haloalkyl, (C₂-C₆)-alkenyl, (C₂-C₆)-alkynyl, (C₁-C₆)-alkoxy-(C₁-C₃)-alkyl, (C₁-C₄)-alkylthio-(C₁-C₃)-alkyl, (C₁-C₄)-alkylsulphoxy-(C₁-C₃)-alkyl, (C₁-C₄)-alkylsulphonyl-(C₁-C₃)-alkyl, (C₁-C₆)-alkoxy, (C₁-C₆)-haloalkoxy, (C₂-C₆)-alkenyloxy, NR¹³R¹⁴, (C₃-C₈)-cycloalkyl, (C₃-C₈)-eycloalkyl-(C₁-C₆)-alkyl, phenyl, phenyl-(C₁-C₃)-alkyl, heteroaryl, heteroaryl-(C₁-C₃)-alkyl, heterocyclyl, heterocyclyl-(C₁-C₃)-alkyl, wherein each of the last-mentioned 8 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
or
R⁸ and R⁹, together with the carbon atom to which they are attached, form a 3- to 6-membered unsaturated, partially saturated or saturated ring, which comprises in each case, in addition to the carbon atoms, p ring members from the group consisting of N(R¹²)ₘ, O and S(O)ₙ, and wherein said ring is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl,
R¹⁰, R¹¹ are each independently (C₁-C₆)-alkyl, (C₂-C₆)-alkenyl, (C₂-C₆)-alkynyl, (C₃-C₈)-cycloalkyl, (C₃-C₈)-cycloalkyl-(C₁-C₆)-alkyl, phenyl, phenyl-(C₁-C₃)-alkyl, heteroaryl, heteroaryl-(C₁-C₃)-alkyl, heterocyclyl, heterocyclyl-(C₁-C₃)-alkyl, wherein each of the last-mentioned 8 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
or
R¹⁰ and R¹¹, together with the sulphur atom to which they are attached, form a 3- to 6-membered unsaturated, partially saturated or saturated ring, which comprises in each case, in addition to the carbon atoms and in addition to the sulphur atom, p ring members from the group consisting of N(R¹²)ₘ, O and S(O)ₙ, and wherein said ring is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy or (C₁-C₄)-alkylsulphonyl,
R¹² is hydrogen, (C₁-C₆)-alkyl or (C₁-C₆)-alkylcarbonyl,
R¹³, R¹⁴ are each independently hydrogen, (C₁-C₆)-alkyl, (C₁-C₆)-alkylcarbonyl, (C₁-C₄)-alkylsulphonyl, phenyl, phenylcarbonyl, wherein each of the last-mentioned two residues is unsubstituted or is substituted by one or more residues from the group consisting of halogen, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl,
or
R¹³ and R¹⁴, together with the nitrogen atom to which they are attached, form a 3- to 8-membered unsaturated, partially saturated or saturated ring, which comprises in each case, in addition to the carbon atoms and in addition to the nitrogen atom, p ring members from the group consisting of N(R¹²)ₘ, O and S(O)ₙ, and wherein said ring is unsubstituted or is substituted by one or more residues from the group consisting of halogen, hydroxyl, (C₁-C₄)-alkyl, (C₁-C₄)-haloalkyl, (C₁-C₄)-alkoxy, (C₁-C₄)-haloalkoxy, and has q oxo groups,
n is independently selected from 0 or 1,
m is independently selected from 0 or 1,
p is independently selected from 0, 1 or 2,
q is independently selected from 0 or 1,
y is 0 or 1.

6. Compound of the formula (G) according to any of Claims 1 to 5, and/or a salt thereof, in which
A is CR⁶R⁷,
W is O,
R¹ is (C₃-C₆)-cycloalkyl, (C₃-C₆)-cycloalkenyl, (C₃-C₆)-cycloalkyl-(C₁-C₃)-alkyl, (C₃-C₆)-cycloalkenyl-(C₁-C₃)-alkyl, phenyl, heteroaryl, heterocyclyl, wherein each of the last-mentioned 7 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
R², R³ are each independently hydrogen, (C₁-C₄)-alkylthiocarbonyl, (C₁-C₄)-haloalkylthiocarbonyl, (C₁-C₆)-alkoxy-(C₁-C₃)-alkylcarbonyl, (C₁-C₄)-alkylthio-(C₁-C₃)-alkylcarbonyl, (C₁-C₄)-alkylsulphoxy-(C₁-C₃)-alkylcarbonyl, (C₁-C₄)-alkylsulphonyl-(C₁-C₃)-alkylcarbonyl, (C₁-C₆)-alkylcarbonyl, (C₁-C₆)-haloalkylcarbonyl, (C₂-C₆)-alkenylcarbonyl, (C₂-C₆)-alkynylcarbonyl, (C₁-C₆)-alkoxycarbonylcarbonyl, (C₁-C₆)-alkoxycarbonyl-(C₁-C₃)-alkylcarbonyl, (C₁-C₆)-alkoxycarbonyl, (C₁-C₆)-haloalkoxycarbonyl, (C₂-C₆)-alkenyloxycarbonyl, (C₃-C₆)-cycloalkylcarbonyl, (C₃-C₆)-cycloalkyl-(C₁-C₆)-alkylcarbonyl, phenylcarbonyl, phenyl-(C₁-C₆)-alkylcarbonyl, hetarylcarbonyl, hetaryl-(C₁-C₆)-alkylcarbonyl, heterocyclylcarbonyl, heterocyclyl-(C₁-C₆)-alkylcarbonyl, wherein each of the last-mentioned 8 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
or
NR²R³ is -N=CR⁸R⁹ or -N=S(O)ₙR¹⁰R¹¹,
R⁶ is hydrogen,
R⁷ is hydrogen or methyl,
R⁸, R⁹ are each independently hydrogen, (C₁-C₆)-alkyl, (C₁-C₆)-haloalkyl, (C₂-C₆)-alkenyl, (C₂-C₆)-alkynyl, (C₁-C₆)-alkoxy-(C₁-C₃)-alkyl, (C₁-C₄)-alkylthio-(C₁-C₃)-alkyl, (C₁-C₄)-alkylsulphoxy-(C₁-C₃)-alkyl, (C₁-C₄)-alkylsulphonyl-(C₁-C₃)-alkyl, (C₁-C₆)-alkoxy, (C₁-C₆)-haloalkoxy, (C₂-C₆)-alkenyloxy, NR¹³R¹⁴, (C₃-C₈)-cycloalkyl, (C₃-C₈)-eycloalkyl-(C₁-C₆)-alkyl, phenyl, phenyl-(C₁-C₃)-alkyl, heteroaryl, heteroaryl-(C₁-C₃)-alkyl, heterocyclyl, heterocyclyl-(C₁-C₃)-alkyl, wherein each of the last-mentioned 8 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
or
R⁸ and R⁹, together with the carbon atom to which they are attached, form a 3- to 6-membered unsaturated, partially saturated or saturated ring, which comprises in each case, in addition to the carbon atoms, p ring members from the group consisting of N(R¹²)ₘ, O and S(O)ₙ, and wherein said ring is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl,
R¹⁰, R¹¹ are each independently (C₁-C₆)-alkyl, (C₂-C₆)-alkenyl, (C₂-C₆)-alkynyl, (C₃-C₈)-cycloalkyl, (C₃-C₈)-cycloalkyl-(C₁-C₆)-alkyl, phenyl, phenyl-(C₁-C₃)-alkyl, heteroaryl, heteroaryl-(C₁-C₃)-alkyl, heterocyclyl, heterocyclyl-(C₁-C₃)-alkyl, wherein each of the last-mentioned 8 residues is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylsulphoxy, (C₁-C₄)-alkylsulphonyl, and wherein heterocyclyl has q oxo groups,
or
R¹⁰ and R¹¹, together with the sulphur atom to which they are attached, form a 3- to 6-membered unsaturated, partially saturated or saturated ring, which comprises in each case, in addition to the carbon atoms and in addition to the sulphur atom, p ring members from the group consisting of N(R¹²)ₘ, O and S(O)ₙ, and wherein said ring is unsubstituted or is substituted by one or more residues from the group consisting of halogen, nitro, hydroxyl, cyano, NR¹³R¹⁴, (C₁-C₄)-alkyl, (C₁-C₄)-haloalkyl, (C₁-C₄)-alkoxy, (C₁-C₄)-haloalkoxy, (C₁-C₄)-alkylthiO, (C₁-C₄)-alkylsulphoxy or (C₁-C₄)-alkylsulphonyl,
R¹² is hydrogen, (C₁-C₆)-alkyl or (C₁-C₆)-alkylcarbonyl,
R¹³, R¹⁴ are each independently hydrogen, (C₁-C₆)-alkyl, (C₁-C₆)-alkylcarbonyl, (C₁-C₄)-alkylsulphonyl, phenyl, phenylcarbonyl, wherein each of the last-mentioned two residues is unsubstituted or is substituted by one or more residues from the group consisting of halogen, (C₁-C₄)-alkyl, (C₁-C₄)-haloalkyl, (C₁-C₄)-alkoxy, (C₁-C₄)-haloalkoxy, (C₁-C₄)-alkylthiO, (C₁-C₄)-alkylsulphoxy, (C₁-C₄)-alkylsulphonyl,
or
R¹³ and R¹⁴, together with the nitrogen atom to which they are attached, form a 3- to 8-membered unsaturated, partially saturated or saturated ring, which comprises in each case, in addition to the carbon atoms and in addition to the nitrogen atom, p ring members from the group consisting of N(R¹²)ₘ, O and S(O)ₙ, and wherein said ring is unsubstituted or is substituted by one or more residues from the group consisting of halogen, hydroxyl, (C₁-C₄)-alkyl, (C₁-C₄)-haloalkyl, (C₁-C₄)-alkoxy, (C₁-C₄)-haloalkoxy, and has q oxo groups,
n is 0,
m is independently selected from 0 or 1,
p is independently selected from 0, 1 or 2,
q is independently selected from 0 or 1,
y is 0 or 1.

7. Compound of the formula (G) according to any of Claims 1 to 6, and/or a salt thereof, in which
n is independently selected from 0 or 1, preferably n is 0,
m is independently selected from 0 or 1, preferably m is 0,
p is independently selected from 0 or 1, and
q is independently selected from 0 or 1, preferably q is 0.

8. Compound of the formula (G) according to any of Claims 1 to 7, and/or a salt thereof, in which y is 1.

9. Use of one or more compounds of the formula (G) and/or salts thereof as defined in any of Claims 1 to 8 as herbicides and/or plant growth regulators.

10. Use of one or more compounds of the formula (G) and/or salts thereof according to Claim 9 for controlling harmful plants or for regulating the growth of plants, wherein one or more compounds of the formula (G) and/or salts thereof are applied onto the harmful plants, plant seeds, the soil in which or on which the plants grow or the area under cultivation.

11. Herbicidal and/or plant growth-regulating composition, **characterized in that** said composition comprises one or more compounds of the formula (G) and/or salts thereof as defined in any of Claims 1 to 8,
and one or more further substances selected from groups (i) and/or (ii):
(i) one or more further agrochemically active substances, preferably selected from the group consisting of insecticides, acaricides, nematicides, further herbicides, fungicides, safeners, fertilizers and/or further growth regulators,
(ii) one or more formulation auxiliaries customary in crop protection.

12. Method for controlling harmful plants or for regulating the growth of plants, **characterized in that** an effective amount of
- one or more compounds of the formula (G) and/or salts thereof, as defined in any of Claims 1 to 8,
or
- a composition according to Claim 11,
is applied to the plants, seeds of plants, the soil in which or on which the plants grow or the area under cultivation.

13. Process for preparing a compound of the formula (G) as defined in any one of Claims 1 to 8, and/or a salt thereof, wherein W is oxygen, **characterized in that**
(a) a compound of formula (G) wherein R² and R³ both are H is obtained in a chemical synthesis comprising the step of cyclization of a compound of the formula (E-XXIV) 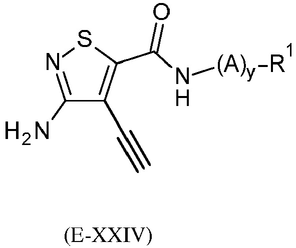 in which R¹, A and y each have the meaning as defined in formula (G),
or
(b) a compound of formula (G) wherein R² and R³ both are H is obtained in a chemical synthesis comprising the step of cyclization of a compound of the formula (E- XXVII) 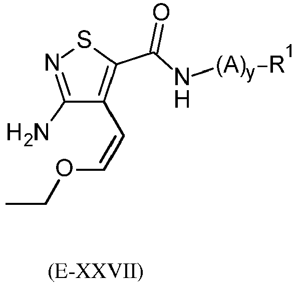 in which R¹, A and y each have the meaning as defined in formula (G),
or
(c) a compound of formula (G), wherein R² and/or R³ are not H is obtained in a chemical synthesis comprising the step of reacting a compound of the formula (G-1) 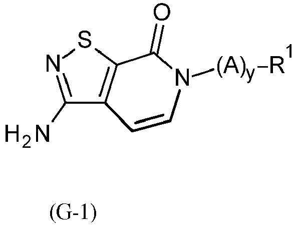 in which R¹, A and y each have the meaning as defined in formula (G),
with an acyl halogenide R²COHal and/or R³COHal, wherein Hal in each case is Cl, Br or I, or an anyhdride (R²CO)₂O, (R³CO)₂O and/or R²CO(O)OCR³, and wherein R² and R³ have the meaning as defined in formula (G), provided that R² and/or R³ are not H.

## Patentansprüche

1. Verbindung der Formel (G) und/oder ein Salz davon, 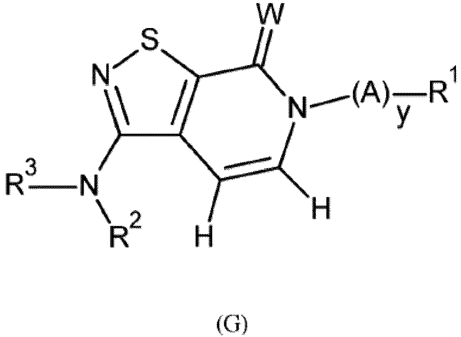 wobei
A für CR⁶R⁷ steht,
W für O oder S steht,
R¹ für (C₁-C₁₂)-Alkyl, (C₁-C₁₂)-Halogenalkyl, (C₂-C₁₂)-Alkenyl, (C₂-C₁₂)-Halogenalkenyl, (C₂-C₁₂)-Alkinyl, (C₂-C₁₂)-Halogenalkinyl, NR¹³R¹⁴, R¹³R¹⁴N-(C₁-C₆)-Alkyl, (C₁-C₆)-Alkoxy, (C₁-C₆)-Halogenalkoxy, (C₁-C₆)-Halogenalkoxy-(C₁-C₃)-alkyl, (C₁-C₆)-Alkoxy-(C₁-C₃)-alkyl, (C₁-C₆)-Alkoxy-(C₂-C₆)-alkoxy-(C₁-C₃)-alkyl, (C₁-C₄)-Alkylthio, (C₁-C₄)-Alkylsulfoxy, (C₁-C₄)-Alkylsulfonyl, (C₁-C₄)-Halogenalkylthio, (C₁-C₄)-Halogenalkylsulfoxy, (C₁-C₄)-Halogenalkylsulfonyl, (C₁-C₄)-Alkylthio-(C₁-C₃)-alkyl, (C₁-C₄)-Alkylsulfoxy-(C₁-C₃)-alkyl, (C₁-C₄)-Alkylsulfonyl-(C₁-C₃)-alkyl, (C₁-C₄)-Halogenalkylthio-(C₁-C₃)-alkyl, (C₁-C₄)-Halogenalkylsulfoxy-(C₁-C₃)-alkyl, (C₁-C₄)-Halogenalkylsulfonyl-(C₁-C₃)-alkyl, (C₃-C₁₂)-Cycloalkyl, (C₃-C₈)-Cycloalkenyl, (C₃-C₁₂)-Cycloalkyl-(C₁-C₆)-alkyl, (C₃-C₈)-Cycloalkenyl-(C₁-C₆)-alkyl, (C₃-C₈)-Cycloalkoxy, (C₃-C₈)-Cycloalkyl-(C₁-C₆)-alkoxy, Aryl, Aryl-(C₁-C₃)-alkyl, Heteroaryl, Heteroaryl-(C₁-C₃)-alkyl, Heterocyclyl, Heterocyclyl-(C₁-C₃)-alkyl, Aryloxy, Heteroaryloxy, Heterocyclyloxy, einen bicyclischen oder einen heterobicyclischen Rest steht, wobei die 17 letztgenannten Reste jeweils unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy, (C₁-C₄)-Alkylsulfonyl, (C₁-C₄)-Halogenalkylthio, (C₁-C₄)-Halogenalkylsulfoxy, (C₁-C₄)-Halogenalkylsulfonyl, (C₁-C₄)-Alkoxycarbonyl, (C₁-C₄)-Halogenalkoxycarbonyl, (C₁-C₄)-Alkylcarboxy, (C₃-C₆)-Cycloalkyl, (C₃-C₆)-Cycloalkyl-(C₁-C₆)-alkyl, (C₁-C₄)-Alkoxycarbonyl-(C₁-C₄)-alkyl, Hydroxycarbonyl, Hydroxycarbonyl-(C₁-C₄)-alkyl, R¹³R¹⁴N-Carbonyl substituiert sind und wobei Heterocyclyl q Oxogruppen aufweist und wobei die oben erwähnten heterocyclischen Reste jeweils zusätzliche zu den Kohlenstoffatomen jeweils p Ringglieder aus der aus N(R¹²)ₘ, O und S(O)ₙ bestehenden Gruppe aufweisen,
R², R³ jeweils unabhängig für Wasserstoff, (C₁-C₁₂)-Alkyl, (C₁-C₁₂)-Halogenalkyl, (C₂-C₁₂)-Alkenyl, (C₂-C₁₂)-Halogenalkenyl, (C₂-C₁₂)-Alkinyl, (C₂-C₁₂)-Halogenalkinyl, (C₁-C₆)-Alkoxy-(C₁-C₃)-alkyl, (C₁-C₆)-Alkoxy-(C₂-C₆)-alkoxy-(C₁-C₃)-alkyl, (C₁-C₆)-Alkoxy-(C₁-C₃)-alkylcarbonyl, (C₁-C₆)-Alkoxy-(C₂-C₆)-alkoxy-(C₁-C₃)-alkylcarbonyl, (C₁-C₆)-Alkoxycarbonyl, (C₁-C₆)-Halogenalkoxycarbonyl, (C₂-C₆)-Alkenyloxycarbonyl, (C₂-C₆)-Halogenalkenyloxycarbonyl, (C₂-C₆)-Alkinyloxycarbonyl, (C₂-C₆)-Halogenalkinyloxycarbonyl, (C₁-C₆)-Alkylcarbonyl, (C₁-C₆)-Halogenalkylcarbonyl, (C₂-C₆)-Alkenylcarbonyl, (C₂-C₆)-Halogenalkenylcarbonyl, (C₂-C₆)-Alkinylcarbonyl, (C₂-C₆)-Halogenalkinylcarbonyl, R¹³R¹⁴N-Carbonyl, (C₁-C₄)-Alkylthio, (C₁-C₄)-Halogenalkylthio, (C₁-C₈)-Alkylthiocarbonyl, (C₁-C₈)-Halogenalkylthiocarbonyl, (C₁-C₄)-Alkylsulfoxy, (C₁-C₄)-Halogenalkylsulfoxy, (C₁-C₄)-Alkylsulfonyl, (C₁-C₄)-Halogenalkylsulfonyl, (C₁-C₄)-Alkylthio-(C₁-C₃)-alkyl, (C₁-C₄)-Alkylsulfoxy-(C₁-C₃)-alkyl, (C₁-C₄)-Alkylsulfonyl-(C₁-C₃)-alkyl, (C₁-C₄)-Alkylthio-(C₁-C₃)-alkylcarbonyl, (C₁-C₄)-Alkylsulfoxy-(C₁-C₃)-alkylcarbonyl, (C₁-C₄)-Alkylsulfonyl-(C₁-C₃)-alkylcarbonyl, (C₁-C₁₂)-Alkylcarbonyl, (C₁-C₁₂)-Halogenalkylcarbonyl, (C₂-C₁₂)-Alkenylcarbonyl, (C₂-C₁₂)-Halogenalkenylcarbonyl, (C₂-C₁₂)-Alkinylcarbonyl, (C₂-C₁₂)-Halogenalkinylcarbonyl, (C₁-C₁₂)-Alkoxycarbonylcarbonyl, (C₁-C₁₂)-Alkoxycarbonyl-(C₁-C₃)-alkylcarbonyl, (C₃-C₈)-Cycloalkyl, (C₃-C₈)-Cycloalkenyl, (C₃-C₈)-Cycloalkyl-(C₁-C₆)-alkyl, (C₃-C₈)-Cycloalkenyl-(C₁-C₆)-alkyl, (C₃-C₈)-Cycloalkylcarbonyl, (C₃-C₈)-Cycloalkenylcarbonyl, (C₃-C₈)-Cycloalkyl-(C₁-C₆)-alkylcarbonyl, (C₃-C₈)-Cycloalkenyl-(C₁-C₆)-alkylcarbonyl, Aryl, Aryl-(C₁-C₃)-alkyl, Heteroaryl, Heteroaryl-(C₁-C₃)-alkyl, Heterocyclyl, Heterocyclyl-(C₁-C₃)-alkyl, Arylcarbonyl, Aryl-(C₁-C₆)-alkylcarbonyl, Heteroarylcarbonyl, Heteroaryl-(C₁-C₆)-alkylcarbonyl, Heterocyclylcarbonyl oder Heterocyclyl-(C₁-C₆)-alkylcarbonyl stehen, wobei die 20 letztgenannten Reste jeweils unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy, (C₁-C₄)-Alkylsulfonyl, (C₁-C₄)-Halogenalkylthio, (C₁-C₄)-Halogenalkylsulfoxy, (C₁-C₄)-Halogenalkylsulfonyl, (C₁-C₄)-Alkoxycarbonyl, (C₁-C₄)-Halogenalkoxycarbonyl, (C₁-C₄)-Alkylcarboxy, (C₃-C₆)-Cycloalkyl, (C₃-C₆)-Cycloalkyl-(C₁-C₆)-alkyl, (C₁-C₄)-Alkoxycarbonyl-(C₁-C₄)-alkyl, Hydroxycarbonyl, Hydroxycarbonyl-(C₁-C₄)-alkyl und R¹³R¹⁴N_Carbonyl substituiert sind und wobei Heterocyclyl q Oxogruppen aufweist,
oder
NR²R³ für -N=CR⁸R⁹ oder -N=S(O)ₙR¹⁰R¹¹ steht,
R⁶, R⁷ jeweils unabhängig für Wasserstoff, Cyano, Halogen, (C₁-C₆)-Alkyl, (C₂-C₆)-Alkenyl, (C₂-C₆)-Alkinyl oder (C₃-C₈)-Cycloalkyl stehen,
oder
R⁶ und R⁷ zusammen mit dem Kohlenstoffatom, an das sie gebunden sind, einen 3- bis 6-gliedrigen carbocyclischen oder heterocyclischen Ring bilden, der jeweils zusätzlich zu den Kohlenstoffatomen p Ringglieder aus der aus N(R¹²)ₘ, O und S(O)ₙ bestehenden Gruppe aufweist, und wobei der Ring unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy, (C₁-C₄)-Alkylsulfonyl, (C₁-C₄)-Halogenalkylthio, (C₁-C₄)-Halogenalkylsulfoxy, (C₁-C₄)-Halogenalkylsulfonyl, (C₁-C₄)-Alkoxycarbonyl, (C₁-C₄)-Halogenalkoxycarbonyl, (C₁-C₄)-Alkylcarboxy, (C₃-C₆)-Cycloalkyl, (C₃-C₆)-Cycloalkyl-(C₁-C₆)-alkyl, (C₁-C₄)-Alkoxycarbonyl-(C₁-C₄)-alkyl, Hydroxycarbonyl, Hydroxycarbonyl-(C₁-C₄)-alkyl und R¹³R¹⁴N-Carbonyl substituiert ist und q Oxogruppen aufweist,
R⁸, R⁹ jeweils unabhängig für Wasserstoff, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₁-C₆)-Alkoxy, (C₁-C₆)-Halogenalkoxy, (C₁-C₆)-Halogenalkoxy-(C₁-C₃)-alkyl, (C₂-C₆)-Alkenyloxy, (C₂-C₆)-Halogenalkenyloxy, (C₂-C₆)-Alkinyloxy, (C₂-C₆)-Halogenalkinyloxy, NR¹³R¹⁴, (C₁-C₆)-Alkoxy-(C₁-C₃)-alkyl, Halogen-(C₁-C₆)-alkoxy-(C₁-C₆)-alkyl, (C₁-C₆)-Alkoxy-(C₂-C₆)-alkoxy-(C₁-C₃)-alkyl, (C₁-C₄)-Alkylthio-(C₁-C₃)-alkyl, (C₁-C₄)-Alkylsulfoxy-(C₁-C₃)-alkyl, (C₁-C₄)-Alkylsulfonyl-(C₁-C₃)-alkyl, (C₃-C₈)-Cycloalkyl, (C₃-C₈)-Cycloalkenyl, (C₃-C₈)-Cycloalkyl-(C₁-C₆)-alkyl, (C₃-C₈)-Cycloalkenyl-(C₁-C₆)-alkyl, Aryl, Aryl-(C₁-C₃)-alkyl, Heteroaryl, Heteroaryl-(C₁-C₃)-alkyl, Heterocyclyl oder Heterocyclyl-(C₁-C₃)-alkyl stehen, wobei die 10 letztgenannten Reste jeweils unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy, (C₁-C₄)-Alkylsulfonyl, (C₁-C₄)-Halogenalkylthio, (C₁-C₄)-Halogenalkylsulfoxy, (C₁-C₄)-Halogenalkylsulfonyl, (C₁-C₄)-Alkoxycarbonyl, (C₁-C₄)-Halogenalkoxycarbonyl, (C₁-C₄)-Alkylcarboxy, (C₃-C₆)-Cycloalkyl, (C₃-C₆)-Cycloalkyl-(C₁-C₆)-alkyl, (C₁-C₄)-Alkoxycarbonyl-(C₁-C₄)-alkyl, Hydroxycarbonyl, Hydroxycarbonyl-(C₁-C₄)-alkyl und R¹³R¹⁴N-Carbonyl substituiert sind und q Oxogruppen aufweisen,
oder R⁸ und R⁹ zusammen mit dem Kohlenstoffatom, an das sie gebunden sind, einen 3- bis 8-gliedrigen ungesättigten, teilweise gesättigten oder gesättigten Ring bilden, der jeweils zusätzlich zu den Kohlenstoffatomen p Ringglieder aus der aus N(R¹²)ₘ, O und S(O)ₙ bestehenden Gruppe aufweist, und wobei der Ring unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy, (C₁-C₄)-Alkylsulfonyl, (C₁-C₄)-Halogenalkylthio, (C₁-C₄)-Halogenalkylsulfoxy, (C₁-C₄)-Halogenalkylsulfonyl, (C₁-C₄)-Alkoxycarbonyl, (C₁-C₄)-Halogenalkoxycarbonyl, (C₁-C₄)-Alkylcarboxy, (C₃-C₆)-Cycloalkyl, (C₃-C₆)-Cycloalkyl-(C₁-C₆)-alkyl, (C₁-C₄)-Alkoxycarbonyl-(C₁-C₄)-alkyl, Hydroxycarbonyl, Hydroxycarbonyl-(C₁-C₄)-alkyl und R¹³R¹⁴N-Carbonyl substituiert ist und q Oxogruppen aufweist,
R¹⁰, R¹¹ jeweils unabhängig für (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₁-C₆)-Alkoxy-(C₁-C₃)-alkyl, Halogen-(C₁-C₆)-alkoxy-(C₁-C₆)-alkyl, (C₁-C₆)-Alkoxy-(C₂-C₆)-alkoxy-(C₁-C₃)-alkyl, (C₁-C₄)-Alkylthio-(C₁-C₃)-alkyl, (C₁-C₄)-Alklysulfoxy-(C₁-C₃)-alkyl, (C₁-C₄)-Alkylsulfonyl-(C₁-C₃)-alkyl, (C₃-C₈)-Cycloalkyl, (C₃-C₈)-Cycloalkenyl, (C₃-C₈)-Cycloalkyl-(C₁-C₆)-alkyl, (C₃-C₈)-Cycloalkenyl-(C₁-C₆)-alkyl, Aryl, Aryl-(C₁-C₃)-alkyl, Heteroaryl, Heteroaryl-(C₁-C₃)-alkyl, Heterocyclyl oder Heterocyclyl-(C₁-C₃)-alkyl stehen, wobei die 10 letztgenannten Reste jeweils unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy, (C₁-C₄)-Alkylsulfonyl, (C₁-C₄)-Halogenalkylthio, (C₁-C₄)-Halogenalkylsulfoxy, (C₁-C₄)-Halogenalkylsulfonyl, (C₁-C₄)-Alkoxycarbonyl, (C₁-C₄)-Halogenalkoxycarbonyl, (C₁-C₄)-Alkylcarboxy, (C₃-C₆)-Cycloalkyl, (C₃-C₆)-Cycloalkyl-(C₁-C₆)-alkyl, (C₁-C₄)-Alkoxycarbonyl-(C₁-C₄)-alkyl, Hydroxycarbonyl, Hydroxycarbonyl-(C₁-C₄)-alkyl und R¹³R¹⁴N_Carbonyl substituiert sind und wobei Heterocyclyl q Oxogruppen aufweist,
oder
R¹⁰ und R¹¹ zusammen mit dem Schwefelatom, an das sie gebunden sind, einen 3- bis 8-gliedrigen ungesättigten, teilweise gesättigten oder gesättigten Ring bilden, der jeweils zusätzlich zu den Kohlenstoffatomen und zusätzlich zu dem Schwefelatom p Ringglieder aus der aus N(R¹²)ₘ, O und S(O)ₙ bestehenden Gruppe umfasst, und wobei der Ring unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy, (C₁-C₄)-Alkylsulfonyl, (C₁-C₄)-Halogenalkylthio, (C₁-C₄)-Halogenalkylsulfoxy, (C₁-C₄)-Halogenalkylsulfonyl, (C₁-C₄)-Alkoxycarbonyl, (C₁-C₄)-Halogenalkoxycarbonyl, (C₁-C₄)-Alkylcarboxy, (C₃-C₆)-Cycloalkyl, (C₃-C₆)-Cycloalkyl-(C₁-C₆)-alkyl, (C₁-C₄)-Alkoxycarbonyl-(C₁-C₄)-alkyl, Hydroxycarbonyl, Hydroxycarbonyl-(C₁-C₄)-alkyl und R¹³R¹⁴-Carbonyl substituiert ist und q Oxogruppen aufweist,
R¹² für Wasserstoff, (C₁-C₁₂)-Alkyl, (C₁-C₁₂)-Halogenalkyl, (C₂-C₁₂)-Alkenyl, (C₂-C₁₂)-Halogenalkenyl, (C₂-C₁₂)-Alkinyl, (C₂-C₁₂)-Halogenalkinyl, (C₃-C₈)-Cycloalkyl, (C₃-C₈)-Halogencycloalkyl, (C₃-C₈)-Cycloalkenyl, (C₃-C₈)-Cycloalkyl-(C₁-C₆)-alkyl, (C₃-C₈)-Cycloalkenyl-(C₁-C₆)-alkyl, (C₁-C₁₂)-Alkylcarbonyl oder (C₁-C₁₂)-Halogenalkylcarbonyl steht,
R¹³, R¹⁴ jeweils unabhängig für Wasserstoff, (C₁-C₁₂)-Alkyl, (C₁-C₁₂)-Halogenalkyl, (C₂-C₁₂)-Alkenyl, (C₂-C₁₂)-Halogenalkenyl, (C₂-C₁₂)-Alkinyl, (C₂-C₁₂)-Halogenalkinyl, (C₁-C₁₂)-Alkylcarbonyl, (C₂-C₁₂)-Alkenylcarbonyl, (C₂-C₁₂)-Alkinylcarbonyl, (C₁-C₁₂)-Halogenalkylcarbonyl, (C₁-C₄)-Alkylsulfonyl, (C₃-C₈)-Cycloalkyl, (C₃-C₈)-Cycloalkenyl, (C₃-C₈)-Cycloalkyl-(C₁-C₆)-alkyl, (C₃-C₈)-Cycloalkenyl-(C₁-C₆)-alkyl, (C₃-C₈)-Cycloalkylcarbonyl, (C₃-C₈)-Cycloalkenylcarbonyl, (C₃-C₈)-Cycloalkyl-(C₁-C₆)-alkylcarbonyl, (C₃-C₈)-Cycloalkenyl-(C₁-C₆)-alkylcarbonyl, Aryl, Arylcarbonyl, Arylsulfonyl, Hetaryl, Hetarylcarbonyl, Hetarylsulfonyl, Heterocyclyl, Heterocyclylcarbonyl oder Heterocyclylsulfonyl stehen, wobei die 17 letztgenannten Reste jeweils unsubstituiert oder durch einen oder mehrere Reste aus der Gruppe bestehend aus Halogen, Nitro, Hydroxyl, Cyano, NH₂, (C₁-C₆)-Alkylamin, (C₁-C₆)-Dialkylamin, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy, (C₁-C₄)-Alkylsulfonyl, (C₁-C₄)-Halogenalkylthio, (C₁-C₄)-Halogenalkylsulfoxy, (C₁-C₄)-Halogenalkylsulfonyl, (C₁-C₄)-Alkoxycarbonyl, (C₁-C₄)-Halogenalkoxycarbonyl, (C₁-C₄)-Alkylcarboxy, (C₃-C₆)-Cycloalkyl, (C₃-C₆)-Cycloalkyl-(C₁-C₆)-alkyl, (C₁-C₄)-Alkoxycarbonyl-(C₁-C₄)-alkyl, Hydroxycarbonyl und Hydroxycarbonyl-(C₁-C₄)-alkyl substituiert sind und wobei Heterocyclyl q Oxogruppen aufweist,
oder
R¹³ und R¹⁴ zusammen mit dem Stickstoffatom, an das sie gebunden sind, einen 3- bis 8-gliedrigen ungesättigten, teilweise gesättigten oder gesättigten Ring bilden, der jeweils zusätzlich zu den Kohlenstoffatomen und zusätzlich zu dem Stickstoffatom p Ringglieder aus der aus N(R¹²)ₘ, O und S(O)ₙ bestehenden Gruppe aufweist, und wobei der Ring unsubstituiert oder durch einen oder mehrere Reste aus der Gruppe bestehend aus Halogen, Nitro, Hydroxyl, Cyano, NH₂, (C₁-C₆)-Alkylamin, (C₁-C₆)-Dialkylamin, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy, (C₁-C₄)-Alkylsulfonyl, (C₁-C₄)-Halogenalkylthio, (C₁-C₄)-Halogenalkylsulfoxy, (C₁-C₄)-Halogenalkylsulfonyl, (C₁-C₄)-Alkoxycarbonyl, (C₁-C₄)-Halogenalkoxycarbonyl, (C₁-C₄)-Alkylcarboxy, (C₃-C₆)-Cycloalkyl, (C₃-C₆)-Cycloalkyl-(C₁-C₆)-alkyl, (C₁-C₄)-Alkoxycarbonyl-(C₁-C₄)-alkyl, Hydroxycarbonyl und Hydroxycarbonyl-(C₁-C₄)-alkyl substituiert ist und q Oxogruppen aufweist,
n unabhängig aus 0, 1 und 2 ausgewählt ist,
m unabhängig aus 0 und 1 ausgewählt ist,
p unabhängig aus 0, 1, 2 und 3 ausgewählt ist,
q unabhängig aus 0, 1 und 2 ausgewählt ist,
y für 0 oder 1 steht.

2. Verbindung der Formel (G) nach Anspruch 1 und/oder ein Salz davon, wobei
A für CR⁶R⁷ steht,
W für O oder S steht,
R¹ für (C₁-C₆)-Alkyl, (C₁-C₆)-Halogenalkyl, (C₂-C₆)-Alkenyl, (C₂-C₆)-Alkinyl, NR¹³R¹⁴, R¹³R¹⁴N-(C₁-C₆)-Alkyl, (C₁-C₆)-Alkoxy, (C₁-C₆)-Halogenalkoxy, (C₁-C₆)-Halogenalkoxy-(C₁-C₃)-alkyl, (C₁-C₆)-Alkoxy-(C₁-C₃)-alkyl, (C₁-C₆)-Alkoxy-(C₂-C₆)-alkoxy-(C₁-C₃)-alkyl, (C₁-C₄)-Alkylthio, (C₁-C₄)-Alkylsulfoxy, (C₁-C₄)-Alkylsulfonyl, (C₁-C₄)-Alkylthio-(C₁-C₃)-alkyl, (C₁-C₄)-Alkylsulfoxy-(C₁-C₃)-alkyl, (C₁-C₄)-Alkylsulfonyl-(C₁-C₃)-alkyl, (C₃-C₈)-Cycloalkyl, (C₃-C₆)-Cycloalkenyl, (C₃-C₆)-Cycloalkyl-(C₁-C₃)-alkyl, (C₃-C₆)-Cycloalkenyl-(C₁-C₃)-alkyl, (C₃-C₆)-Cycloalkoxy, Phenyl, Heteroaryl, Heterocyclyl, Phenoxy, Heteroaryloxy, Heterocyclyloxy oder einen carbobicyclischen Rest steht, wobei die 12 letztgenannten Reste jeweils unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy und (C₁-C₄)-Alkylsulfonyl, substituiert sind und wobei Heterocyclyl q Oxogruppen aufweist,
R², R³ jeweils unabhängig für Wasserstoff, (C₁-C₆)-Alkyl, (C₁-C₆)-Halogenalkyl, (C₂-C₆)-Alkenyl, (C₂-C₆)-Alkinyl, (C₁-C₆)-Alkoxy-(C₁-C₃)-alkyl, (C₁-C₆)-Alkoxy-(C₁-C₃)-alkylcarbonyl, (C₁-C₄)-Alkylthio, (C₁-C₄)-Halogenalkylthio, (C₁-C₄)-Alkylthiocarbonyl, (C₁-C₄)-Halogenalkylthiocarbonyl, (C₁-C₄)-Alkylthio-(C₁-C₃)-alkyl, (C₁-C₄)-Alkylsulfoxy-(C₁-C₃)-alkyl, (C₁-C₄)-Alkylsulfonyl-(C₁-C₃)-alkyl, (C₁-C₄)-Alkylthio-(C₁-C₃)-alkylcarbonyl, (C₁-C₄)-Alkylsulfoxy-(C₁-C₃)-alkylcarbonyl, (C₁-C₄)-Alkylsulfonyl-(C₁-C₃)-alkylcarbonyl, (C₁-C₆)-Alkylcarbonyl, (C₁-C₆)-Halogenalkylcarbonyl, (C₂-C₆)-Alkenylcarbonyl, (C₂-C₆)-Alkinylcarbonyl, (C₁-C₆)-Alkoxycarbonylcarbonyl, (C₁-C₆)-Alkoxycarbonyl-(C₁-C₃)-alkylcarbonyl, (C₁-C₆)-Alkoxycarbonyl, (C₁-C₆)-Halogenalkoxycarbonyl, (C₂-C₆)-Alkenyloxycarbonyl, (C₃-C₆)-Cycloalkyl, (C₃-C₆)-Cycloalkyl-(C₁-C₆)-alkyl, (C₃-C₆)-Cycloalkylcarbonyl, (C₃-C₆)-Cycloalkyl-(C₁-C₆)-alkylcarbonyl, Phenyl, Phenyl-(C₁-C₃)-alkyl, Heteroaryl, Heteroaryl-(C₁-C₃)-alkyl, Heterocyclyl, Heterocyclyl-(C₁-C₃)-alkyl, Phenylcarbonyl, Phenyl-(C₁-C₆)-alkylcarbonyl, Hetarylcarbonyl, Hetaryl-(C₁-C₆)-alkylcarbonyl, Heterocyclylcarbonyl oder Heterocyclyl-(C₁-C₆)-alkylcarbonyl stehen, wobei die 16 letztgenannten Reste jeweils unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy und (C₁-C₄)-Alkylsulfonyl substituiert sind und wobei Heterocyclyl q Oxogruppen aufweist,
oder
NR²R³ für -N=CR⁸R⁹ oder -N=S(O)ₙR¹⁰R¹¹ steht,
R⁶, R⁷ jeweils unabhängig für Wasserstoff oder (C₁-C₆)-Alkyl stehen,
R⁸, R⁹ jeweils unabhängig für Wasserstoff, (C₁-C₆)-Alkyl, (C₁-C₆)-Halogenalkyl, (C₂-C₆)-Alkenyl, (C₂-C₆)-Alkinyl, (C₁-C₆)-Alkoxy-(C₁-C₃)-alkyl, (C₁-C₄)-Alkylthio-(C₁-C₃)-alkyl, (C₁-C₄)-Alkylsulfoxy-(C₁-C₃)-alkyl, (C₁-C₄)-Alkylsulfonyl-(C₁-C₃)-alkyl, (C₁-C₆)-Alkoxy, (C₁-C₆)-Halogenalkoxy, (C₂-C₆)-Alkenyloxy, NR¹³R¹⁴, (C₃-C₈)-Cycloalkyl, (C₃-C₈)-Cycloalkyl-(C₁-C₆)-alkyl, Phenyl, Phenyl-(C₁-C₃)-alkyl, Heteroaryl, Heteroaryl-(C₁-C₃)-alkyl, Heterocyclyl oder Heterocyclyl-(C₁-C₃)-alkyl stehen, wobei die 8 letztgenannten Reste jeweils unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy und (C₁-C₄)-Alkylsulfonyl substituiert sind und wobei Heterocyclyl q Oxogruppen aufweist,
oder
R⁸ und R⁹ zusammen mit dem Kohlenstoffatom, an das sie gebunden sind, einen 3- bis 6-gliedrigen ungesättigten, teilweise gesättigten oder gesättigten Ring bilden, der jeweils zusätzlich zu den Kohlenstoffatomen p Ringglieder aus der aus N(R¹²)ₘ, O und S(O)ₙ bestehenden Gruppe umfasst, und wobei der Ring unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfbxy und (C₁-C₄)-Alkylsulfonyl substituiert ist und wobei Heterocyclyl q Oxogruppen aufweist,
R¹⁰, R¹¹ jeweils unabhängig für (C₁-C₆)-Alkyl, (C₂-C₆)-Alkenyl, (C₂-C₆)-Alkinyl, (C₁-C₆)-Alkoxy-(C₁-C₃)-alkyl, (C₁-C₄)-Alkylthio-(C₁-C₃)-alkyl, (C₁-C₄)-Alkylsulfoxy-(C₁-C₃)-alkyl, (C₁-C₄)-Alkylsulfonyl-(C₁-C₃)-alkyl, (C₃-C₈)-Cycloalkyl, (C₃-C₈)-Cycloalkyl-(C₁-C₆)-alkyl, Phenyl, Phenyl-(C₁-C₃)-alkyl, Heteroaryl, Heteroaryl-(C₁-C₃)-alkyl, Heterocyclyl oder Heterocyclyl-(C₁-C₃)-alkyl stehen, wobei die 8 letztgenannten Reste jeweils unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfbxy und (C₁-C₄)-Alkylsulfonyl substituiert sind und wobei Heterocyclyl q Oxogruppen aufweist,
oder
R¹⁰ und R¹¹ zusammen mit dem Schwefelatom, an das sie gebunden sind, einen 3- bis 6-gliedrigen ungesättigten, teilweise gesättigten oder gesättigten Ring bilden, der jeweils zusätzlich zu den Kohlenstoffatomen und zusätzlich zu dem Schwefelatom p Ringglieder aus der aus N(R¹²)ₘ, O und S(O)ₙ bestehenden Gruppe umfasst, und wobei der Ring unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy und (C₁-C₄)-Alkylsulfonyl substituiert ist,
R¹² für Wasserstoff, (C₁-C₆)-Alkyl oder (C₁-C₆)-Alkylcarbonyl steht,
R¹³, R¹⁴ jeweils unabhängig für Wasserstoff, (C₁-C₆)-Alkyl, (C₁-C₆)-Alkylcarbonyl, (C₁-C₄)-Alkylsulfonyl, Phenyl oder Phenylcarbonyl stehen, wobei die zwei letztgenannten Reste jeweils unsubstituiert oder durch einen oder mehrere Reste aus der Gruppe bestehend aus Halogen, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy und (C₁-C₄)-Alkylsulfonyl substituiert sind,
oder
R¹³ und R¹⁴ zusammen mit dem Stickstoffatom, an das sie gebunden sind, einen 3- bis 8-gliedrigen ungesättigten, teilweise gesättigten oder gesättigten Ring bilden, der jeweils zusätzlich zu den Kohlenstoffatomen und zusätzlich zu dem Stickstoffatom p Ringglieder aus der aus N(R¹²)ₘ, O und S(O)ₙ bestehenden Gruppe umfasst, und wobei der Ring unsubstituiert oder durch einen oder mehrere Reste aus der Gruppe bestehend aus Halogen, Hydroxyl, (C₁-C₄)-Alkyl, (C₁-C₄)-Halogenalkyl, (C₁-C₄)-Alkoxy und (C₁-C₄)-Halogenalkoxy substituiert ist und q Oxogruppen aufweist,
n unabhängig aus 0, 1 und 2 ausgewählt ist,
m unabhängig aus 0 und 1 ausgewählt ist,
p unabhängig aus 0, 1 und 2 ausgewählt ist,
q unabhängig aus 0, 1 und 2 ausgewählt ist,
y für 0 oder 1 steht.

3. Verbindung der Formel (G) nach Anspruch 1 oder 2 und/oder ein Salz davon, wobei
A für CR⁶R⁷ steht,
W für O oder S steht,
R¹ für (C₁-C₆)-Alkyl, (C₁-C₆)-Halogenalkyl, (C₂-C₆)-Alkenyl, (C₂-C₆)-Alkinyl, NR¹³R¹⁴, R¹³R¹⁴N-(C₁-C₆)-Alkyl, (C₁-C₆)-Alkoxy, (C₁-C₆)-Halogenalkoxy, (C₁-C₆)-Alkoxy-(C₁-C₃)-alkyl, (C₁-C₆)-Alkoxy-(C₂-C₆)-alkoxy-(C₁-C₃)-alkyl, (C₁-C₄)-Alkylthio, (C₁-C₄)-Alkylsulfoxy, (C₁-C₄)-Alkylsulfonyl, (C₁-C₄)-Alkylthio-(C₁-C₃)-alkyl, (C₁-C₄)-Alkylsulfoxy-(C₁-C₃)-alkyl, (C₁-C₄)-Alkylsulfonyl-(C₁-C₃)-alkyl, (C₃-C₈)-Cycloalkyl, (C₃-C₆)-Cycloalkenyl, (C₃-C₆)-Cycloalkyl-(C₁-C₃)-alkyl, (C₃-C₆)-Cycloalkenyl-(C₁-C₃)-alkyl, (C₃-C₆)-Cycloalkoxy, Phenyl, Heteroaryl, Heterocyclyl, Phenoxy, Heteroaryloxy oder Heterocyclyloxy steht, wobei die 11 letztgenannten Reste jeweils unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy und (C₁-C₄)-Alkylsulfonyl, substituiert sind und wobei Heterocyclyl q Oxogruppen aufweist,
R², R³ jeweils unabhängig für Wasserstoff, (C₁-C₆)-Alkyl, (C₁-C₆)-Halogenalkyl, (C₂-C₆)-Alkenyl, (C₂-C₆)-Alkinyl, (C₁-C₆)-Alkoxy-(C₁-C₃)-alkyl, (C₁-C₆)-Alkoxy-(C₁-C₃)-alkylcarbonyl, (C₁-C₄)-Alkylthio, (C₁-C₄)-Halogenalkylthio, (C₁-C₄)-Alkylthiocarbonyl, (C₁-C₄)-Halogenalkylthiocarbonyl, (C₁-C₄)-Alkylthio-(C₁-C₃)-alkyl, (C₁-C₄)-Alkylsulfoxy-(C₁-C₃)-alkyl, (C₁-C₄)-Alkylsulfonyl-(C₁-C₃)-alkyl, (C₁-C₄)-Alkylthio-(C₁-C₃)-alkylcarbonyl, (C₁-C₄)-Alkylsulfoxy-(C₁-C₃)-alkylcarbonyl, (C₁-C₄)-Alkylsulfonyl-(C₁-C₃)-alkylcarbonyl, (C₁-C₆)-Alkylcarbonyl, (C₁-C₆)-Halogenalkylcarbonyl, (C₂-C₆)-Alkenylcarbonyl, (C₂-C₆)-Alkinylcarbonyl, (C₁-C₆)-Alkoxycarbonylcarbonyl, (C₁-C₆)-Alkoxycarbonyl-(C₁-C₃)-alkylcarbonyl, (C₁-C₆)-Alkoxycarbonyl, (C₁-C₆)-Halogenalkoxycarbonyl, (C₂-C₆)-Alkenyloxycarbonyl, (C₃-C₆)-Cycloalkyl, (C₃-C₆)-Cycloalkyl-(C₁-C₆)-alkyl, (C₃-C₆)-Cycloalkylcarbonyl, (C₃-C₆)-Cycloalkyl-(C₁-C₆)-alkylcarbonyl, Phenyl, Phenyl-(C₁-C₃)-alkyl, Heteroaryl, Heteroaryl-(C₁-C₃)-alkyl, Heterocyclyl, Heterocyclyl-(C₁-C₃)-alkyl, Phenylcarbonyl, Phenyl-(C₁-C₆)-alkylcarbonyl, Hetarylcarbonyl, Hetaryl-(C₁-C₆)-alkylcarbonyl, Heterocyclylcarbonyl oder Heterocyclyl-(C₁-C₆)-alkylcarbonyl stehen, wobei die 16 letztgenannten Reste jeweils unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy und (C₁-C₄)-Alkylsulfonyl substituiert sind und wobei Heterocyclyl q Oxogruppen aufweist,
oder
NR²R³ für -N=CR⁸R⁹ oder -N=S(O)ₙR¹⁰R¹¹ steht,
R⁶, R⁷ jeweils unabhängig für Wasserstoff oder (C₁-C₄)-Alkyl stehen, R⁶ und R⁷ vorzugsweise unabhängig für Wasserstoff oder Methyl stehen,
R⁸, R⁹ jeweils unabhängig für Wasserstoff, (C₁-C₆)-Alkyl, (C₁-C₆)-Halogenalkyl, (C₂-C₆)-Alkenyl, (C₂-C₆)-Alkinyl, (C₁-C₆)-Alkoxy-(C₁-C₃)-alkyl, (C₁-C₄)-Alkylthio-(C₁-C₃)-alkyl, (C₁-C₄)-Alkylsulfoxy-(C₁-C₃)-alkyl, (C₁-C₄)-Alkylsulfonyl-(C₁-C₃)-alkyl, (C₁-C₆)-Alkoxy, (C₁-C₆)-Halogenalkoxy, (C₂-C₆)-Alkenyloxy, NR¹³R¹⁴, (C₃-C₈)-Cycloalkyl, (C₃-C₈)-Cycloalkyl-(C₁-C₆)-alkyl, Phenyl, Phenyl-(C₁-C₃)-alkyl, Heteroaryl, Heteroaryl-(C₁-C₃)-alkyl, Heterocyclyl oder Heterocyclyl-(C₁-C₃)-alkyl stehen, wobei die 8 letztgenannten Reste jeweils unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy und (C₁-C₄)-Alkylsulfonyl substituiert sind und wobei Heterocyclyl q Oxogruppen aufweist,
oder
R⁸ und R⁹ zusammen mit dem Kohlenstoffatom, an das sie gebunden sind, einen 3- bis 6-gliedrigen ungesättigten, teilweise gesättigten oder gesättigten Ring bilden, der jeweils zusätzlich zu den Kohlenstoffatomen p Ringglieder aus der aus N(R¹²)ₘ, O und S(O)ₙ bestehenden Gruppe umfasst, und wobei der Ring unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy und (C₁-C₄)-Alkylsulfonyl substituiert ist,
R¹⁰, R¹¹ jeweils unabhängig für (C₁-C₆)-Alkyl, (C₂-C₆)-Alkenyl, (C₂-C₆)-Alkinyl, (C₁-C₆)-Alkoxy-(C₁-C₃)-alkyl, (C₁-C₄)-Alkylthio-(C₁-C₃)-alkyl, (C₁-C₄)-Alkylsulfoxy-(C₁-C₃)-alkyl, (C₁-C₄)-Alkylsulfonyl-(C₁-C₃)-alkyl, (C₃-C₈)-Cycloalkyl, (C₃-C₈)-Cycloalkyl-(C₁-C₆)-alkyl, Phenyl, Phenyl-(C₁-C₃)-alkyl, Heteroaryl, Heteroaryl-(C₁-C₃)-alkyl, Heterocyclyl oder Heterocyclyl-(C₁-C₃)-alkyl stehen, wobei die 8 letztgenannten Reste jeweils unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy und (C₁-C₄)-Alkylsulfonyl substituiert sind und wobei Heterocyclyl q Oxogruppen aufweist,
oder
R¹⁰ und R¹¹ zusammen mit dem Schwefelatom, an das sie gebunden sind, einen 3- bis 6-gliedrigen ungesättigten, teilweise gesättigten oder gesättigten Ring bilden, der jeweils zusätzlich zu den Kohlenstoffatomen und zusätzlich zu dem Schwefelatom p Ringglieder aus der aus N(R¹²)ₘ, O und S(O)ₙ bestehenden Gruppe umfasst, und wobei der Ring unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy und (C₁-C₄)-Alkylsulfonyl substituiert ist,
R¹² für Wasserstoff, (C₁-C₆)-Alkyl oder (C₁-C₆)-Alkylcarbonyl steht,
R¹³, R¹⁴ jeweils unabhängig für Wasserstoff, (C₁-C₆)-Alkyl, (C₁-C₆)-Alkylcarbonyl, (C₁-C₄)-Alkylsulfonyl, Phenyl oder Phenylcarbonyl stehen, wobei die zwei letztgenannten Reste jeweils unsubstituiert oder durch einen oder mehrere Reste aus der Gruppe bestehend aus Halogen, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy und (C₁-C₄)-Alkylsulfonyl substituiert sind,
oder
R¹³ und R¹⁴ zusammen mit dem Stickstoffatom, an das sie gebunden sind, einen 3- bis 8-gliedrigen ungesättigten, teilweise gesättigten oder gesättigten Ring bilden, der jeweils zusätzlich zu den Kohlenstoffatomen und zusätzlich zu dem Stickstoffatom p Ringglieder aus der aus N(R¹²)ₘ, O und S(O)ₙ bestehenden Gruppe umfasst, und wobei der Ring unsubstituiert oder durch einen oder mehrere Reste aus der Gruppe bestehend aus Halogen, Hydroxyl, (C₁-C₄)-Alkyl, (C₁-C₄)-Halogenalkyl, (C₁-C₄)-Alkoxy und (C₁-C₄)-Halogenalkoxy substituiert ist und q Oxogruppen aufweist,
n unabhängig aus 0, 1 und 2 ausgewählt ist,
m unabhängig aus 0 und 1 ausgewählt ist,
p unabhängig aus 0, 1 und 2 ausgewählt ist,
q unabhängig aus 0 und 1 ausgewählt ist,
y für 0 oder 1 steht.

4. Verbindung der Formel (G) nach einem der Ansprüche 1 bis 3 und/oder ein Salz davon, wobei
A für CR⁶R⁷ steht,
W für O oder S steht, vorzugsweise O, steht
R¹ für (C₁-C₆)-Alkyl, (C₁-C₆)-Halogenalkyl, (C₂-C₆)-Alkenyl, (C₁-C₆)-Alkoxy-(C₁-C₃)-alkyl, (C₁-C₆)-Alkoxy-(C₂-C₆)-alkoxy-(C₁-C₃)-alkyl, (C₁-C₄)-Alkylsulfonyl, (C₁-C₄)-Alkylthio-(C₁-C₃)-alkyl, (C₁-C₄)-Alkylsulfoxy-(C₁-C₃)-alkyl, (C₁-C₄)-Alkylsulfonyl-(C₁-C₃)-alkyl, (C₃-C₈)-Cycloalkyl, (C₃-C₆)-Cycloalkenyl, (C₃-C₆)-Cycloalkyl-(C₁-C₃)-alkyl, (C₃-C₆)-Cycloalkenyl-(C₁-C₃)-alkyl, (C₃-C₆)-Cycloalkoxy, Phenyl, Heteroaryl, Heterocyclyl, Phenoxy, Heteroaryloxy oder Heterocyclyloxy steht, wobei die 11 letztgenannten Reste jeweils unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfbxy und (C₁-C₄)-Alkylsulfonyl, substituiert sind und wobei Heterocyclyl q Oxogruppen aufweist,
R², R³ jeweils unabhängig für Wasserstoff, (C₂-C₆)-Alkinyl, (C₁-C₄)-Alkylthio, (C₁-C₄)-Halogenalkylthio, (C₁-C₄)-Alkylthiocarbonyl, (C₁-C₄)-Halogenalkylthiocarbonyl, (C₁-C₆)-Alkoxy-(C₁-C₃)-alkylcarbonyl, (C₁-C₄)-Alkylthio-(C₁-C₃)-alkylcarbonyl, (C₁-C₄)-Alkylsulfoxy-((C₁-C₃)-alkylcarbonyl, (C₁-C₄)-Alkylsulfonyl-(C₁-C₃)-alkylcarbonyl, (C₁-C₆)-Alkylcarbonyl, (C₁-C₆)-Halogenalkylcarbonyl, (C₂-C₆)-Alkenylcarbonyl, (C₂-C₆)-Alkinylcarbonyl, (C₁-C₆)-Alkoxycarbonylcarbonyl, (C₁-C₆)-Alkoxycarbonyl-(C₁-C₃)-alkylcarbonyl, (C₁-C₆)-Alkoxycarbonyl, (C₁-C₆)-Halogenalkoxycarbonyl, (C₂-C₆)-Alkenyloxycarbonyl, (C₃-C₆)-Cycloalkylcarbonyl, (C₃-C₆)-Cycloalkyl-(C₁-C₆)-alkylcarbonyl, Phenylcarbonyl, Phenyl-(C₁-C₆)-alkylcarbonyl, Hetarylcarbonyl, Hetaryl-(C₁-C₆)-alkylcarbonyl, Heterocyclylcarbonyl oder Heterocyclyl-(C₁-C₆)-alkylcarbonyl stehen, wobei die 8 letztgenannten Reste jeweils unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy und (C₁-C₄)-Alkylsulfonyl substituiert sind und wobei Heterocyclyl q Oxogruppen aufweist,
oder
NR²R³ für -N=CR⁸R⁹ oder -N=S(O)ₙR¹⁰R¹¹ steht,
R⁶ für Wasserstoff steht,
R⁷ für Wasserstoff oder Methyl steht,
R⁸, R⁹ jeweils unabhängig für Wasserstoff, (C₁-C₆)-Alkyl, (C₁-C₆)-Halogenalkyl, (C₂-C₆)-Alkenyl, (C₂-C₆)-Alkinyl, (C₁-C₆)-Alkoxy-(C₁-C₃)-alkyl, (C₁-C₄)-Alkylthio-(C₁-C₃)-alkyl, (C₁-C₄)-Alkylsulfoxy-(C₁-C₃)-alkyl, (C₁-C₄)-Alkylsulfonyl-(C₁-C₃)-alkyl, (C₁-C₆)-Alkoxy, (C₁-C₆)-Halogenalkoxy, (C₂-C₆)-Alkenyloxy, NR¹³R¹⁴, (C₃-C₈)-Cycloalkyl, (C₃-C₈)-Cycloalkyl-(C₁-C₆)-alkyl, Phenyl, Phenyl-(C₁-C₃)-alkyl, Heteroaryl, Heteroaryl-(C₁-C₃)-alkyl, Heterocyclyl oder Heterocyclyl-(C₁-C₃)-alkyl stehen, wobei die 8 letztgenannten Reste jeweils unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy und (C₁-C₄)-Alkylsulfonyl substituiert sind und wobei Heterocyclyl q Oxogruppen aufweist,
oder
R⁸ und R⁹ zusammen mit dem Kohlenstoffatom, an das sie gebunden sind, einen 3- bis 6-gliedrigen ungesättigten, teilweise gesättigten oder gesättigten Ring bilden, der jeweils zusätzlich zu den Kohlenstoffatomen p Ringglieder aus der aus N(R¹²)ₘ, O und S(O)ₙ bestehenden Gruppe umfasst, und wobei der Ring unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfbxy und (C₁-C₄)-Alkylsulfonyl substituiert ist,
R¹⁰, R¹¹ jeweils unabhängig für (C₁-C₆)-Alkyl, (C₂-C₆)-Alkenyl, (C₂-C₆)-Alkinyl, (C₁-C₆)-Alkoxy-(C₁-C₃)-alkyl, (C₁-C₄)-Alkylthio-(C₁-C₃)-alkyl, (C₁-C₄)-Alkylsulfoxy-(C₁-C₃)-alkyl, (C₁-C₄)-Alkylsulfonyl-(C₁-C₃)-alkyl, (C₃-C₈)-Cycloalkyl, (C₃-C₈)-Cycloalkyl-(C₁-C₆)-alkyl, Phenyl, Phenyl-(C₁-C₃)-alkyl, Heteroaryl, Heteroaryl-(C₁-C₃)-alkyl, Heterocyclyl und Heterocyclyl-(C₁-C₃)-alkyl stehen, wobei die 8 letztgenannten Reste jeweils unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy und (C₁-C₄)-Alkylsulfonyl substituiert sind und wobei Heterocyclyl q Oxogruppen aufweist,
oder
R¹⁰ und R¹¹ zusammen mit dem Schwefelatom, an das sie gebunden sind, einen 3- bis 6-gliedrigen ungesättigten, teilweise gesättigten oder gesättigten Ring bilden, der jeweils zusätzlich zu den Kohlenstoffatomen und zusätzlich zu dem Schwefelatom p Ringglieder aus der aus N(R¹²)ₘ, O und S(O)ₙ bestehenden Gruppe umfasst, und wobei der Ring unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy und (C₁-C₄)-Alkylsulfonyl substituiert ist,
R¹² für Wasserstoff, (C₁-C₆)-Alkyl oder (C₁-C₆)-Alkylcarbonyl steht,
R¹³, R¹⁴ jeweils unabhängig für Wasserstoff, (C₁-C₆)-Alkyl, (C₁-C₆)-Alkylcarbonyl, (C₁-C₄)-Alkylsulfonyl, Phenyl oder Phenylcarbonyl stehen, wobei die zwei letztgenannten Reste jeweils unsubstituiert oder durch einen oder mehrere Reste aus der Gruppe bestehend aus Halogen, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy und (C₁-C₄)-Alkylsulfonyl substituiert sind,
oder
R¹³ und R¹⁴ zusammen mit dem Stickstoffatom, an das sie gebunden sind, einen 3- bis 8-gliedrigen ungesättigten, teilweise gesättigten oder gesättigten Ring bilden, der jeweils zusätzlich zu den Kohlenstoffatomen und zusätzlich zu dem Stickstoffatom p Ringglieder aus der aus N(R¹²)ₘ, O und S(O)ₙ bestehenden Gruppe umfasst, und wobei der Ring unsubstituiert oder durch einen oder mehrere Reste aus der Gruppe bestehend aus Halogen, Hydroxyl, (C₁-C₄)-Alkyl, (C₁-C₄)-Halogenalkyl, (C₁-C₄)-Alkoxy und (C₁-C₄)-Halogenalkoxy substituiert ist und q Oxogruppen aufweist,
n unabhängig aus 0, 1 und 2 ausgewählt ist,
m unabhängig aus 0 und 1 ausgewählt ist,
p unabhängig aus 0, 1 und 2 ausgewählt ist,
q unabhängig aus 0 und 1 ausgewählt ist,
y für 0 oder 1 steht.

5. Verbindung der Formel (G) nach einem der Ansprüche 1 bis 4 und/oder ein Salz davon, wobei
A für CR⁶R⁷ steht,
W für O oder S steht, vorzugsweise O, steht
R¹ für (C₃-C₈)-Cycloalkyl, (C₃-C₆)-Cycloalkenyl, (C₃-C₆)-Cycloalkyl-(C₁-C₃)-alkyl, (C₃-C₆)-Cycloalkenyl-(C₁-C₃)-alkyl, Phenyl, Heteroaryl oder Heterocyclyl steht, wobei die 7 letztgenannten Reste jeweils unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy und (C₁-C₄)-Alkylsulfonyl, substituiert sind und wobei Heterocyclyl q Oxogruppen aufweist,
R², R³ jeweils unabhängig für Wasserstoff, (C₂-C₆)-Alkinyl, (C₁-C₄)-Alkylthio, (C₁-C₄)-Halogenalkylthio (wobei (C₁-C₄-Halogenalkylthio besonders bevorzugt für SCF₃ steht), (C₁-C₄)-Alkylthiocarbonyl, (C₁-C₄)-Halogenalkylthiocarbonyl, (C₁-C₆)-Alkoxy-(C₁-C₃)-alkylcarbonyl, (C₁-C₄)-Alkylthio-(C₁-C₃)-alkylcarbonyl, (C₁-C₄)-Alkylsulfoxy-((C₁-C₃)-alkylcarbonyl, (C₁-C₄)-Alkylsulfonyl-(C₁-C₃)-alkylcarbonyl, (C₁-C₆)-Alkylcarbonyl, (C₁-C₆)-Halogenalkylcarbonyl, (C₂-C₆)-Alkenylcarbonyl, (C₂-C₆)-Alkinylcarbonyl, (C₁-C₆)-Alkoxycarbonylcarbonyl, (C₁-C₆)-Alkoxycarbonyl-(C₁-C₃)-alkylcarbonyl, (C₁-C₆)-Alkoxycarbonyl, (C₁-C₆)-Halogenalkoxycarbonyl, (C₂-C₆)-Alkenyloxycarbonyl, (C₃-C₆)-Cycloalkylcarbonyl, (C₃-C₆)-Cycloalkyl-(C₁-C₆)-alkylcarbonyl, Phenylcarbonyl, Phenyl-(C₁-C₆)-alkylcarbonyl, Hetarylcarbonyl, Hetaryl-(C₁-C₆)-alkylcarbonyl, Heterocyclylcarbonyl oder Heterocyclyl-(C₁-C₆)-alkylcarbonyl stehen, wobei die 8 letztgenannten Reste jeweils unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy und (C₁-C₄)-Alkylsulfonyl substituiert sind und wobei Heterocyclyl q Oxogruppen aufweist,
oder
NR²R³ für -N=CR⁸R⁹ oder -N=S(O)ₙR¹⁰R¹¹ steht,
R⁶ für Wasserstoff steht,
R⁷ für Wasserstoff oder Methyl steht,
R⁸, R⁹ jeweils unabhängig für Wasserstoff, (C₁-C₆)-Alkyl, (C₁-C₆)-Halogenalkyl, (C₂-C₆)-Alkenyl, (C₂-C₆)-Alkinyl, (C₁-C₆)-Alkoxy-(C₁-C₃)-alkyl, (C₁-C₄)-Alkylthio-(C₁-C₃)-alkyl, (C₁-C₄)-Alkylsulfoxy-(C₁-C₃)-alkyl, (C₁-C₄)-Alkylsulfonyl-(C₁-C₃)-alkyl, (C₁-C₆)-Alkoxy, (C₁-C₆)-Halogenalkoxy, (C₂-C₆)-Alkenyloxy, NR¹³R¹⁴, (C₃-C₈)-Cycloalkyl, (C₃-C₈)-Cycloalkyl-(C₁-C₆)-alkyl, Phenyl, Phenyl-(C₁-C₃)-alkyl, Heteroaryl, Heteroaryl-(C₁-C₃)-alkyl, Heterocyclyl oder Heterocyclyl-(C₁-C₃)-alkyl stehen, wobei die 8 letztgenannten Reste jeweils unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy und (C₁-C₄)-Alkylsulfonyl substituiert sind und wobei Heterocyclyl q Oxogruppen aufweist,
oder
R⁸ und R⁹ zusammen mit dem Kohlenstoffatom, an das sie gebunden sind, einen 3- bis 6-gliedrigen ungesättigten, teilweise gesättigten oder gesättigten Ring bilden, der jeweils zusätzlich zu den Kohlenstoffatomen p Ringglieder aus der aus N(R¹²)ₘ, O und S(O)ₙ bestehenden Gruppe umfasst, und wobei der Ring unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy und (C₁-C₄)-Alkylsulfonyl substituiert ist,
R¹⁰, R¹¹ jeweils unabhängig für (C₁-C₆)-Alkyl, (C₂-C₆)-Alkenyl, (C₂-C₆)-Alkinyl, (C₃-C₈)-Cycloalkyl, (C₃-C₈)-Cycloalkyl-(C₁-C₆)-alkyl, Phenyl, Phenyl-(C₁-C₃)-alkyl, Heteroaryl, Heteroaryl-(C₁-C₃)-alkyl, Heterocyclyl oder Heterocyclyl-(C₁-C₃)-alkyl stehen, wobei die 8 letztgenannten Reste jeweils unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy und (C₁-C₄)-Alkylsulfonyl substituiert sind und wobei Heterocyclyl q Oxogruppen aufweist,
oder
R¹⁰ und R¹¹ zusammen mit dem Schwefelatom, an das sie gebunden sind, einen 3- bis 6-gliedrigen ungesättigten, teilweise gesättigten oder gesättigten Ring bilden, der jeweils zusätzlich zu den Kohlenstoffatomen und zusätzlich zu dem Schwefelatom p Ringglieder aus der aus N(R¹²)ₘ, O und S(O)ₙ bestehenden Gruppe umfasst, und wobei der Ring unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy und (C₁-C₄)-Alkylsulfonyl substituiert ist,
R¹² für Wasserstoff, (C₁-C₆)-Alkyl oder (C₁-C₆)-Alkylcarbonyl steht,
R¹³, R¹⁴ jeweils unabhängig für Wasserstoff, (C₁-C₆)-Alkyl, (C₁-C₆)-Alkylcarbonyl, (C₁-C₄)-Alkylsulfonyl, Phenyl oder Phenylcarbonyl stehen, wobei die zwei letztgenannten Reste jeweils unsubstituiert oder durch einen oder mehrere Reste aus der Gruppe bestehend aus Halogen, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy und (C₁-C₄)-Alkylsulfonyl substituiert sind,
oder
R¹³ und R¹⁴ zusammen mit dem Stickstoffatom, an das sie gebunden sind, einen 3- bis 8-gliedrigen ungesättigten, teilweise gesättigten oder gesättigten Ring bilden, der jeweils zusätzlich zu den Kohlenstoffatomen und zusätzlich zu dem Stickstoffatom p Ringglieder aus der aus N(R¹²)ₘ, O und S(O)ₙ bestehenden Gruppe umfasst, und wobei der Ring unsubstituiert oder durch einen oder mehrere Reste aus der Gruppe bestehend aus Halogen, Hydroxyl, (C₁-C₄)-Alkyl, (C₁-C₄)-Halogenalkyl, (C₁-C₄)-Alkoxy und (C₁-C₄)-Halogenalkoxy substituiert ist und q Oxogruppen aufweist,
n unabhängig aus 0 und 1 ausgewählt ist,
m unabhängig aus 0 und 1 ausgewählt ist,
p unabhängig aus 0, 1 und 2 ausgewählt ist,
q unabhängig aus 0 und 1 ausgewählt ist,
y für 0 oder 1 steht.

6. Verbindung der Formel (G) nach einem der Ansprüche 1 bis 5 und/oder ein Salz davon, wobei
A für CR⁶R⁷ steht,
W für O steht
R¹ für (C₃-C₆)-Cycloalkyl, (C₃-C₆)-Cycloalkenyl, (C₃-C₆)-Cycloalkyl-(C₁-C₃)-alkyl, (C₃-C₆)-Cycloalkenyl-(C₁-C₃)-alkyl, Phenyl, Heteroaryl oder Heterocyclyl steht, wobei die 7 letztgenannten Reste jeweils unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy und (C₁-C₄)-Alkylsulfonyl, substituiert sind und wobei Heterocyclyl q Oxogruppen aufweist,
R², R³ jeweils unabhängig für Wasserstoff, (C₁-C₄)-Alkylthiocarbonyl, (C₁-C₄)-Halogenalkylthiocarbonyl, (C₁-C₆)-Alkoxy-(C₁-C₃)-alkylcarbonyl, (C₁-C₄)-Alkylthio-(C₁-C₃)-alkylcarbonyl, (C₁-C₄)-Alkylsulfoxy-((C₁-C₃)-alkylcarbonyl, (C₁-C₄)-Alkylsulfonyl-(C₁-C₃)-alkylcarbonyl, (C₁-C₆)-Alkylcarbonyl, (C₁-C₆)-Halogenalkylcarbonyl, (C₂-C₆)-Alkenylcarbonyl, (C₂-C₆)-Alkinylcarbonyl, (C₁-C₆)-Alkoxycarbonylcarbonyl, (C₁-C₆)-Alkoxycarbonyl-(C₁-C₃)-alkylcarbonyl, (C₁-C₆)-Alkoxycarbonyl, (C₁-C₆)-Halogenalkoxycarbonyl, (C₂-C₆)-Alkenyloxycarbonyl, (C₃-C₆)-Cycloalkylcarbonyl, (C₃-C₆)-Cycloalkyl-(C₁-C₆)-alkylcarbonyl, Phenylcarbonyl, Phenyl-(C₁-C₆)-alkylcarbonyl, Hetarylcarbonyl, Hetaryl-(C₁-C₆)-alkylcarbonyl, Heterocyclylcarbonyl oder Heterocyclyl-(C₁-C₆)-alkylcarbonyl stehen, wobei die 8 letztgenannten Reste jeweils unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy und (C₁-C₄)-Alkylsulfonyl substituiert sind und wobei Heterocyclyl q Oxogruppen aufweist,
oder
NR²R³ für -N=CR⁸R⁹ oder -N=S(O)ₙR¹⁰R¹¹ steht,
R⁶ für Wasserstoff steht,
R⁷ für Wasserstoff oder Methyl steht,
R⁸, R⁹ jeweils unabhängig für Wasserstoff, (C₁-C₆)-Alkyl, (C₁-C₆)-Halogenalkyl, (C₂-C₆)-Alkenyl, (C₂-C₆)-Alkinyl, (C₁-C₆)-Alkoxy-(C₁-C₃)-alkyl, (C₁-C₄)-Alkylthio-(C₁-C₃)-alkyl, (C₁-C₄)-Alkylsulfoxy-(C₁-C₃)-alkyl, (C₁-C₄)-Alkylsulfonyl-(C₁-C₃)-alkyl, (C₁-C₆)-Alkoxy, (C₁-C₆)-Halogenalkoxy, (C₂-C₆)-Alkenyloxy, NR¹³R¹⁴, (C₃-C₈)-Cycloalkyl, (C₃-C₈)-Cycloalkyl-(C₁-C₆)-alkyl, Phenyl, Phenyl-(C₁-C₃)-alkyl, Heteroaryl, Heteroaryl-(C₁-C₃)-alkyl, Heterocyclyl oder Heterocyclyl-(C₁-C₃)-alkyl stehen, wobei die 8 letztgenannten Reste jeweils unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy und (C₁-C₄)-Alkylsulfonyl substituiert sind und wobei Heterocyclyl q Oxogruppen aufweist,
oder
R⁸ und R⁹ zusammen mit dem Kohlenstoffatom, an das sie gebunden sind, einen 3- bis 6-gliedrigen ungesättigten, teilweise gesättigten oder gesättigten Ring bilden, der jeweils zusätzlich zu den Kohlenstoffatomen p Ringglieder aus der aus N(R¹²)ₘ, O und S(O)ₙ bestehenden Gruppe umfasst, und wobei der Ring unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfbxy und (C₁-C₄)-Alkylsulfonyl substituiert ist,
R¹⁰, R¹¹ jeweils unabhängig für (C₁-C₆)-Alkyl, (C₂-C₆)-Alkenyl, (C₂-C₆)-Alkinyl, (C₃-C₈)-Cycloalkyl, (C₃-C₈)-Cycloalkyl-(C₁-C₆)-alkyl, Phenyl, Phenyl-(C₁-C₃)-alkyl, Heteroaryl, Heteroaryl-(C₁-C₃)-alkyl, Heterocyclyl oder Heterocyclyl-(C₁-C₃)-alkyl stehen, wobei die 8 letztgenannten Reste jeweils unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy und (C₁-C₄)-Alkylsulfonyl substituiert sind und wobei Heterocyclyl q Oxogruppen aufweist,
oder
R¹⁰ und R¹¹ zusammen mit dem Schwefelatom, an das sie gebunden sind, einen 3- bis 6-gliedrigen ungesättigten, teilweise gesättigten oder gesättigten Ring bilden, der jeweils zusätzlich zu den Kohlenstoffatomen und zusätzlich zu dem Schwefelatom p Ringglieder aus der aus N(R¹²)ₘ, O und S(O)ₙ bestehenden Gruppe umfasst, und wobei der Ring unsubstituiert oder durch einen oder mehrere Reste aus der Gruppe bestehend aus Halogen, Nitro, Hydroxyl, Cyano, NR¹³R¹⁴, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy und (C₁-C₄)-Alkylsulfonyl substituiert ist,
R¹² für Wasserstoff, (C₁-C₆)-Alkyl oder (C₁-C₆)-Alkylcarbonyl steht,
R¹³, R¹⁴ jeweils unabhängig für Wasserstoff, (C₁-C₆)-Alkyl, (C₁-C₆)-Alkylcarbonyl, (C₁-C₄)-Alkylsulfonyl, Phenyl oder Phenylcarbonyl stehen, wobei die zwei letztgenannten Reste jeweils unsubstituiert oder durch einen oder mehrere Reste aus der Gruppe bestehend aus Halogen, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Alkylsulfoxy und (C₁-C₄)-Alkylsulfonyl substituiert sind,
oder
R¹³ und R¹⁴ zusammen mit dem Stickstoffatom, an das sie gebunden sind, einen 3- bis 8-gliedrigen ungesättigten, teilweise gesättigten oder gesättigten Ring bilden, der jeweils zusätzlich zu den Kohlenstoffatomen und zusätzlich zu dem Stickstoffatom p Ringglieder aus der aus N(R¹²)ₘ, O und S(O)ₙ bestehenden Gruppe umfasst, und wobei der Ring unsubstituiert oder durch einen oder mehrere Reste aus der Gruppe bestehend aus Halogen, Hydroxyl, (C₁-C₄)-Alkyl, (C₁-C₄)-Halogenalkyl, (C₁-C₄)-Alkoxy und (C₁-C₄)-Halogenalkoxy substituiert ist und q Oxogruppen aufweist,
n für 0 steht,
m unabhängig aus 0 und 1 ausgewählt ist,
p unabhängig aus 0, 1 und 2 ausgewählt ist,
q unabhängig aus 0 und 1 ausgewählt ist,
y für 0 oder 1 steht.

7. Verbindung der Formel (G) nach einem der Ansprüche 1 bis 6 und/oder ein Salz davon, wobei
n unabhängig aus 0 und 1 ausgewählt ist, n vorzugsweise für 0 steht,
m unabhängig aus 0 und 1 ausgewählt ist, m vorzugsweise für 0 steht,
p unabhängig aus 0 und 1 ausgewählt ist und
q unabhängig aus 0 und 1 ausgewählt ist, q vorzugsweise für 0 steht.

8. Verbindung der Formel (G) nach einem der Ansprüche 1 bis 7 und/oder ein Salz davon, wobei y für 1 steht.

9. Verwendung einer oder mehrerer Verbindungen der Formel (G) und/oder Salze davon, wie in einem der Ansprüche 1 bis 8 definiert, als Herbizide und/oder Pflanzenwachstumsregulatoren.

10. Verwendung einer oder mehrerer Verbindungen der Formel (G) und/oder Salzen davon nach Anspruch 9 zur Bekämpfung von Schadpflanzen oder zum Regulieren des Wachstums von Pflanzen, wobei man eine oder mehrere Verbindungen der Formel (G) und/oder Salze davon auf die Schadpflanzen, Pflanzensamen, den Boden, in dem oder auf dem die Pflanzen wachsen, oder die Kulturfläche ausbringt.

11. Herbizide und/oder pflanzenwachstumsregulierende Zusammensetzung, **dadurch gekennzeichnet, dass** die Zusammensetzung eine oder mehrere Verbindungen der Formel (G) und/oder Salze davon, wie in einem der Ansprüche 1 bis 8 definiert,
und eine oder mehrere weitere Substanzen ausgewählt aus den Gruppen (i) und/oder (ii) umfasst:
(i) eine oder mehrere weitere agrochemische Wirkstoffe, vorzugsweise ausgewählt aus der Gruppe bestehend aus Insektiziden, Akariziden, Nematiziden, weiteren Herbiziden, Fungiziden, Safenern, Düngemitteln und/oder weiteren Wachstumsregulatoren,
(ii) ein oder mehrere im Pflanzenschutz übliche Formulierungshilfsstoffe.

12. Verfahren zur Bekämpfung von Schadpflanzen oder zum Regulieren des Wachstums von Pflanzen, **dadurch gekennzeichnet, dass** man eine wirksame Menge von
- einer oder mehreren Verbindungen der Formel (G) und/oder Salzen davon, wie in einem der Ansprüche 1 bis 8 definiert,
oder
- einer Zusammensetzung nach Anspruch 11
auf die Pflanzen, Samen von Pflanzen, den Boden, in oder auf dem die Pflanzen wachsen, oder die Kulturfläche ausbringt.

13. Verfahren zur Herstellung einer wie in einem der Ansprüche 1 bis 8 definierten Verbindung der Formel (G) und/oder eines Salzes davon, wobei W für Sauerstoff steht, **dadurch gekennzeichnet, dass** man
(a) eine Verbindung der Formel (G), in welcher R² und R³ beide für H stehen, in einer chemischen Synthese erhält, die den Schritt der Cyclisierung einer Verbindung der Formel (E-XXIV) 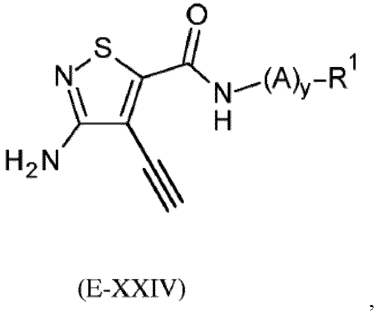 in welcher R¹, A und y jeweils die in Formel (G) definierte Bedeutung haben, umfasst,
oder
(b) eine Verbindung der Formel (G), in welcher R² und R³ beide für H stehen, in einer chemischen Synthese erhält, die den Schritt der Cyclisierung einer Verbindung der Formel (E-XXVII) 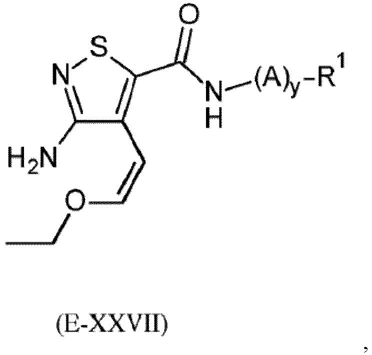 in welcher R¹, A und y jeweils die in Formel (G) definierte Bedeutung haben, umfasst,
oder
(c) eine Verbindung der Formel (G), in welcher R² und/oder R³ nicht für H steht/stehen, in einer chemischen Synthese erhält, die den Schritt der Umsetzung einer Verbindung der Formel (G-1) 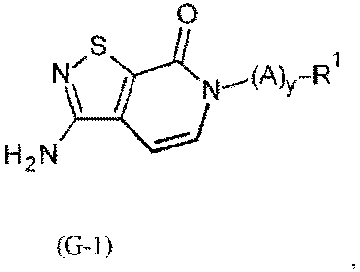 in welcher R¹, A und y jeweils die in Formel (G) definierte Bedeutung haben,
mit einem Acylhalogenid R²COHal und/oder R³COHal, wobei Hal jeweils für Cl, Br oder I steht, oder einem Anhydrid (R²CO)₂O, (R³CO)₂O und/oder R²CO(O)OCR³, wobei R² und R³ jeweils die in Formel (G) definierte Bedeutung haben, mit der Maßgabe, dass R² und/oder R³ nicht für H steht/stehen, umfasst.

## Revendications

1. Composé de formule (G) et/ou sel correspondant, 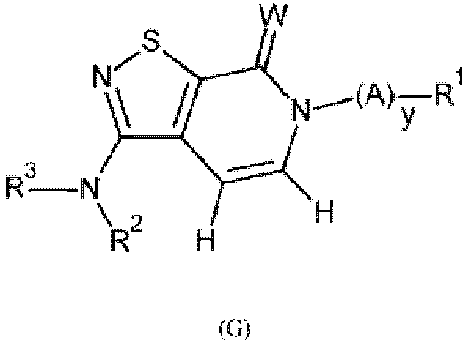 dans laquelle
A est CR⁶R⁷,
W est O ou S,
R¹ est (C₁-C₁₂)-alkyle, (C₁-C₁₂)-halogénoalkyle, (C₂-C₁₂)-alcényle, (C₂-C₁₂)-halogénoalcényle, (C₂-C₁₂)-alcynyle, (C₂-C₁₂)-halogénoalcynyle, NR¹³R¹⁴, R¹³R¹⁴N-(C₁-C₆)-alkyle, (C₁-C₆)-alcoxy, (C₁-C₆)-halogénoalcoxy, (C₁-C₆)-halogénoalcoxy-(C₁-C₃)-alkyle, (C₁-C₆)-alcoxy-(C₁-C₃)-alkyle, (C₁-C₆)-alcoxy-(C₂-C₆)-alcoxy-(C₁-C₃)-alkyle, (C₁-C₄)-alkylthio, (C₁-C₄)-alkylsulfoxy, (C₁-C₄)-alkylsulfonyle, (C₁-C₄)-halogénoalkylthio, (C₁-C₄)-halogenoalkylsulfoxy, (C₁-C₄)-halogénoalkylsulfonyle, (C₁-C₄)-alkylthio-(C₁-C₃)-alkyle, (C₁-C₄)-alkylsulfoxy-(C₁-C₃)-alkyle, (C₁-C₄)-alkylsulfonyl-(C₁-C₃)-alkyle, (C₁-C₄)-halogénoalkylthio-(C₁-C₃)-alkyle, (C₁-C₄)-halogénoalkylsulfoxy-(C₁-C₃)-alkyle, (C₁-C₄)-halogénoalkylsulfonyl-(C₁-C₃)-alkyle, (C₃-C₁₂)-cycloalkyle, (C₃-C₈)-cycloalcényle, (C₃-C₁₂)-cycloalkyl-(C₁-C₆)-alkyle, (C₃-C₈)-cycloalcényl-(C₁-C₆)-alkyle, (C₃-C₈)-cycloalcoxy, (C₃-C₈)-cycloalkyl-(C₁-C₆)-alcoxy, aryle, aryl-(C₁-C₃)-alkyle, hétéroaryle, hétéroaryl-(C₁-C₃)-alkyle, hétérocyclyle, hétérocyclyl-(C₁-C₃)-alkyle, aryloxy, hétéroaryloxy, hétérocyclyloxy, un radical bicyclique ou un radical hétérobicyclique, chacun des 17 radicaux mentionnés en dernier étant non substitué ou étant substitué par un ou plusieurs radicaux choisis dans le groupe constitué par halogène, nitro, hydroxyle, cyano, NR¹³R¹⁴, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy, (C₁-C₄)-alkylsulfonyle, (C₁-C₄)-halogénoalkylthio, (C₁-C₄)-halogénoalkylsulfoxy, (C₁-C₄)-halogénoalkylsulfonyle, (C₁-C₄)-alcoxycarbonyle, (C₁-C₄)-halogénoalcoxycarbonyle, (C₁-C₄)-alkylcarboxy, (C₃-C₆)-cycloalkyle, (C₃-C₆)-cycloalkyl-(C₁-C₆)-alkyle, (C₁-C₄)-alcoxycarbonyl-(C₁-C₄)-alkyle, hydroxycarbonyle, hydroxycarbonyl-(C₁-C₄)-alkyle, R¹³R¹⁴N-carbonyle et hétérocyclyle possédant q groupes oxo, et chacun parmi les radicaux hétérocycliques mentionnés précédemment, en plus des atomes de carbone, possédant dans chaque cas p éléments de cycle du groupe constitué par N(R¹²)ₘ, O et S(O)ₙ,
R², R³ sont chacun indépendamment hydrogène, (C₁-C₁₂)-alkyle, (C₁-C₁₂)-halogénoalkyle, (C₂-C₁₂)-alcényle, (C₂-C₁₂)-halogénoalcényle, (C₂-C₁₂)-alcynyle, (C₂-C₁₂)-halogénoalcynyle, (C₁-C₆)-alcoxy-(C₁-C₃)-alkyle, (C₁-C₆)-alcoxy-(C₂-C₆)-alcoxy-(C₁-C₃)-alkyle, (C₁-C₆)-alcoxy-(C₁-C₃)-alkylcarbonyle, (C₁-C₆)-alcoxy-(C₂-C₆)-alcoxy-(C₁-C₃)-alkylcarbonyle, (C₁-C₆)-alcoxycarbonyle, (C₁-C₆)-halogénoalcoxycarbonyle, (C₂-C₆)-alcényloxycarbonyle, (C₂-C₆)-halogénoalcényloxycarbonyle, (C₂-C₆)-alcynyloxycarbonyle, (C₂-C₆)-halogénoalcynyloxycarbonyle, (C₁-C₆)-alkylcarbonyle, (C₁-C₆)-halogénoalkylcarbonyle, (C₂-C₆)-alcénylcarbonyle, (C₂-C₆)-halogénoalcénylcarbonyle, (C₂-C₆)-alcynylcarbonyle, (C₂-C₆)-halogénoalcynylcarbonyle, R¹³R¹⁴N-carbonyle, (C₁-C₄)-alkylthio, (C₁-C₄)-halogénoalkylthio, (C₁-C₈)-alkylthiocarbonyle, (C₁-C₈)-halogénoalkylthiocarbonyle, (C₁-C₄)-alkylsulfoxy, (C₁-C₄)-halogénoalkylsulfoxy, (C₁-C₄)-alkylsulfonyle, (C₁-C₄)-halogénoalkylsulfonyle, (C₁-C₄)-alkylthio-(C₁-C₃)-alkyle, (C₁-C₄)-alkylsulfoxy-(C₁-C₃)-alkyle, (C₁-C₄)-alkylsulfonyl-(C₁-C₃)-alkyle, (C₁-C₄)-alkylthio-(C₁-C₃)-alkylcarbonyle, (C₁-C₄)-alkylsulfoxy-(C₁-C₃)-alkylcarbonyle, (C₁-C₄)-alkylsulfonyl-(C₁-C₃)-alkylcarbonyle, (C₁-C₁₂)-alkylcarbonyle, (C₁-C₁₂)-halogénoalkylcarbonyle, (C₂-C₁₂)-alcénylcarbonyle, (C₂-C₁₂)-halogénoalcénylcarbonyle, (C₂-C₁₂)-alcynylcarbonyle, (C₂-C₁₂)-halogénoalcynylcarbonyle, (C₁-C₁₂)-alcoxycarbonylcarbonyle, (C₁-C₁₂)-alcoxycarbonyl-(C₁-C₃)-alkylcarbonyle, (C₃-C₈)-cycloalkyle, (C₃-C₈)-cycloalcényle, (C₃-C₈)-cycloalkyl-(C₁-C₆)-alkyle, (C₃-C₈)-cycloalcényl-(C₁-C₆)-alkyle, (C₃-C₈)-cycloalkylcarbonyle, (C₃-C₈)-cycloalcénylcarbonyle, (C₃-C₈)-cycloalkyl-(C₁-C₆)-alkylcarbonyle, (C₃-C₈)-cycloalcényl-(C₁-C₆)-alkylcarbonyle, aryle, aryl-(C₁-C₃)-alkyle, hétéroaryle, hétéroaryl-(C₁-C₃)-alkyle, hétérocyclyle, hétérocyclyl-(C₁-C₃)-alkyle, arylcarbonyle, aryl-(C₁-C₆)-alkylcarbonyle, hétéroarylcarbonyle, heteroaryl-(C₁-C₆)-alkylcarbonyle, hétérocyclylcarbonyle ou hétérocyclyl-(C₁-C₆)-alkylcarbonyle, chacun des 20 radicaux mentionnés en dernier étant non substitué ou étant substitué par un ou plusieurs radicaux choisis dans le groupe constitué par halogène, nitro, hydroxyle, cyano, NR¹³R¹⁴, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy, (C₁-C₄)-alkylsulfonyle, (C₁-C₄)-halogénoalkylthio, (C₁-C₄)-halogénoalkylsulfoxy, (C₁-C₄)-halogénoalkylsulfonyle, (C₁-C₄)-alcoxycarbonyle, (C₁-C₄)-halogénoalcoxycarbonyle, (C₁-C₄)-alkylcarboxy, (C₃-C₆)-cycloalkyle, (C₃-C₆)-cycloalkyl-(C₁-C₆)-alkyle, (C₁-C₄)-alcoxycarbonyl-(C₁-C₄)-alkyle, hydroxycarbonyle, hydroxycarbonyl-(C₁-C₄)-alkyle et R¹³R¹⁴N-carbonyle, et hétérocyclyle possédant q groupes oxo,
ou
NR²R³ est -N=CR⁸R⁹ ou -N=S(O)ₙR¹⁰R¹¹,
R⁶, R⁷ sont chacun indépendamment hydrogène, cyano, halogène, (C₁-C₆)-alkyle, (C₂-C₆)-alcényle, (C₂-C₆)-alcynyle ou (C₃-C₈)-cycloalkyle, ou R⁶ et R⁷, conjointement avec l'atome de carbone auquel ils sont fixés, forment un cycle carbone cyclique ou hétérocyclique à 3 à 6 chaînons, qui comprend dans chaque cas, en plus des atomes de carbone, p éléments de cycle du groupe constitué par N(R¹²)ₘ, O et S(O)ₙ, et ledit cycle étant non substitué ou étant substitué par un ou plusieurs radicaux du groupe constitué par halogène, nitro, hydroxyle, cyano, NR¹³R¹⁴, (C₁-C-₄-alkyle, (C₁-C₄)-halogénoalkyle, (C₁-C₄)-alcoxy, (C₁-C-₄-halogénoalcoxy, (C₁-C₄)-alkylthio, (C₁-C₄)-alkylsulfoxy, (C₁-C₄)-alkylsulfonyle, (C₁-C₄)-halogénoalkylthio, (C₁-C₄)-halogénoalkylsulfoxy, (C₁-C₄)-halogénoalkylsulfonyle, (C₁-C₄)-alcoxycarbonyle, (C₁-C₄)-halogénoalcoxycarbonyle, (C₁-C₄)-alkylcarboxy, (C₃-C₆)-cycloalkyle, (C₃-C₆)-cycloalkyl-(C₁-C₆)-alkyle, (C₁-C₄)-alcoxycarbonyl-(C₁-C₄)-alkyle, hydroxycarbonyle, hydroxycarbonyl-(C₁-C₄)-alkyle et R¹³R¹⁴N-carbonyle et possédant q groupes oxo,
R⁸, R⁹ sont chacun indépendamment hydrogène, (C₁-C₆)-alkyle, (C₁-C₆)-halogénoalkyle, (C₂-C₆)-alcényle, (C₂-C₆)-halogénoalcényle, (C₂-C₆)-alcynyle, (C₂-C₆)-halogénoalcynyle, (C₁-C₆)-alcoxy, (C₁-C₆)-halogénoalcoxy, (C₁-C₆)-halogénoalcoxy-(C₁-C₃)-alkyle, (C₂-C₆)-alcényloxy, (C₂-C₆)-halogénoalcényloxy, (C₂-C₆)-alcynyloxy, (C₂-C₆)-halogénoalcynyloxy, NR¹³R¹⁴, (C₁-C₆)-alcoxy-(C₁-C₃)-alkyle, halogen-(C₁-C₆)-alcoxy-(C₁-C₆)-alkyle, (C₁-C₆)-alcoxy-(C₂-C₆)-alcoxy-(C₁-C₃)-alkyle, (C₁-C₄)-alkylthio-(C₁-C₃)-alkyle, (C₁-C₄)-alkylsulfoxy-(C₁-C₃)-alkyle, (C₁-C₄)-alkylsulfonyl-(C₁-C₃)-alkyle, (C₃-C₈)-cycloalkyle, (C₃-C₈)-cycloalcényle, (C₃-C₈)-cycloalkyl-(C₁-C₆)-alkyle, (C₃-C₈)-cycloalcényl-(C₁-C₆)-alkyle, aryle, aryl-(C₁-C₃)-alkyle, hétéroaryle, heteroaryl-(C₁-C₃)-alkyle, hétérocyclyle ou hétérocyclyl-(C₁-C₃)-alkyle, chacun des 10 radicaux mentionnés en dernier étant non substitué ou étant substitué par un ou plusieurs radicaux du groupe constitué par halogène, nitro, hydroxyle, cyano, NR¹³R¹⁴, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy, (C₁-C₄)-alkylsulfonyle, (C₁-C₄)-halogénoalkylthio, (C₁-C₄)-halogénoalkylsulfoxy, (C₁-C₄)-halogénoalkylsulfonyle, (C₁-C₄)-alcoxycarbonyle, (C₁-C₄)-halogénoalcoxycarbonyle, (C₁-C₄)-alkylcarboxy, (C₃-C₆)-cycloalkyle, (C₃-C₆)-cycloalkyl-(C₁-C₆)-alkyle, (C₁-C₄)-alcoxycarbonyl-(C₁-C₄)-alkyle, hydroxycarbonyle, hydroxycarbonyl-(C₁-C₄)-alkyle et R¹³R¹⁴N-carbonyle et possédant q groupes oxo,
ou
R⁸ et R⁹, conjointement avec l'atome de carbone auquel ils sont fixés, forment un cycle insaturé, partiellement saturé ou saturé à 3 à 8 chaînons, qui comprend dans chaque cas, en plus des atomes de carbone, p éléments de cycle du groupe constitué par N(R¹²)ₘ, O et S(O)ₙ, et ledit cycle étant non substitué ou étant substitué par un ou plusieurs radicaux du groupe constitué par halogène, nitro, hydroxyle, cyano, NR¹³R¹⁴, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy, (C₁-C₄)-alkylsulfonyle, (C₁-C₄)-halogénoalkylthio, (C₁-C₄)-halogénoalkylsulfoxy, (C₁-C₄)-halogénoalkylsulfonyle, (C₁-C₄)-alcoxycarbonyle, (C₁-C₄)-halogénoalcoxycarbonyle, (C₁-C₄)-alkylcarboxy, (C₃-C₆)-cycloalkyle, (C₃-C₆)-cycloalkyl-(C₁-C₆)-alkyle, (C₁-C₄)-alcoxycarbonyl-(C₁-C₄)-alkyle, hydroxycarbonyle, hydroxycarbonyl-(C₁-C₄)-alkyle et R¹³R¹⁴N-carbonyle et possédant q groupes oxo,
R¹⁰, R¹¹ sont chacun indépendamment (C₁-C₆)-alkyle, (C₁-C₆)-halogénoalkyle, (C₂-C₆)-alcényle, (C₂-C₆)-halogénoalcényle, (C₂-C₆)-alcynyle, (C₂-C₆)-halogénoalcynyle, (C₁-C₆)-alcoxy-(C₁-C₃)-alkyle, halogen-(C₁-C₆)-alcoxy-(C₁-C₆)-alkyle, (C₁-C₆)-alcoxy-(C₂-C₆)-alcoxy-(C₁-C₃)-alkyle, (C₁-C₄)-alkylthio-(C₁-C₃)-alkyle, (C₁-C₄)-alkylsulfoxy-(C₁-C₃)-alkyle, (C₁-C₄)-alkylsulfonyl-(C₁-C₃)-alkyle, (C₃-C₈)-cycloalkyle, (C₃-C₈)-cycloalcényle, (C₃-C₈)-cycloalkyl-(C₁-C₆)-alkyle, (C₃-C₈)-cycloalcényl-(C₁-C₆)-alkyle, aryle, aryl-(C₁-C₃)-alkyle, hétéroaryle, hétéroaryl-(C₁-C₃)-alkyle, hétérocyclyle ou hétérocyclyl-(C₁-C₃)-alkyle, chacun des 10 radicaux mentionnés en dernier étant non substitué ou étant substitué par un ou plusieurs radicaux du groupe constitué par halogène, nitro, hydroxyle, cyano, NR¹³R¹⁴, (C₁-C₄)-alkyle, (C₁-C-₄-halogénoalkyle, (C₁-C₄)-alcoxy, (C₁-C₄)-halogénoalcoxy, (C₁-C₄)-alkylthio, (C₁-C₄)-alkylsulfoxy, (C₁-C₄)-alkylsulfonyle, (C₁-C₄)-halogénoalkylthio, (C₁-C₄)-halogénoalkylsulfoxy, (C₁-C₄)-halogénoalkylsulfonyle, (C₁-C₄)-alcoxycarbonyle, (C₁-C₄)-halogénoalcoxycarbonyle, (C₁-C₄)-alkylcarboxy, (C₃-C₆)-cycloalkyle, (C₃-C₆)-cycloalkyl-(C₁-C₆)-alkyle, (C₁-C₄)-alcoxycarbonyl-(C₁-C₄)-alkyle, hydroxycarbonyle, hydroxycarbonyl-(C₁-C₄)-alkyle et R¹³R¹⁴N-carbonyle et hétérocyclyle possédant q groupes oxo,
ou
R¹⁰ et R¹¹, conjointement avec l'atome de soufre auquel ils sont fixés, forment un cycle insaturé, partiellement saturé ou saturé à 3 à 8 chaînons, qui comprend dans chaque cas, en plus des atomes de carbone et en plus de l'atome de soufre, p éléments de cycle du groupe constitué par N(R¹²)ₘ, O et S(O)ₙ, et ledit cycle étant non substitué ou étant substitué par un ou plusieurs radicaux du groupe constitué par halogène, nitro, hydroxyle, cyano, NR¹³R¹⁴, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy, (C₁-C₄)-alkylsulfonyle, (C₁-C₄)-halogénoalkylthio, (C₁-C₄)-halogénoalkylsulfbxy, (C₁-C₄)-halogénoalkylsulfonyle, (C₁-C₄)-alcoxycarbonyle, (C₁-C₄)-halogénoalcoxycarbonyle, (C₁-C₄)-alkylcarboxy, (C₃-C₆)-cycloalkyle, (C₃-C₆)-cycloalkyl-(C₁-C₆)-alkyle, (C₁-C₄)-alcoxycarbonyl-(C₁-C₄)-alkyle, hydroxycarbonyle, hydroxycarbonyl-(C₁-C₄)-alkyle et R¹³R¹⁴N-carbonyle et possédant q groupes oxo,
R¹² est hydrogène, (C₁-C₁₂)-alkyle, (C₁-C₁₂)-halogénoalkyle, (C₂-C₁₂)-alcényle, (C₂-C₁₂)-halogénoalcényle, (C₂-C₁₂)-alcynyle, (C₂-C₁₂)-halogénoalcynyle, (C₃-C₈)-cycloalkyle, (C₃-C₈)-halogénocycloalkyle, (C₃-C₈)-cycloalcényle, (C₃-C₈)-cycloalkyl-(C₁-C₆)-alkyle, (C₃-C₈)-cycloalcényl-(C₁-C₆)-alkyle, (C₁-C₁₂)-alkylcarbonyle ou (C₁-C₁₂)-halogénoalkylcarbonyle,
R¹³, R¹⁴ sont à chaque fois indépendamment hydrogène, (C₁-C₁₂)-alkyle, (C₁-C₁₂)-halogénoalkyle, (C₂-C₁₂)-alcényle, (C₂-C₁₂)-halogénoalcényle, (C₂-C₁₂)-alcynyle, (C₂-C₁₂)-halogénoalcynyle, (C₁-C₁₂)-alkylcarbonyle, (C₂-C₁₂)-alcénylcarbonyle, (C₂-C₁₂)-alcynylcarbonyle, (C₁-C₁₂)-halogénoalkylcarbonyle, (C₁-C₄)-alkylsulfonyle, (C₃-C₈)-cycloalkyle, (C₃-C₈)-cycloalcényle, (C₃-C₈)-cycloalkyl-(C₁-C₆)-alkyle, (C₃-C₈)-cycloalcényl-(C₁-C₆)-alkyle, (C₃-C₈)-cycloalkylcarbonyle, (C₃-C₈)-cycloalcénylcarbonyle, (C₃-C₈)-cycloalkyl-(C₁-C₆)-alkylcarbonyle, (C₃-C₈)-cycloalcényl-(C₁-C₆)-alkylcarbonyle, aryle, arylcarbonyle, arylsulfonyle, hetaryle, hetarylcarbonyle, hetarylsulfonyle, hétérocyclyle, hétérocyclylcarbonyle ou hétérocyclylsulfonyle, chacun des 17 radicaux mentionnés en dernier étant non substitué ou étant substitué par un ou plusieurs radicaux du groupe constitué par halogène, nitro, hydroxyle, cyano, NH₂, (C₁-C₆)-alkylamine, (C₁-C₆)-dialkylamine, (C₁-C-₄-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy, (C₁-C₄)-alkylsulfonyle, (C₁-C₄)-halogénoalkylthio, (C₁-C₄)-halogénoalkylsulfoxy, (C₁-C₄)-halogénoalkylsulfonyle, (C₁-C₄)-alcoxycarbonyle, (C₁-C₄)-halogénoalcoxycarbonyle, (C₁-C₄)-alkylcarboxy, (C₃-C₆)-cycloalkyle, (C₃-C₆)-cycloalkyl-(C₁-C₆)-alkyle, (C₁-C₄)-alcoxycarbonyl-(C₁-C₄)-alkyle, hydroxycarbonyle et hydroxycarbonyl-(C₁-C₄)-alkyle et hétérocyclyle possédant q groupes oxo,
ou
R¹³ et R¹⁴, conjointement avec l'atome d'azote auquel ils sont fixés, forment un cycle insaturé, partiellement saturé ou saturé à 3 à 8 chaînons, qui comprend dans chaque cas, en plus des atomes de carbone et en plus de l'atome d'azote, p éléments de cycle du groupe constitué par N(R¹²)ₘ, O et S(O)ₙ, et ledit cycle étant non substitué ou étant substitué par un ou plusieurs radicaux du groupe constitué par halogène, nitro, hydroxyle, cyano, NH₂, (C₁-C₆)-alkylamine, (C₁-C₆)-dialkylamine, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy, (C₁-C₄)-alkylsulfonyle, (C₁-C₄)-halogénoalkylthio, (C₁-C₄)-halogénoalkylsulfoxy, (C₁-C₄)-halogénoalkylsulfonyle, (C₁-C₄)-alcoxycarbonyle, (C₁-C₄)-halogénoalcoxycarbonyle, (C₁-C₄)-alkylcarboxy, (C₃-C₆)-cycloalkyle, (C₃-C₆)-cycloalkyl-(C₁-C₆)-alkyle, (C₁-C₄)-alcoxycarbonyl-(C₁-C₄)-alkyle, hydroxycarbonyle et hydroxycarbonyl-(C₁-C₄)-alkyle et possédant q groupes oxo,
n est indépendamment choisi parmi 0, 1 et 2,
m est indépendamment choisi parmi 0 et 1,
p est indépendamment choisi parmi 0, 1, 2 et 3,
q est indépendamment choisi parmi 0, 1 et 2,
y est 0 ou 1.

2. Composé de formule (G) selon la revendication 1 et/ou sel correspondant, dans laquelle
A est CR⁶R⁷,
W est O ou S,
R¹ est (C₁-C₆)-alkyle, (C₁-C₆)-halogénoalkyle, (C₂-C₆)-alcényle, (C₂-C₆)-alcynyle, NR¹³R¹⁴, R¹³R¹⁴N-(C₁-C₆)-alkyle, (C₁-C₆)-alcoxy, (C₁-C₆)-halogénoalcoxy, (C₁-C₆)-halogénoalcoxy-(C₁-C₃)-alkyle, (C₁-C₆)-alcoxy-(C₁-C₃)-alkyle, (C₁-C₆)-alcoxy-(C₂-C₆)-alcoxy-(C₁-C₃)-alkyle, (C₁-C₄)-alkylthio, (C₁-C₄)-alkylsulfoxy, (C₁-C₄)-alkylsulfonyle, (C₁-C₄)-alkylthio-(C₁-C₃)-alkyle, (C₁-C₄)-alkylsulfoxy-(C₁-C₃)-alkyle, (C₁-C₄)-alkylsulfonyl-(C₁-C₃)-alkyle, (C₃-C₈)-cycloalkyle, (C₃-C₆)-cycloalcényle, (C₃-C₆)-cycloalkyl-(C₁-C₃)-alkyle, (C₃-C₆)-cycloalcényl-(C₁-C₃)-alkyle, (C₃-C₆)-cycloalcoxy, phényle, hétéroaryle, hétérocyclyle, phénoxy, hétéroaryloxy, hétérocyclyloxy ou un radical carbobicyclique, chacun des 12 radicaux mentionnés en dernier étant non substitué ou étant substitué par un ou plusieurs radicaux choisis dans le groupe constitué par halogène, nitro, hydroxyle, cyano, NR¹³R¹⁴, (C₁-C-₄-alkyle, (C₁-C-₄-halogénoalkyle, (C₁-C₄)-alcoxy, (C₁-C-₄-halogénoalcoxy, (C₁-C₄)-alkylthio, (C₁-C₄)-alkylsulfoxy et (C₁-C₄)-alkylsulfonyle, et hétérocyclyle possédant q groupes oxo,
R², R³ sont chacun indépendamment hydrogène, (C₁-C₆)-alkyle, (C₁-C₆)-halogénoalkyle, (C₂-C₆)-alcényle, (C₂-C₆)-alcynyle, (C₁-C₆)-alcoxy-(C₁-C₃)-alkyle, (C₁-C₆)-alcoxy-(C₁-C₃)-alkylcarbonyle, (C₁-C₄)-alkylthio, (C₁-C₄)-halogénoalkylthio, (C₁-C₄)-alkylthiocarbonyle, (C₁-C₄)-halogénoalkylthiocarbonyle, (C₁-C₄)-alkylthio-(C₁-C₃)-alkyle, (C₁-C₄)-alkylsulfoxy-(C₁-C₃)-alkyle, (C₁-C₄)-alkylsulfonyl-(C₁-C₃)-alkyle, (C₁-C₄)-alkylthio-(C₁-C₃)-alkylcarbonyle, (C₁-C₄)-alkylsulfoxy-(C₁-C₃)-alkylcarbonyle, (C₁-C₄)-alkylsulfonyl-(C₁-C₃)-alkylcarbonyle, (C₁-C₆)-alkylcarbonyle, (C₁-C₆)-halogénoalkylcarbonyle, (C₂-C₆)-alcénylcarbonyle, (C₂-C₆)-alcynylcarbonyle, (C₁-C₆)-alcoxycarbonylcarbonyle, (C₁-C₆)-alcoxycarbonyl-(C₁-C₃)-alkylcarbonyle, (C₁-C₆)-alcoxycarbonyle, (C₁-C₆)-halogénoalcoxycarbonyle, (C₂-C₆)-alcényloxycarbonyle, (C₃-C₆)-cycloalkyle, (C₃-C₆)-cycloalkyl-(C₁-C₆)-alkyle, (C₃-C₆)-cycloalkylcarbonyle, (C₃-C₆)-cycloalkyl-(C₁-C₆)-alkylcarbonyle, phényle, phényl-(C₁-C₃)-alkyle, hétéroaryle, hétéroaryl-(C₁-C₃)-alkyle, hétérocyclyle, hétérocyclyl-(C₁-C₃)-alkyle, phénylcarbonyle, phényl-(C₁-C₆)-alkylcarbonyle, hetarylcarbonyle, hetaryl-(C₁-C₆)-alkylcarbonyle, hétérocyclylcarbonyle ou hétérocyclyl-(C₁-C₆)-alkylcarbonyle, chacun des 16 radicaux mentionnés en dernier étant non substitué ou étant substitué par un ou plusieurs radicaux choisis dans le groupe constitué par halogène, nitro, hydroxyle, cyano, NR¹³R¹⁴, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy et (C₁-C₄)-alkylsulfonyle, et hétérocyclyle possédant q groupes oxo,
ou
NR²R³ est -N=CR⁸R⁹ ou -N=S(O)ₙR¹⁰R¹¹,
R⁶, R⁷ sont chacun indépendamment hydrogène ou (C₁-C₆)-alkyle,
R⁸, R⁹ sont chacun indépendamment hydrogène, (C₁-C₆)-alkyle, (C₁-C₆)-halogénoalkyle, (C₂-C₆)-alcényle, (C₂-C₆)-alcynyle, (C₁-C₆)-alcoxy-(C₁-C₃)-alkyle, (C₁-C₄)-alkylthio-(C₁-C₃)-alkyle, (C₁-C₄)-alkylsulfoxy-(C₁-C₃)-alkyle, (C₁-C₄)-alkylsulfonyl-(C₁-C₃)-alkyle, (C₁-C₆)-alcoxy, (C₁-C₆)-halogénoalcoxy, (C₂-C₆)-alcényloxy, NR¹³R¹⁴, (C₃-C₈)-cycloalkyle, (C₃-C₈)-cycloalkyl-(C₁-C₆)-alkyle, phényle, phényl-(C₁-C₃)-alkyle, hétéroaryle, hétéroaryl-(C₁-C₃)-alkyle, hétérocyclyle ou hétérocyclyl-(C₁-C₃)-alkyle, chacun des 8 radicaux mentionnés en dernier étant non substitué ou étant substitué par un ou plusieurs radicaux du groupe constitué par halogène, nitro, hydroxyle, cyano, NR¹³R¹⁴, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy et (C₁-C₄)-alkylsulfonyle, et hétérocyclyle possédant q groupes oxo,
ou
R⁸ et R⁹, conjointement avec l'atome de carbone auquel ils sont fixés, forment un cycle insaturé, partiellement saturé ou saturé à 3 à 6 chaînons, qui comprend dans chaque cas, en plus des atomes de carbone, p éléments de cycle du groupe constitué par N(R¹²)ₘ, O et S(O)ₙ, et ledit cycle étant non substitué ou étant substitué par un ou plusieurs radicaux du groupe constitué par halogène, nitro, hydroxyle, cyano, NR¹³R¹⁴, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy et (C₁-C₄)-alkylsulfonyle, et hétérocyclyle possédant q groupes oxo,
R¹⁰, R¹¹ sont chacun indépendamment (C₁-C₆)-alkyle, (C₂-C₆)-alcényle, (C₂-C₆)-alcynyle, (C₁-C₆)-alcoxy-(C₁-C₃)-alkyle, (C₁-C₄)-alkylthio-(C₁-C₃)-alkyle, (C₁-C₄)-alkylsulfoxy-(C₁-C₃)-alkyle, (C₁-C₄)-alkylsulfonyl-(C₁-C₃)-alkyle, (C₃-C₈)-cycloalkyle, (C₃-C₈)-cycloalkyl-(C₁-C₆)-alkyle, phényle, phényl-(C₁-C₃)-alkyle, hétéroaryle, hétéroaryl-(C₁-C₃)-alkyle, hétérocyclyle ou hétérocyclyl-(C₁-C₃)-alkyle, chacun des 8 radicaux mentionnés en dernier étant non substitué ou étant substitué par un ou plusieurs radicaux du groupe constitué par halogène, nitro, hydroxyle, cyano, NR¹³R¹⁴, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy et (C₁-C₄)-alkylsulfonyle, et hétérocyclyle possédant q groupes oxo,
ou
R¹⁰ et R¹¹, conjointement avec l'atome de soufre auquel ils sont fixés, forment un cycle insaturé, partiellement saturé ou saturé à 3 à 6 chaînons, qui comprend dans chaque cas, en plus des atomes de carbone et en plus de l'atome de soufre, p éléments de cycle du groupe constitué par N(R¹²)ₘ, O et S(O)ₙ, et ledit cycle étant non substitué ou étant substitué par un ou plusieurs radicaux du groupe constitué par halogène, nitro, hydroxyle, cyano, NR¹³R¹⁴, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy et (C₁-C₄)-alkylsulfonyle,
R¹² est hydrogène, (C₁-C₆)-alkyle ou (C₁-C₆)-alkylcarbonyle,
R¹³, R¹⁴ sont chacun indépendamment hydrogène, (C₁-C₆)-alkyle, (C₁-C₆)-alkylcarbonyle, (C₁-C₄)-alkylsulfonyle, phényle, phénylcarbonyle, chacun des deux radicaux mentionnés en dernier étant non substitué ou étant substitué par un ou plusieurs radicaux du groupe constitué par halogène, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy et (C₁-C₄)-alkylsulfonyle,
ou
R¹³ et R¹⁴, conjointement avec l'atome d'azote auquel ils sont fixés, forment un cycle insaturé, partiellement saturé ou saturé à 3 à 8 chaînons, qui comprend dans chaque cas, en plus des atomes de carbone et en plus de l'atome d'azote, p éléments de cycle du groupe constitué par N(R¹²)ₘ, O et S(O)ₙ, et ledit cycle étant non substitué ou étant substitué par un ou plusieurs radicaux du groupe constitué par halogène, hydroxyle, (C₁-C₄)-alkyle, (C₁-C₄)-halogénoalkyle, (C₁-C₄)-alcoxy et (C₁-C₄)-halogénoalcoxy et possédant q groupes oxo,
n est indépendamment choisi parmi 0, 1 et 2,
m est indépendamment choisi parmi 0 et 1,
p est indépendamment choisi parmi 0, 1 et 2,
q est indépendamment choisi parmi 0, 1 et 2,
y est 0 ou 1.

3. Composé de formule (G) selon la revendication 1 ou 2, et/ou sel correspondant, dans laquelle
A est CR⁶R⁷,
W est O ou S,
R¹ est (C₁-C₆)-alkyle, (C₁-C₆)-halogénoalkyle, (C₂-C₆)-alcényle, (C₂-C₆)-alcynyle, NR¹³R¹⁴, R¹³R¹⁴N-(C₁-C₆)-alkyle, (C₁-C₆)-alcoxy, (C₁-C₆)-halogénoalcoxy, (C₁-C₆)-alcoxy-(C₁-C₃)-alkyle, (C₁-C₆)-alcoxy-(C₂-C₆)-alcoxy-(C₁-C₃)-alkyle, (C₁-C₄)-alkylthio, (C₁-C₄)-alkylsulfoxy, (C₁-C₄)-alkylsulfonyle, (C₁-C₄)-alkylthio-(C₁-C₃)-alkyle, (C₁-C₄)-alkylsulfoxy-(C₁-C₃)-alkyle, (C₁-C₄)-alkylsulfonyl-(C₁-C₃)-alkyle, (C₃-C₈)-cycloalkyle, (C₃-C₆)-cycloalcényle, (C₃-C₆)-cycloalkyl-(C₁-C₃)-alkyle, (C₃-C₆)-cycloalcényl-(C₁-C₃)-alkyle, (C₃-C₆)-cycloalcoxy, phényle, hétéroaryle, hétérocyclyle, phénoxy, hétéroaryloxy ou hétérocyclyloxy, chacun des 11 radicaux mentionnés en dernier étant non substitué ou étant substitué par un ou plusieurs radicaux du groupe constitué par halogène, nitro, hydroxyle, cyano, NR¹³R¹⁴, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy et (C₁-C₄)-alkylsulfonyle, et hétérocyclyle possédant q groupes oxo,
R², R³ sont chacun indépendamment hydrogène, (C₁-C₆)-alkyle, (C₁-C₆)-halogénoalkyle, (C₂-C₆)-alcényle, (C₂-C₆)-alcynyle, (C₁-C₆)-alcoxy-(C₁-C₃)-alkyle, (C₁-C₆)-alcoxy-(C₁-C₃)-alkylcarbonyle, (C₁-C₄)-alkylthio, (C₁-C₄)-halogénoalkylthio, (C₁-C₄)-alkylthiocarbonyle, (C₁-C₄)-halogénoalkylthiocarbonyle, (C₁-C₄)-alkylthio-(C₁-C₃)-alkyle, (C₁-C₄)-alkylsulfoxy-(C₁-C₃)-alkyle, (C₁-C₄)-alkylsulfonyl-(C₁-C₃)-alkyle, (C₁-C₄)-alkylthio-(C₁-C₃)-alkylcarbonyle, (C₁-C₄)-alkylsulfoxy-(C₁-C₃)-alkylcarbonyle, (C₁-C₄)-alkylsulfonyl-(C₁-C₃)-alkylcarbonyle, (C₁-C₆)-alkylcarbonyle, (C₁-C₆)-halogénoalkylcarbonyle, (C₂-C₆)-alcénylcarbonyle, (C₂-C₆)-alcynylcarbonyle, (C₁-C₆)-alcoxycarbonylcarbonyle, (C₁-C₆)-alcoxycarbonyl-(C₁-C₃)-alkylcarbonyle, (C₁-C₀)-alcoxycarbonyle, (C₁-C₆)-halogénoalcoxycarbonyle, (C₂-C₆)-alcényloxycarbonyle, (C₃-C₆)-cycloalkyle, (C₃-C₆)-cycloalkyl-(C₁-C₆)-alkyle, (C₃-C₆)-cycloalkylcarbonyle, (C₃-C₆)-cycloalkyl-(C₁-C₆)-alkylcarbonyle, phényle, phényl-(C₁-C₃)-alkyle, hétéroaryle, hétéroaryl-(C₁-C₃)-alkyle, hétérocyclyle, hétérocyclyl-(C₁-C₃)-alkyle, phénylcarbonyle, phényl-(C₁-C₆)-alkylcarbonyle, hetarylcarbonyle, hetaryl-(C₁-C₆)-alkylcarbonyle, hétérocyclylcarbonyle ou hétérocyclyl-(C₁-C₆)-alkylcarbonyle, chacun des 16 radicaux mentionnés en dernier étant non substitué ou étant substitué par un ou plusieurs radicaux du groupe constitué par halogène, nitro, hydroxyle, cyano, NR¹³R¹⁴, (C₁-C-₄-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy et (C₁-C₄)-alkylsulfonyle, et hétérocyclyle possédant q groupes oxo,
ou
NR²R³ est -N=CR⁸R⁹ ou -N=S(O)ₙR¹⁰R¹¹,
R⁶, R⁷ sont chacun indépendamment hydrogène ou (C₁-C₆)-alkyle, préférablement R⁶ et R⁷ sont indépendamment hydrogène ou méthyle,
R⁸, R⁹ sont chacun indépendamment hydrogène, (C₁-C₆)-alkyle, (C₁-C₆)-halogénoalkyle, (C₂-C₆)-alcényle, (C₂-C₆)-alcynyle, (C₁-C₆)-alcoxy-(C₁-C₃)-alkyle, (C₁-C₄)-alkylthio-(C₁-C₃)-alkyle, (C₁-C₄)-alkylsulfoxy-(C₁-C₃)-alkyle, (C₁-C₄)-alkylsulfonyl-(C₁-C₃)-alkyle, (C₁-C₆)-alcoxy, (C₁-C₆)-halogénoalcoxy, (C₂-C₆)-alcényloxy, NR¹³R¹⁴, (C₃-C₈)-cycloalkyle, (C₃-C₈)-cycloalkyl-(C₁-C₆)-alkyle, phényle, phényl-(C₁-C₃)-alkyle, hétéroaryle, hétéroaryl-(C₁-C₃)-alkyle, hétérocyclyle ou hétérocyclyl-(C₁-C₃)-alkyle, chacun des 8 radicaux mentionnés en dernier étant non substitué ou substitué par un ou plusieurs radicaux du groupe constitué par halogène, nitro, hydroxyle, cyano, NR¹³R¹⁴, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy et (C₁-C₄)-alkylsulfonyle, et hétérocyclyle possédant q groupes oxo,
ou
R⁸ et R⁹, conjointement avec l'atome de carbone auquel ils sont fixés, forment un cycle insaturé, partiellement saturé ou saturé à 3 à 6 chaînons, qui comprend dans chaque cas, en plus des atomes de carbone, p éléments de cycle du groupe constitué par N(R¹²)ₘ, O et S(O)ₙ, et ledit cycle étant non substitué ou étant substitué par un ou plusieurs radicaux du groupe constitué par halogène, nitro, hydroxyle, cyano, NR¹³R¹⁴, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy et (C₁-C₄)-alkylsulfonyle,
R¹⁰, R¹¹ sont chacun indépendamment (C₁-C₆)-alkyle, (C₂-C₆)-alcényle, (C₂-C₆)-alcynyle, (C₁-C₆)-alcoxy-(C₁-C₃)-alkyle, (C₁-C₄)-alkylthio-(C₁-C₃)-alkyle, (C₁-C₄)-alkylsulfoxy-(C₁-C₃)-alkyle, (C₁-C₄)-alkylsulfonyl-(C₁-C₃)-alkyle, (C₃-C₈)-cycloalkyle, (C₃-C₈)-cycloalkyl-(C₁-C₆)-alkyle, phényle, phényl-(C₁-C₃)-alkyle, hétéroaryle, hétéroaryl-(C₁-C₃)-alkyle, hétérocyclyle ou hétérocyclyl-(C₁-C₃)-alkyle, chacun des 8 radicaux mentionnés en dernier étant non substitué ou étant substitué par un ou plusieurs radicaux du groupe constitué par halogène, nitro, hydroxyle, cyano, NR¹³R¹⁴, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy et (C₁-C₄)-alkylsulfonyle, et hétérocyclyle possédant q groupes oxo,
ou
R¹⁰ et R¹¹, conjointement avec l'atome de soufre auquel ils sont fixés, forment un cycle insaturé, partiellement saturé ou saturé à 3 à 6 chaînons, qui comprend dans chaque cas, en plus des atomes de carbone et en plus de l'atome de soufre, p éléments de cycle du groupe constitué par N(R¹²)ₘ, O et S(O)ₙ, et ledit cycle étant non substitué ou étant substitué par un ou plusieurs radicaux du groupe constitué par halogène, nitro, hydroxyle, cyano, NR¹³R¹⁴, (C₁-C-₄-alkyle, (C₁-C-₄-halogénoalkyle, (C₁-C₄)-alcoxy, (C₁-C₄)-halogénoalcoxy, (C₁-C₄)-alkylthio, (C₁-C₄)-alkylsulfoxy et (C₁-C₄)-alkylsulfonyle,
R¹² est hydrogène, (C₁-C₆)-alkyle ou (C₁-C₆)-alkylcarbonyle,
R¹³, R¹⁴ sont chacun indépendamment hydrogène, (C₁-C₆)-alkyle, (C₁-C₆)-alkylcarbonyle, (C₁-C₄)-alkylsulfonyle, phényle ou phénylcarbonyle, chacun des deux radicaux mentionnés en dernier étant non substitué ou étant substitué par un ou plusieurs radicaux du groupe constitué par halogène, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy et (C₁-C₄)-alkylsulfonyle,
ou
R¹³ et R¹⁴, conjointement avec l'atome d'azote auquel ils sont fixés, forment un cycle insaturé, partiellement saturé ou saturé à 3 à 8 chaînons, qui comprend dans chaque cas, en plus des atomes de carbone et en plus de l'atome d'azote, p éléments de cycle du groupe constitué par N(R¹²)ₘ, O et S(O)ₙ, et ledit cycle étant non substitué ou étant substitué par un ou plusieurs radicaux du groupe constitué par halogène, hydroxyle, (C₁-C₄)-alkyle, (C₁-C₄)-halogénoalkyle, (C₁-C₄)-alcoxy et (C₁-C₄)-halogénoalcoxy et possédant q groupes oxo,
n est indépendamment choisi parmi 0, 1 et 2,
m est indépendamment choisi parmi 0 et 1,
p est indépendamment choisi parmi 0, 1 et 2,
q est indépendamment choisi parmi 0, 1 et 2,
y est 0 ou 1.

4. Composé de formule (G) selon l'une quelconque des revendications 1 à 3, et/ou sel correspondant, dans laquelle
A est CR⁶R⁷,
W est O ou S, préférablement O,
R¹ est (C₁-C₆)-alkyle, (C₁-C₆)-halogénoalkyle, (C₂-C₆)-alcényle, (C₁₀-C₆)-alcoxy-(C₁-C₃)-alkyle, (C₁-C₆)-alcoxy-(C₂-C₆)-alcoxy-(C₁-C₃)-alkyle, (C₁-C₄)-alkylsulfonyle, (C₁-C₄)-alkylthio-(C₁-C₃)-alkyle, (C₁-C₄)-alkylsulfoxy-(C₁-C₃)-alkyle, (C₁-C₄)-alkylsulfonyl-(C₁-C₃)-alkyle, (C₃-C₈)-cycloalkyle, (C₃-C₆)-cycloalcényle, (C₃-C₆)-cycloalkyl-(C₁-C₃)-alkyle, (C₃-C₆)-cycloalcényl-(C₁-C₃)-alkyle, (C₃-C₆)-cycloalcoxy, phényle, hétéroaryle, hétérocyclyle, phénoxy, hétéroaryloxy ou hétérocyclyloxy, chacun des 11 radicaux mentionnés en dernier étant non substitué ou étant substitué par un ou plusieurs radicaux du groupe constitué par halogène, nitro, hydroxyle, cyano, NR¹³R¹⁴, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy et (C₁-C₄)-alkylsulfonyle, et hétérocyclyle possédant q groupes oxo,
R², R³ sont chacun indépendamment hydrogène, (C₂-C₆)-alcynyle, (C₁-C₄)-alkylthio, (C₁-C₄)-halogénoalkylthio, (C₁-C₄)-alkylthiocarbonyle, (C₁-C₄)-halogénoalkylthiocarbonyle, (C₁-C₆)-alcoxy-(C₁-C₃)-alkylcarbonyle, (C₁-C₄)-alkylthio-(C₁-C₃)-alkylcarbonyle, (C₁-C₄)-alkylsulfoxy-((C₁-C₃)-alkylcarbonyle, (C₁-C₄)-alkylsulfonyl-(C₁-C₃)-alkylcarbonyle, (C₁-C₆)-alkylcarbonyle, (C₁-C₆)-halogénoalkylcarbonyle, (C₂-C₆)-alcénylcarbonyle, (C₂-C₆)-alcynylcarbonyle, (C₁-C₆)-alcoxycarbonylcarbonyle, (C₁-C₆)-alcoxycarbonyl-(C₁-C₃)alkylcarbonyle, (C₁-C₆)-alcoxycarbonyle, (C₁-C₆)-halogénoalcoxycarbonyle, (C₂-C₆)-alcényloxycarbonyle, (C₃-C₆)-cycloalkylcarbonyle, (C₃-C₆)-cycloalkyl-(C₁-C₆)-alkylcarbonyle, phénylcarbonyle, phényl-(C₁-C₆)-alkylcarbonyle, hetarylcarbonyle, hetaryl-(C₁-C₆)-alkylcarbonyle, hétérocyclylcarbonyle ou hétérocyclyl-(C₁-C₆)-alkylcarbonyle, chacun des 8 radicaux mentionnés en dernier étant non substitué ou étant substitué par un ou plusieurs radicaux du groupe constitué par halogène, nitro, hydroxyle, cyano, NR¹³R¹⁴, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy et (C₁-C₄)-alkylsulfonyle et hétérocyclyle possédant q groupes oxo,
ou
NR²R³ est -N=CR⁸R⁹ ou -N=S(O)ₙR¹⁰R¹¹,
R⁶ est hydrogène,
R⁷ est hydrogène ou méthyle,
R⁸, R⁹ sont chacun indépendamment hydrogène, (C₁-C₆)-alkyle, (C₁-C₆)-halogénoalkyle, (C₂-C₆)-alcényle, (C₂-C₆)-alcynyle, (C₁-C₆)-alcoxy-(C₁-C₃)-alkyle, (C₁-C₄)-alkylthio-(C₁-C₃)-alkyle, (C₁-C₄)-alkylsulfoxy-(C₁-C₃)-alkyle, (C₁-C₄)-alkylsulfonyl-(C₁-C₃)-alkyle, (C₁-C₆)-alcoxy, (C₁-C₆)-halogénoalcoxy, (C₂-C₆)-alcényloxy, NR¹³R¹⁴, (C₃-C₈)-cycloalkyle, (C₃-C₈)-cycloalkyl-(C₁-C₆)-alkyle, phényle, phényl-(C₁-C₃)-alkyle, hétéroaryle, hétéroaryl-(C₁-C₃)-alkyle, hétérocyclyle ou hétérocyclyl-(C₁-C₃)-alkyle, chacun des 8 radicaux mentionnés en dernier étant non substitué ou étant substitué par un ou plusieurs radicaux du groupe constitué par halogène, nitro, hydroxyle, cyano, NR¹³R¹⁴, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy et (C₁-C₄)-alkylsulfonyle, et hétérocyclyle possédant q groupes oxo,
ou
R⁸ et R⁹, conjointement avec l'atome de carbone auquel ils sont fixés, forment un cycle insaturé, partiellement saturé ou saturé à 3 à 6 chaînons, qui comprend dans chaque cas, en plus des atomes de carbone, p éléments de cycle du groupe constitué par N(R¹²)ₘ, O et S(O)ₙ, et ledit cycle étant non substitué ou étant substitué par un ou plusieurs radicaux du groupe constitué par halogène, nitro, hydroxyle, cyano, NR¹³R¹⁴, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy et (C₁-C₄)-alkylsulfonyle,
R¹⁰, R¹¹ sont chacun indépendamment (C₁-C₆)-alkyle, (C₂-C₆)-alcényle, (C₂-C₆)-alcynyle, (C₁-C₆)-alcoxy-(C₁-C₃)-alkyle, (C₁-C₄)-alkylthio-(C₁-C₃)-alkyle, (C₁-C₄)-alkylsulfoxy-(C₁-C₃)-alkyle, (C₁-C₄)-alkylsulfonyl-(C₁-C₃)-alkyle, (C₃-C₈)-cycloalkyle, (C₃-C₈)-cycloalkyl-(C₁-C₆)-alkyle, phényle, phényl-(C₁-C₃)-alkyle, hétéroaryle, hétéroaryl-(C₁-C₃)-alkyle, hétérocyclyle et hétérocyclyl-(C₁-C₃)-alkyle, chacun des 8 radicaux mentionnés en dernier étant non substitué ou étant substitué par un ou plusieurs radicaux du groupe constitué par halogène, nitro, hydroxyle, cyano, NR¹³R¹⁴, (C₁-C-₄-alkyle, (C₁-C₄)-halogénoalkyle, (C₁-C₄)-alcoxy, (C₁-C-₄-halogénoalcoxy, (C₁-C₄)-alkylthio, (C₁-C₄)-alkylsulfoxy et (C₁-C₄)-alkylsulfonyle, et hétérocyclyle possédant q groupes oxo,
ou
R¹⁰ et R¹¹, conjointement avec l'atome de soufre auquel ils sont fixés, forment un cycle insaturé, partiellement saturé ou saturé à 3 à 6 chaînons, qui comprend dans chaque cas, en plus des atomes de carbone et en plus de l'atome de soufre, p éléments de cycle du groupe constitué par N(R¹²)ₘ, O et S(O)ₙ, et ledit cycle étant non substitué ou étant substitué par un ou plusieurs radicaux du groupe constitué par halogène, nitro, hydroxyle, cyano, NR¹³R¹⁴, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy et (C₁-C₄)-alkylsulfonyle,
R¹² est hydrogène, (C₁-C₆)-alkyle ou (C₁-C₆)-alkylcarbonyle,
R¹³, R¹⁴ sont chacun indépendamment hydrogène, (C₁-C₆)-alkyle, (C₁-C₆)-alkylcarbonyle, (C₁-C₄)-alkylsulfonyle, phényle ou phénylcarbonyle, chacun des deux radicaux mentionnés en dernier étant non substitué ou étant substitué par un ou plusieurs radicaux du groupe constitué par halogène, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy et (C₁-C₄)-alkylsulfonyle,
ou
R¹³ et R¹⁴, conjointement avec l'atome d'azote auquel ils sont fixés, forment un cycle insaturé, partiellement saturé ou saturé à 3 à 8 chaînons, qui comprend dans chaque cas, en plus des atomes de carbone et en plus de l'atome d'azote, p éléments de cycle du groupe constitué par N(R¹²)ₘ, O et S(O)ₙ, et ledit cycle étant non substitué ou étant substitué par un ou plusieurs radicaux du groupe constitué par halogène, hydroxyle, (C₁-C₄)-alkyle, (C₁-C₄)-halogénoalkyle, (C₁-C₄)-alcoxy et (C₁-C₄)-halogénoalcoxy, et possédant q groupes oxo,
n est indépendamment choisi parmi 0, 1 et 2,
m est indépendamment choisi parmi 0 et 1,
p est indépendamment choisi parmi 0, 1 et 2,
q est indépendamment choisi parmi 0 et 1,
y est 0 ou 1.

5. Composé de formule (G) selon l'une quelconque des revendications 1 à 4, et/ou sel correspondant, dans laquelle
A est CR⁶R⁷,
W est O ou S, préférablement O,
R¹ est (C₃-C₈)-cycloalkyle, (C₃-C₆)-cycloalcényle, (C₃-C₆)-cycloalkyl-(C₁-C₃)-alkyle, (C₃-C₆)-cycloalcényl-(C₁-C₃)-alkyle, phényle, hétéroaryle ou hétérocyclyle, chacun des 7 radicaux mentionnés en dernier étant non substitué ou étant substitué par un ou plusieurs radicaux du groupe constitué par halogène, nitro, hydroxyle, cyano, NR¹³R¹⁴, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy et (C₁-C₄)-alkylsulfonyle, et hétérocyclyle possédant q groupes oxo,
R², R³ sont chacun indépendamment hydrogène, (C₂-C₆)-alcynyle, (C₁-C₄)-alkylthio, (C₁-C₄)-halogénoalkylthio ((C₁-C₄)-halogénoalkylthio étant plus préférablement SCF₃), (C₁-C₄)-alkylthiocarbonyle, (C₁-C₄)-halogénoalkylthiocarbonyle, (C₁-C₆)-alcoxy-(C₁-C₃)-alkylcarbonyle, (C₁-C₄)-alkylthio-(C₁-C₃)-alkylcarbonyle, (C₁-C₄)-alkylsulfoxy-((C₁-C₃)-alkylcarbonyle, (C₁-C₄)-alkylsulfonyl-(C₁-C₃)-alkylcarbonyle, (C₁-C₆)-alkylcarbonyle, (C₁-C₆)-halogénoalkylcarbonyle, (C₂-C₆)-alcénylcarbonyle, (C₂-C₆)-alcynylcarbonyle, (C₁-C₆)-alcoxycarbonylcarbonyle, (C₁-C₆)-alcoxycarbonyl-(C₁-C₃)alkylcarbonyle, (C₁-C₆)-alcoxycarbonyle, (C₁-C₆)-halogénoalcoxycarbonyle, (C₂-C₆)-alcényloxycarbonyle, (C₃-C₆)-cycloalkylcarbonyle, (C₃-C₆)-cycloalkyl-(C₁-C₆)-alkylcarbonyle, phénylcarbonyle, phényl-(C₁-C₆)-alkylcarbonyle, hetarylcarbonyle, hetaryl-(C₁-C₆)-alkylcarbonyle, hétérocyclylcarbonyle ou hétérocyclyl-(C₁-C₆)-alkylcarbonyle, chacun des 8 radicaux mentionnés en dernier étant non substitué ou étant substitué par un ou plusieurs radicaux du groupe constitué par halogène, nitro, hydroxyle, cyano, NR¹³R¹⁴, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy et (C₁-C₄)-alkylsulfonyle, et hétérocyclyle possédant q groupes oxo,
ou
NR²R³ est -N=CR⁸R⁹ ou -N=S(O)ₙR¹⁰R¹¹,
R⁶ est hydrogène,
R⁷ est hydrogène ou méthyle,
R⁸, R⁹ sont chacun indépendamment hydrogène, (C₁-C₆)-alkyle, (C₁-C₆)-halogénoalkyle, (C₂-C₆)-alcényle, (C₂-C₆)-alcynyle, (C₁-C₆)-alcoxy-(C₁-C₃)-alkyle, (C₁-C₄)-alkylthio-(C₁-C₃)-alkyle, (C₁-C₄)-alkylsulfoxy-(C₁-C₃)-alkyle, (C₁-C₄)-alkylsulfonyl-(C₁-C₃)-alkyle, (C₁-C₆)-alcoxy, (C₁-C₆)-halogénoalcoxy, (C₂-C₆)-alcényloxy, NR¹³R¹⁴, (C₃-C₈)-cycloalkyle, (C₃-C₈)-cycloalkyl-(C₁-C₆)-alkyle, phényle, phényl-(C₁-C₃)-alkyle, hétéroaryle, hétéroaryl-(C₁-C₃)-alkyle, hétérocyclyle ou hétérocyclyl-(C₁-C₃)-alkyle, chacun des 8 radicaux mentionnés en dernier étant non substitué ou étant substitué par un ou plusieurs radicaux du groupe constitué par halogène, nitro, hydroxyle, cyano, NR¹³R¹⁴, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy et (C₁-C₄)-alkylsulfonyle, et hétérocyclyle possédant q groupes oxo,
ou
R⁸ et R⁹, conjointement avec l'atome de carbone auquel ils sont fixés, forment un cycle insaturé, partiellement saturé ou saturé à 3 à 6 chaînons, qui comprend dans chaque cas, en plus des atomes de carbone, p éléments de cycle du groupe constitué par N(R¹²)ₘ, O et S(O)ₙ, et ledit cycle étant non substitué ou étant substitué par un ou plusieurs radicaux du groupe constitué par halogène, nitro, hydroxyle, cyano, NR¹³R¹⁴, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy et (C₁-C₄)-alkylsulfonyle,
R¹⁰, R¹¹ sont chacun indépendamment (C₁-C₆)-alkyle, (C₂-C₆)-alcényle, (C₂-C₆)-alcynyle, (C₃-C₈)-cycloalkyle, (C₃-C₈)-cycloalkyl-(C₁-C₆)-alkyle, phényle, phényl-(C₁-C₃)-alkyle, hétéroaryle, hétéroaryl-(C₁-C₃)-alkyle, hétérocyclyle ou hétérocyclyl-(C₁-C₃)-alkyle, chacun des 8 radicaux mentionnés en dernier étant non substitué ou étant substitué par un ou plusieurs radicaux du groupe constitué par halogène, nitro, hydroxyle, cyano, NR¹³R¹⁴, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy et (C₁-C₄)-alkylsulfonyle, et hétérocyclyle possédant q groupes oxo,
ou
R¹⁰ et R¹¹, conjointement avec l'atome de soufre auquel ils sont fixés, forment un cycle insaturé, partiellement saturé ou saturé à 3 à 6 chaînons, qui comprend dans chaque cas, en plus des atomes de carbone et en plus de l'atome de soufre, p éléments de cycle du groupe constitué par N(R¹²)ₘ, O et S(O)ₙ, et ledit cycle étant non substitué ou étant substitué par un ou plusieurs radicaux du groupe constitué par halogène, nitro, hydroxyle, cyano, NR¹³R¹⁴, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy et (C₁-C₄)-alkylsulfonyle,
R¹² est hydrogène, (C₁-C₆)-alkyle ou (C₁-C₆)-alkylcarbonyle,
R¹³, R¹⁴ sont chacun indépendamment hydrogène, (C₁-C₆)-alkyle, (C₁-C₆)-alkylcarbonyle, (C₁-C₄)-alkylsulfonyle, phényle ou phénylcarbonyle, chacun des deux radicaux mentionnés en dernier étant non substitué ou étant substitué par un ou plusieurs radicaux du groupe constitué par halogène, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy et (C₁-C₄)-alkylsulfonyle,
ou
R¹³ et R¹⁴, conjointement avec l'atome d'azote auquel ils sont fixés, forment un cycle insaturé, partiellement saturé ou saturé à 3 à 8 chaînons, qui comprend dans chaque cas, en plus des atomes de carbone et en plus de l'atome d'azote, p éléments de cycle du groupe constitué par N(R¹²)ₘ, O et S(O)ₙ, et ledit cycle étant non substitué ou étant substitué par un ou plusieurs radicaux du groupe constitué par halogène, hydroxyle, (C₁-C₄)-alkyle, (C₁-C₄)-halogénoalkyle, (C₁-C₄)-alcoxy et (C₁-C₄)-halogénoalcoxy, et possédant q groupes oxo,
n est indépendamment choisi parmi 0 et 1,
m est indépendamment choisi parmi 0 et 1,
p est indépendamment choisi parmi 0, 1 et 2,
q est indépendamment choisi parmi 0 et 1,
y est 0 ou 1.

6. Composé de formule (G) selon l'une quelconque des revendications 1 à 5, et/ou sel correspondant, dans laquelle
A est CR⁶R⁷,
W est O,
R¹ est (C₃-C₆)-cycloalkyle, (C₃-C₆)-cycloalcényle, (C₃-C₆)-cycloalkyl-(C₁-C₃)-alkyle, (C₃-C₆)-cycloalcényl-(C₁-C₃)-alkyle, phényle, hétéroaryle ou hétérocyclyle, chacun des 7 radicaux mentionnés en dernier étant non substitué ou étant substitué par un ou plusieurs radicaux du groupe constitué par halogène, nitro, hydroxyle, cyano, NR¹³R¹⁴, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy et (C₁-C₄)-alkylsulfonyle, et hétérocyclyle possédant q groupes oxo,
R², R³ sont chacun indépendamment hydrogène, (C₁-C₄)-alkylthiocarbonyle, (C₁-C₄)-halogénoalkylthiocarbonyle, (C₁-C₆)-alcoxy-(C₁-C₃)-alkylcarbonyle, (C₁-C₄)-alkylthio-(C₁-C₃)-alkylcarbonyle, (C₁-C₄)-alkylsulfoxy-((C₁-C₃)-alkylcarbonyle, (C₁-C₄)-alkylsulfonyl-(C₁-C₃)-alkylcarbonyle, (C₁-C₆)-alkylcarbonyle, (C₁-C₆)-halogénoalkylcarbonyle, (C₂-C₆)-alcénylcarbonyle, (C₂-C₆)-alcynylcarbonyle, (C₁-C₆)-alcoxycarbonylcarbonyle, (C₁-C₆)-alcoxycarbonyl-(C₁-C₃)alkylcarbonyle, (C₁-C₆)-alcoxycarbonyle, (C₁-C₆)-halogénoalcoxycarbonyle, (C₂-C₆)-alcényloxycarbonyle, (C₃-C₆)-cycloalkylcarbonyle, (C₃-C₆)-cycloalkyl-(C₁-C₆)-alkylcarbonyle, phénylcarbonyle, phényl-(C₁-C₆)-alkylcarbonyle, hetarylcarbonyle, hetaryl-(C₁-C₆)-alkylcarbonyle, hétérocyclylcarbonyle ou hétérocyclyl-(C₁-C₆)-alkylcarbonyle, chacun des 8 radicaux mentionnés en dernier étant non substitué ou étant substitué par un ou plusieurs radicaux du groupe constitué par halogène, nitro, hydroxyle, cyano, NR¹³R¹⁴, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy et (C₁-C₄)-alkylsulfonyle, et hétérocyclyle possédant q groupes oxo,
ou
NR²R³ est -N=CR⁸R⁹ ou -N=S(O)ₙR¹⁰R¹¹,
R⁶ est hydrogène,
R⁷ est hydrogène ou méthyle,
R⁸, R⁹ sont chacun indépendamment hydrogène, (C₁-C₆)-alkyle, (C₁-C₆)-halogénoalkyle, (C₂-C₆)-alcényle, (C₂-C₆)-alcynyle, (C₁-C₆)-alcoxy-(C₁-C₃)-alkyle, (C₁-C₄)-alkylthio-(C₁-C₃)-alkyle, (C₁-C₄)-alkylsulfoxy-(C₁-C₃)-alkyle, (C₁-C₄)-alkylsulfonyl-(C₁-C₃)-alkyle, (C₁-C₆)-alcoxy, (C₁-C₆)-halogénoalcoxy, (C₂-C₆)-alcényloxy, NR¹³R¹⁴, (C₃-C₈)-cycloalkyle, (C₃-C₈)-cycloalkyl-(C₁-C₆)-alkyle, phényle, phényl-(C₁-C₃)-alkyle, hétéroaryle, hétéroaryl-(C₁-C₃)-alkyle, hétérocyclyle ou hétérocyclyl-(C₁-C₃)-alkyle, chacun des 8 radicaux mentionnés en dernier étant non substitué ou étant substitué par un ou plusieurs radicaux du groupe constitué par halogène, nitro, hydroxyle, cyano, NR¹³R¹⁴, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy et (C₁-C₄)-alkylsulfonyle, et hétérocyclyle possédant q groupes oxo,
ou
R⁸ et R⁹, conjointement avec l'atome de carbone auquel ils sont fixés, forment un cycle insaturé, partiellement saturé ou saturé à 3 à 6 chaînons, qui comprend dans chaque cas, en plus des atomes de carbone, p éléments de cycle du groupe constitué par N(R¹²)ₘ, O et S(O)ₙ, et ledit cycle étant non substitué ou étant substitué par un ou plusieurs radicaux du groupe constitué par halogène, nitro, hydroxyle, cyano, NR¹³R¹⁴, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy et (C₁-C₄)-alkylsulfonyle,
R¹⁰, R¹¹ sont chacun indépendamment (C₁-C₆)-alkyle, (C₂-C₆)-alcényle, (C₂-C₆)-alcynyle, (C₃-C₈)-cycloalkyle, (C₃-C₈)-cycloalkyl-(C₁-C₆)-alkyle, phényle, phényl-(C₁-C₃)-alkyle, hétéroaryle, hétéroaryl-(C₁-C₃)-alkyle, hétérocyclyle ou hétérocyclyl-(C₁-C₃)-alkyle, chacun des 8 radicaux mentionnés en dernier étant non substitué ou étant substitué par un ou plusieurs radicaux du groupe constitué par halogène, nitro, hydroxyle, cyano, NR¹³R¹⁴, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy et (C₁-C₄)-alkylsulfonyle, et hétérocyclyle possédant q groupes oxo,
ou
R¹⁰ et R¹¹, conjointement avec l'atome de soufre auquel ils sont fixés, forment un cycle insaturé, partiellement saturé ou saturé à 3 à 6 chaînons, qui comprend dans chaque cas, en plus des atomes de carbone et en plus de l'atome de soufre, p éléments de cycle du groupe constitué par N(R¹²)ₘ, O et S(O)ₙ, et ledit cycle étant non substitué ou étant substitué par un ou plusieurs radicaux du groupe constitué par halogène, nitro, hydroxyle, cyano, NR¹³R¹⁴, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy et (C₁-C₄)-alkylsulfonyle,
R¹² est hydrogène, (C₁-C₆)-alkyle ou (C₁-C₆)-alkylcarbonyle,
R¹³, R¹⁴ sont chacun indépendamment hydrogène, (C₁-C₆)-alkyle, (C₁-C₆)-alkylcarbonyle, (C₁-C₄)-alkylsulfonyle, phényle ou phénylcarbonyle, chacun des deux radicaux mentionnés en dernier étant non substitué ou étant substitué par un ou plusieurs radicaux du groupe constitué par halogène, (C₁-C₄)-alkyle, (C₁-C₄)-halogénoalkyle, (C₁-C₄)-alcoxy, (C₁-C₄)-halogénoalcoxy, (C₁-C₄)-alkylthio, (C₁-C₄)-alkylsulfoxy et (C₁-C₄)-alkylsulfonyle,
ou
R¹³ et R¹⁴, conjointement avec l'atome d'azote auquel ils sont fixés, forment un cycle insaturé, partiellement saturé ou saturé à 3 à 8 chaînons, qui comprend dans chaque cas, en plus des atomes de carbone et en plus de l'atome d'azote, p éléments de cycle du groupe constitué par N(R¹²)ₘ, O et S(O)ₙ, et ledit cycle étant non substitué ou étant substitué par un ou plusieurs radicaux du groupe constitué par halogène, hydroxyle,(C₁-C₄)-alkyle, (C₁-C₄)-halogénoalkyle, (C₁-C₄)-alcoxy et (C₁-C₄)-halogénoalcoxy, et possédant q groupes oxo,
n est 0,
m est indépendamment choisi parmi 0 et 1,
p est indépendamment choisi parmi 0, 1 et 2,
q est indépendamment choisi parmi 0 et 1,
y est 0 ou 1.

7. Composé de formule (G) selon l'une quelconque des revendications 1 à 6, et/ou sel correspondant, dans laquelle
n est indépendamment choisi parmi 0 et 1, préférablement n est 0,
m est indépendamment choisi parmi 0 et 1, préférablement m est 0,
p est indépendamment choisi parmi 0 et 1, et
q est indépendamment choisi parmi 0 et 1, préférablement q est 0.

8. Composé de formule (G) selon l'une quelconque des revendications 1 à 7, et/ou sel correspondant, dans laquelle y est 1.

9. Utilisation d'un ou plusieurs composés de formule (G), et/ou de sels correspondants tels que définis dans l'une quelconque des revendications 1 à 8 en tant qu'herbicides et/ou régulateurs de croissance végétale.

10. Utilisation d'un ou plusieurs composés de formule (G), et/ou de sels correspondants selon la revendication 9 pour la lutte contre des végétaux nuisibles ou pour la régulation de la croissance de végétaux, un ou plusieurs composés de formule (G) et/ou sels correspondants étant appliqués sur les végétaux nuisibles, les graines végétales, le sol dans lequel ou sur lequel les végétaux croissent ou sur la zone cultivée.

11. Composition herbicide et/ou de régulation de croissance végétale, **caractérisée en ce que** ladite composition comprend un ou plusieurs composés de formule (G) et/ou des sels correspondants tels que définis dans l'une quelconque des revendications 1 à 8,
et une ou plusieurs substances supplémentaires choisies parmi les groupes (i) et (ii) :
(i) une ou plusieurs substances actives sur le plan agrochimique supplémentaires, préférablement choisies dans le groupe constitué par des insecticides, des acaricides, des nématicides, des herbicides supplémentaires, des fongicides, des phytoprotecteurs, des fertilisants et/ou des régulateurs de croissance supplémentaires,
(ii) un ou plusieurs auxiliaires de formulation habituels dans la protection de cultures.

12. Procédé pour la lutte contre des végétaux nuisibles ou pour la régulation de la croissance de végétaux, **caractérisé en ce qu'**une quantité efficace
- d'un ou plusieurs composés de formule (G) et/ou de sels correspondants, tels que définis dans l'une quelconque des revendications 1 à 8,
ou
- d'une composition selon la revendication 11,
est appliquée aux végétaux, aux graines de végétaux, au sol dans lequel ou sur lequel les végétaux croissent ou à la surface cultivée.

13. Procédé pour la préparation d'un composé de formule (G) tel que défini dans l'une quelconque des revendications 1 à 8, et/ou d'un sel correspondant, W étant oxygène, **caractérisé en ce que**
(a) un composé de formule (G) dans laquelle R² et R³ sont tous deux H est obtenu dans une synthèse chimique comprenant l'étape de cyclisation d'un composé de formule (E-XXIV) 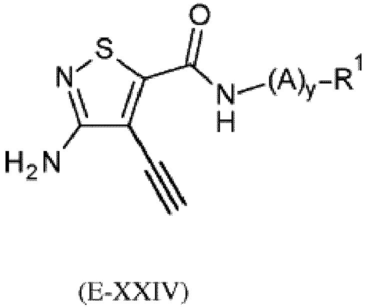 dans laquelle R¹, A et y possèdent chacun la signification telle que définie dans la formule (G),
ou
(b) un composé de formule (G) dans laquelle R² et R³ sont tous deux H est obtenu dans une synthèse chimique comprenant l'étape de cyclisation d'un composé de formule (E-XXVII) 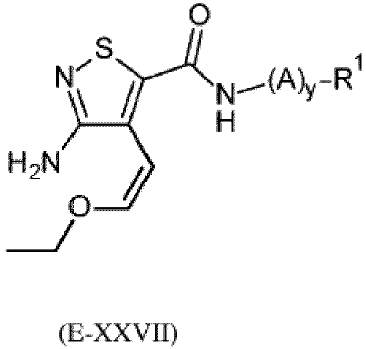 dans laquelle R¹, A et y possèdent chacun la signification telle que définie dans la formule (G),
ou
(c) un composé de formule (G) dans laquelle R² et/ou R³ ne sont pas H est obtenu dans une synthèse chimique comprenant l'étape de mise en réaction d'un composé de formule (G-1) 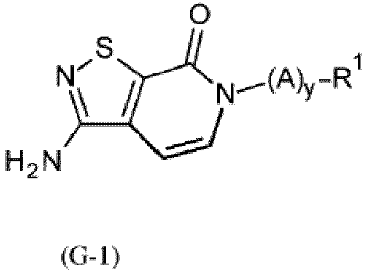 dans laquelle R¹, A et y possèdent chacun la signification telle que définie dans la formule (G),
avec un halogénure d'acyle R²COHal et/ou R³COHal, Hal étant dans chaque cas Cl, Br ou I, ou avec un anhydride (R²CO)₂O, (R³CO)₂O et/ou R²CO(O)OCR³, et R² et R³ possédant la signification telle que définie dans la formule (G), étant entendu que R² et/ou R³ ne sont pas H.
